(19) Europäisches Patentamt
European Patent Office
Office européen des brevets

(11) Publication number : **0 245 058 B1**

(12) # EUROPEAN PATENT SPECIFICATION

(45) Date of publication of patent specification : **18.03.92 Bulletin 92/12**

(21) Application number : **87303961.4**

(22) Date of filing : **01.05.87**

(51) Int. Cl.⁵ : **C07D 403/12,** C07D 409/12, C07D 401/12, C07D 491/04, C07F 7/18, C07D 231/18, C07D 233/34, C07D 213/71, C07D 213/89, A01N 47/36, // (C07D491/04, 307:00, 239:00), (C07D491/04, 311:00, 239:00)

(54) **Herbicidal heterocyclic sulfonamides.**

The file contains technical information submitted after the application was filed and not included in this specification

(30) Priority : **02.05.86 US 859275**
**13.06.86 US 874307**
**13.03.87 US 22365**
**30.03.87 US 29434**

(43) Date of publication of application : **11.11.87 Bulletin 87/46**

(45) Publication of the grant of the patent : **18.03.92 Bulletin 92/12**

(84) Designated Contracting States : **BE CH DE FR GB GR IT LI LU NL SE**

(56) References cited :
**EP-A- 0 013 480**
**EP-A- 0 030 142**
**EP-A- 0 046 677**
**EP-A- 0 087 780**
**EP-A- 0 097 122**
**EP-A- 0 101 407**
**EP-A- 0 107 624**
**PATENT ABSTRACTS OF JAPAN, vol. 9, no. 88 (C-276)[1811], 17th April 1985; & JP - A - 59 219 281**
**PATENT ABSTRACTS OF JAPAN, vol. 9, no. 218 (C-301)[1941], 5th September 1985; JP - A - 60 789 80**
**PATENT ABSTRACTS OF JAPAN, vol. 10, no. 350 (C-387)[2406], 26th November 1986; & JP - A - 61 151 188**

(73) Proprietor : **E.I. DU PONT DE NEMOURS AND COMPANY**
**1007 Market Street**
**Wilmington Delaware 19898 (US)**

(72) Inventor : **Wexler Barry Arthur**
**2205 Patwynn Road**
**Wilmington Delaware 19810 (US)**

(74) Representative : **Hildyard, Edward Martin et al**
**Frank B. Dehn & Co. European Patent Attorneys Imperial House 15-19 Kingsway**
**London WC2B 6UZ (GB)**

EP 0 245 058 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

Background of the Invention

This invention relates to novel ketone pyrazole, thiophene, and pyridine sulfonylurea herbicidal compounds, agriculturally suitable compositions thereof and a method of using them to control the growth of undesired vegetation.

New compounds effective for controlling the growth of undesired vegetation are in constant demand. In the most common situation, such compounds are sought to selectively control the growth of weeds in useful crops such as cotton, rice, corn, wheat and soybeans, to name a few. Unchecked weed growth in such crops can cause significant losses, reducing profit to the farmer and increasing costs to the consumer. In other situations, herbicides are desired which will control all plant growth. Examples of areas in which complete control of all vegetation is desired are areas around railroad tracks and industrial storage areas. There are many products commercially available for these purposes, but the search continues for products which are more effective, less costly and environmentally safe.

The "sulfonylurea" herbicides are an extremely potent class of herbicides discovered within the last few years. A multitude of structural variations exist within the class of herbicides, but they generally consist of a sulfonylurea bridge, $-SO_2NHCONH-$, linking two aromatic or heteroaromatic rings.

EP-A95,925 which was published 7.12.83 discloses herbicidal sulfonylureas of formula

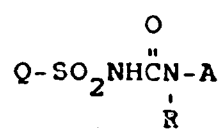

wherein

Q is, in part,

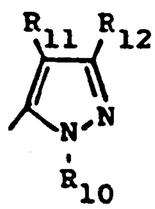

Q-4

,

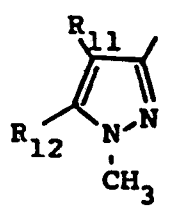

Q-5

or

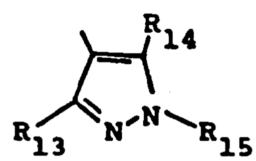

Q-6

;

$R_{10}$ is H, $C_1$-$C_4$ alkyl, $C_3$-$C_4$ alkenyl, $C_3$-$C_4$ alkynyl, $CO_2R_{24}$, $SO_2NR_{20}R_{21}$ or $SO_2R_{22}$;

$R_{11}$ is H, $C_1$-$C_3$ alkyl, F, Cl, Br, $NO_2$, $OR_{16}$, $CO_2R_{24}$, $S(O)_mR_{25}$ or $SO_2NR_{20}R_{21}$;

provided that when $R_{10}$ is other than $C_1$-$C_3$ alkyl, then $R_{11}$ is H, Cl, $OCH_3$, $NO_2$, or $CH_3$;

$R_{12}$ is H or $CH_3$;

$R_{13}$ and $R_{14}$ are independently H, $C_1$-$C_3$ alkyl, $OR_{16}$, F, Cl, Br, $NO_2$, $CO_2R_{24}$, S(O) $R_{25}$ or $SO_2NR_{20}R_{21}$;

provided that, when either of $R_{13}$ or $R_{14}$ is $CO_2R_{24}$, $S(O)_mR_{25}$ or $SO_2NR_{20}R_{21}$, then the other is H, Cl, $CH_3$, $OCH_3$ or $NO_2$; and

$R_{15}$ is H or $CH_3$.

EP-A-87,780 (published 7.9.83) discloses herbicidal sulfonylureas of formula

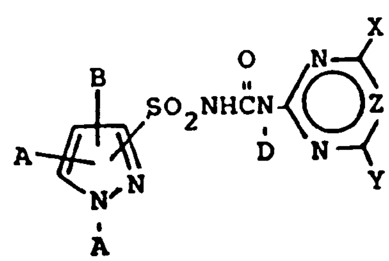

wherein

A is H, $C_1$-$C_8$ alkyl or optionally substituted phenyl;

B and C are independently H, halogen, $NO_2$, $C_1$-$C_8$ alkyl, arylalkyl, $C_1$-$C_8$ alkoxy, haloalkyl, $CO_2R$, $CONR_1R_2$, $S(O)_nR_3$, $SO_2NR_4R_5$, or optionally substituted phenyl.

ZA 83/3850 (published 28.11.83) discloses compounds of formula

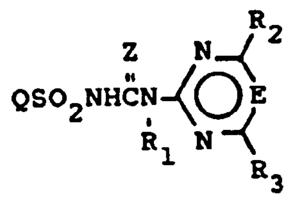

wherein

Q is a five-membered, heterocyclic radical which is bound by way of a carbon atom and contains 2 or 3 heteroatoms and which may be optionally substituted by halogen, pseudohalogen, nitro, alkyl, hydroxyl, haloalkyl, alkoxy, alkylthio, haloalkoxy, haloalkylthio, amino, alkylamino, dialkylamino, alkylcarbonylamino, alkylcarbonyl, alkoxycarbonyl, alkoxyalkyl, alkylthiocarbonyl, carbamoyl, alkylaminocarbonyl, dialkylaminocarbonyl, alkylsulfinyl, alkylsulfonyl, alkenyloxy or alkynyloxy; and groups such as phenyl, phenoxy or phenylthio, which are unsubstituted or substituted by halogen, nitro, cyano, alkyl, alkoxy, haloalkyl, alkylcarbonyl, alkoxycarbonyl or haloalkoxy; and also benzyl unsubstituted or substituted by halogen and/or alkyl.

U.S. 4,127,405 and U.S. 4,169,719 disclose herbicidal thiophenesulfonamides, wherein the thiophene ring may be optionally substituted with $CH_3$, Cl or Br.

U.S. 4,398,939 discloses herbicidal thiophenesulfonamides, wherein the thiophene ring is substituted with substituent groups selected from $C_1$-$C_4$ alkyl, $C_3$ alkenyl, $OCH_3$, $NO_2$, Cl, Br, $SO_2N(C_1$-$C_3$ alkyl$)_2$ or $SO_2N(OCH_3)CH_3$.

U.S. 4,481,029 discloses herbicidal thiophenesulfonamides, wherein the thiophene ring is substituted with carboxylic acid, carboxylic ester and alkylcarbonyl groups or derivatives thereof.

U.S. 4,441,910 discloses herbicidal thiophenesulfonamides, wherein the thiophene ring is substituted with the group represented by $R_6S(O)_n$ wherein $R_6$ is $C_1$-$C_4$ alkyl, $C_3$-$C_4$ alkenyl, cyclopentyl or cyclopropylmethyl.

European Publication No. 13,480 (published July 23, 1980) discloses herbicidal pyridine-2-, -3- and -4-sulfonylureas, wherein the pyridine ring may be substituted by Cl, Br, F, $C_1$-$C_4$ alkyl, $C_1$-$C_4$ alkoxy, $C_1$-$C_4$ alkylthio, $NO_2$ or a carboxylic ester group.

U.S. 4,456,469 (issued 29.6.84) discloses herbicidal pyridine-3-sulfonylureas substituted by $C_1$-$C_6$ alkyl-, $C_3$-$C_6$ alkenyl-, $C_2$-$C_4$ alkoxyalkyl- and $C_5$-$C_6$ cycloalkylsulfonyl groups.

U.S. 4,518,776 (Swiss priority 19.7.82) discloses, in part, a process for the preparation of compounds of formula

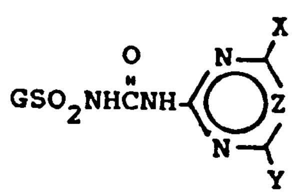

wherein

G is 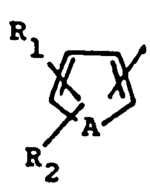 or 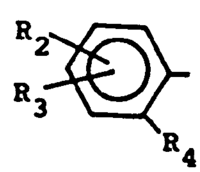 ;

$R_1$ is H, $C_1$-$C_4$ alkyl, halogen, $NO_2$, CN, $NH_2$, $S(O)_nC_1$-$C_4$ alkyl, $SO_2C_1$-$C_4$ alkoxy, $SO_2$-di-$C_1$-$C_4$ alkylamino, CHO, $CONH_2$, $DC_3$-$C_5$ alkynyl, $CODC_3$-$C_5$ alkynyl, $DC_1$-$C_4$ alkyl, $DC_3$-$C_5$ alkenyl, $COC_1$-$C_4$ alkyl,

$CODC_1$-$C_4$ alkyl or $CODC_3$-$C_5$ alkenyl;

n is 1 or 2;

D is O, S, NH or $NC_1$-$C_4$ alkyl;

$R_2$ is H, halogen, $CF_3$, $NO_2$, $C_1$-$C_4$ alkyl or $C_1$-$C_4$ alkoxy; and

A is O, S, $NR_5$ or -C=N-.

U.S. 4.521,597 discloses, in part, a process for the preparation of compounds of formula

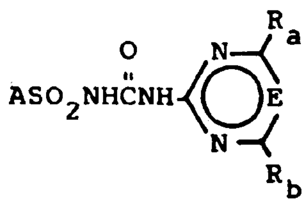

wherein

A is 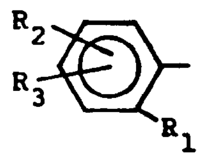 , 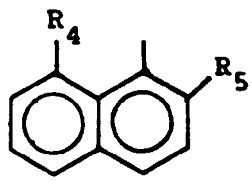 or

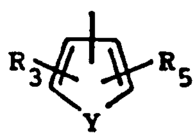 ;

$R_3$ is H, halogen, $NO_2$, $OCH_3$ or $CF_3$;

$R_5$ is H, F, Cl, Br, $NO_2$, $C_1$-$C_5$ alkyl, $C_1$-$C_5$ alkoxy, $CF_3$, $S(O)_mC_1$-$C_5$ alkyl, $COR_7$ or $SO_2NR_8R_9$;

Y is O, S or $C(R_5)$=N; and

$R_7$ is H, $C_1$-$C_5$ alkyl, $C_1$-$C_5$ haloalkyl, $C_1$-$C_5$ alkoxy, $C_1$-$C_5$ haloalkoxy, $C_2$-$C_{10}$ alkoxyalkoxy, $C_3$-$C_5$ alkenyloxy, $C_3$-$C_5$ alkynyloxy, phenoxy, benzyloxy, $C_1$-$C_5$ alkylthio or $NR_8R_9$.

U.S. 4,549,898 discloses herbicidal sulfonylureas of formula

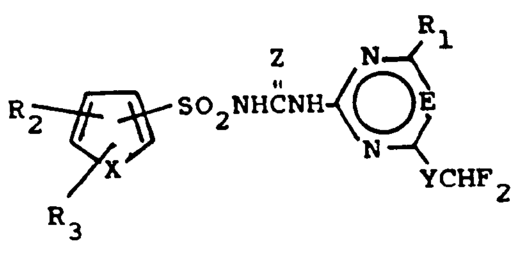

wherein

X is O, S, $NR_4$ or $C(R_5)$=N;

Y is O or S;

Z is O or S;

E is N or CH;

$R_1$ is H, $C_1$-$C_4$ alkyl, $C_1$-$C_4$ haloalkyl, $C_1$-$C_4$ haloalkoxy, $C_1$-$C_4$ alkoxy, halogen, $C_1$-$C_4$ alkylthio, $NR_6R_7$ or alkoxyalkyl containing not more than 4 carbon atoms;

$R_2$ is H, $C_1$-$C_3$ alkyl, $C_1$-$C_3$ haloalkyl, halogen, $NO_2$, $C_1$-$C_3$ alkoxy, $C(W)R_5$, $SO_2NR_5R_7$, $S(O)_n$-$C_1$-$C_3$ alkyl or $COR_9$;

$R_3$ is H, halogen, $C_1$-$C_3$ alkyl, $OCH_3$ or $CF_3$;

$R_5$ is H, $NO_2$, F, Cl, Br, $CH_3$, $CF_3$, $S(O)_nC_1$-$C_3$ alkyl, $COC_1$-$C_4$ alkoxy or $C_1$-$C_3$ alkoxy;

$R_8$ is H, $C_1$-$C_6$ alkyl, $C_1$-$C_4$ haloalkyl, $C_3$-$C_6$ cycloalkyl, $C_4$-$C_7$ cycloalkylalkyl or alkoxyalkyl containing not more than 4 carbon atoms; and

W is O or $NOR_{10}$.

Japanese Patent Application Number 58-70407 (SHO 59-219,281, laid open 10.12.84 discloses pyrazole-5-sulfonylureas of formula

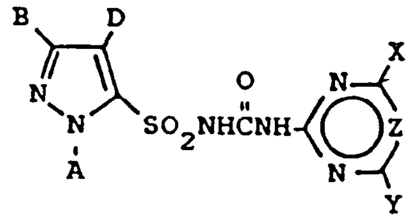

wherein

A is H, lower alkyl or phenyl;

B is H or lower alkyl;

D is H, $CO_2R$ or COAr, halogen, $NO_2$ or $SO_2NR^1R^2$;

and

Ar is phenyl optionally substituted with halogen.

U.S. 4,370,480 discloses herbicidal sulfonylureas of formula

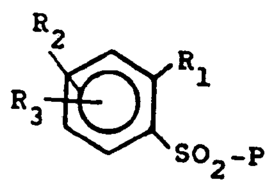

wherein

$R_1$ is 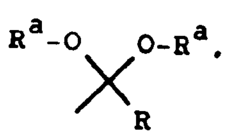, 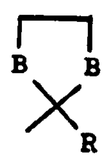, 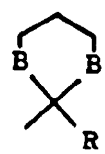 or 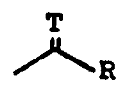;

R is H, $C_1$-$C_2$ alkyl, $C_2$-$C_6$ alkenyl, $C_2$-$C_6$ alkynyl;
$C_1$-$C_4$ alkyl substituted with one to four substituents selected from 0-3 F, 0-3 Cl, 0-3 Br, 0-2 $OCH_3$, 0-1 cyano, 0-1 $CO_2R'_1$ where $R'_1$ is $C_1$-$C_3$ alkyl, $CO_2R'_1$, $C_2$-$C_4$ alkenyl substituted with 1-3 Cl, $C_3$-$C_6$ cycloalkyl, $C_5$-$C_6$ cycloalkenyl, $C_5$-$C_6$ cycloalkyl substituted with substituents selected from 1-3 $CH_3$ or one of $CH_3CH_2$, Cl, $OCH_3$, $C_4$-$C_7$ cycloalkylalkyl,

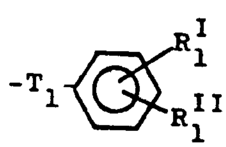 where $T_1$ is $\underset{R'_2}{C}=CH-$, $-\underset{R'_2}{CH}-(CH_2)-$

or a single bond; and

T is O or $NOR'''_1$.

Japanese Patent Application Number 84-273152 (Sho 86-151188, laid open July 9, 1986) discloses the following compound

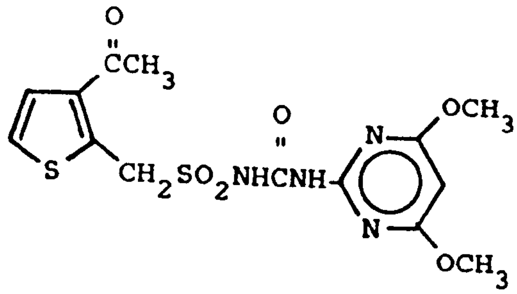

Still further herbicidal sulfonylureas are disclosed in EP-A-0 030 142; EP-A-0 046 677; EP-A-0 097 122; EP-A-0 101 407; EP-A-0 107 624; JP-A-61 151 188 and JP-A-60 78980.

## SUMMARY OF THE INVENTION

This invention relates to novel compounds of Formula I, agriculturally suitable compositions containing them, and their method-of-use as general preemergence and/or postemergence herbicides or plant growth regulants.

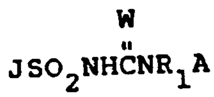

I

wherein

J is 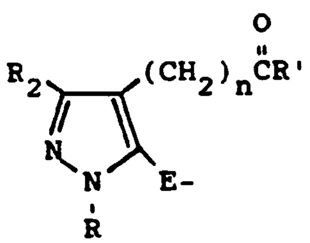

J-1

,

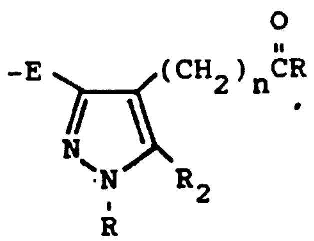

J-2

,

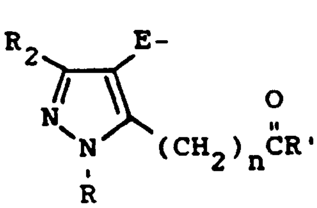

J-3

,

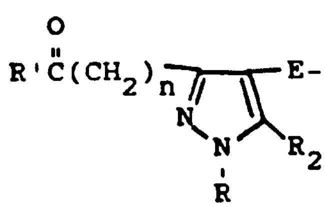

J-4

,

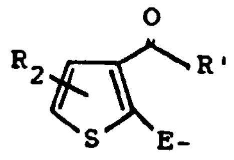

,

J-5

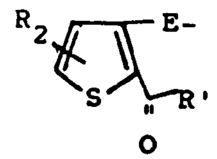

,

J-6

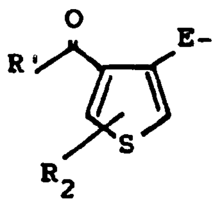

,

J-7

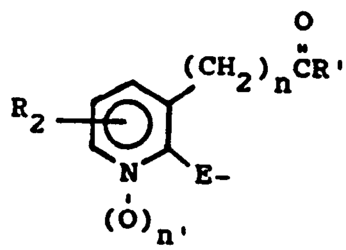

,

J-8

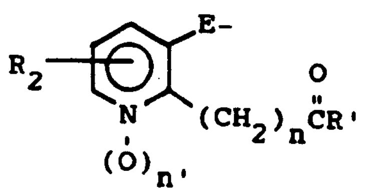

,

J-9

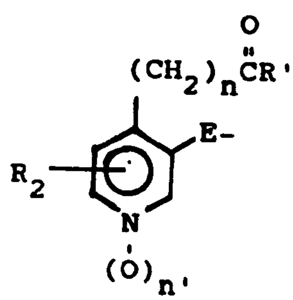

or

J-10

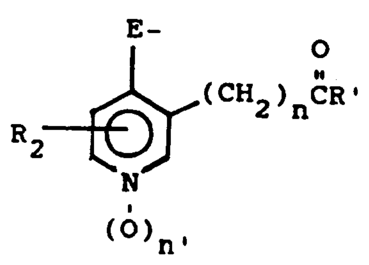

;

J-11

R is H, $C_1$-$C_3$ alkyl, phenyl, $SO_2NR_aR_b$, $C_1$-$C_2$ haloalkyl, $C_2$-$C_4$ alkoxyalkyl, $C_2$-$C_3$ cyanoalkyl, $C_2$-$C_4$ alkylthioalkyl, $C_2$-$C_4$ alkylsulfinylalkyl, $C_2$-$C_4$ alkylsulfonylalkyl, $CO_2C_1$-$C_2$ alkyl, $C_1$-$C_4$ alkylcarbonyl, $C_1$-$C_2$ alkylsulfonyl, $C_3$-$C_4$ alkenyl, $C_3$-$C_4$ alkynyl or $C_1$-$C_2$ alkyl substituted with $CO_2C_1$-$C_2$ alkyl;

$R_1$ is H or $CH_3$;

$R_2$ is H, $C_1$-$C_3$ alkyl, $C_1$-$C_3$ haloalkyl, halogen, nitro, $C_1$-$C_3$ alkoxy, $SO_2NR_cR_d$, $C_1$-$C_3$ alkylthio, $C_1$-$C_3$ alkylsulfinyl, $C_1$-$C_3$ alkylsulfonyl, CN, $CO_2R_e$, $C_1$-$C_3$ haloalkoxy, $C_1$-$C_3$ haloalkylthio, amino, $C_1$-$C_2$ alkylamino, di($C_1$-

$C_3$ alkyl)amino or $C_1$-$C_2$ alkyl substituted with $C_1$-$C_2$ alkoxy, $C_1$-$C_2$ haloalkoxy, $C_1$-$C_2$ alkylthio, $C_1$-$C_2$ haloalkylthio, CN, OH or SH;

$R_a$ and $R_b$ are independently $C_1$-$C_2$ alkyl;

$R_c$ is H, $C_1$-$C_4$ alkyl, $C_2$-$C_3$ cyanoalkyl, methoxy or ethoxy;

$R_c$ is H, $C_1$-$C_4$ alkyl or $C_3$-$C_4$ alkenyl; or

$R_c$ and $R_d$ may be taken together as $-(CH_2)_3-$, $-(CH_2)_4-$, $-(CH_2)_5-$ or $-CH_2CH_2OCH_2CH_2-$;

$R_e$ is $C_1$-$C_4$ alkyl, $C_3$-$C_4$ alkenyl, $C_3$-$C_4$ alkynyl, $C_2$-$C_4$ haloalkyl, $C_1$-$C_2$ cyanoalkyl. $C_5$-$C_6$ cycloalkyl, $C_4$-$C_7$ cycloalkylalkyl or $C_2$-$C_4$ alkoxyalkyl;

R′ is $C_1$-$C_5$ alkyl, $C_1$-$C_5$ haloalkyl, $C_1$-$C_5$ alkyl substituted with one or two $R_3$ groups, $C_2$-$C_5$ alkenyl, $C_2$-$C_5$ haloalkenyl, $C_3$-$C_5$ alkenyl substituted with one or two $R_3$ groups, $C_3$-$C_5$ alkynyl, $C_3$-$C_5$ haloalkynyl, $C_3$-$C_5$ alkynyl substituted with one or two $R_3$ groups, $C_3$-$C_5$ cycloalkyl, $C_3$-$C_5$ halocycloalkyl, $C_3$-$C_5$ cycloalkyl substituted with one or two $R_4$ groups, $C_4$-$C_7$ cycloalkylalkyl, $C_4$-$C_7$ halocycloalkylalkyl, $C_4$-$C_7$ cycloalkylalkyl substituted with one or two $R_4$ groups, phenyl or benzyl;

$R_3$ is $C_1$-$C_3$ alkoxy, $C_1$-$C_3$ alkylthio, $C_1$-$C_3$ haloalkoxy, $C_1$-$C_3$ alkylsulfinyl, $C_1$-$C_3$ alkylsulfonyl, CN, $NO_2$, OH, $OR_5$ or di-($C_1$-$C_3$ alkyl)amino;

$R_4$ is $C_1$-$C_3$ alkyl, $C_1$-$C_3$ alkoxy, $C_1$-$C_3$ haloalkoxy, CN, $NO_2$, OH, $OR_5$ or di-($C_1$-$C_3$ alkyl)amino;

$R_5$ is $SO_2CH_3$, $Si(CH_3)_3$, $C_2$-$C_3$ alkylcarbonyl or $CO_2C_1$-$C_2$ alkyl;

E is a single bond or $CH_2$;

W is O or S;

n is O or 1;

n′ is 0 or 1;

A is 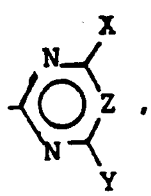 ,

A-1

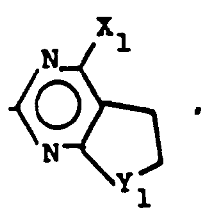 ,

A-2

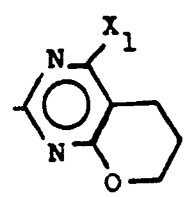 ,

A-3

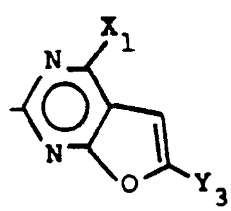 ,

A-4

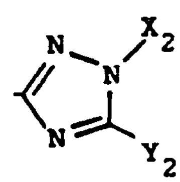 ,

A-5

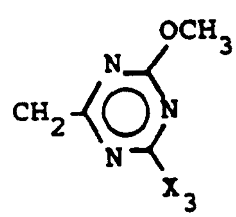 ;

A-6

or 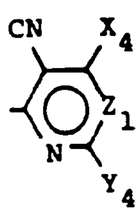 ;

A-7

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio,

halogen, $C_2$-$C_5$ alkoxyalkyl, $C_2$-$C_5$ alkoxyalkoxy, amino, $C_1$-$C_3$ alkylamino, di($C_1$-$C_3$ alkyl)amino or $C_3$-$C_5$ cycloalkyl;

Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, $C_2$-$C_5$ alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkylthioalkyl, $C_1$-$C_4$ haloalkyl, $C_2$-$C_4$ alkynyl, azido, cyano, $C_2$-$C_5$ alkylsulfinylalkyl, $C_2$-$C_5$ alkylsulfonylalkyl,

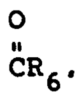, 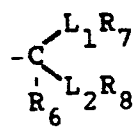, 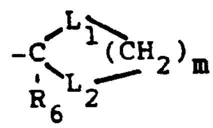, 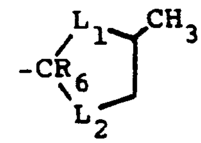

or $N(OCH_3)CH_3$;

m is 2 or 3;

$L_1$ and $L_2$ are independently O or S:

$R_6$ is H or $C_1$-$C_3$ alkyl;

$R_7$ and $R_6$ are independently $C_1$-$C_3$ alkyl;

Z is CH or N;

$Z_1$ is CH or N;

$Y_1$ is O or $CH_2$;

$X_1$ is $CH_3$, $OCH_3$, $OC_2H_5$ or $OCF_2H$;

$X_2$ is $CH_3$, $C_2H_5$ or $CH_2CF_3$;

$Y_2$ is $OCH_3$, $OC_2H_5$, $SCH_3$, $SC_2H_5$, $CH_3$ or $CH_2CH_3$;

$X_3$ is $CH_3$ or $OCH_3$;

$Y_3$ is H or $CH_3$;

$X_4$ is $CH_3$, $OCH_3$, $OC_2H_5$, $CH_2OCH_3$ or Cl; and

$Y_4$ is $CH_3$, $OCH_3$, $OC_2H_5$ or Cl;

and their agriculturally suitable salts; provided that

a) when X is Cl, F, Br or I, then Z is CH and Y is $OCH_3$, $OC_2H_5$, $N(OCH_3)CH_3$, $NHCH_3$,$N(CH_3)_2$ or $OCF_2H$;

b) when X or Y is $C_1$ haloalkoxy, then Z is CH;

c) $X_4$ and $Y_4$ are not simultaneously Cl;

d) when W is S, then $R_1$ is H, A is A-1 and Y is $CH_3$, $OCH_3$, $OC_2H_5$, $CH_2OCH_3$, $C_2H_5$, $CF_3$, $SCH_3$, $OCH_2CH{=}CH_2$, $OCH_2C{\equiv}CH$, $OCH_2CH_2OCH_3$, $CH(OCH_3)_2$ or 1,3-dioxolan-2-yl;

e) when the total number of carbons of X and Y is greater than four, then the number of carbons of R must be less than or equal to two:

f) when J is J-1, J-2, J-3 or J-4 then R′ is other than phenyl;

g) when J is J-5, J-6 or J-7 wherein E is a single bond, then R′ is other than $C_1$-$C_5$ alkyl, $C_3$-$C_5$ alkenyl, phenyl, benzyl, cyclopentyl or $C_4$-$C_7$ cycloalkylalkyl;

h) when either or both of X and Y are $OCF_2H$ then J is J-1, J-2, J-3, J-4, J-8, J-9, J-10 or J-11; and

i) when A is A-7 and $Z_1$ is M, then J is J-1, J-2, J-3 or J-4 and R′ is $C_3$-$C_5$ cycloalkyl;

j) when the total number of carbon atoms of X and Y is greater than four, then the total number of carbon atoms of $R_2$ and R′ must be less than or equal to 7.

In the above definitions, the term "alkyl", used either alone or in compound words such as "alkylthio" or "haloalkyl", denotes straight chain or branched alkyl, e.g. methyl, ethyl, n-propyl, isopropyl or the different butyl and pentyl isomers.

Alkoxy denotes methoxy, ethoxy, n-propyloxy, isopropyloxy and the different butyl isomers.

Alkenyl denotes straight chain or branched alkenes, e.g., 1-propenyl, 2-propenyl, 3-propenyl and the different butenyl and pentenyl isomers.

Alkynyl denotes straight chain or branched alkynes, e.g. ethynyl, 1-propynyl, 2-propynyl and the different butynyl and pentynyl isomers.

Alkylsulfonyl denotes methylsulfonyl, ethylsulfonyl and the different propylsulfonyl isomers.

Alkylthio, alkylsulfinyl, alkylamino, etc. are defined analogously to the above examples.

Cycloalkyl denotes cyclopropyl, cyclobutyl, cyclopentyl and cyclohexyl.

The term "halogen", either alone or in compound words such as "haloalkyl", denotes fluorine, chlorine, bromine or iodine. Further, when used in compound words such as "haloalkyl" said alkyl may be partially halogenated or fully substituted with halogen atoms and said halogen atoms may be the same or different. Examples

of haloalkyl include $CH_2CH_2F$, $CF_2CF_3$ and $CH_2CHFCl$.

The total number of carbon atoms in a substituent group is indicated by the $C_i$-$C_j$ prefix where i and j are numbers from 1 to 7. For example, $C_1$-$C_3$ alkylsulfonyl would designate methylsulfonyl through propylsulfonyl, $C_2$ alkoxyalkoxy would designate $OCH_2OCH_3$, $C_2$ cyanoalkyl would designate $CH_2CN$ and $C_3$ cyanoalkyl would designate $CH_2CH_2CN$ and $CH(CN)CH_3$.

Compounds preferred for reasons of increased ease of synthesis and/or greater herbicidal efficacy are:

1. Compounds of Formula I wherein in addition the provisos (a) to (j) there is additionally the following proviso (k) when J is J-5, $R_1$ is H, $R_2$ is H, E is $CH_2$, A is A-1, X is $OCH_3$, Y is $OCH_3$ and Z is CH, then R′ is other than $CH_3$.
2. Compounds of Formula I where E is a single bond; and
W is 0.
3. Compounds of Formula I where E is $CH_2$; and
W is 0.
4. Compounds of Preferred 2 where
$R_2$ is H, $C_1$-$C_3$ alkyl, halogen, $C_1$-$C_3$ alkyl substituted with 1 to 3 halogen atoms selected from 1 to 3 Cl, 1 to 3 F or 1 Br, $OCH_3$, $SO_2NHCH_3$, $SO_2N(CH_3)_2$, $S(O)_nCH_3$, $CO_2CH_3$, $CO_2CH_2CH_3$, $OCF_2H$, $CH_2OCH_3$ or $CH_2CN$;
R is H, $C_1$-$C_3$ alkyl, phenyl, $CH_2CF_3$ or $CH_2CH{=}CH_2$;
X is $C_1$-$C_2$ alkyl, $C_1$-$C_2$ alkoxy, Cl, F, Br, I, $OCF_2H$, $CH_2F$, $CF_3$, $OCH_2CH_2F$, $OCH_2CHF_2$, $OCH_2CF_3$, $CH_2Cl$ or $CH_2Br$; and
Y is H, $C_1$-$C_2$ alkyl, $C_1$-$C_2$ alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH{=}CH_2$, $OCH_2C{\equiv}CH$, $OCH_2CH_2OCH_3$, $CH_2SCH_3$,

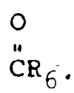, 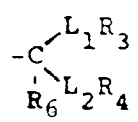, 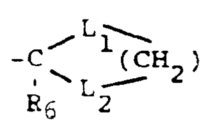, 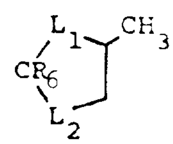,

$OCF_2H$, $OCF_2Br$. $SCF_2H$, cyclopropyl, $C{\equiv}CH$ or $C{\equiv}CCH_3$.
5. Compounds of Preferred 4 where
R′ is $C_1$-$C_4$ alkyl, $C_1$-$C_3$ alkyl substituted with 1 to 3 halogen atoms selected from 1 to 3 Cl, 1 to 3 F or 1 Br, $C_2$-$C_4$ alkoxyalkyl, $C_2$-$C_4$ alkylthioalkyl, $C_2$-$C_4$ cyanoalkyl, $C_2$-$C_4$ alkenyl, $C_2$-$C_3$ alkenyl substituted with 1 to 3 halogen atoms selected from 1 to 3 Cl, 1 to 3 F or 1 Br, $C_3$-$C_4$ alkynyl, $C_3$-$C_5$ cycloalkyl, $C_3$-$C_5$ cycloalkyl substituted with 1 to 3 halogen atoms selected from 1 to 3 Cl, 1 to 3 F or 1 Br or cyclopropylmetnyl.
6. Compounds of Preferred 5 where
A is A-1;
n is O:
X is $CH_3$, $OCH_3$, $OCH_2CH_3$, Cl or $OCF_2H$; and
Y is $CH_3$, $OCH_3$, $C_2H_5$, $CH_2OCH_3$, $NHCH_3$, $CH(OCH_3)_2$ or cyclopropyl.
7. Compounds of Preferred 6 where
$R_1$ is H;
$R_2$ is H, Cl, Br, $OCH_3$ or $CH_3$; and
R′is $C_1$-$C_3$ alkyl, $C_1$-$C_3$ alkyl substituted with 1 to 3 F, $C_2$-$C_3$ alkoxyalkyl, $C_2$-$C_3$ alkylthioalkyl, $C_2$-$C_3$ cyanoalkyl, $C_2$-$C_3$ alkenyl, propargyl, $C_3$-$C_5$ cycloalkyl or cyclopropylmethyl.
8. Compounds of Preferred 7 where J is J-1.
9. Compounds of Preferred 7 where J is J-2.
10. Compounds of Preferred 7 where J is J-3.
11. Compounds of Preferred 7 where J is J-4.
12. Compounds of Preferred 7 where J is J-5.
13. Compounds of Preferred 7 where J is J-6.
14. Compounds of Preferred 7 where J is J-7.
15. Compounds of Preferred 7 where J is J-8.
16. Compounds of Preferred 7 where J is J-9.
17. Compounds of Preferred 7 where J is J-10.
18. Compounds of Preferred 7 where J is J-11.
19. Compounds of Preferred 8 where
R′ is $C_1$-$C_3$ alkyl.

20. Compounds of Preferred 8 where

R′ is $C_1$-$C_3$ alkyl substituted with 1 to 3 F, $C_2$-$C_3$ alkoxyalkyl, $C_2$-$C_3$ alkylthioalkyl, $C_2$-$C_3$ cyanoalkyl, $C_2$-$C_3$ alkenyl, propargyl, $C_3$-$C_5$ cycloalkyl or cyclopropylmethyl.

21. Compounds of Preferred 8 where

R′ is $C_3$-$C_5$ cycloalkyl.

22. Compounds of Preferred 3 where

R is H, $C_1$-$C_3$ alkyl, phenyl, $CH_2CF_3$ or $CH_2CH{=}CH_2$;

$R_2$ is H, Cl, Br, $OCH_3$ or $CH_3$;

R′ is $C_1$-$C_3$ alkyl, $C_1$-$C_3$ alkyl substituted with 1 to 3 F, $C_2$-$C_3$ alkoxyalkyl, $C_2$-$C_3$ alkylthioalkyl, $C_2$-$C_3$ cyanoalkyl, $C_2$-$C_3$ alkenyl, propargyl, $C_3$-$C_5$ cycloalkyl or cyclopropylmethyl.

n is O;

A is A-1:

X is $CH_3$, $OCH_3$, $OCH_2CH_3$, Cl or $OCF_2H$; and

Y is CH3, $OCH_3$, $C_2H_5$, $CH_2OCH_3$, $NHCH_3$, $CH(OCH_3)_2$ or cyclopropyl.

23. Compounds of Formula I where

J is J-1, J-2, J-3 or J-4; and

R′ is $C_1$-$C_5$ haloalkyl, $C_1$-$C_5$ alkyl substituted with one or two $R_3$ groups, $C_2$-$C_5$ alkenyl, $C_2$-$C_5$ haloalkenyl, $C_3$-$C_5$ alkenyl substituted with one or two $R_3$ groups, $C_3$-$C_5$ alkynyl, $C_3$-$C_5$ haloalkynyl, $C_3$-$C_5$ alkynyl substituted with one or two $R_3$ groups, $C_3$-$C_5$ cycloalkyl, $C_3$-$C_5$ halocycloalkyl, $C_3$-$C_5$ cycloalkyl substituted with one or two $R_4$ groups, $C_4$-$C_7$ cycloalkylalkyl substituted with one or two $R_4$ groups, phenyl or benzyl.

24. Compounds of Formula I where J is J-5, J-6 or J-7.

25. Compounds of Formula I where when J is J-8, J-9, J-10 or J-11.

Compounds of the invention specifically preferred for reasons of greatest ease of synthesis and/or greatest herbicidal efficacy are 4-(cyclopropylcarbonyl)-N-[(4,6-dimethoxy-pyrimidin-2-yl)aminocarbinyl]-1-methyl-1H-pyrazole-5-sulfonamide, m.p. 189-192°C (d); 4-(1-oxopropyl)-N-[(4,6-dimethoxypyrimidin-2-yl)aminocarbonyl]-1-methyl-1H-pyrazole-5-sulfonamide, m.p. 189-192°C(d), and 2-(cyclopropylcarbonyl)-N-[(4,6-dimethoxypyrimidin-2-yl)aminocarbonyl]-3-thiophenesulfonamide, m.p. 165-168°C.

The compounds of this invention are highly active as preemergent and/or postemergent herbicides or plant growth regulants with selectivity on rice, corn, wheat, soybeans and barley.

## DETAILED DESCRIPTION OF THE INVENTION

### Synthesis

Compounds of Formula I can be prepared by one or more of the procedures shown in Equations 1, 4, and 5. J, $R_1$,and A are as previously defined.

**Equation 1**

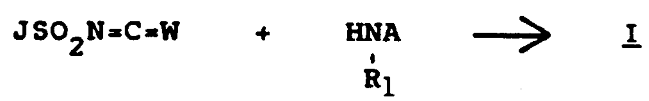

II III

The reaction of Equation 1 is best carried out in an inert aprotic organic solvent such as dichloromethane, 1,2-dichloroethane, tetrahydrofuran, or acetonitrile, at a temperature between 20° and 85°C. The order of addition is not critical; however, it is often convenient to add the sulfonyl isocyanate or a solution of it in the reaction solvent, to a stirred suspension of the amine.

In some cases, the desired product is insoluble in the reaction solvent at ambient temperature and crystallizes from it in pure form. Products soluble in the reaction solvent are isolated by evaporation of the solvent. Compounds of Formula I then may be purified by trituration of the evaporation residue with solvents such as 1-chlorobutane or ethyl ether and filtration, by recrystallization from mixtures of solvents such as 1,2-dichloroethane, 1-chlorobutane and heptane or by chromatography on silica gel.

Sulfonyl isocyanates (II, W is O) are known in the art and are prepared from the corresponding sulfonamides (IV) by one of the following two general methods.

Equation 2

$$\underset{\underline{IV}}{JSO_2NH_2} \xrightarrow[COCl_2,\ cat.]{CH_3(CH_2)_3NCO} \underline{II},\ W \text{ is } O$$

The sulfonamide IV is reacted with an alkyl isocyanate (e.g., n-butyl isocyanate) in a solvent whose boiling point is above 135°C, such as xylene. The reaction can optionally be carried out in the presence of a catalytic amount of 1,4-diaza[2.2.2]bicyclooctane (DABCO). The reaction mixture is heated to 135-140°C and held at that temperature for 5-60 minutes, after which phosgene is slowly added at such a rate that the temperature remains between 133 and 135°C. When the consumption of phosgene has ceased, the mixture is cooled and filtered to remove insoluble material. Finally, the solvent, alkyl isocyanate, and excess phosgene are evaporated, leaving the sulfonyl isocyanate (II).

If desired, the alkyl isocyanate-sulfonamide adduct can be made and isolated before reaction with the phosgene. In this case the sulfonamide (IV), alkyl isocyanate, and anhydrous base (e.g, $K_2CO_3$) in a polar, aprotic solvent (e.g. acetone, butanone, or acetonitrile) are mixed and heated under reflux for 1 to 6 hours. The reaction mixture is then diluted with water, and the pH is adjusted to about 3 with acid (e.g. HCl, $H_2SO_4$). The adduct is filtered out and dried, and then reacted with phosgene as described above. This procedure modification is especially useful when sulfonamide (IV) is high melting and has low solubility in the phosgenation solvent.

Sulfonyl isocyanates (II, W is O) can also be prepared by the following method.

Equation 3

$$\text{(a)}\quad \underline{IV} \xrightarrow{SOCl_2} \underset{\underline{V}}{JSO_2NSO}$$

$$\text{(b)}\quad \underline{V} \xrightarrow{COCl_2,\ pyridine\ cat.} \underline{II},\ W \text{ is } O$$

The sulfonamide (IV) is heated at reflux in an excess of thionyl chloride. The reaction is continued until the sulfonamide protons are no longer detectable in the proton magnetic resonance spectrum. From 16 hours to 5 days is typically sufficient for complete conversion to the thionylamide (V) (Equation 3a).

The thionyl chloride is evaporated and the residue is treated with an inert solvent (e.g. toluene) containing at least one equivalent (typically 2-3 equivalents) of phosgene. A catalytic amount of pyridine (typically 0.1 equivalent) is added, and the mixture is heated to about 60-140°C, with 80-100°C preferred. Conversion to the isocyanate (II, W is O) is usually substantially complete within 15 minutes to 3 hours (Equation 3b). The mixture is then cooled and filtered, and the solvent is evaporated, leaving the sulfonyl isocyanate (II, W is O).

Sulfonyl isothiocyanates (II, W is S) are known in the art and are prepared from the corresponding sulfonamides (IV) by reaction with carbon disulfide and potassium hydroxide followed by treatment of the resulting dipotassium salt VI with phosgene. Such a procedure is described in Arch. Pharm. 299, 174 (1966).

Many of the compounds of Formula I can be prepared by the procedure shown in Equation 4.

Equation 4

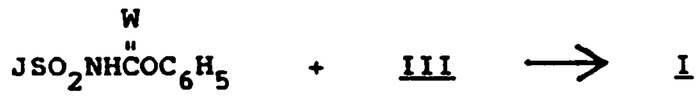

VI

The reaction of Equation 4 is carried out by contacting phenylcarbamates or phenylthiocarbamates of Formula VI with aminoheterocycles of Formula III in an inert organic solvent such as dioxane or tetrahydrofuran at temperatures of about 20-100°C for a period of about one-half to twenty-four hours. The product can be isolated by evaporation of the reaction solvent and purified by methods previously described.

Phenylcarbamates and phenylthiocarbamates of Formula VI can be prepared by the methods described, or modifications thereof known to those skilled in the art, in U.S. 4,443,243.

Alternatively, many of the compounds of Formula I can be prepared by the method described in Equation 5.

Equation 5

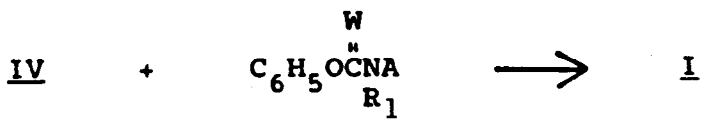

VII

The reaction of Equation 5 can be carried out by contacting equimolar amounts of a sulfonamide of Formula IV with a heterocyclic phenylcarbamate or phenylthiocarbamate of Formula VII in the presence of a base such as 1,8-diazabicyclo[5.4.0]undec-7-ene (DBU), by methods analogous to those described in South African Patent Application 83/0441. The phenylcarbamates and phenylthiocarbamates of Formula VII can be prepared by methods, or modifications thereof known to those skilled in the art, described in South African Patent Application 82/5671 and South African Patent Application 82/5045.

The sulfonamides IV of this invention may be prepared in a variety of ways some of which are described in Equations 6 through 15.

For example, the 4-keto-5-sulfonamide isomer 1 may be prepared as outlined in Equation 6.

Equation 6

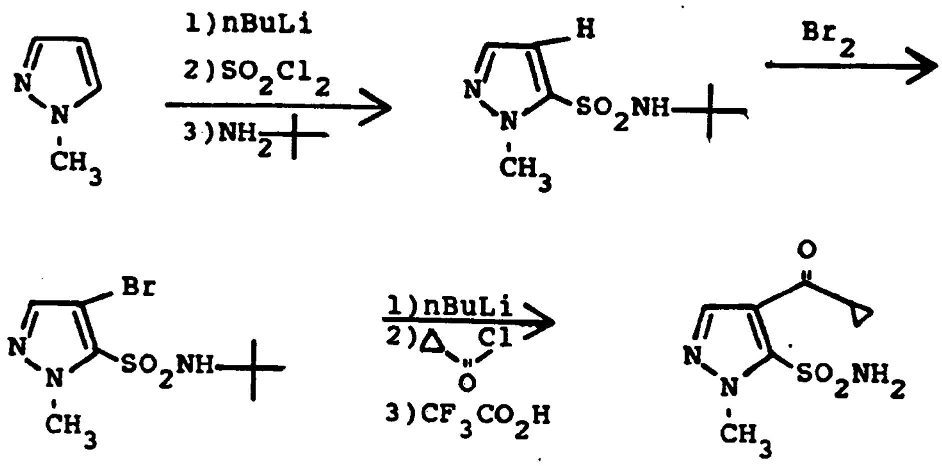


2

1

Preparation of the intermediates such as bromide 2 may be found in EPA-95,925. Exposure of bromide 2 to nBuLi followed by addition of the resulting anion to cyclopropyl acid chloride affords the protected sulfonamide. Deprotection of the sulfonamide affords the desired sulfonamide 1.

Introduction of various R and $R_2$ groups to sulfonamides such as 1 may be accomplished in several ways. For example, the sequence in Equation 6 could also be performed on 3-chloro-1-methylpyrazole or 1,3-dimethylpyrazole affording 3 and 4 respectively. Chloride 3 may then be used to further elaborate

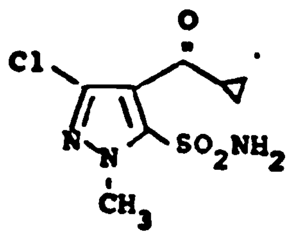

3

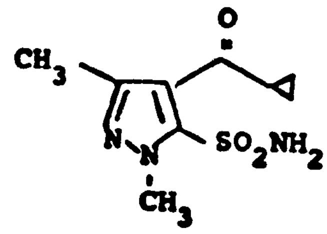

4

$R_2$ as outlined in Equation 7.

Equation 7

3

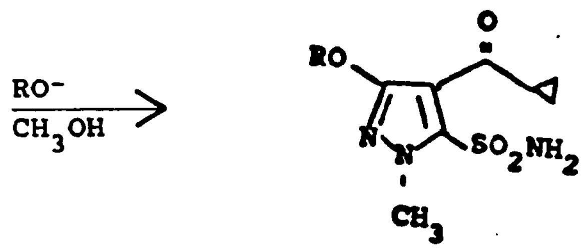


5

The N-substituent of compounds such as 1, 3, 4 and 5 may also be varied by applying the same sequence of reactions as outlined in Equation 6 to various N-substituted pyrazoles. For example, pyrazole may be alkylated with dimethylsulfamoylchloride to afford pyrazole 6. Pyrazole 6 is then converted to sulfonamide 7 as outlined in Equation 8.

Equation 8

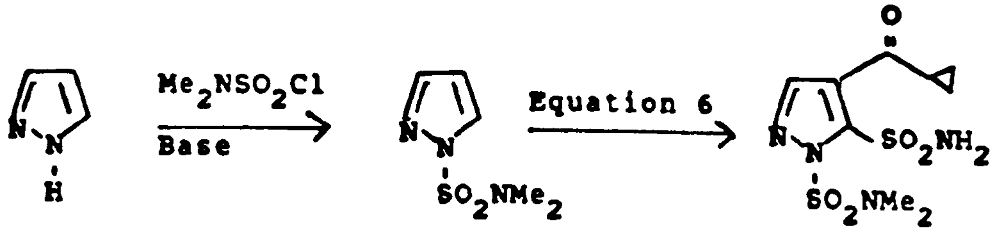


6 7

In the case where either R or $R_2$ are sensitive to nBuLi (i.e. $R_2$ is $CO_2CH_3$ or Br) then the lithiating reagent of choice is lithium diisopropylamide (LDA). Utilizing the same sequence as outlined in Equation 6 but substituting LDA for nBuLi affords sulfonamides such as 8. This is outlined in Equation 9.

Equation 9

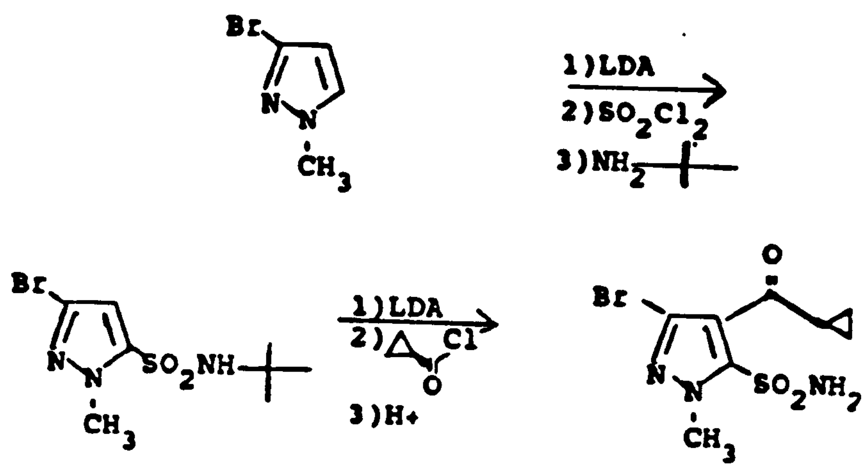


8

The isomeric 5-keto-4-sulfonamide pyrazoles may be prepared as outlined in Equations 10 and 11.

Equation 10

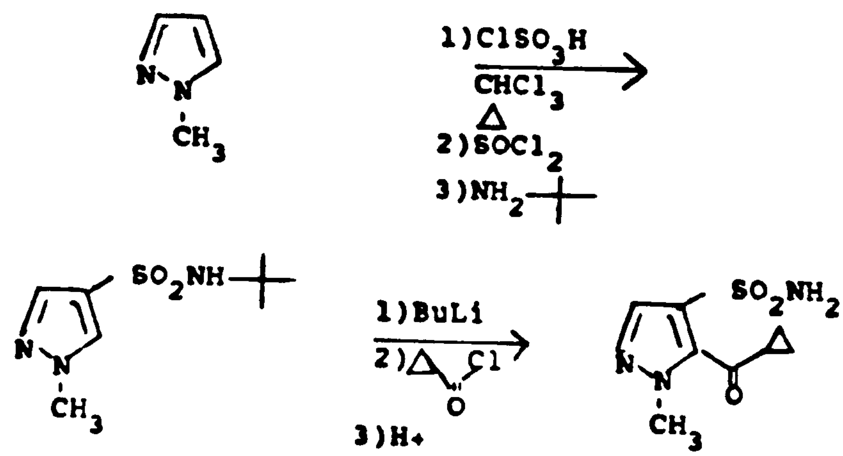


9

In Equation 10 the sequential order of group introduction is reversed to that of Equation 6. The introduction of various R and $R_2$ groups may be accomplished in the same manor as previously described for the 4-ketoisomer in Equations 7, 8 and 9.

An alternate synthesis of sulfonamides such as 9 is outlined in Equation 11.

Equation 11

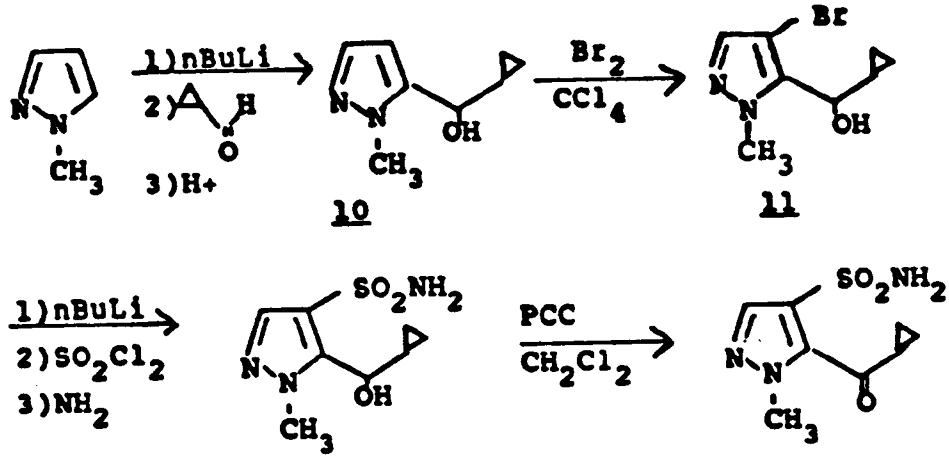


10

11

12

9

Oxidations of alcohols to ketones such as 12 to 9 are well known in the art. For further discussion pertaining to the oxidation of alcohols to ketones, see R. H. Cornforth, J. W. Cornforth and G. Popjak, Tetrahedron, 18, 1351 (1962).

The isomeric 3-keto-4-sulfonamide such as 13 may be prepared as outlined in Equation 12.

Equation 12

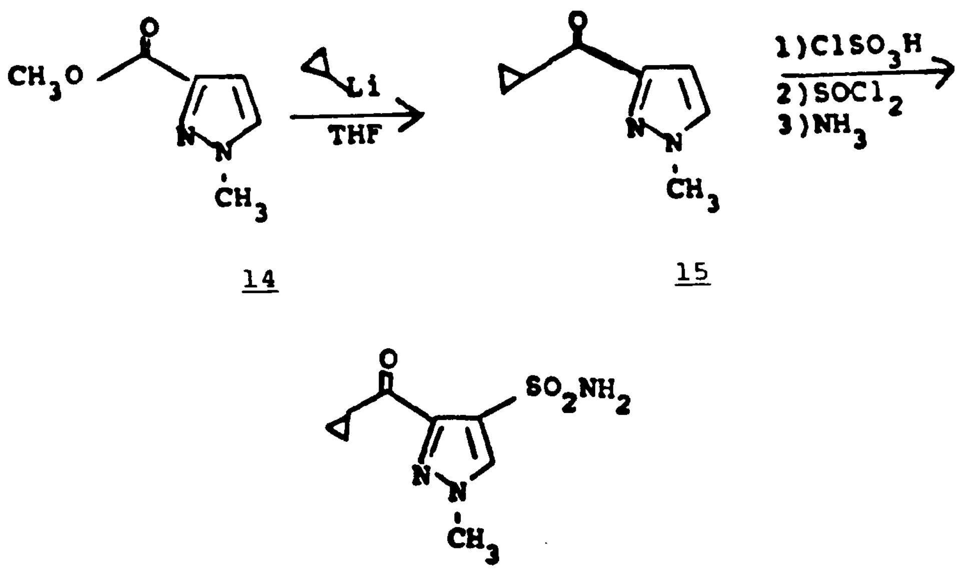


14

15

13

In the above example of Equation 12, as before, minor variations of starting material allows for the introduction of different R and $R_2$ groups. The starting pyrazoles 14 or 15 may be prepared via the condensation of a hydrazine with a triketo species as outlined in Equation 13.

Equation 13

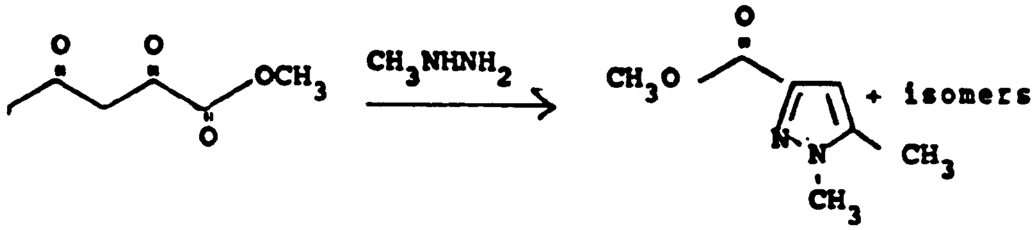


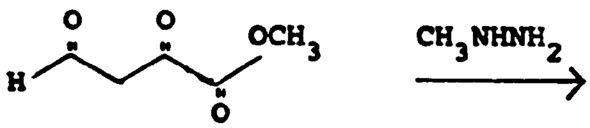


14

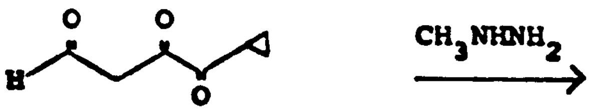


15

The final pyrazole isomer of the invention such as sulfonamide 16 may be prepared as outlined in Equation 14.

Equation 14

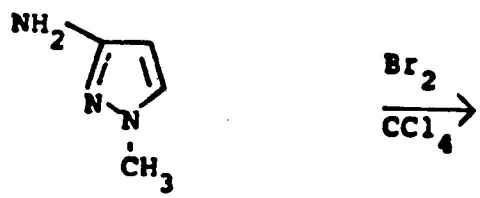


17

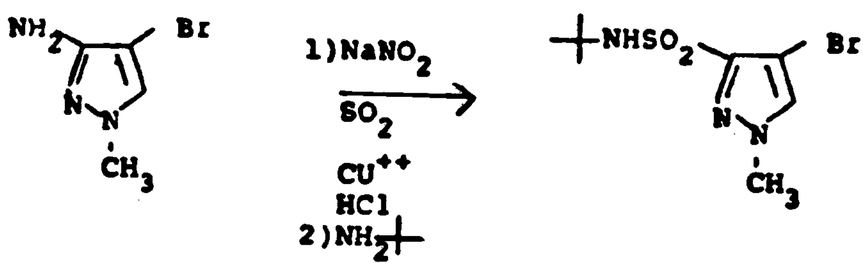


18

19

19

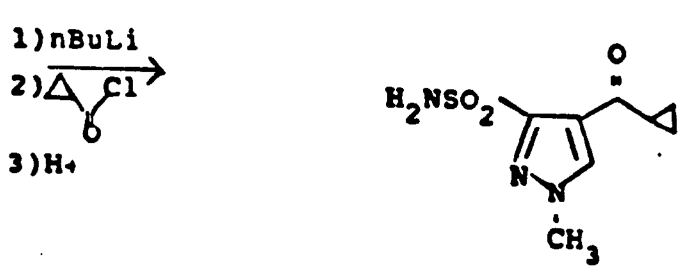


16

Again, as described previously, alteration of the starting material allows for the preparation of compounds such as 16 where R and/or $R_2$ may be varied. For example, utilizing phenylhydrazine and a chloronitrite results in pyrazole 20 and subsequently sulfonamide, 21. This is outlined in Equation 15.

Equation 15

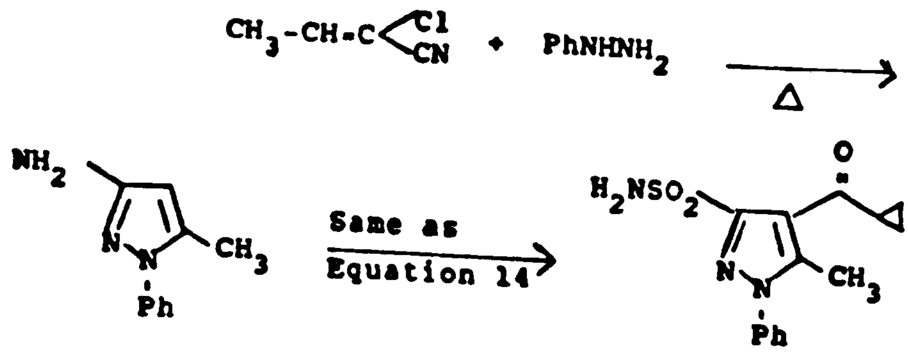


20

21

In all the above examples, substitution of cyclopropyl acid chloride with other acid chlorides would result in the corresponding ketones such as compounds 22, 23, 24, 25 and 26.

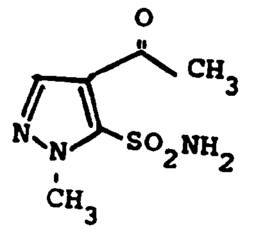

22

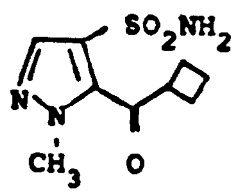

23

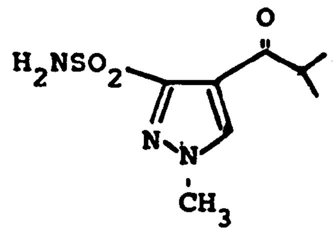

24

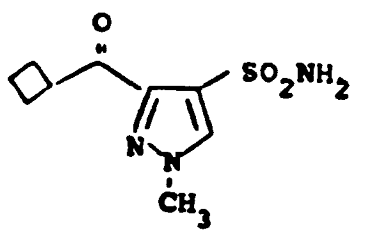

25

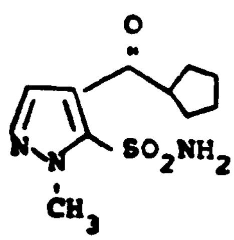

26

For further details pertaining to the synthesis of pyrazoles see EP-A-87,780, South African Patent Application 833,350, EP-A-95,925 and T. L. Jacobs, "Heterocyclic compounds", R. C. Elderfield ed., Vol. 5, pp. 45-161, Wiley, New York, 1957.

For further details pertaining to carbanions see J. Stowell, "Carbanions in Organic Synthesis", Wiley-Interscience, New York, 1979.

Thiophene sulfonamides such as 27 may be prepared as outlined in Equation 16.

## Equation 16

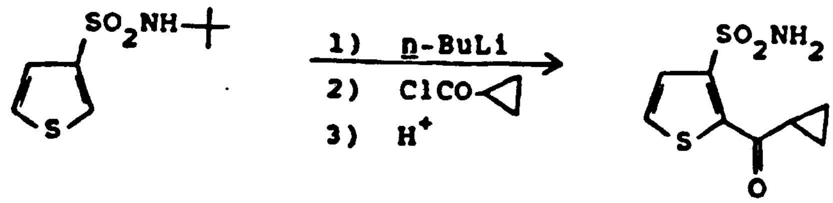


28 27

Introduction of various $R_2$ groups on the thiophene ring may be accomplished in several ways. For example, the sequence in Equation 16 may also be performed on the 4-substituted analogs resulting in the corresponding sulfonamides such as 29 and 30.

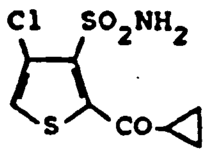

29

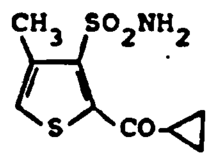

30

The isomeric 2-thiophenesulfonamide such as 31 may be prepared as outlined in Equation 17.

## Equation 17

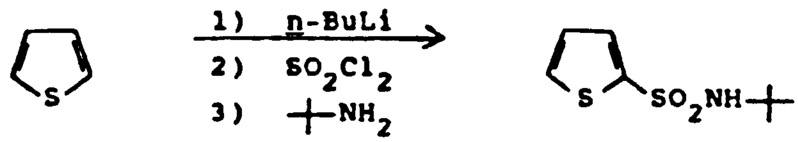


32

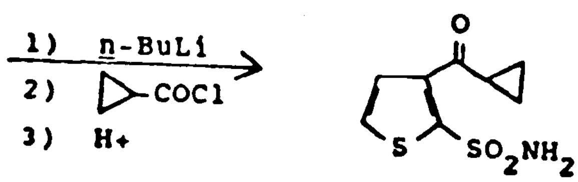


31

An alternate synthesis of sulfonamides such as 31 is outlined in Equation 18.

## Equation 18

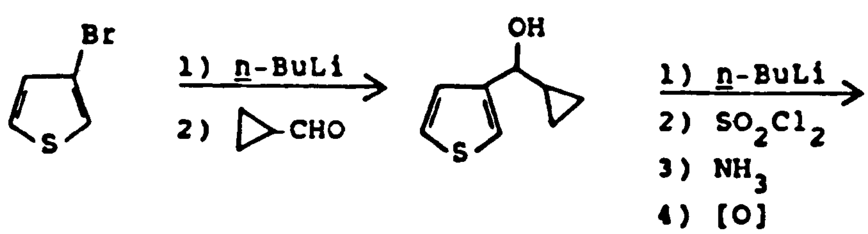


31

The isomeric 3-thiophenesulfonamide such as 33 may be prepared as outlined in Equation 19.

Equation 19

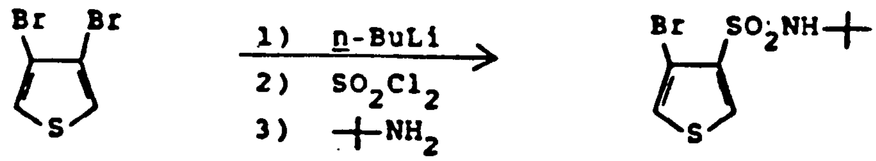


34 35

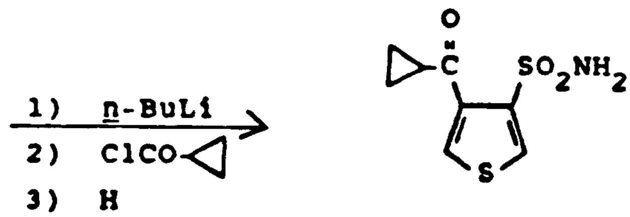


33

Introduction of various $R_2$ groups onto the thiophene ring may be accomplished by varying the starting material as previously described.

Further details pertaining to the preparation and functional group manipulation of thiophenes may be found in U.S. Patent 4,481,029.

Preparation of the pyridinesulfonamtdes of this invention, such as pyridine 36 may be carried out in a variety of ways. For example, Meerwein reaction of 37 followed by ortho lithiation affords sulfonamides such as 36, as outlined in Equation 20.

Equation 20

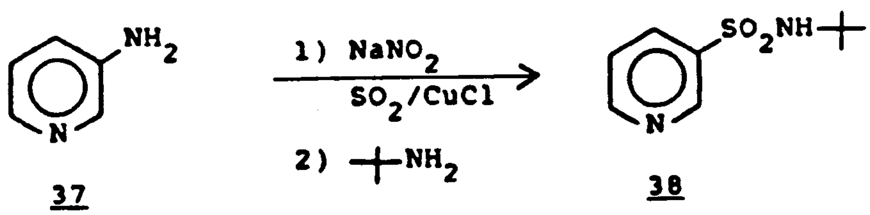


37 38

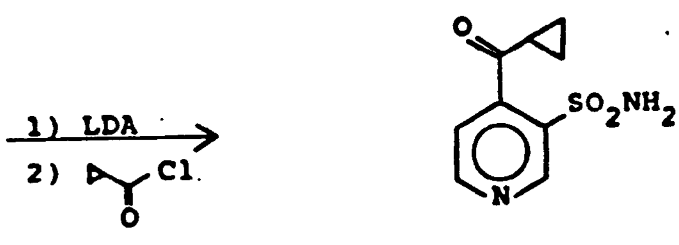


36

Prior to removal of the tert-butyl group it may be necessary to protect the ketone functionality as the ethylene ketal which may then be removed subsequently at a later time.

The isomeric sulfonamide, 39, may be prepared as outlined in Equation 21.

Equation 21

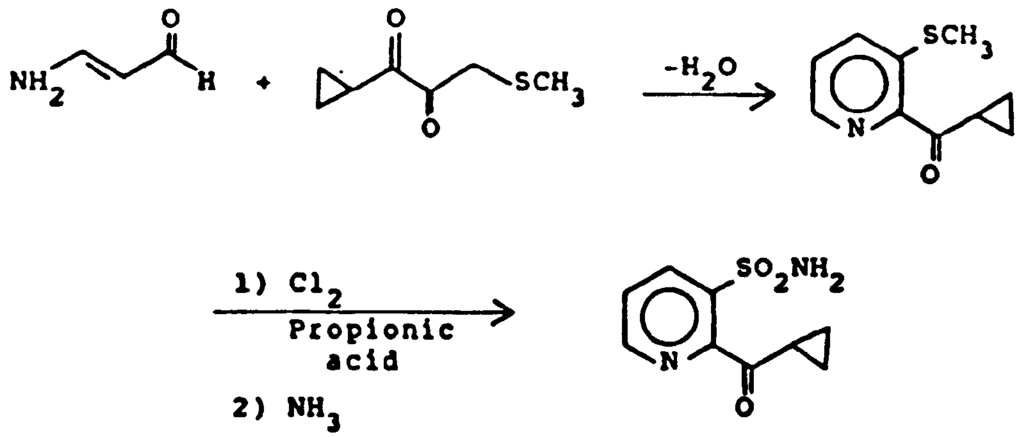


39

The isomeric sulfonamide 40 may be prepared as outlined in Equation 22.

Equation 22

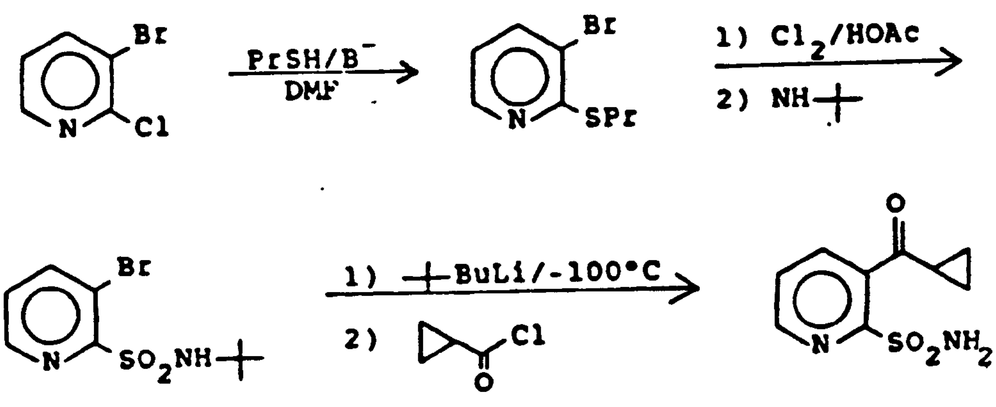


40

Introduction of various substituents on the pyridine ring system as well as variation of R′ may be accomplished as described previously for the pyrazole system.

For further details pertaining to the synthesis of pyridines see, E. Beritmaier, S. Gassenmann and E. Bayer, Tetrahedron 26, 5907 (1970); B. Blank et al., J. Med. Chem., 1, 1065 (1974); M. Mallet and G. Queguiner, Tetrahedron, 41, 3433 (1985) and J. Delarge and C. L. Lapiere, Annales Pharm. France, 36, 369 (1978).

The synthesis of heterocyclic amines such as those represented by Formula III has been reviewed in "The Chemistry of Heterocyclic Compounds," a series published by Interscience Publ., New York and London. Aminopyrimidines are described by D. J. Brown in "The Pyrimidines," Vol. XVI of the series mentioned above which is herein incorporated by reference. The 2-amino-1,3,5-triazines of Formula III, where A is A-1 and Z is N, can be prepared according to methods described by E. M. Smolin and L. Rapaport in "s-Triazines and Derivatives," Vol. XIII.

Pyrimidines of Formula III, where A is A-1 and Y is an acetal or thioacetal substituent, can be prepared by methods taught in European Patent Application no. 84,224 (published July 27, 1983).

Pyrimidines of Formula III, where A is A-1 and Y is cyclopropyl or $OCF_2H$ can be synthesized according to the methods taught in United States Patent 4,515,626 and United States Patent 4,540,782, respectively.

Compounds of Formula III, where A is A-2 or A-3, can be prepared by procedures disclosed in United States Patent 4,339,267.

Compounds of Formula III, where A is A-4, can be prepared by methods taught in United States Patent 4,487,626.

Additional references dealing with the synthesis of bicyclic pyrimidines of formula III, where A is A-2, A-3, or A-4 are Braker, Sheehan, Spitzmiller and Lott, J. Am. Chem. Soc., 69, 3072 (1947); Mitler and Bhattachanya, Quart. J. Indian Chem. Soc., 4, 152 (1927); Shrage and Hitchings, J. Org. Chem., 16, 1153 (1951); Caldwell, Kornfeld and Donnell, J. Am. Chem. Soc., 63, 2188 (1941); and Fissekis, Myles and Brown, J. Org. Chem., 29, 2670 (1964).

Compounds of Formula III, where A is A-5, can be prepared by methods taught in United States Patent 4,421,550.

Compounds of Formula III, where A is A-6, can be prepared by methods taught in the United States Patent 4,496,392.

Compounds of Formula III, where A is A-7 can be prepared by methods taught in EP-A-125,864.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide, carbonate or hydroxide). Quaternary amine salts can be made by similar techniques.

Salts of compounds of Formula I can also be prepared by exchange of one cation for another. Cationic exchange can be effected by direct contact of an aqueous solution of a salt of a compound of Formula I (e.g., alkali or quaternary amine salt) with a solution containing the cation to be exchanged. This method is most effective when the desired salt containing the exchanged cation is insoluble in water and can be separated by filtration.

Exchange may also be effected by passing an aqueous solution of a salt of a compound of formula I (e.g., an alkali metal or quaternary amine salt) through a column packed with a cation exchange resin containing the cation to be exchanged for that of the original salt and the desired product is eluted from the column. This method is particularly useful when the desired salt is water-soluble, e.g., a potassium sodium or calcium salt.

Acid addition salts, useful in this invention, can be obtained by reacting a compound of Formula I with a suitable acid, e.g., p-toluenesulfonic acid, trichloroacetic acid or the like.

The preparation of the compounds of this invention is further illustrated by the following specific examples. Unless otherwise indicated, temperatures are in degrees centigrade.

## Example 1

### Preparation of 4-cyclopropylcarbonyl-1-methyl-5-pyrazole t-butyl sulfonamide

To a cooled -78°C, solution of n-BuLi (3.7 g, 57.2 mmol) in approximately 350 ml of tetrahydrofuran is added 1-methyl-4-bromo-5-pyrazole t-butylsulfonamide (7.5 g, 25.4 mmol) dropwise. The solution is stirred for 15 minutes and then added via cannula to freshly distilled cyclopropyl acid chloride (6 g, 57.2 mmol) cooled to -78°C. The resulting solution was quenched with brine, separated, dryed and concentrated in vaccuo. The resulting oil was flash chromatographed (50:50 (v/v)) ethylacetate-hexane to afford 2.7 g of a white solid, m.p. 113-115°C.

## Example 2

### Preparation of 4-cyclopropylcarbonyl-1-methyl-5-pyrazolesulfonamide

To a stirring solution of trifluoroacetic acid was added 4-cyclopropyl-1-methyl-5-pyrazole t-butylsulfonamide. The solution was stirred overnight at room temperature. The reaction mixture was concentrated in vaccuo and the resulting solids were triturated with n-butylchloride, m.p. 125-127°C; NMR (200 MHz, $CDCl_3$) 1.1 (m, 2H), 1.3 (m, 2H), 2.4 (m, 1H), 4.2 (s, 3H), 6.4 (br. s, 2H), 8.07 (s, 1H).

## Example 3

### Preparation of 4-(Cyclopropylcarbonyl)-N-[(4,6-dimethoxypyrimidin-2-yl)aminocarbonyl]-1-methyl-1H-pyrazole-5-sulfonamide

To a stirring mixture of the sulfonamide from Example 2 (175 mg, 0.76 mmol) and the phenylcarbamate of 4,6-dimethoxy-2-aminopyrimidine (210 mg, 0.76 mmol) in 3 ml of acetonitrile was added diazobicycloundecane (116 mg, 0.76 mmol). The solution was stirred for approximately 10 minutes. Acidification of the reaction mixture and filtration of the resulting solids afforded 300 mg of the desired compound m.p. 189-192°C NMR (200 MHz, $CDCl_3$) 1.0 (m, 2H), 1.1 (m, 2H), 2.4 (m, 1H), 4.0 (s, 6H), 4.35 (s, 1H), 5.80 (s. 1H), 7.4 (br. S, 1H), 8.05 (s,

1H), 12.9 (s, 1H).

Example 4

Preparation of 4-Acetyl-1-methyl-1H-pyrazole-5-sulfonamide

To a stirring solution of n-BuLi (3.7 g, 57.2 mmol) in 350 mL of tetrahydrofuran cooled to -95°C is added 4-bromo-1-methyl-1H-pyrazole-5-t-butylsulfonamide (7.5 g, 25.4 mmol). The solution is cannulated into a stirring solution of acetyl chloride (76.2 mmol) cooled to -78°C. The reaction is stirred for 1/2 hour at -78°C then quenched with saturated sodium chloride. The organic layer is separated, dried and concentrated. The resulting crude oil was added to $CF_3CO_2H$ and allowed to stir for 24 hours. The acid was removed under vacuum and the resulting oil was flash chromatographed. The resulting solid, m.p. 153-162°C, was mostly the closed hemiaminal, which was used directly in the next reaction.

Example 5

Preparation of 4-Acetyl-N-[(4,6-dimethoxy-2-pyrimidinyl)aminocarbonyl]-1-methyl-1H-pyrazole-5-sulfonamide

To a mixture of the hemiaminal from Example 4 (200 mg, 0.98 mmol), the phenyl carbamate of 4,6-dimethoxy-2-aminopyrimidine (271 mg, 0.98 mmol) 3 mL of acetonitrile, and DBU (212 mg, 0.98 mmol) was added. The reaction was diluted with 3 mL of water and 3 mL of 5% hydrochloric acid. The resulting solids were collected to afford 200 mg of a white solid, m.p. 179-182°C; NMR (200 MHz, $CDCl_3$) δ 2.46 (s, 3H), 4.06 (s, 6H), 4.37 (s, 3H), 5.81 (s, 1H), 7.4 (br.s, 1H), 7.9 (s, 1H) and 13.0 (br.s, 1H); IR (KBr) 1730 $cm^{-1}$.

Example 6

Preparation of N-(1,1-Dimethylethyl)-1-methyl-4-(1-oxobutyl)-1H-pyrazole-5-sulfonamide

To a solution of n-BuLi (2.1 g, 33.7 mmol) cooled to -78°C in 250 mL of tetrahydrofuran is added the t-butyl protected 4-bromo-1-methyl-5-pyrazolesulfonamide (4.5 g, 15.2 mmol). The solution is then added to butyric anhydride (2.9 g, 18.2 mmol) at -78°C. Standard work-up afforded 4.4 g of an oil which was a mixture of the desired product and debrominated starting material. This material was not purified, but used as is in the next reaction.

Example 7

Preparation of 1-Methyl-4-(1-oxobutyl)-1H-pyrazole-5-sulfonamide

The mixture from the previous Example 6 (3.9 g) was added to $CF_3CO_2H$ (TFA) and stirred for 4.5 hours. Removal of the TFA afforded a brown oil. Flash chromatography (15:85 EtOAC:hexane (v/v)) yielded 1.0 g of a white solid, m.p. 97-99°C. NMR (200 MHz, $CDCl_3$) δ 0.97 (t, 3H), 1.67 (m, 2H), 2.83 (t, 3H), 4.19 (s, 3H), 6.40 (br.s, 2H) and 7.91 (s, 1H).

Example 8

Preparation of N-[(4-Methoxy-6-methyl-1,3,5-triazin-2-yl)aminocarbonyl]-1-methyl-4-(1-oxobutyl)-1H-pyrazole-5-sulfonamide

To a mixture of sulfonamide (150 mg, 0.61 mmol) and the phenyl carbamate of 4-methyl-6-methoxy-2-aminotriazine (158 mg, 0.61 mmol) in 3 mL of acetonitrile is added DBU (93 mg, 0.61 mmol). The solution is diluted with 3 mL of $H_2O$ and 3 mL of 5% HCl, and the resulting solids are collected, m.p. 174-176°C. NMR (200 MHz, $CDCl_3$) δ 0.88 (s, 3H), 1.68 (m, 2H), 2.7 (s, 3H), 2.79 (t, 2H), 4.19 (s, 3H), 4.33 (s, 3H), 7.7 (br.s. 1H), 7.9 (s, 1H) and 12.9 (br.s, 1H).

Example 9

Preparation of 2-(cyclopropylcarbonyl)-N-(1-1-dimethylethyl)-3-thiophenesulfonamide

To a stirring solution of n-BuLi (6.6g, 102 mmol) in 300 ml of tetrahydrofuran is added the t-butyl protected 3-thiophenesulfonamide (10.0g, 45.7 mmol). The solution was warmed to -30°C and then recooled to -78°C. The solution was cannulated into a mixture of cyclopropane carbonylic acid chloride (5.7g. 51.7 mmol) in 50 ml of tetrahydrofuran @ -78°C. The reaction was quenched with brine, separated and dryed over magnesium sulfate, concentration of the organic in vaccuo afforded 17g of anvil. Flash chromatography (25:75 EtCAC/hexane (v/v)) afforded 44 g of the desired product. NMR (200MHz, $CDC_3$) δ 1.26 (m, 13H), 2.5 (m, 1H), 6.5 (br. S, 1H), 7.5 (d, 1H), 7.67 (d, 1H):

Example 10

Preparation of 2-(cyclopropylcarbonyl)-N-[(4,6-dimethoxy-1,3,5-triazin-2-yl)aminocarbonyl]-3-thiophenesulfonamide

To a stirring solution of sulfonamide (300 mg, 1.3 mmol), the phenylcarbamate of 4,6-dimethoxy-2-aminotriazine (358 mg, 1.3 mmol) in 5 ml of acetonitrile was added DBU (197 mg, 1.3 mmol). The same work up as in example 5 afforded 390 mg of the desired product. m.p. 146-148°C, NMR (200 mHz, $CDCl_3$) δ, 1.1 (m,2H), 1.3 (m,2H), 2.5 (m,1H), 4.1 (S,6H), 7.5 hr,s,1H), 7.6 (d,1H), 7.9 (d,1H), 12.3 (s,1H):

Using the procedures from Equations 1 to 22 and Examples 1 to 10 the compounds of Tables I to XV can be prepared.

Tables

Table I

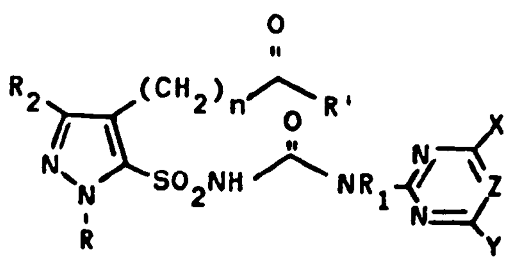

Table II

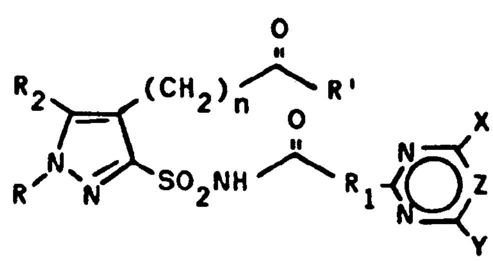

Table III

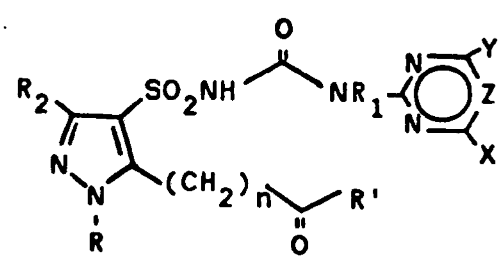

Table IV 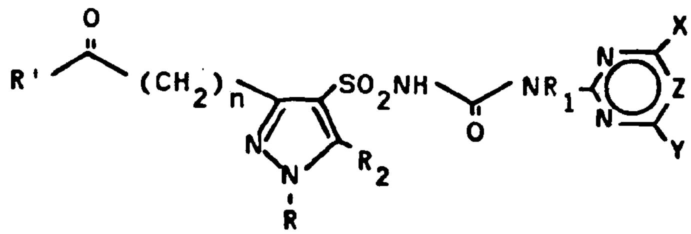

## Tables (continued)

Table V 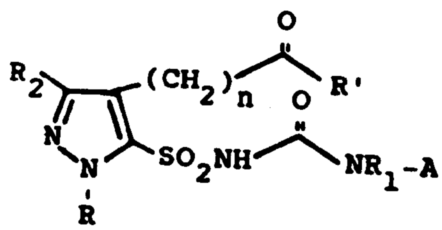

Table Va 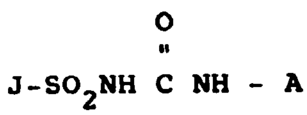

Table VI 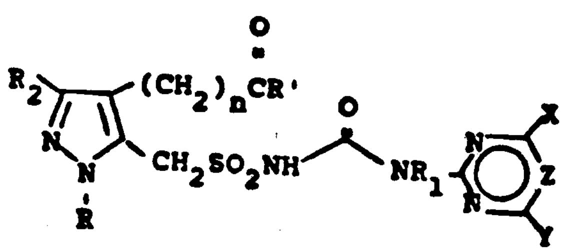

Table VII 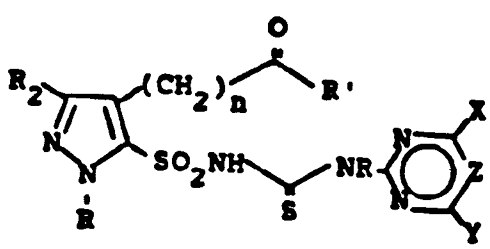

Table VIII 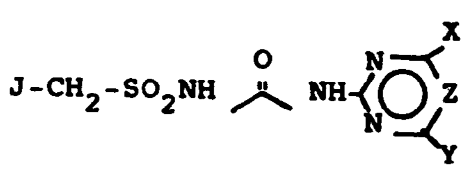

Table IX

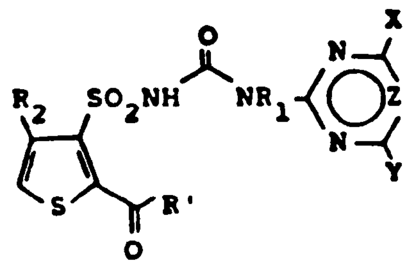

Tables (continued)

Table X

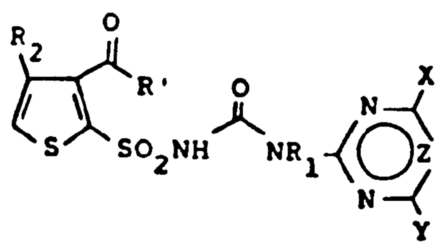

Table XI

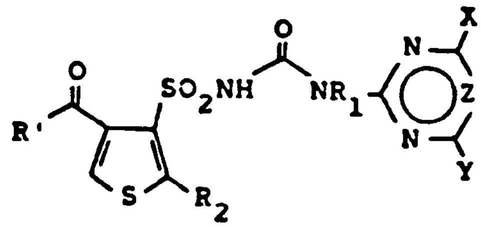

Table XII

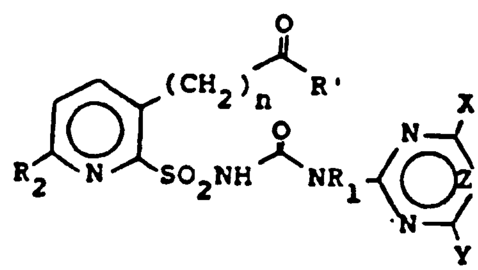

Table XIII

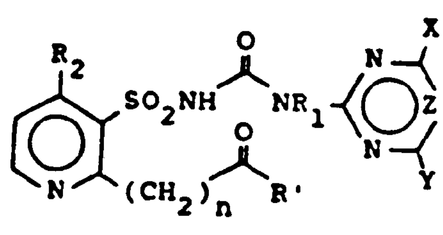

Table XIV

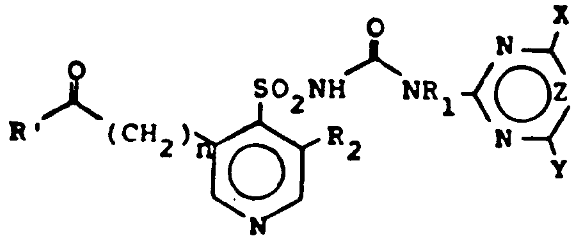

Tables (continued)

Table XV

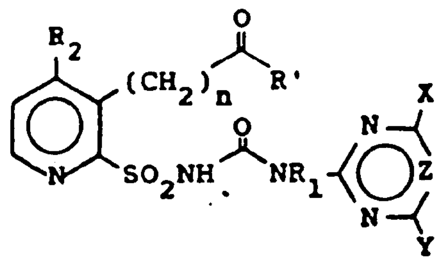

Table I

| R | $R_1$ | n | $R_2$ | R' | X | Y | Z | m.p.(°C) |
|---|---|---|---|---|---|---|---|---|
| $CH_3$ | H | O | H | cyclopropyl | $CH_3$ | $CH_3$ | CH | 162-165 |
| $CH_3$ | H | O | H | cyclopropyl | $CH_3$ | $OCH_3$ | CH | 155-157 |
| $CH_3$ | H | O | H | cyclopropyl | $OCH_3$ | $OCH_3$ | CH | 189-192 |
| $CH_3$ | H | O | H | cyclopropyl | $CH_3$ | $CH_3$ | N | |
| $CH_3$ | H | O | H | cyclopropyl | $CH_3$ | $OCH_3$ | N | 134-136 |
| $CH_3$ | H | O | H | cyclopropyl | $OCH_3$ | $OCH_3$ | N | 164-166 |
| $CH_3$ | H | O | H | cyclopropyl | Cl | $OCH_3$ | CH | 197-199 |
| $CH_3$ | H | O | H | cyclobutyl | $CH_3$ | $CH_3$ | CH | 190-194 |
| $CH_3$ | H | O | H | cyclobutyl | $CH_3$ | $OCH_3$ | CH | 200-203 |
| $CH_3$ | H | O | H | cyclobutyl | $OCH_3$ | $OCH_3$ | CH | 196-199 |
| $CH_3$ | H | O | H | cyclobutyl | $CH_3$ | $CH_3$ | N | |
| $CH_3$ | H | O | H | cyclobutyl | $CH_3$ | $OCH_3$ | N | 175-178 |
| $CH_3$ | H | O | H | cyclobutyl | $OCH_3$ | $OCH_3$ | N | 168-170 |
| $CH_3$ | H | O | H | cyclobutyl | Cl | $OCH_3$ | CH | 210-212 |
| $CH_3$ | H | O | H | cyclopentyl | $CH_3$ | $CH_3$ | CH | |
| $CH_3$ | H | O | H | cyclopentyl | $CH_3$ | $OCH_3$ | CH | |
| $CH_3$ | H | O | H | cyclopentyl | $OCH_3$ | $OCH_3$ | CH | |
| $CH_3$ | H | O | H | cyclopentyl | $CH_3$ | $CH_3$ | N | |
| $CH_3$ | H | O | H | cyclopentyl | $CH_3$ | $OCH_3$ | N | |
| $CH_3$ | H | O | H | cyclopentyl | $OCH_3$ | $OCH_3$ | N | |
| $CH_3$ | H | O | H | cyclopentyl | Cl | $OCH_3$ | CH | |
| $CH_3$ | H | O | H | cyclopropyl | $OCH_3$ | $OCH_2CH_3$ | CH | |
| $CH_3$ | H | O | H | cyclopropyl | cyclopropyl | $OCH_3$ | CH | |
| $CH_3$ | H | O | H | cyclopropyl | $OCH_3$ | $CH(OCH_3)_2$ | CH | |
| $CH_3$ | H | O | H | cyclopropyl | $NHCH_3$ | $OCH_2CH_3$ | N | |
| $CH_3$ | H | O | H | cyclopropyl | $NHCH_3$ | $OCH_3$ | N | |
| $CH_3$ | H | O | H | cyclopropyl | $OCH_3$ | $OCH_2CH_3$ | N | |
| $CH_3$ | H | O | H | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | $CH_3$ | cyclopropyl | $CH_3$ | $CH_3$ | CH | |
| $CH_3$ | H | O | $CH_3$ | cyclopropyl | $CH_3$ | $OCH_3$ | CH | |
| $CH_3$ | H | O | $CH_3$ | cyclopropyl | $OCH_3$ | $OCH_3$ | CH | |
| $CH_3$ | H | O | $CH_3$ | cyclopropyl | $CH_3$ | $CH_3$ | N | |
| $CH_3$ | H | O | $CH_3$ | cyclopropyl | $CH_3$ | $OCH_3$ | N | |
| $CH_3$ | H | O | $CH_3$ | cyclopropyl | $OCH_3$ | $OCH_3$ | N | |
| $CH_3$ | H | O | $CH_3$ | cyclopropyl | Cl | $OCH_3$ | CH | |
| $CH_3$ | H | O | $CH_3$ | cyclobutyl | $CH_3$ | $CH_3$ | CH | |
| $CH_3$ | H | O | $CH_3$ | cyclobutyl | $CH_3$ | $OCH_3$ | CH | |
| $CH_3$ | H | O | $CH_3$ | cyclobutyl | $OCH_3$ | $OCH_3$ | CH | |
| $CH_3$ | H | O | $CH_3$ | cyclobutyl | $CH_3$ | $CH_3$ | N | |
| $CH_3$ | H | O | $CH_3$ | cyclobutyl | $CH_3$ | $OCH_3$ | N | |
| $CH_3$ | H | O | $CH_3$ | cyclobutyl | $OCH_3$ | $OCH_3$ | N | |
| $CH_3$ | H | O | $CH_3$ | cyclobutyl | Cl | $OCH_3$ | CH | |
| $CH_3$ | H | O | $CH_3$ | cyclopentyl | $CH_3$ | $CH_3$ | CH | |
| $CH_3$ | H | O | $CH_3$ | cyclopentyl | $CH_3$ | $OCH_3$ | CH | |
| $CH_3$ | H | O | $CH_3$ | cyclopentyl | $OCH_3$ | $OCH_3$ | CH | |
| $CH_3$ | H | O | $CH_3$ | cyclopentyl | $CH_3$ | $CH_3$ | N | |
| $CH_3$ | H | O | $CH_3$ | cyclopentyl | $CH_3$ | $OCH_3$ | N | |
| $CH_3$ | H | O | $CH_3$ | cyclopentyl | $OCH_3$ | $OCH_3$ | N | |
| $CH_3$ | H | O | $CH_3$ | cyclopentyl | Cl | $OCH_3$ | CH | |
| $CH_3$ | H | O | $CH_3$ | cyclopropyl | $OCH_3$ | $OCH_2CH_3$ | CH | |
| $CH_3$ | H | O | $CH_3$ | cyclopropyl | cyclopropyl | $OCH_3$ | CH | |
| $CH_3$ | H | O | $CH_3$ | cyclopropyl | $OCH_3$ | $CH(OCH_3)_2$ | CH | |
| $CH_3$ | H | O | $CH_3$ | cyclopropyl | $NHCH_3$ | $OCH_2CH_3$ | N | |
| $CH_3$ | H | O | $CH_3$ | cyclopropyl | $NHCH_3$ | $OCH_3$ | N | |
| $CH_3$ | H | O | $CH_3$ | cyclopropyl | $OCH_3$ | $OCH_2CH_3$ | N | |
| $CH_3$ | H | O | $CH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | Cl | cyclopropyl | $CH_3$ | $CH_3$ | CH | 167-169 |
| $CH_3$ | H | O | Cl | cyclopropyl | $CH_3$ | $OCH_3$ | CH | 178-180 |
| $CH_3$ | H | O | Cl | cyclopropyl | $OCH_3$ | $OCH_3$ | CH | 193-195 |
| $CH_3$ | H | O | Cl | cyclopropyl | $CH_3$ | $CH_3$ | N | |
| $CH_3$ | H | O | Cl | cyclopropyl | $CH_3$ | $OCH_3$ | N | |
| $CH_3$ | H | O | Cl | cyclopropyl | $OCH_3$ | $OCH_3$ | N | |
| $CH_3$ | H | O | Cl | cyclopropyl | Cl | $OCH_3$ | CH | |
| $CH_3$ | H | O | Cl | cyclobutyl | $CH_3$ | $CH_3$ | CH | |
| $CH_3$ | H | O | Cl | cyclobutyl | $CH_3$ | $OCH_3$ | CH | |
| $CH_3$ | H | O | Cl | cyclobutyl | $OCH_3$ | $OCH_3$ | CH | |
| $CH_3$ | H | O | Cl | cyclobutyl | $CH_3$ | $CH_3$ | N | |
| $CH_3$ | H | O | Cl | cyclobutyl | $CH_3$ | $OCH_3$ | N | |
| $CH_3$ | H | O | Cl | cyclobutyl | $OCH_3$ | $OCH_3$ | N | |
| $CH_3$ | H | O | Cl | cyclobutyl | Cl | $OCH_3$ | CH | |
| $CH_3$ | H | O | Cl | cyclopentyl | $CH_3$ | $CH_3$ | CH | |
| $CH_3$ | H | O | Cl | cyclopentyl | $CH_3$ | $OCH_3$ | CH | |
| $CH_3$ | H | O | Cl | cyclopentyl | $OCH_3$ | $OCH_3$ | CH | |
| $CH_3$ | H | O | Cl | cyclopentyl | $CH_3$ | $CH_3$ | N | |
| $CH_3$ | H | O | Cl | cyclopentyl | $CH_3$ | $OCH_3$ | N | |
| $CH_3$ | H | O | Cl | cyclopentyl | $OCH_3$ | $OCH_3$ | N | |
| $CH_3$ | H | O | Cl | cyclopentyl | Cl | $OCH_3$ | CH | |
| $CH_3$ | H | O | Cl | cyclopropyl | $OCH_3$ | $OCH_2CH_3$ | CH | |
| $CH_3$ | H | O | Cl | cyclopropyl | cyclopropyl | $OCH_3$ | CH | |
| $CH_3$ | H | O | Cl | cyclopropyl | $OCH_3$ | $CH(OCH_3)_2$ | CH | |
| $CH_3$ | H | O | Cl | cyclopropyl | $NHCH_3$ | $OCH_2CH_3$ | N | |
| $CH_3$ | H | O | Cl | cyclopropyl | $NHCH_3$ | $OCH_3$ | N | |
| $CH_3$ | H | O | Cl | cyclopropyl | $OCH_3$ | $OCH_2CH_3$ | N | |
| $CH_3$ | H | O | Cl | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | Br | cyclopropyl | $CH_3$ | $CH_3$ | CH | |
| $CH_3$ | H | O | Br | cyclopropyl | $CH_3$ | $OCH_3$ | CH | |
| $CH_3$ | H | O | Br | cyclopropyl | $OCH_3$ | $OCH_3$ | CH | |
| $CH_3$ | H | O | Br | cyclopropyl | $CH_3$ | $CH_3$ | N | |
| $CH_3$ | H | O | Br | cyclopropyl | $CH_3$ | $OCH_3$ | N | |
| $CH_3$ | H | O | Br | cyclopropyl | $OCH_3$ | $OCH_3$ | N | |
| $CH_3$ | H | O | Br | cyclopropyl | Cl | $OCH_3$ | CH | |
| $CH_3$ | H | O | Br | cyclobutyl | $CH_3$ | $CH_3$ | CH | |
| $CH_3$ | H | O | Br | cyclobutyl | $CH_3$ | $OCH_3$ | CH | |
| $CH_3$ | H | O | Br | cyclobutyl | $OCH_3$ | $OCH_3$ | CH | |
| $CH_3$ | H | O | Br | cyclobutyl | $CH_3$ | $CH_3$ | N | |
| $CH_3$ | H | O | Br | cyclobutyl | $CH_3$ | $OCH_3$ | N | |
| $CH_3$ | H | O | Br | cyclobutyl | $OCH_3$ | $OCH_3$ | N | |
| $CH_3$ | H | O | Br | cyclobutyl | Cl | $OCH_3$ | CH | |
| $CH_3$ | H | O | Br | cyclopentyl | $CH_3$ | $CH_3$ | CH | |
| $CH_3$ | H | O | Br | cyclopentyl | $CH_3$ | $OCH_3$ | CH | |
| $CH_3$ | H | O | Br | cyclopentyl | $OCH_3$ | $OCH_3$ | CH | |
| $CH_3$ | H | O | Br | cyclopentyl | $CH_3$ | $CH_3$ | N | |
| $CH_3$ | H | O | Br | cyclopentyl | $CH_3$ | $OCH_3$ | N | |
| $CH_3$ | H | O | Br | cyclopentyl | $OCH_3$ | $OCH_3$ | N | |
| $CH_3$ | H | O | Br | cyclopentyl | Cl | $OCH_3$ | CH | |
| $CH_3$ | H | O | Br | cyclopropyl | $OCH_3$ | $OCH_2CH_3$ | CH | |
| $CH_3$ | H | O | Br | cyclopropyl | cyclopropyl | $OCH_3$ | CH | |
| $CH_3$ | H | O | Br | cyclopropyl | $OCH_3$ | $CH(OCH_3)_2$ | CH | |
| $CH_3$ | H | O | Br | cyclopropyl | $NHCH_3$ | $OCH_2CH_3$ | N | |
| $CH_3$ | H | O | Br | cyclopropyl | $NHCH_3$ | $OCH_3$ | N | |
| $CH_3$ | H | O | Br | cyclopropyl | $OCH_3$ | $OCH_2CH_3$ | N | |
| $CH_3$ | H | O | Br | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | $CH_2F$ | cyclopropyl | $CH_3$ | $CH_3$ | CH | |
| $CH_3$ | H | O | $CH_2F$ | cyclopropyl | $CH_3$ | $OCH_3$ | CH | |
| $CH_3$ | H | O | $CH_2F$ | cyclopropyl | $OCH_3$ | $OCH_3$ | CH | |
| $CH_3$ | H | O | $CH_2F$ | cyclopropyl | $CH_3$ | $CH_3$ | N | |
| $CH_3$ | H | O | $CH_2F$ | cyclopropyl | $CH_3$ | $OCH_3$ | N | |
| $CH_3$ | H | O | $CH_2F$ | cyclopropyl | $OCH_3$ | $OCH_3$ | N | |
| $CH_3$ | H | O | $CH_2F$ | cyclopropyl | Cl | $OCH_3$ | CH | |
| $CH_3$ | H | O | $CH_2F$ | cyclobutyl | $CH_3$ | $CH_3$ | CH | |
| $CH_3$ | H | O | $CH_2F$ | cyclobutyl | $CH_3$ | $OCH_3$ | CH | |
| $CH_3$ | H | O | $CH_2F$ | cyclobutyl | $OCH_3$ | $OCH_3$ | CH | |
| $CH_3$ | H | O | $CH_2F$ | cyclobutyl | $CH_3$ | $CH_3$ | N | |
| $CH_3$ | H | O | $CH_2F$ | cyclobutyl | $CH_3$ | $OCH_3$ | N | |
| $CH_3$ | H | O | $CH_2F$ | cyclobutyl | $OCH_3$ | $OCH_3$ | N | |
| $CH_3$ | H | O | $CH_2F$ | cyclobutyl | Cl | $OCH_3$ | CH | |
| $CH_3$ | H | O | $CH_2F$ | cyclopentyl | $CH_3$ | $CH_3$ | CH | |
| $CH_3$ | H | O | $CH_2F$ | cyclopentyl | $CH_3$ | $OCH_3$ | CH | |
| $CH_3$ | H | O | $CH_2F$ | cyclopentyl | $OCH_3$ | $OCH_3$ | CH | |
| $CH_3$ | H | O | $CH_2F$ | cyclopentyl | $CH_3$ | $CH_3$ | N | |
| $CH_3$ | H | O | $CH_2F$ | cyclopentyl | $CH_3$ | $OCH_3$ | N | |
| $CH_3$ | H | O | $CH_2F$ | cyclopentyl | $OCH_3$ | $OCH_3$ | N | |
| $CH_3$ | H | O | $CH_2F$ | cyclopentyl | Cl | $OCH_3$ | CH | |
| $CH_3$ | H | O | $CH_2F$ | cyclopropyl | $OCH_3$ | $OCH_2CH_3$ | CH | |
| $CH_3$ | H | O | $CH_2F$ | cyclopropyl | cyclopropyl | $OCH_3$ | CH | |
| $CH_3$ | H | O | $CH_2F$ | cyclopropyl | $OCH_3$ | $CH(OCH_3)_2$ | CH | |
| $CH_3$ | H | O | $CH_2F$ | cyclopropyl | $NHCH_3$ | $OCH_2CH_3$ | N | |
| $CH_3$ | H | O | $CH_2F$ | cyclopropyl | $NHCH_3$ | $OCH_3$ | N | |
| $CH_3$ | H | O | $CH_2F$ | cyclopropyl | $OCH_3$ | $OCH_2CH_3$ | N | |
| $CH_3$ | H | O | $CH_2F$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | $OCH_3$ | cyclopropyl | $CH_3$ | $CH_3$ | CH | |
| $CH_3$ | H | O | $OCH_3$ | cyclopropyl | $CH_3$ | $OCH_3$ | CH | |
| $CH_3$ | H | O | $OCH_3$ | cyclopropyl | $OCH_3$ | $OCH_3$ | CH | |
| $CH_3$ | H | O | $OCH_3$ | cyclopropyl | $CH_3$ | $CH_3$ | N | |
| $CH_3$ | H | O | $OCH_3$ | cyclopropyl | $CH_3$ | $OCH_3$ | N | |
| $CH_3$ | H | O | $OCH_3$ | cyclopropyl | $OCH_3$ | $OCH_3$ | N | |
| $CH_3$ | H | O | $OCH_3$ | cyclopropyl | Cl | $OCH_3$ | CH | |
| $CH_3$ | H | O | $OCH_3$ | cyclobutyl | $CH_3$ | $CH_3$ | CH | |
| $CH_3$ | H | O | $OCH_3$ | cyclobutyl | $CH_3$ | $OCH_3$ | CH | |
| $CH_3$ | H | O | $OCH_3$ | cyclobutyl | $OCH_3$ | $OCH_3$ | CH | |
| $CH_3$ | H | O | $OCH_3$ | cyclobutyl | $CH_3$ | $CH_3$ | N | |
| $CH_3$ | H | O | $OCH_3$ | cyclobutyl | $CH_3$ | $OCH_3$ | N | |
| $CH_3$ | H | O | $OCH_3$ | cyclobutyl | $OCH_3$ | $OCH_3$ | N | |
| $CH_3$ | H | O | $OCH_3$ | cyclobutyl | Cl | $OCH_3$ | CH | |
| $CH_3$ | H | O | $OCH_3$ | cyclopentyl | $CH_3$ | $CH_3$ | CH | |
| $CH_3$ | H | O | $OCH_3$ | cyclopentyl | $CH_3$ | $OCH_3$ | CH | |
| $CH_3$ | H | O | $OCH_3$ | cyclopentyl | $OCH_3$ | $OCH_3$ | CH | |
| $CH_3$ | H | O | $OCH_3$ | cyclopentyl | $CH_3$ | $CH_3$ | N | |
| $CH_3$ | H | O | $OCH_3$ | cyclopentyl | $CH_3$ | $OCH_3$ | N | |
| $CH_3$ | H | O | $OCH_3$ | cyclopentyl | $OCH_3$ | $OCH_3$ | N | |
| $CH_3$ | H | O | $OCH_3$ | cyclopentyl | Cl | $OCH_3$ | CH | |
| $CH_3$ | H | O | $OCH_3$ | cyclopropyl | $OCH_3$ | $OCH_2CH_3$ | CH | |
| $CH_3$ | H | O | $OCH_3$ | cyclopropyl | cyclopropyl | $OCH_3$ | CH | |
| $CH_3$ | H | O | $OCH_3$ | cyclopropyl | $OCH_3$ | $CH(OCH_3)_2$ | CH | |
| $CH_3$ | H | O | $OCH_3$ | cyclopropyl | $NHCH_3$ | $OCH_2CH_3$ | N | |
| $CH_3$ | H | O | $OCH_3$ | cyclopropyl | $NHCH_3$ | $OCH_3$ | N | |
| $CH_3$ | H | O | $OCH_3$ | cyclopropyl | $OCH_3$ | $OCH_2CH_3$ | N | |
| $CH_3$ | H | O | $OCH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | $SO_2N(CH_3)_2$ | cyclopropyl | $CH_3$ | $CH_3$ | CH | |
| $CH_3$ | H | O | $SO_2N(CH_3)_2$ | cyclopropyl | $CH_3$ | $OCH_3$ | CH | |
| $CH_3$ | H | O | $SO_2N(CH_3)_2$ | cyclopropyl | $OCH_3$ | $OCH_3$ | CH | |
| $CH_3$ | H | O | $SO_2N(CH_3)_2$ | cyclopropyl | $CH_3$ | $CH_3$ | N | |
| $CH_3$ | H | O | $SO_2N(CH_3)_2$ | cyclopropyl | $CH_3$ | $OCH_3$ | N | |
| $CH_3$ | H | O | $SO_2N(CH_3)_2$ | cyclopropyl | $OCH_3$ | $OCH_3$ | N | |
| $CH_3$ | H | O | $SO_2N(CH_3)_2$ | cyclopropyl | Cl | $OCH_3$ | CH | |
| $CH_3$ | H | O | $SO_2N(CH_3)_2$ | cyclobutyl | $CH_3$ | $CH_3$ | CH | |
| $CH_3$ | H | O | $SO_2N(CH_3)_2$ | cyclobutyl | $CH_3$ | $OCH_3$ | CH | |
| $CH_3$ | H | O | $SO_2N(CH_3)_2$ | cyclobutyl | $OCH_3$ | $OCH_3$ | CH | |
| $CH_3$ | H | O | $SO_2N(CH_3)_2$ | cyclobutyl | $CH_3$ | $CH_3$ | N | |
| $CH_3$ | H | O | $SO_2N(CH_3)_2$ | cyclobutyl | $CH_3$ | $OCH_3$ | N | |
| $CH_3$ | H | O | $SO_2N(CH_3)_2$ | cyclobutyl | $OCH_3$ | $OCH_3$ | N | |
| $CH_3$ | H | O | $SO_2N(CH_3)_2$ | cyclobutyl | Cl | $OCH_3$ | CH | |
| $CH_3$ | H | O | $SO_2N(CH_3)_2$ | cyclopentyl | $CH_3$ | $CH_3$ | CH | |
| $CH_3$ | H | O | $SO_2N(CH_3)_2$ | cyclopentyl | $CH_3$ | $OCH_3$ | CH | |
| $CH_3$ | H | O | $SO_2N(CH_3)_2$ | cyclopentyl | $OCH_3$ | $OCH_3$ | CH | |
| $CH_3$ | H | O | $SO_2N(CH_3)_2$ | cyclopentyl | $CH_3$ | $CH_3$ | N | |
| $CH_3$ | H | O | $SO_2N(CH_3)_2$ | cyclopentyl | $CH_3$ | $OCH_3$ | N | |
| $CH_3$ | H | O | $SO_2N(CH_3)_2$ | cyclopentyl | $OCH_3$ | $OCH_3$ | N | |
| $CH_3$ | H | O | $SO_2N(CH_3)_2$ | cyclopentyl | Cl | $OCH_3$ | CH | |
| $CH_3$ | H | O | $SO_2N(CH_3)_2$ | cyclopropyl | $OCH_3$ | $OCH_2CH_3$ | CH | |
| $CH_3$ | H | O | $SO_2N(CH_3)_2$ | cyclopropyl | cyclopropyl | $OCH_3$ | CH | |
| $CH_3$ | H | O | $SO_2N(CH_3)_2$ | cyclopropyl | $OCH_3$ | $CH(OCH_3)_2$ | CH | |
| $CH_3$ | H | O | $SO_2N(CH_3)_2$ | cyclopropyl | $NHCH_3$ | $OCH_2CH_3$ | N | |
| $CH_3$ | H | O | $SO_2N(CH_3)_2$ | cyclopropyl | $NHCH_3$ | $OCH_3$ | N | |
| $CH_3$ | H | O | $SO_2N(CH_3)_2$ | cyclopropyl | $OCH_3$ | $OCH_2CH_3$ | N | |
| $CH_3$ | H | O | $SO_2N(CH_3)_2$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | $SCH_3$ | cyclopropyl | $CH_3$ | $CH_3$ | CH | |
| $CH_3$ | H | O | $SCH_3$ | cyclopropyl | $CH_3$ | $OCH_3$ | CH | |
| $CH_3$ | H | O | $SCH_3$ | cyclopropyl | $OCH_3$ | $OCH_3$ | CH | |
| $CH_3$ | H | O | $SCH_3$ | cyclopropyl | $CH_3$ | $CH_3$ | N | |
| $CH_3$ | H | O | $SCH_3$ | cyclopropyl | $CH_3$ | $OCH_3$ | N | |
| $CH_3$ | H | O | $SCH_3$ | cyclopropyl | $OCH_3$ | $OCH_3$ | N | |
| $CH_3$ | H | O | $SCH_3$ | cyclopropyl | Cl | $OCH_3$ | CH | |
| $CH_3$ | H | O | $SCH_3$ | cyclobutyl | $CH_3$ | $CH_3$ | CH | |
| $CH_3$ | H | O | $SCH_3$ | cyclobutyl | $CH_3$ | $OCH_3$ | CH | |
| $CH_3$ | H | O | $SCH_3$ | cyclobutyl | $OCH_3$ | $OCH_3$ | CH | |
| $CH_3$ | H | O | $SCH_3$ | cyclobutyl | $CH_3$ | $CH_3$ | N | |
| $CH_3$ | H | O | $SCH_3$ | cyclobutyl | $CH_3$ | $OCH_3$ | N | |
| $CH_3$ | H | O | $SCH_3$ | cyclobutyl | $OCH_3$ | $OCH_3$ | N | |
| $CH_3$ | H | O | $SCH_3$ | cyclobutyl | Cl | $OCH_3$ | CH | |
| $CH_3$ | H | O | $SCH_3$ | cyclopentyl | $CH_3$ | $CH_3$ | CH | |
| $CH_3$ | H | O | $SCH_3$ | cyclopentyl | $CH_3$ | $OCH_3$ | CH | |
| $CH_3$ | H | O | $SCH_3$ | cyclopentyl | $OCH_3$ | $OCH_3$ | CH | |
| $CH_3$ | H | O | $SCH_3$ | cyclopentyl | $CH_3$ | $CH_3$ | N | |
| $CH_3$ | H | O | $SCH_3$ | cyclopentyl | $CH_3$ | $OCH_3$ | N | |
| $CH_3$ | H | O | $SCH_3$ | cyclopentyl | $OCH_3$ | $OCH_3$ | N | |
| $CH_3$ | H | O | $SCH_3$ | cyclopentyl | Cl | $OCH_3$ | CH | |
| $CH_3$ | H | O | $SCH_3$ | cyclopropyl | $OCH_3$ | $OCH_2CH_3$ | CH | |
| $CH_3$ | H | O | $SCH_3$ | cyclopropyl | cyclopropyl | $OCH_3$ | CH | |
| $CH_3$ | H | O | $SCH_3$ | cyclopropyl | $OCH_3$ | $CH(OCH_3)_2$ | CH | |
| $CH_3$ | H | O | $SCH_3$ | cyclopropyl | $NHCH_3$ | $OCH_2CH_3$ | N | |
| $CH_3$ | H | O | $SCH_3$ | cyclopropyl | $NHCH_3$ | $OCH_3$ | N | |
| $CH_3$ | H | O | $SCH_3$ | cyclopropyl | $OCH_3$ | $OCH_2CH_3$ | N | |
| $CH_3$ | H | O | $SCH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 0 | $SOCH_3$ | cyclopropyl | $CH_3$ | $CH_3$ | CH | |
| $CH_3$ | H | 0 | $SOCH_3$ | cyclopropyl | $CH_3$ | $OCH_3$ | CH | |
| $CH_3$ | H | 0 | $SOCH_3$ | cyclopropyl | $OCH_3$ | $OCH_3$ | CH | |
| $CH_3$ | H | 0 | $SOCH_3$ | cyclopropyl | $CH_3$ | $CH_3$ | N | |
| $CH_3$ | H | 0 | $SOCH_3$ | cyclopropyl | $CH_3$ | $OCH_3$ | N | |
| $CH_3$ | H | 0 | $SOCH_3$ | cyclopropyl | $OCH_3$ | $OCH_3$ | N | |
| $CH_3$ | H | 0 | $SOCH_3$ | cyclopropyl | Cl | $OCH_3$ | CH | |
| $CH_3$ | H | 0 | $SOCH_3$ | cyclobutyl | $CH_3$ | $CH_3$ | CH | |
| $CH_3$ | H | 0 | $SOCH_3$ | cyclobutyl | $CH_3$ | $OCH_3$ | CH | |
| $CH_3$ | H | 0 | $SOCH_3$ | cyclobutyl | $OCH_3$ | $OCH_3$ | CH | |
| $CH_3$ | H | 0 | $SOCH_3$ | cyclobutyl | $CH_3$ | $CH_3$ | N | |
| $CH_3$ | H | 0 | $SOCH_3$ | cyclobutyl | $CH_3$ | $OCH_3$ | N | |
| $CH_3$ | H | 0 | $SOCH_3$ | cyclobutyl | $OCH_3$ | $OCH_3$ | N | |
| $CH_3$ | H | 0 | $SOCH_3$ | cyclobutyl | Cl | $OCH_3$ | CH | |
| $CH_3$ | H | 0 | $SOCH_3$ | cyclopentyl | $CH_3$ | $CH_3$ | CH | |
| $CH_3$ | H | 0 | $SOCH_3$ | cyclopentyl | $CH_3$ | $OCH_3$ | CH | |
| $CH_3$ | H | 0 | $SOCH_3$ | cyclopentyl | $OCH_3$ | $OCH_3$ | CH | |
| $CH_3$ | H | 0 | $SOCH_3$ | cyclopentyl | $CH_3$ | $CH_3$ | N | |
| $CH_3$ | H | 0 | $SOCH_3$ | cyclopentyl | $CH_3$ | $OCH_3$ | N | |
| $CH_3$ | H | 0 | $SOCH_3$ | cyclopentyl | $OCH_3$ | $OCH_3$ | N | |
| $CH_3$ | H | 0 | $SOCH_3$ | cyclopentyl | Cl | $OCH_3$ | CH | |
| $CH_3$ | H | 0 | $SOCH_3$ | cyclopropyl | $OCH_3$ | $OCH_2CH_3$ | CH | |
| $CH_3$ | H | 0 | $SOCH_3$ | cyclopropyl | cyclopropyl | $OCH_3$ | CH | |
| $CH_3$ | H | 0 | $SOCH_3$ | cyclopropyl | $OCH_3$ | $CH(OCH_3)_2$ | CH | |
| $CH_3$ | H | 0 | $SOCH_3$ | cyclopropyl | $NHCH_3$ | $OCH_2CH_3$ | N | |
| $CH_3$ | H | 0 | $SOCH_3$ | cyclopropyl | $NHCH_3$ | $OCH_3$ | N | |
| $CH_3$ | H | 0 | $SOCH_3$ | cyclopropyl | $OCH_3$ | $OCH_2CH_3$ | N | |
| $CH_3$ | H | 0 | $SOCH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | $SO_2CH_3$ | cyclopropyl | $CH_3$ | $CH_3$ | CH | |
| $CH_3$ | H | O | $SO_2CH_3$ | cyclopropyl | $CH_3$ | $OCH_3$ | CH | |
| $CH_3$ | H | O | $SO_2CH_3$ | cyclopropyl | $OCH_3$ | $OCH_3$ | CH | |
| $CH_3$ | H | O | $SO_2CH_3$ | cyclopropyl | $CH_3$ | $CH_3$ | N | |
| $CH_3$ | H | O | $SO_2CH_3$ | cyclopropyl | $CH_3$ | $OCH_3$ | N | |
| $CH_3$ | H | O | $SO_2CH_3$ | cyclopropyl | $OCH_3$ | $OCH_3$ | N | |
| $CH_3$ | H | O | $SO_2CH_3$ | cyclopropyl | Cl | $OCH_3$ | CH | |
| $CH_3$ | H | O | $SO_2CH_3$ | cyclobutyl | $CH_3$ | $CH_3$ | CH | |
| $CH_3$ | H | O | $SO_2CH_3$ | cyclobutyl | $CH_3$ | $OCH_3$ | CH | |
| $CH_3$ | H | O | $SO_2CH_3$ | cyclobutyl | $OCH_3$ | $OCH_3$ | CH | |
| $CH_3$ | H | O | $SO_2CH_3$ | cyclobutyl | $CH_3$ | $CH_3$ | N | |
| $CH_3$ | H | O | $SO_2CH_3$ | cyclobutyl | $CH_3$ | $OCH_3$ | N | |
| $CH_3$ | H | O | $SO_2CH_3$ | cyclobutyl | $OCH_3$ | $OCH_3$ | N | |
| $CH_3$ | H | O | $SO_2CH_3$ | cyclobutyl | Cl | $OCH_3$ | CH | |
| $CH_3$ | H | O | $SO_2CH_3$ | cyclopentyl | $CH_3$ | $CH_3$ | CH | |
| $CH_3$ | H | O | $SO_2CH_3$ | cyclopentyl | $CH_3$ | $OCH_3$ | CH | |
| $CH_3$ | H | O | $SO_2CH_3$ | cyclopentyl | $OCH_3$ | $OCH_3$ | CH | |
| $CH_3$ | H | O | $SO_2CH_3$ | cyclopentyl | $CH_3$ | $CH_3$ | N | |
| $CH_3$ | H | O | $SO_2CH_3$ | cyclopentyl | $CH_3$ | $OCH_3$ | N | |
| $CH_3$ | H | O | $SO_2CH_3$ | cyclopentyl | $OCH_3$ | $OCH_3$ | N | |
| $CH_3$ | H | O | $SO_2CH_3$ | cyclopentyl | Cl | $OCH_3$ | CH | |
| $CH_3$ | H | O | $SO_2CH_3$ | cyclopropyl | $OCH_3$ | $OCH_2CH_3$ | CH | |
| $CH_3$ | H | O | $SO_2CH_3$ | cyclopropyl | cyclopropyl | $OCH_3$ | CH | |
| $CH_3$ | H | O | $SO_2CH_3$ | cyclopropyl | $OCH_3$ | $CH(OCH_3)_2$ | CH | |
| $CH_3$ | H | O | $SO_2CH_3$ | cyclopropyl | $NHCH_3$ | $OCH_2CH_3$ | N | |
| $CH_3$ | H | O | $SO_2CH_3$ | cyclopropyl | $NHCH_3$ | $OCH_3$ | N | |
| $CH_3$ | H | O | $SO_2CH_3$ | cyclopropyl | $OCH_3$ | $OCH_2CH_3$ | N | |
| $CH_3$ | H | O | $SO_2CH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | CN | cyclopropyl | $CH_3$ | $CH_3$ | CH | |
| $CH_3$ | H | O | CN | cyclopropyl | $CH_3$ | $OCH_3$ | CH | |
| $CH_3$ | H | O | CN | cyclopropyl | $OCH_3$ | $OCH_3$ | CH | |
| $CH_3$ | H | O | CN | cyclopropyl | $CH_3$ | $CH_3$ | N | |
| $CH_3$ | H | O | CN | cyclopropyl | $CH_3$ | $OCH_3$ | N | |
| $CH_3$ | H | O | CN | cyclopropyl | $OCH_3$ | $OCH_3$ | N | |
| $CH_3$ | H | O | CN | cyclopropyl | Cl | $OCH_3$ | CH | |
| $CH_3$ | H | O | CN | cyclobutyl | $CH_3$ | $CH_3$ | CH | |
| $CH_3$ | H | O | CN | cyclobutyl | $CH_3$ | $OCH_3$ | CH | |
| $CH_3$ | H | O | CN | cyclobutyl | $OCH_3$ | $OCH_3$ | CH | |
| $CH_3$ | H | O | CN | cyclobutyl | $CH_3$ | $CH_3$ | N | |
| $CH_3$ | H | O | CN | cyclobutyl | $CH_3$ | $OCH_3$ | N | |
| $CH_3$ | H | O | CN | cyclobutyl | $OCH_3$ | $OCH_3$ | N | |
| $CH_3$ | H | O | CN | cyclobutyl | Cl | $OCH_3$ | CH | |
| $CH_3$ | H | O | CN | cyclopentyl | $CH_3$ | $CH_3$ | CH | |
| $CH_3$ | H | O | CN | cyclopentyl | $CH_3$ | $OCH_3$ | CH | |
| $CH_3$ | H | O | CN | cyclopentyl | $OCH_3$ | $OCH_3$ | CH | |
| $CH_3$ | H | O | CN | cyclopentyl | $CH_3$ | $CH_3$ | N | |
| $CH_3$ | H | O | CN | cyclopentyl | $CH_3$ | $OCH_3$ | N | |
| $CH_3$ | H | O | CN | cyclopentyl | $OCH_3$ | $OCH_3$ | N | |
| $CH_3$ | H | O | CN | cyclopentyl | Cl | $OCH_3$ | CH | |
| $CH_3$ | H | O | CN | cyclopropyl | $OCH_3$ | $OCH_2CH_3$ | CH | |
| $CH_3$ | H | O | CN | cyclopropyl | cyclopropyl | $OCH_3$ | CH | |
| $CH_3$ | H | O | CN | cyclopropyl | $OCH_3$ | $CH(OCH_3)_2$ | CH | |
| $CH_3$ | H | O | CN | cyclopropyl | $NHCH_3$ | $OCH_2CH_3$ | N | |
| $CH_3$ | H | O | CN | cyclopropyl | $NHCH_3$ | $OCH_3$ | N | |
| $CH_3$ | H | O | CN | cyclopropyl | $OCH_3$ | $OCH_2CH_3$ | N | |
| $CH_3$ | H | O | CN | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 0 | $CO_2CH_3$ | cyclopropyl | $CH_3$ | $CH_3$ | CH | |
| $CH_3$ | H | 0 | $CO_2CH_3$ | cyclopropyl | $CH_3$ | $OCH_3$ | CH | |
| $CH_3$ | H | 0 | $CO_2CH_3$ | cyclopropyl | $OCH_3$ | $OCH_3$ | CH | |
| $CH_3$ | H | 0 | $CO_2CH_3$ | cyclopropyl | $CH_3$ | $CH_3$ | N | |
| $CH_3$ | H | 0 | $CO_2CH_3$ | cyclopropyl | $CH_3$ | $OCH_3$ | N | |
| $CH_3$ | H | 0 | $CO_2CH_3$ | cyclopropyl | $OCH_3$ | $OCH_3$ | N | |
| $CH_3$ | H | 0 | $CO_2CH_3$ | cyclopropyl | Cl | $OCH_3$ | CH | |
| $CH_3$ | H | 0 | $CO_2CH_3$ | cyclobutyl | $CH_3$ | $CH_3$ | CH | |
| $CH_3$ | H | 0 | $CO_2CH_3$ | cyclobutyl | $CH_3$ | $OCH_3$ | CH | |
| $CH_3$ | H | 0 | $CO_2CH_3$ | cyclobutyl | $OCH_3$ | $OCH_3$ | CH | |
| $CH_3$ | H | 0 | $CO_2CH_3$ | cyclobutyl | $CH_3$ | $CH_3$ | N | |
| $CH_3$ | H | 0 | $CO_2CH_3$ | cyclobutyl | $CH_3$ | $OCH_3$ | N | |
| $CH_3$ | H | 0 | $CO_2CH_3$ | cyclobutyl | $OCH_3$ | $OCH_3$ | N | |
| $CH_3$ | H | 0 | $CO_2CH_3$ | cyclobutyl | Cl | $OCH_3$ | CH | |
| $CH_3$ | H | 0 | $CO_2CH_3$ | cyclopentyl | $CH_3$ | $CH_3$ | CH | |
| $CH_3$ | H | 0 | $CO_2CH_3$ | cyclopentyl | $CH_3$ | $OCH_3$ | CH | |
| $CH_3$ | H | 0 | $CO_2CH_3$ | cyclopentyl | $OCH_3$ | $OCH_3$ | CH | |
| $CH_3$ | H | 0 | $CO_2CH_3$ | cyclopentyl | $CH_3$ | $CH_3$ | N | |
| $CH_3$ | H | 0 | $CO_2CH_3$ | cyclopentyl | $CH_3$ | $OCH_3$ | N | |
| $CH_3$ | H | 0 | $CO_2CH_3$ | cyclopentyl | $OCH_3$ | $OCH_3$ | N | |
| $CH_3$ | H | 0 | $CO_2CH_3$ | cyclopentyl | Cl | $OCH_3$ | CH | |
| $CH_3$ | H | 0 | $CO_2CH_3$ | cyclopropyl | $OCH_3$ | $OCH_2CH_3$ | CH | |
| $CH_3$ | H | 0 | $CO_2CH_3$ | cyclopropyl | cyclopropyl | $OCH_3$ | CH | |
| $CH_3$ | H | 0 | $CO_2CH_3$ | cyclopropyl | $OCH_3$ | $CH(OCH_3)_2$ | CH | |
| $CH_3$ | H | 0 | $CO_2CH_3$ | cyclopropyl | $NHCH_3$ | $OCH_2CH_3$ | N | |
| $CH_3$ | H | 0 | $CO_2CH_3$ | cyclopropyl | $NHCH_3$ | $OCH_3$ | N | |
| $CH_3$ | H | 0 | $CO_2CH_3$ | cyclopropyl | $OCH_3$ | $OCH_2CH_3$ | N | |
| $CH_3$ | H | 0 | $CO_2CH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | $N(CH_3)_2$ | cyclopropyl | $CH_3$ | $CH_3$ | CH | |
| $CH_3$ | H | O | $N(CH_3)_2$ | cyclopropyl | $CH_3$ | $OCH_3$ | CH | |
| $CH_3$ | H | O | $N(CH_3)_2$ | cyclopropyl | $OCH_3$ | $OCH_3$ | CH | |
| $CH_3$ | H | O | $N(CH_3)_2$ | cyclopropyl | $CH_3$ | $CH_3$ | N | |
| $CH_3$ | H | O | $N(CH_3)_2$ | cyclopropyl | $CH_3$ | OCH | N | |
| $CH_3$ | H | O | $N(CH_3)_2$ | cyclopropyl | $OCH_3$ | $OCH_3$ | N | |
| $CH_3$ | H | O | $N(CH_3)_2$ | cyclopropyl | Cl | $OCH_3$ | CH | |
| $CH_3$ | H | O | $N(CH_3)_2$ | cyclobutyl | $CH_3$ | $CH_3$ | CH | |
| $CH_3$ | H | O | $N(CH_3)_2$ | cyclobutyl | $CH_3$ | $OCH_3$ | CH | |
| $CH_3$ | H | O | $N(CH_3)_2$ | cyclobutyl | $OCH_3$ | $OCH_3$ | CH | |
| $CH_3$ | H | O | $N(CH_3)_2$ | cyclobutyl | $CH_3$ | $CH_3$ | N | |
| $CH_3$ | H | O | $N(CH_3)_2$ | cyclobutyl | $CH_3$ | $OCH_3$ | N | |
| $CH_3$ | H | O | $N(CH_3)_2$ | cyclobutyl | $OCH_3$ | $OCH_3$ | N | |
| $CH_3$ | H | O | $N(CH_3)_2$ | cyclobutyl | Cl | $OCH_3$ | CH | |
| $CH_3$ | H | O | $N(CH_3)_2$ | cyclopentyl | $CH_3$ | $CH_3$ | CH | |
| $CH_3$ | H | O | $N(CH_3)_2$ | cyclopentyl | $CH_3$ | $OCH_3$ | CH | |
| $CH_3$ | H | O | $N(CH_3)_2$ | cyclopentyl | $OCH_3$ | $OCH_3$ | CH | |
| $CH_3$ | H | O | $N(CH_3)_2$ | cyclopentyl | $CH_3$ | $CH_3$ | N | |
| $CH_3$ | H | O | $N(CH_3)_2$ | cyclopentyl | $CH_3$ | $OCH_3$ | N | |
| $CH_3$ | H | O | $N(CH_3)_2$ | cyclopentyl | $OCH_3$ | $OCH_3$ | N | |
| $CH_3$ | H | O | $N(CH_3)_2$ | cyclopentyl | Cl | $OCH_3$ | CH | |
| $CH_3$ | H | O | $N(CH_3)_2$ | cyclopropyl | $OCH_3$ | $OCH_2CH_3$ | CH | |
| $CH_3$ | H | O | $N(CH_3)_2$ | cyclopropyl | cyclopropyl | $OCH_3$ | CH | |
| $CH_3$ | H | O | $N(CH_3)_2$ | cyclopropyl | $OCH_3$ | $CH(OCH_3)_2$ | CH | |
| $CH_3$ | H | O | $N(CH_3)_2$ | cyclopropyl | $NHCH_3$ | $OCH_2CH_3$ | N | |
| $CH_3$ | H | O | $N(CH_3)_2$ | cyclopropyl | $NHCH_3$ | $OCH_3$ | N | |
| $CH_3$ | H | O | $N(CH_3)_2$ | cyclopropyl | $OCH_3$ | $OCH_2CH_3$ | N | |
| $CH_3$ | H | O | $N(CH_3)_2$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | $CH_2CN$ | cyclopropyl | $CH_3$ | $CH_3$ | CH | |
| $CH_3$ | H | O | $CH_2CN$ | cyclopropyl | $CH_3$ | $OCH_3$ | CH | |
| $CH_3$ | H | O | $CH_2CN$ | cyclopropyl | $OCH_3$ | $OCH_3$ | CH | |
| $CH_3$ | H | O | $CH_2CN$ | cyclopropyl | $CH_3$ | $CH_3$ | N | |
| $CH_3$ | H | O | $CH_2CN$ | cyclopropyl | $CH_3$ | $OCH_3$ | N | |
| $CH_3$ | H | O | $CH_2CN$ | cyclopropyl | $OCH_3$ | $OCH_3$ | N | |
| $CH_3$ | H | O | $CH_2CN$ | cyclopropyl | Cl | $OCH_3$ | CH | |
| $CH_3$ | H | O | $CH_2CN$ | cyclobutyl | $CH_3$ | $CH_3$ | CH | |
| $CH_3$ | H | O | $CH_2CN$ | cyclobutyl | $CH_3$ | $OCH_3$ | CH | |
| $CH_3$ | H | O | $CH_2CN$ | cyclobutyl | $OCH_3$ | $OCH_3$ | CH | |
| $CH_3$ | H | O | $CH_2CN$ | cyclobutyl | $CH_3$ | $CH_3$ | N | |
| $CH_3$ | H | O | $CH_2CN$ | cyclobutyl | $CH_3$ | $OCH_3$ | N | |
| $CH_3$ | H | O | $CH_2CN$ | cyclobutyl | $OCH_3$ | $OCH_3$ | N | |
| $CH_3$ | H | O | $CH_2CN$ | cyclobutyl | Cl | $OCH_3$ | CH | |
| $CH_3$ | H | O | $CH_2CN$ | cyclopentyl | $CH_3$ | $CH_3$ | CH | |
| $CH_3$ | H | O | $CH_2CN$ | cyclopentyl | $CH_3$ | $OCH_3$ | CH | |
| $CH_3$ | H | O | $CH_2CN$ | cyclopentyl | $OCH_3$ | $OCH_3$ | CH | |
| $CH_3$ | H | O | $CH_2CN$ | cyclopentyl | $CH_3$ | $CH_3$ | N | |
| $CH_3$ | H | O | $CH_2CN$ | cyclopentyl | $CH_3$ | $OCH_3$ | N | |
| $CH_3$ | H | O | $CH_2CN$ | cyclopentyl | $OCH_3$ | $OCH_3$ | N | |
| $CH_3$ | H | O | $CH_2CN$ | cyclopentyl | Cl | $OCH_3$ | CH | |
| $CH_3$ | H | O | $CH_2CN$ | cyclopropyl | $OCH_3$ | $OCH_2CH_3$ | CH | |
| $CH_3$ | H | O | $CH_2CN$ | cyclopropyl | cyclopropyl | $OCH_3$ | CH | |
| $CH_3$ | H | O | $CH_2CN$ | cyclopropyl | $OCH_3$ | $CH(OCH_3)_2$ | CH | |
| $CH_3$ | H | O | $CH_2CN$ | cyclopropyl | $NHCH_3$ | $OCH_2CH_3$ | N | |
| $CH_3$ | H | O | $CH_2CN$ | cyclopropyl | $NHCH_3$ | $OCH_3$ | N | |
| $CH_3$ | H | O | $CH_2CN$ | cyclopropyl | $OCH_3$ | $OCH_2CH_3$ | N | |
| $CH_3$ | H | O | $CH_2CN$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | $CH_2OCH_3$ | cyclopropyl | $CH_3$ | $CH_3$ | CH | |
| $CH_3$ | H | O | $CH_2OCH_3$ | cyclopropyl | $CH_3$ | $OCH_3$ | CH | |
| $CH_3$ | H | O | $CH_2OCH_3$ | cyclopropyl | $OCH_3$ | $OCH_3$ | CH | |
| $CH_3$ | H | O | $CH_2OCH_3$ | cyclopropyl | $CH_3$ | $CH_3$ | N | |
| $CH_3$ | H | O | $CH_2OCH_3$ | cyclopropyl | $CH_3$ | $OCH_3$ | N | |
| $CH_3$ | H | O | $CH_2OCH_3$ | cyclopropyl | $OCH_3$ | $OCH_3$ | N | |
| $CH_3$ | H | O | $CH_2OCH_3$ | cyclopropyl | Cl | $OCH_3$ | CH | |
| $CH_3$ | H | O | $CH_2OCH_3$ | cyclobutyl | $CH_3$ | $CH_3$ | CH | |
| $CH_3$ | H | O | $CH_2OCH_3$ | cyclobutyl | $CH_3$ | $OCH_3$ | CH | |
| $CH_3$ | H | O | $CH_2OCH_3$ | cyclobutyl | $OCH_3$ | $OCH_3$ | CH | |
| $CH_3$ | H | O | $CH_2OCH_3$ | cyclobutyl | $CH_3$ | $CH_3$ | N | |
| $CH_3$ | H | O | $CH_2OCH_3$ | cyclobutyl | $CH_3$ | $OCH_3$ | N | |
| $CH_3$ | H | O | $CH_2OCH_3$ | cyclobutyl | $OCH_3$ | $OCH_3$ | N | |
| $CH_3$ | H | O | $CH_2OCH_3$ | cyclobutyl | Cl | $OCH_3$ | CH | |
| $CH_3$ | H | O | $CH_2OCH_3$ | cyclopentyl | $CH_3$ | $CH_3$ | CH | |
| $CH_3$ | H | O | $CH_2OCH_3$ | cyclopentyl | $CH_3$ | $OCH_3$ | CH | |
| $CH_3$ | H | O | $CH_2OCH_3$ | cyclopentyl | $OCH_3$ | $OCH_3$ | CH | |
| $CH_3$ | H | O | $CH_2OCH_3$ | cyclopentyl | $CH_3$ | $CH_3$ | N | |
| $CH_3$ | H | O | $CH_2OCH_3$ | cyclopentyl | $CH_3$ | $OCH_3$ | N | |
| $CH_3$ | H | O | $CH_2OCH_3$ | cyclopentyl | $OCH_3$ | $OCH_3$ | N | |
| $CH_3$ | H | O | $CH_2OCH_3$ | cyclopentyl | Cl | $OCH_3$ | CH | |
| $CH_3$ | H | O | $CH_2OCH_3$ | cyclopropyl | $OCH_3$ | $OCH_2CH_3$ | CH | |
| $CH_3$ | H | O | $CH_2OCH_3$ | cyclopropyl | cyclopropyl | $OCH_3$ | CH | |
| $CH_3$ | H | O | $CH_2OCH_3$ | cyclopropyl | $OCH_3$ | $CH(OCH_3)_2$ | CH | |
| $CH_3$ | H | O | $CH_2OCH_3$ | cyclopropyl | $NHCH_3$ | $OCH_2CH_3$ | N | |
| $CH_3$ | H | O | $CH_2OCH_3$ | cyclopropyl | $NHCH_3$ | $OCH_3$ | N | |
| $CH_3$ | H | O | $CH_2OCH_3$ | cyclopropyl | $OCH_3$ | $OCH_2CH_3$ | N | |
| $CH_3$ | H | O | $CH_2OCH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | $CH(OH)CH_3$ | cyclopropyl | $CH_3$ | $CH_3$ | CH | |
| $CH_3$ | H | O | $CH(OH)CH_3$ | cyclopropyl | $CH_3$ | $OCH_3$ | CH | |
| $CH_3$ | H | O | $CH(OH)CH_3$ | cyclopropyl | $OCH_3$ | $OCH_3$ | CH | |
| $CH_3$ | H | O | $CH(OH)CH_3$ | cyclopropyl | $CH_3$ | $CH_3$ | N | |
| $CH_3$ | H | O | $CH(OH)CH_3$ | cyclopropyl | $CH_3$ | $OCH_3$ | N | |
| $CH_3$ | H | O | $CH(OH)CH_3$ | cyclopropyl | $OCH_3$ | $OCH_3$ | N | |
| $CH_3$ | H | O | $CH(OH)CH_3$ | cyclopropyl | Cl | $OCH_3$ | CH | |
| $CH_3$ | H | O | $CH(OH)CH_3$ | cyclobutyl | $CH_3$ | $CH_3$ | CH | |
| $CH_3$ | H | O | $CH(OH)CH_3$ | cyclobutyl | $CH_3$ | $OCH_3$ | CH | |
| $CH_3$ | H | O | $CH(OH)CH_3$ | cyclobutyl | $OCH_3$ | $OCH_3$ | CH | |
| $CH_3$ | H | O | $CH(OH)CH_3$ | cyclobutyl | $CH_3$ | $CH_3$ | N | |
| $CH_3$ | H | O | $CH(OH)CH_3$ | cyclobutyl | $CH_3$ | $OCH_3$ | N | |
| $CH_3$ | H | O | $CH(OH)CH_3$ | cyclobutyl | $OCH_3$ | $OCH_3$ | N | |
| $CH_3$ | H | O | $CH(OH)CH_3$ | cyclobutyl | Cl | $OCH_3$ | CH | |
| $CH_3$ | H | O | $CH(OH)CH_3$ | cyclopentyl | $CH_3$ | $CH_3$ | CH | |
| $CH_3$ | H | O | $CH(OH)CH_3$ | cyclopentyl | $CH_3$ | $OCH_3$ | CH | |
| $CH_3$ | H | O | $CH(OH)CH_3$ | cyclopentyl | $OCH_3$ | $OCH_3$ | CH | |
| $CH_3$ | H | O | $CH(OH)CH_3$ | cyclopentyl | $CH_3$ | $CH_3$ | N | |
| $CH_3$ | H | O | $CH(OH)CH_3$ | cyclopentyl | $CH_3$ | $OCH_3$ | N | |
| $CH_3$ | H | O | $CH(OH)CH_3$ | cyclopentyl | $OCH_3$ | $OCH_3$ | N | |
| $CH_3$ | H | O | $CH(OH)CH_3$ | cyclopentyl | Cl | $OCH_3$ | CH | |
| $CH_3$ | H | O | $CH(OH)CH_3$ | cyclopropyl | $OCH_3$ | $OCH_2CH_3$ | CH | |
| $CH_3$ | H | O | $CH(OH)CH_3$ | cyclopropyl | cyclopropyl | $OCH_3$ | CH | |
| $CH_3$ | H | O | $CH(OH)CH_3$ | cyclopropyl | $OCH_3$ | $CH(OCH_3)_2$ | CH | |
| $CH_3$ | H | O | $CH(OH)CH_3$ | cyclopropyl | $NHCH_3$ | $OCH_2CH_3$ | N | |
| $CH_3$ | H | O | $CH(OH)CH_3$ | cyclopropyl | $NHCH_3$ | $OCH_3$ | N | |
| $CH_3$ | H | O | $CH(OH)CH_3$ | cyclopropyl | $OCH_3$ | $OCH_2CH_3$ | N | |
| $CH_3$ | H | O | $CH(OH)CH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_2CH_3$ | H | O | H | cyclopropyl | $CH_3$ | $CH_3$ | CH | |
| $CH_2CH_3$ | H | O | H | cyclopropyl | $CH_3$ | $OCH_3$ | CH | |
| $CH_2CH_3$ | H | O | H | cyclopropyl | $OCH_3$ | $OCH_3$ | CH | |
| $CH_2CH_3$ | H | O | H | cyclopropyl | $CH_3$ | $CH_3$ | N | |
| $CH_2CH_3$ | H | O | H | cyclopropyl | $CH_3$ | $OCH_3$ | N | |
| $CH_2CH_3$ | H | O | H | cyclopropyl | $OCH_3$ | $OCH_3$ | N | |
| $CH_2CH_3$ | H | O | H | cyclopropyl | Cl | $OCH_3$ | CH | |
| $CH_2CH_3$ | H | O | H | cyclobutyl | $CH_3$ | $CH_3$ | CH | |
| $CH_2CH_3$ | H | O | H | cyclobutyl | $CH_3$ | $OCH_3$ | CH | |
| $CH_2CH_3$ | H | O | H | cyclobutyl | $OCH_3$ | $OCH_3$ | CH | |
| $CH_2CH_3$ | H | O | H | cyclobutyl | $CH_3$ | $CH_3$ | N | |
| $CH_2CH_3$ | H | O | H | cyclobutyl | $CH_3$ | $OCH_3$ | N | |
| $CH_2CH_3$ | H | O | H | cyclobutyl | $OCH_3$ | $OCH_3$ | N | |
| $CH_2CH_3$ | H | O | H | cyclobutyl | Cl | $OCH_3$ | CH | |
| $CH_2CH_3$ | H | O | H | cyclopentyl | $CH_3$ | $CH_3$ | CH | |
| $CH_2CH_3$ | H | O | H | cyclopentyl | $CH_3$ | $OCH_3$ | CH | |
| $CH_2CH_3$ | H | O | H | cyclopentyl | $OCH_3$ | $OCH_3$ | CH | |
| $CH_2CH_3$ | H | O | H | cyclopentyl | $CH_3$ | $CH_3$ | N | |
| $CH_2CH_3$ | H | O | H | cyclopentyl | $CH_3$ | $OCH_3$ | N | |
| $CH_2CH_3$ | H | O | H | cyclopentyl | $OCH_3$ | $OCH_3$ | N | |
| $CH_2CH_3$ | H | O | H | cyclopentyl | Cl | OCH | CH | |
| $CH_2CH_3$ | H | O | H | cyclopropyl | $OCH_3$ | $OCH_2CH_3$ | CH | |
| $CH_2CH_3$ | H | O | H | cyclopropyl | cyclopropyl | $OCH_3$ | CH | |
| $CH_2CH_3$ | H | O | H | cyclopropyl | $OCH_3$ | $CH(OCH_3)_2$ | CH | |
| $CH_2CH_3$ | H | O | H | cyclopropyl | $NHCH_3$ | $OCH_2CH_3$ | N | |
| $CH_2CH_3$ | H | O | H | cyclopropyl | $NHCH_3$ | $OCH_3$ | N | |
| $CH_2CH_3$ | H | O | H | cyclopropyl | $OCH_3$ | $OCH_2CH_3$ | N | |
| $CH_2CH_3$ | H | O | H | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| Ph | H | O | H | cyclopropyl | $CH_3$ | $CH_3$ | CH | |
| Ph | H | O | H | cyclopropyl | $CH_3$ | $OCH_3$ | CH | |
| Ph | H | O | H | cyclopropyl | $OCH_3$ | $OCH_3$ | CH | |
| Ph | H | O | H | cyclopropyl | $CH_3$ | $CH_3$ | N | |
| Ph | H | O | H | cyclopropyl | $CH_3$ | $OCH_3$ | N | |
| Ph | H | O | H | cyclopropyl | $OCH_3$ | $OCH_3$ | N | |
| Ph | H | O | H | cyclopropyl | Cl | $OCH_3$ | CH | |
| Ph | H | O | H | cyclobutyl | $CH_3$ | $CH_3$ | CH | |
| Ph | H | O | H | cyclobutyl | $CH_3$ | $OCH_3$ | CH | |
| Ph | H | O | H | cyclobutyl | $OCH_3$ | $OCH_3$ | CH | |
| Ph | H | O | H | cyclobutyl | $CH_3$ | $CH_3$ | N | |
| Ph | H | O | H | cyclobutyl | $CH_3$ | $OCH_3$ | N | |
| Ph | H | O | H | cyclobutyl | $OCH_3$ | $OCH_3$ | N | |
| Ph | H | O | H | cyclobutyl | Cl | $OCH_3$ | CH | |
| Ph | H | O | H | cyclopentyl | $CH_3$ | $CH_3$ | CH | |
| Ph | H | O | H | cyclopentyl | $CH_3$ | $OCH_3$ | CH | |
| Ph | H | O | H | cyclopentyl | $OCH_3$ | $OCH_3$ | CH | |
| Ph | H | O | H | cyclopentyl | $CH_3$ | $CH_3$ | N | |
| Ph | H | O | H | cyclopentyl | $CH_3$ | $OCH_3$ | N | |
| Ph | H | O | H | cyclopentyl | $OCH_3$ | $OCH_3$ | N | |
| Ph | H | O | H | cyclopentyl | Cl | $OCH_3$ | CH | |
| Ph | H | O | H | cyclopropyl | $OCH_3$ | $OCH_2CH_3$ | CH | |
| Ph | H | O | H | cyclopropyl | cyclopropyl | $OCH_3$ | CH | |
| Ph | H | O | H | cyclopropyl | $OCH_3$ | $CH(OCH_3)_2$ | CH | |
| Ph | H | O | H | cyclopropyl | $NHCH_3$ | $OCH_2CH_3$ | N | |
| Ph | H | O | H | cyclopropyl | $NHCH_3$ | $OCH_3$ | N | |
| Ph | H | O | H | cyclopropyl | $OCH_3$ | $OCH_2CH_3$ | N | |
| Ph | H | O | H | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CO_2CH_3$ | H | O | H | cyclopropyl | $CH_3$ | $CH_3$ | CH | |
| $CO_2CH_3$ | H | O | H | cyclopropyl | $CH_3$ | $OCH_3$ | CH | |
| $CO_2CH_3$ | H | O | H | cyclopropyl | $OCH_3$ | $OCH_3$ | CH | |
| $CO_2CH_3$ | H | O | H | cyclopropyl | $CH_3$ | $CH_3$ | N | |
| $CO_2CH_3$ | H | O | H | cyclopropyl | $CH_3$ | $OCH_3$ | N | |
| $CO_2CH_3$ | H | O | H | cyclopropyl | $OCH_3$ | $OCH_3$ | N | |
| $CO_2CH_3$ | H | O | H | cyclopropyl | Cl | $OCH_3$ | CH | |
| $CO_2CH_3$ | H | O | H | cyclobutyl | $CH_3$ | $CH_3$ | CH | |
| $CO_2CH_3$ | H | O | H | cyclobutyl | $CH_3$ | $OCH_3$ | CH | |
| $CO_2CH_3$ | H | O | H | cyclobutyl | $OCH_3$ | $OCH_3$ | CH | |
| $CO_2CH_3$ | H | O | H | cyclobutyl | $CH_3$ | $CH_3$ | N | |
| $CO_2CH_3$ | H | O | H | cyclobutyl | $CH_3$ | $OCH_3$ | N | |
| $CO_2CH_3$ | H | O | H | cyclobutyl | $OCH_3$ | $OCH_3$ | N | |
| $CO_2CH_3$ | H | O | H | cyclobutyl | Cl | $OCH_3$ | CH | |
| $CO_2CH_3$ | H | O | H | cyclopentyl | $CH_3$ | $CH_3$ | CH | |
| $CO_2CH_3$ | H | O | H | cyclopentyl | $CH_3$ | $OCH_3$ | CH | |
| $CO_2CH_3$ | H | O | H | cyclopentyl | $OCH_3$ | $OCH_3$ | CH | |
| $CO_2CH_3$ | H | O | H | cyclopentyl | $CH_3$ | $CH_3$ | N | |
| $CO_2CH_3$ | H | O | H | cyclopentyl | $CH_3$ | $OCH_3$ | N | |
| $CO_2CH_3$ | H | O | H | cyclopentyl | $OCH_3$ | $OCH_3$ | N | |
| $CO_2CH_3$ | H | O | H | cyclopentyl | Cl | $OCH_3$ | CH | |
| $CO_2CH_3$ | H | O | H | cyclopropyl | $OCH_3$ | $OCH_2CH_3$ | CH | |
| $CO_2CH_3$ | H | O | H | cyclopropyl | cyclopropyl | $OCH_3$ | CH | |
| $CO_2CH_3$ | H | O | H | cyclopropyl | $OCH_3$ | $CH(OCH_3)_2$ | CH | |
| $CO_2CH_3$ | H | O | H | cyclopropyl | $NHCH_3$ | $OCH_2CH_3$ | N | |
| $CO_2CH_3$ | H | O | H | cyclopropyl | $NHCH_3$ | $OCH_3$ | N | |
| $CO_2CH_3$ | H | O | H | cyclopropyl | $OCH_3$ | $OCH_2CH_3$ | N | |
| $CO_2CH_3$ | H | O | H | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $SO_2N(CH_3)_2$ | H | O | H | cyclopropyl | $CH_3$ | $CH_3$ | CH | |
| $SO_2N(CH_3)_2$ | H | O | H | cyclopropyl | $CH_3$ | $OCH_3$ | CH | |
| $SO_2N(CH_3)_2$ | H | O | H | cyclopropyl | $OCH_3$ | $OCH_3$ | CH | |
| $SO_2N(CH_3)_2$ | H | O | H | cyclopropyl | $CH_3$ | $CH_3$ | N | |
| $SO_2N(CH_3)_2$ | H | O | H | cyclopropyl | $CH_3$ | $OCH_3$ | N | |
| $SO_2N(CH_3)_2$ | H | O | H | cyclopropyl | $OCH_3$ | $OCH_3$ | N | |
| $SO_2N(CH_3)_2$ | H | O | H | cyclopropyl | Cl | $OCH_3$ | CH | |
| $SO_2N(CH_3)_2$ | H | O | H | cyclobutyl | $CH_3$ | $CH_3$ | CH | |
| $SO_2N(CH_3)_2$ | H | O | H | cyclobutyl | $CH_3$ | $OCH_3$ | CH | |
| $SO_2N(CH_3)_2$ | H | O | H | cyclobutyl | $OCH_3$ | $OCH_3$ | CH | |
| $SO_2N(CH_3)_2$ | H | O | H | cyclobutyl | $CH_3$ | $CH_3$ | N | |
| $SO_2N(CH_3)_2$ | H | O | H | cyclobutyl | $CH_3$ | $OCH_3$ | N | |
| $SO_2N(CH_3)_2$ | H | O | H | cyclobutyl | $OCH_3$ | $OCH_3$ | N | |
| $SO_2N(CH_3)_2$ | H | O | H | cyclobutyl | Cl | $OCH_3$ | CH | |
| $SO_2N(CH_3)_2$ | H | O | H | cyclopentyl | $CH_3$ | $CH_3$ | CH | |
| $SO_2N(CH_3)_2$ | H | O | H | cyclopentyl | $CH_3$ | $OCH_3$ | CH | |
| $SO_2N(CH_3)_2$ | H | O | H | cyclopentyl | $OCH_3$ | $OCH_3$ | CH | |
| $SO_2N(CH_3)_2$ | H | O | H | cyclopentyl | $CH_3$ | $CH_3$ | N | |
| $SO_2N(CH_3)_2$ | H | O | H | cyclopentyl | $CH_3$ | $OCH_3$ | N | |
| $SO_2N(CH_3)_2$ | H | O | H | cyclopentyl | $OCH_3$ | $OCH_3$ | N | |
| $SO_2N(CH_3)_2$ | H | O | H | cyclopentyl | Cl | $OCH_3$ | CH | |
| $SO_2N(CH_3)_2$ | H | O | H | cyclopropyl | $OCH_3$ | $OCH_2CH_3$ | CH | |
| $SO_2N(CH_3)_2$ | H | O | H | cyclopropyl | cyclopropyl | $OCH_3$ | CH | |
| $SO_2N(CH_3)_2$ | H | O | H | cyclopropyl | $OCH_3$ | $CH(OCH_3)_2$ | CH | |
| $SO_2N(CH_3)_2$ | H | O | H | cyclopropyl | $NHCH_3$ | $OCH_2CH_3$ | N | |
| $SO_2N(CH_3)_2$ | H | O | H | cyclopropyl | $NHCH_3$ | $OCH_3$ | N | |
| $SO_2N(CH_3)_2$ | H | O | H | cyclopropyl | $OCH_3$ | $OCH_2CH_3$ | N | |
| $SO_2N(CH_3)_2$ | H | O | H | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_2F$ | H | O | H | cyclopropyl | $CH_3$ | $CH_3$ | CH | |
| $CH_2F$ | H | O | H | cyclopropyl | $CH_3$ | $OCH_3$ | CH | |
| $CH_2F$ | H | O | H | cyclopropyl | $OCH_3$ | $OCH_3$ | CH | |
| $CH_2F$ | H | O | H | cyclopropyl | $CH_3$ | $CH_3$ | N | |
| $CH_2F$ | H | O | H | cyclopropyl | $CH_3$ | $OCH_3$ | N | |
| $CH_2F$ | H | O | H | cyclopropyl | $OCH_3$ | $OCH_3$ | N | |
| $CH_2F$ | H | O | H | cyclopropyl | Cl | $OCH_3$ | CH | |
| $CH_2F$ | H | O | H | cyclobutyl | $CH_3$ | $CH_3$ | CH | |
| $CH_2F$ | H | O | H | cyclobutyl | $CH_3$ | $OCH_3$ | CH | |
| $CH_2F$ | H | O | H | cyclobutyl | $OCH_3$ | $OCH_3$ | CH | |
| $CH_2F$ | H | O | H | cyclobutyl | $CH_3$ | $CH_3$ | N | |
| $CH_2F$ | H | O | H | cyclobutyl | $CH_3$ | $OCH_3$ | N | |
| $CH_2F$ | H | O | H | cyclobutyl | $OCH_3$ | $OCH_3$ | N | |
| $CH_2F$ | H | O | H | cyclobutyl | Cl | $OCH_3$ | CH | |
| $CH_2F$ | H | O | H | cyclopentyl | $CH_3$ | $CH_3$ | CH | |
| $CH_2F$ | H | O | H | cyclopentyl | $CH_3$ | $OCH_3$ | CH | |
| $CH_2F$ | H | O | H | cyclopentyl | $OCH_3$ | $OCH_3$ | CH | |
| $CH_2F$ | H | O | H | cyclopentyl | $CH_3$ | $CH_3$ | N | |
| $CH_2F$ | H | O | H | cyclopentyl | $CH_3$ | $OCH_3$ | N | |
| $CH_2F$ | H | O | H | cyclopentyl | $OCH_3$ | $OCH_3$ | N | |
| $CH_2F$ | H | O | H | cyclopentyl | Cl | $OCH_3$ | CH | |
| $CH_2F$ | H | O | H | cyclopropyl | $OCH_3$ | $OCH_2CH_3$ | CH | |
| $CH_2F$ | H | O | H | cyclopropyl | cyclopropyl | $OCH_3$ | CH | |
| $CH_2F$ | H | O | H | cyclopropyl | $OCH_3$ | $CH(OCH_3)_2$ | CH | |
| $CH_2F$ | H | O | H | cyclopropyl | $NHCH_3$ | $OCH_2CH_3$ | N | |
| $CH_2F$ | H | O | H | cyclopropyl | $NHCH_3$ | $OCH_3$ | N | |
| $CH_2F$ | H | O | H | cyclopropyl | $OCH_3$ | $OCH_2CH_3$ | N | |
| $CH_2F$ | H | O | H | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_2CH=CH_2$ | H | O | H | cyclopropyl | $CH_3$ | $CH_3$ | CH | |
| $CH_2CH=CH_2$ | H | O | H | cyclopropyl | $CH_3$ | $OCH_3$ | CH | |
| $CH_2CH=CH_2$ | H | O | H | cyclopropyl | $OCH_3$ | $OCH_3$ | CH | |
| $CH_2CH=CH_2$ | H | O | H | cyclopropyl | $CH_3$ | $CH_3$ | N | |
| $CH_2CH=CH_2$ | H | O | H | cyclopropyl | $CH_3$ | $OCH_3$ | N | |
| $CH_2CH=CH_2$ | H | O | H | cyclopropyl | $OCH_3$ | $OCH_3$ | N | |
| $CH_2CH=CH_2$ | H | O | H | cyclopropyl | Cl | $OCH_3$ | CH | |
| $CH_2CH=CH_2$ | H | O | H | cyclobutyl | $CH_3$ | $CH_3$ | CH | |
| $CH_2CH=CH_2$ | H | O | H | cyclobutyl | $CH_3$ | $OCH_3$ | CH | |
| $CH_2CH=CH_2$ | H | O | H | cyclobutyl | $OCH_3$ | $OCH_3$ | CH | |
| $CH_2CH=CH_2$ | H | O | H | cyclobutyl | $CH_3$ | $CH_3$ | N | |
| $CH_2CH=CH_2$ | H | O | H | cyclobutyl | $CH_3$ | $OCH_3$ | N | |
| $CH_2CH=CH_2$ | H | O | H | cyclobutyl | $OCH_3$ | $OCH_3$ | N | |
| $CH_2CH=CH_2$ | H | O | H | cyclobutyl | Cl | $OCH_3$ | CH | |
| $CH_2CH=CH_2$ | H | O | H | cyclopentyl | $CH_3$ | $CH_3$ | CH | |
| $CH_2CH=CH_2$ | H | O | H | cyclopentyl | $CH_3$ | $OCH_3$ | CH | |
| $CH_2CH=CH_2$ | H | O | H | cyclopentyl | $OCH_3$ | $OCH_3$ | CH | |
| $CH_2CH=CH_2$ | H | O | H | cyclopentyl | $CH_3$ | $CH_3$ | N | |
| $CH_2CH=CH_2$ | H | O | H | cyclopentyl | $CH_3$ | $OCH_3$ | N | |
| $CH_2CH=CH_2$ | H | O | H | cyclopentyl | $OCH_3$ | $OCH_3$ | N | |
| $CH_2CH=CH_2$ | H | O | H | cyclopentyl | Cl | $OCH_3$ | CH | |
| $CH_2CH=CH_2$ | H | O | H | cyclopropyl | $OCH_3$ | $OCH_2CH_3$ | CH | |
| $CH_2CH=CH_2$ | H | O | H | cyclopropyl | cyclopropyl | $OCH_3$ | CH | |
| $CH_2CH=CH_2$ | H | O | H | cyclopropyl | $OCH_3$ | $CH(OCH_3)_2$ | CH | |
| $CH_2CH=CH_2$ | H | O | H | cyclopropyl | $NHCH_3$ | $OCH_2CH_3$ | N | |
| $CH_2CH=CH_2$ | H | O | H | cyclopropyl | $NHCH_3$ | $OCH_3$ | N | |
| $CH_2CH=CH_2$ | H | O | H | cyclopropyl | $OCH_3$ | $OCH_2CH_3$ | N | |
| $CH_2CH=CH_2$ | H | O | H | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_2C{\equiv}CH$ | H | O | H | cyclopropyl | $CH_3$ | $CH_3$ | CH | |
| $CH_2C{\equiv}CH$ | H | O | H | cyclopropyl | $CH_3$ | $OCH_3$ | CH | |
| $CH_2C{\equiv}CH$ | H | O | H | cyclopropyl | $OCH_3$ | $OCH_3$ | CH | |
| $CH_2C{\equiv}CH$ | H | O | H | cyclopropyl | $CH_3$ | $CH_3$ | N | |
| $CH_2C{\equiv}CH$ | H | O | H | cyclopropyl | $CH_3$ | $OCH_3$ | N | |
| $CH_2C{\equiv}CH$ | H | O | H | cyclopropyl | $OCH_3$ | $OCH_3$ | N | |
| $CH_2C{\equiv}CH$ | H | O | H | cyclopropyl | Cl | $OCH_3$ | CH | |
| $CH_2C{\equiv}CH$ | H | O | H | cyclobutyl | $CH_3$ | $CH_3$ | CH | |
| $CH_2C{\equiv}CH$ | H | O | H | cyclobutyl | $CH_3$ | $OCH_3$ | CH | |
| $CH_2C{\equiv}CH$ | H | O | H | cyclobutyl | $OCH_3$ | $OCH_3$ | CH | |
| $CH_2C{\equiv}CH$ | H | O | H | cyclobutyl | $CH_3$ | $CH_3$ | N | |
| $CH_2C{\equiv}CH$ | H | O | H | cyclobutyl | $CH_3$ | $OCH_3$ | N | |
| $CH_2C{\equiv}CH$ | H | O | H | cyclobutyl | $OCH_3$ | $OCH_3$ | N | |
| $CH_2C{\equiv}CH$ | H | O | H | cyclobutyl | Cl | $OCH_3$ | CH | |
| $CH_2C{\equiv}CH$ | H | O | H | cyclopentyl | $CH_3$ | $CH_3$ | CH | |
| $CH_2C{\equiv}CH$ | H | O | H | cyclopentyl | $CH_3$ | $OCH_3$ | CH | |
| $CH_2C{\equiv}CH$ | H | O | H | cyclopentyl | $OCH_3$ | $OCH_3$ | CH | |
| $CH_2C{\equiv}CH$ | H | O | H | cyclopentyl | $CH_3$ | $CH_3$ | N | |
| $CH_2C{\equiv}CH$ | H | O | H | cyclopentyl | $CH_3$ | $OCH_3$ | N | |
| $CH_2C{\equiv}CH$ | H | O | H | cyclopentyl | $OCH_3$ | $OCH_3$ | N | |
| $CH_2C{\equiv}CH$ | H | O | H | cyclopentyl | Cl | $OCH_3$ | CH | |
| $CH_2C{\equiv}CH$ | H | O | H | cyclopropyl | $OCH_3$ | $OCH_2CH_3$ | CH | |
| $CH_2C{\equiv}CH$ | H | O | H | cyclopropyl | cyclopropyl | $OCH_3$ | CH | |
| $CH_2C{\equiv}CH$ | H | O | H | cyclopropyl | $OCH_3$ | $CH(OCH_3)_2$ | CH | |
| $CH_2C{\equiv}CH$ | H | O | H | cyclopropyl | $NHCH_3$ | $OCH_2CH_3$ | N | |
| $CH_2C{\equiv}CH$ | H | O | H | cyclopropyl | $NHCH_3$ | $OCH_3$ | N | |
| $CH_2C{\equiv}CH$ | H | O | H | cyclopropyl | $OCH_3$ | $OCH_2CH_3$ | N | |
| $CH_2C{\equiv}CH$ | H | O | H | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| H | H | O | Cl | cyclopropyl | $CH_3$ | $CH_3$ | CH | |
| H | H | O | Cl | cyclopropyl | $CH_3$ | $OCH_3$ | CH | |
| H | H | O | Cl | cyclopropyl | $OCH_3$ | $OCH_3$ | CH | |
| H | H | O | Cl | cyclopropyl | $CH_3$ | $CH_3$ | N | |
| H | H | O | Cl | cyclopropyl | $CH_3$ | $OCH_3$ | N | |
| H | H | O | Cl | cyclopropyl | $OCH_3$ | $OCH_3$ | N | |
| H | H | O | Cl | cyclopropyl | Cl | $OCH_3$ | CH | |
| H | H | O | Cl | cyclobutyl | $CH_3$ | $CH_3$ | CH | |
| H | H | O | Cl | cyclobutyl | $CH_3$ | $OCH_3$ | CH | |
| H | H | O | Cl | cyclobutyl | $OCH_3$ | $OCH_3$ | CH | |
| H | H | O | Cl | cyclobutyl | $CH_3$ | $CH_3$ | N | |
| H | H | O | Cl | cyclobutyl | $CH_3$ | $OCH_3$ | N | |
| H | H | O | Cl | cyclobutyl | $OCH_3$ | $OCH_3$ | N | |
| H | H | O | Cl | cyclobutyl | Cl | $OCH_3$ | CH | |
| H | H | O | Cl | cyclopentyl | $CH_3$ | $CH_3$ | CH | |
| H | H | O | Cl | cyclopentyl | $CH_3$ | $OCH_3$ | CH | |
| H | H | O | Cl | cyclopentyl | $OCH_3$ | $OCH_3$ | CH | |
| H | H | O | Cl | cyclopentyl | $CH_3$ | $CH_3$ | N | |
| H | H | O | Cl | cyclopentyl | $CH_3$ | $OCH_3$ | N | |
| H | H | O | Cl | cyclopentyl | $OCH_3$ | $OCH_3$ | N | |
| H | H | O | Cl | cyclopentyl | Cl | $OCH_3$ | CH | |
| H | H | O | Cl | cyclopropyl | $OCH_3$ | $OCH_2CH_3$ | CH | |
| H | H | O | Cl | cyclopropyl | cyclopropyl | $OCH_3$ | CH | |
| H | H | O | Cl | cyclopropyl | $OCH_3$ | $CH(OCH_3)_2$ | CH | |
| H | H | O | Cl | cyclopropyl | $NHCH_3$ | $OCH_2CH_3$ | N | |
| H | H | O | Cl | cyclopropyl | $NHCH_3$ | $OCH_3$ | N | |
| H | H | O | Cl | cyclopropyl | $OCH_3$ | $OCH_2CH_3$ | N | |
| H | H | O | Cl | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_2CH_3$ | H | 0 | Cl | cyclopropyl | $CH_3$ | $CH_3$ | CH | |
| $CH_2CH_3$ | H | 0 | Cl | cyclopropyl | $CH_3$ | $OCH_3$ | CH | |
| $CH_2CH_3$ | H | 0 | Cl | cyclopropyl | $OCH_3$ | $OCH_3$ | CH | |
| $CH_2CH_3$ | H | 0 | Cl | cyclopropyl | $CH_3$ | $CH_3$ | N | |
| $CH_2CH_3$ | H | 0 | Cl | cyclopropyl | $CH_3$ | $OCH_3$ | N | |
| $CH_2CH_3$ | H | 0 | Cl | cyclopropyl | $OCH_3$ | $OCH_3$ | N | |
| $CH_2CH_3$ | H | 0 | Cl | cyclopropyl | Cl | $OCH_3$ | CH | |
| $CH_2CH_3$ | H | 0 | Cl | cyclobutyl | $CH_3$ | $CH_3$ | CH | |
| $CH_2CH_3$ | H | 0 | Cl | cyclobutyl | $CH_3$ | $OCH_3$ | CH | |
| $CH_2CH_3$ | H | 0 | Cl | cyclobutyl | $OCH_3$ | $OCH_3$ | CH | |
| $CH_2CH_3$ | H | 0 | Cl | cyclobutyl | $CH_3$ | $CH_3$ | N | |
| $CH_2CH_3$ | H | 0 | Cl | cyclobutyl | $CH_3$ | $OCH_3$ | N | |
| $CH_2CH_3$ | H | 0 | Cl | cyclobutyl | $OCH_3$ | $OCH_3$ | N | |
| $CH_2CH_3$ | H | 0 | Cl | cyclobutyl | Cl | $OCH_3$ | CH | |
| $CH_2CH_3$ | H | 0 | Cl | cyclopentyl | $CH_3$ | $CH_3$ | CH | |
| $CH_2CH_3$ | H | 0 | Cl | cyclopentyl | $CH_3$ | $OCH_3$ | CH | |
| $CH_2CH_3$ | H | 0 | Cl | cyclopentyl | $OCH_3$ | $OCH_3$ | CH | |
| $CH_2CH_3$ | H | 0 | Cl | cyclopentyl | $CH_3$ | $CH_3$ | N | |
| $CH_2CH_3$ | H | 0 | Cl | cyclopentyl | $CH_3$ | $OCH_3$ | N | |
| $CH_2CH_3$ | H | 0 | Cl | cyclopentyl | $OCH_3$ | $OCH_3$ | N | |
| $CH_2CH_3$ | H | 0 | Cl | cyclopentyl | Cl | $OCH_3$ | CH | |
| $CH_2CH_3$ | H | 0 | Cl | cyclopropyl | $OCH_3$ | $OCH_2CH_3$ | CH | |
| $CH_2CH_3$ | H | 0 | Cl | cyclopropyl | cyclopropyl | $OCH_3$ | CH | |
| $CH_2CH_3$ | H | 0 | Cl | cyclopropyl | $OCH_3$ | $CH(OCH_3)_2$ | CH | |
| $CH_2CH_3$ | H | 0 | Cl | cyclopropyl | $NHCH_3$ | $OCH_2CH_3$ | N | |
| $CH_2CH_3$ | H | 0 | Cl | cyclopropyl | $NHCH_3$ | $OCH_3$ | N | |
| $CH_2CH_3$ | H | 0 | Cl | cyclopropyl | $OCH_3$ | $OCH_2CH_3$ | N | |
| $CH_2CH_3$ | H | 0 | Cl | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_2CH_2CH_3$ | H | 0 | Cl | cyclopropyl | $CH_3$ | $CH_3$ | CH | |
| $CH_2CH_2CH_3$ | H | 0 | Cl | cyclopropyl | $CH_3$ | $OCH_3$ | CH | |
| $CH_2CH_2CH_3$ | H | 0 | Cl | cyclopropyl | $OCH_3$ | $OCH_3$ | CH | |
| $CH_2CH_2CH_3$ | H | 0 | Cl | cyclopropyl | $CH_3$ | $CH_3$ | N | |
| $CH_2CH_2CH_3$ | H | 0 | Cl | cyclopropyl | $CH_3$ | $OCH_3$ | N | |
| $CH_2CH_2CH_3$ | H | 0 | Cl | cyclopropyl | $OCH_3$ | $OCH_3$ | N | |
| $CH_2CH_2CH_3$ | H | 0 | Cl | cyclopropyl | Cl | $OCH_3$ | CH | |
| $CH_2CH_2CH_3$ | H | 0 | Cl | cyclobutyl | $CH_3$ | $CH_3$ | CH | |
| $CH_2CH_2CH_3$ | H | 0 | Cl | cyclobutyl | $CH_3$ | $OCH_3$ | CH | |
| $CH_2CH_2CH_3$ | H | 0 | Cl | cyclobutyl | $OCH_3$ | $OCH_3$ | CH | |
| $CH_2CH_2CH_3$ | H | 0 | Cl | cyclobutyl | $CH_3$ | $CH_3$ | N | |
| $CH_2CH_2CH_3$ | H | 0 | Cl | cyclobutyl | $CH_3$ | $OCH_3$ | N | |
| $CH_2CH_2CH_3$ | H | 0 | Cl | cyclobutyl | $OCH_3$ | $OCH_3$ | N | |
| $CH_2CH_2CH_3$ | H | 0 | Cl | cyclobutyl | Cl | $OCH_3$ | CH | |
| $CH_2CH_2CH_3$ | H | 0 | Cl | cyclopentyl | $CH_3$ | $CH_3$ | CH | |
| $CH_2CH_2CH_3$ | H | 0 | Cl | cyclopentyl | $CH_3$ | $OCH_3$ | CH | |
| $CH_2CH_2CH_3$ | H | 0 | Cl | cyclopentyl | $OCH_3$ | $OCH_3$ | CH | |
| $CH_2CH_2CH_3$ | H | 0 | Cl | cyclopentyl | $CH_3$ | $CH_3$ | N | |
| $CH_2CH_2CH_3$ | H | 0 | Cl | cyclopentyl | $CH_3$ | $OCH_3$ | N | |
| $CH_2CH_2CH_3$ | H | 0 | Cl | cyclopentyl | $OCH_3$ | $OCH_3$ | N | |
| $CH_2CH_2CH_3$ | H | 0 | Cl | cyclopentyl | Cl | $OCH_3$ | CH | |
| $CH_2CH_2CH_3$ | H | 0 | Cl | cyclopropyl | $OCH_3$ | $OCH_2CH_3$ | CH | |
| $CH_2CH_2CH_3$ | H | 0 | Cl | cyclopropyl | cyclopropyl | $OCH_3$ | CH | |
| $CH_2CH_2CH_3$ | H | 0 | Cl | cyclopropyl | $OCH_3$ | $CH(OCH_3)_2$ | CH | |
| $CH_2CH_2CH_3$ | H | 0 | Cl | cyclopropyl | $NHCH_3$ | $OCH_2CH_3$ | N | |
| $CH_2CH_2CH_3$ | H | 0 | Cl | cyclopropyl | $NHCH_3$ | $OCH_3$ | N | |
| $CH_2CH_2CH_3$ | H | 0 | Cl | cyclopropyl | $OCH_3$ | $OCH_2CH_3$ | N | |
| $CH_2CH_2CH_3$ | H | 0 | Cl | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_2SO_2CH_3$ | H | 0 | Cl | cyclopropyl | $CH_3$ | $CH_3$ | CH | |
| $CH_2SO_2CH_3$ | H | 0 | Cl | cyclopropyl | $CH_3$ | $OCH_3$ | CH | |
| $CH_2SO_2CH_3$ | H | 0 | Cl | cyclopropyl | $OCH_3$ | $OCH_3$ | CH | |
| $CH_2SO_2CH_3$ | H | 0 | Cl | cyclopropyl | $CH_3$ | $CH_3$ | N | |
| $CH_2SO_2CH_3$ | H | 0 | Cl | cyclopropyl | $CH_3$ | $OCH_3$ | N | |
| $CH_2SO_2CH_3$ | H | 0 | Cl | cyclopropyl | $OCH_3$ | $OCH_3$ | N | |
| $CH_2SO_2CH_3$ | H | 0 | Cl | cyclopropyl | Cl | $OCH_3$ | CH | |
| $CH_2SO_2CH_3$ | H | 0 | Cl | cyclobutyl | $CH_3$ | $CH_3$ | CH | |
| $CH_2SO_2CH_3$ | H | 0 | Cl | cyclobutyl | $CH_3$ | $OCH_3$ | CH | |
| $CH_2SO_2CH_3$ | H | 0 | Cl | cyclobutyl | $OCH_3$ | $OCH_3$ | CH | |
| $CH_2SO_2CH_3$ | H | 0 | Cl | cyclobutyl | $CH_3$ | $CH_3$ | N | |
| $CH_2SO_2CH_3$ | H | 0 | Cl | cyclobutyl | $CH_3$ | $OCH_3$ | N | |
| $CH_2SO_2CH_3$ | H | 0 | Cl | cyclobutyl | $OCH_3$ | $OCH_3$ | N | |
| $CH_2SO_2CH_3$ | H | 0 | Cl | cyclobutyl | Cl | $OCH_3$ | CH | |
| $CH_2SO_2CH_3$ | H | 0 | Cl | cyclopentyl | $CH_3$ | $CH_3$ | CH | |
| $CH_2SO_2CH_3$ | H | 0 | Cl | cyclopentyl | $CH_3$ | $OCH_3$ | CH | |
| $CH_2SO_2CH_3$ | H | 0 | Cl | cyclopentyl | $OCH_3$ | $OCH_3$ | CH | |
| $CH_2SO_2CH_3$ | H | 0 | Cl | cyclopentyl | $CH_3$ | $CH_3$ | N | |
| $CH_2SO_2CH_3$ | H | 0 | Cl | cyclopentyl | $CH_3$ | $OCH_3$ | N | |
| $CH_2SO_2CH_3$ | H | 0 | Cl | cyclopentyl | $OCH_3$ | $OCH_3$ | N | |
| $CH_2SO_2CH_3$ | H | 0 | Cl | cyclopentyl | Cl | $OCH_3$ | CH | |
| $CH_2SO_2CH_3$ | H | 0 | Cl | cyclopropyl | $OCH_3$ | $OCH_2CH_3$ | CH | |
| $CH_2SO_2CH_3$ | H | 0 | Cl | cyclopropyl | cyclopropyl | $OCH_3$ | CH | |
| $CH_2SO_2CH_3$ | H | 0 | Cl | cyclopropyl | $OCH_3$ | $CH(OCH_3)_2$ | CH | |
| $CH_2SO_2CH_3$ | H | 0 | Cl | cyclopropyl | $NHCH_3$ | $OCH_2CH_3$ | N | |
| $CH_2SO_2CH_3$ | H | 0 | Cl | cyclopropyl | $NHCH_3$ | $OCH_3$ | N | |
| $CH_2SO_2CH_3$ | H | 0 | Cl | cyclopropyl | $OCH_3$ | $OCH_2CH_3$ | N | |
| $CH_2SO_2CH_3$ | H | 0 | Cl | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| Ph | H | 0 | Cl | cyclopropyl | $CH_3$ | $CH_3$ | CH | |
| Ph | H | 0 | Cl | cyclopropyl | $CH_3$ | $OCH_3$ | CH | |
| Ph | H | 0 | Cl | cyclopropyl | $OCH_3$ | $OCH_3$ | CH | |
| Ph | H | 0 | Cl | cyclopropyl | $CH_3$ | $CH_3$ | N | |
| Ph | H | 0 | Cl | cyclopropyl | $CH_3$ | $OCH_3$ | N | |
| Ph | H | 0 | Cl | cyclopropyl | $OCH_3$ | $OCH_3$ | N | |
| Ph | H | 0 | Cl | cyclopropyl | Cl | $OCH_3$ | CH | |
| Ph | H | 0 | Cl | cyclobutyl | $CH_3$ | $CH_3$ | CH | |
| Ph | H | 0 | Cl | cyclobutyl | $CH_3$ | $OCH_3$ | CH | |
| Ph | H | 0 | Cl | cyclobutyl | $OCH_3$ | $OCH_3$ | CH | |
| Ph | H | 0 | Cl | cyclobutyl | $CH_3$ | $CH_3$ | N | |
| Ph | H | 0 | Cl | cyclobutyl | $CH_3$ | $OCH_3$ | N | |
| Ph | H | 0 | Cl | cyclobutyl | $OCH_3$ | $OCH_3$ | N | |
| Ph | H | 0 | Cl | cyclobutyl | Cl | $OCH_3$ | CH | |
| Ph | H | 0 | Cl | cyclopentyl | $CH_3$ | $CH_3$ | CH | |
| Ph | H | 0 | Cl | cyclopentyl | $CH_3$ | $OCH_3$ | CH | |
| Ph | H | 0 | Cl | cyclopentyl | $OCH_3$ | $OCH_3$ | CH | |
| Ph | H | 0 | Cl | cyclopentyl | $CH_3$ | $CH_3$ | N | |
| Ph | H | 0 | Cl | cyclopentyl | $CH_3$ | $OCH_3$ | N | |
| Ph | H | 0 | Cl | cyclopentyl | $OCH_3$ | $OCH_3$ | N | |
| Ph | H | 0 | Cl | cyclopentyl | Cl | $OCH_3$ | CH | |
| Ph | H | 0 | Cl | cyclopropyl | $OCH_3$ | $OCH_2CH_3$ | CH | |
| Ph | H | 0 | Cl | cyclopropyl | cyclopropyl | $OCH_3$ | CH | |
| Ph | H | 0 | Cl | cyclopropyl | $OCH_3$ | $CH(OCH_3)_2$ | CH | |
| Ph | H | 0 | Cl | cyclopropyl | $NHCH_3$ | $OCH_2CH_3$ | N | |
| Ph | H | 0 | Cl | cyclopropyl | $NHCH_3$ | $OCH_3$ | N | |
| Ph | H | 0 | Cl | cyclopropyl | $OCH_3$ | $OCH_2CH_3$ | N | |
| Ph | H | 0 | Cl | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| H | H | 0 | $CH_3$ | cyclopropyl | $CH_3$ | $CH_3$ | CH | |
| H | H | 0 | $CH_3$ | cyclopropyl | $CH_3$ | $OCH_3$ | CH | |
| H | H | 0 | $CH_3$ | cyclopropyl | $OCH_3$ | $OCH_3$ | CH | |
| H | H | 0 | $CH_3$ | cyclopropyl | $CH_3$ | $CH_3$ | N | |
| H | H | 0 | $CH_3$ | cyclopropyl | $CH_3$ | $OCH_3$ | N | |
| H | H | 0 | $CH_3$ | cyclopropyl | $OCH_3$ | $OCH_3$ | N | |
| H | H | 0 | $CH_3$ | cyclopropyl | Cl | $OCH_3$ | CH | |
| H | H | 0 | $CH_3$ | cyclobutyl | $CH_3$ | $CH_3$ | CH | |
| H | H | 0 | $CH_3$ | cyclobutyl | $CH_3$ | $OCH_3$ | CH | |
| H | H | 0 | $CH_3$ | cyclobutyl | $OCH_3$ | $OCH_3$ | CH | |
| H | H | 0 | $CH_3$ | cyclobutyl | $CH_3$ | $CH_3$ | N | |
| H | H | 0 | $CH_3$ | cyclobutyl | $CH_3$ | $OCH_3$ | N | |
| H | H | 0 | $CH_3$ | cyclobutyl | $OCH_3$ | $OCH_3$ | N | |
| H | H | 0 | $CH_3$ | cyclobutyl | Cl | $OCH_3$ | CH | |
| H | H | 0 | $CH_3$ | cyclopentyl | $CH_3$ | $CH_3$ | CH | |
| H | H | 0 | $CH_3$ | cyclopentyl | $CH_3$ | $OCH_3$ | CH | |
| H | H | 0 | $CH_3$ | cyclopentyl | $OCH_3$ | $OCH_3$ | CH | |
| H | H | 0 | $CH_3$ | cyclopentyl | $CH_3$ | $CH_3$ | N | |
| H | H | 0 | $CH_3$ | cyclopentyl | $CH_3$ | $OCH_3$ | N | |
| H | H | 0 | $CH_3$ | cyclopentyl | $OCH_3$ | $OCH_3$ | N | |
| H | H | 0 | $CH_3$ | cyclopentyl | Cl | $OCH_3$ | CH | |
| H | H | 0 | $CH_3$ | cyclopropyl | $OCH_3$ | $OCH_2CH_3$ | CH | |
| H | H | 0 | $CH_3$ | cyclopropyl | cyclopropyl | $OCH_3$ | CH | |
| H | H | 0 | $CH_3$ | cyclopropyl | $OCH_3$ | $CH(OCH_3)_2$ | CH | |
| H | H | 0 | $CH_3$ | cyclopropyl | $NHCH_3$ | $OCH_2CH_3$ | N | |
| H | H | 0 | $CH_3$ | cyclopropyl | $NHCH_3$ | $OCH_3$ | N | |
| H | H | 0 | $CH_3$ | cyclopropyl | $OCH_3$ | $OCH_2CH_3$ | N | |
| H | H | 0 | $CH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_2CH_3$ | H | O | $CH_3$ | cyclopropyl | $CH_3$ | $CH_3$ | CH | |
| $CH_2CH_3$ | H | O | $CH_3$ | cyclopropyl | $CH_3$ | $OCH_3$ | CH | |
| $CH_2CH_3$ | H | O | $CH_3$ | cyclopropyl | $OCH_3$ | $OCH_3$ | CH | |
| $CH_2CH_3$ | H | O | $CH_3$ | cyclopropyl | $CH_3$ | $CH_3$ | N | |
| $CH_2CH_3$ | H | O | $CH_3$ | cyclopropyl | $CH_3$ | $OCH_3$ | N | |
| $CH_2CH_3$ | H | O | $CH_3$ | cyclopropyl | $OCH_3$ | $OCH_3$ | N | |
| $CH_2CH_3$ | H | O | $CH_3$ | cyclopropyl | Cl | $OCH_3$ | CH | |
| $CH_2CH_3$ | H | O | $CH_3$ | cyclobutyl | $CH_3$ | $CH_3$ | CH | |
| $CH_2CH_3$ | H | O | $CH_3$ | cyclobutyl | $CH_3$ | $OCH_3$ | CH | |
| $CH_2CH_3$ | H | O | $CH_3$ | cyclobutyl | $OCH_3$ | $OCH_3$ | CH | |
| $CH_2CH_3$ | H | O | $CH_3$ | cyclobutyl | $CH_3$ | $CH_3$ | N | |
| $CH_2CH_3$ | H | O | $CH_3$ | cyclobutyl | $CH_3$ | $OCH_3$ | N | |
| $CH_2CH_3$ | H | O | $CH_3$ | cyclobutyl | $OCH_3$ | $OCH_3$ | N | |
| $CH_2CH_3$ | H | O | $CH_3$ | cyclobutyl | Cl | $OCH_3$ | CH | |
| $CH_2CH_3$ | H | O | $CH_3$ | cyclopentyl | $CH_3$ | $CH_3$ | CH | |
| $CH_2CH_3$ | H | O | $CH_3$ | cyclopentyl | $CH_3$ | $OCH_3$ | CH | |
| $CH_2CH_3$ | H | O | $CH_3$ | cyclopentyl | $OCH_3$ | $OCH_3$ | CH | |
| $CH_2CH_3$ | H | O | $CH_3$ | cyclopentyl | $CH_3$ | $CH_3$ | N | |
| $CH_2CH_3$ | H | O | $CH_3$ | cyclopentyl | $CH_3$ | $OCH_3$ | N | |
| $CH_2CH_3$ | H | O | $CH_3$ | cyclopentyl | $OCH_3$ | $OCH_3$ | N | |
| $CH_2CH_3$ | H | O | $CH_3$ | cyclopentyl | Cl | $OCH_3$ | CH | |
| $CH_2CH_3$ | H | O | $CH_3$ | cyclopropyl | $OCH_3$ | $OCH_2CH_3$ | CH | |
| $CH_2CH_3$ | H | O | $CH_3$ | cyclopropyl | cyclopropyl | $OCH_3$ | CH | |
| $CH_2CH_3$ | H | O | $CH_3$ | cyclopropyl | $OCH_3$ | $CH(OCH_3)_2$ | CH | |
| $CH_2CH_3$ | H | O | $CH_3$ | cyclopropyl | $NHCH_3$ | $OCH_2CH_3$ | N | |
| $CH_2CH_3$ | H | O | $CH_3$ | cyclopropyl | $NHCH_3$ | $OCH_3$ | N | |
| $CH_2CH_3$ | H | O | $CH_3$ | cyclopropyl | $OCH_3$ | $OCH_2CH_3$ | N | |
| $CH_2CH_3$ | H | O | $CH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_2CH=CH_2$ | H | O | $CH_3$ | cyclopropyl | $CH_3$ | $CH_3$ | CH | |
| $CH_2CH=CH_2$ | H | O | $CH_3$ | cyclopropyl | $CH_3$ | $OCH_3$ | CH | |
| $CH_2CH=CH_2$ | H | O | $CH_3$ | cyclopropyl | $OCH_3$ | $OCH_3$ | CH | |
| $CH_2CH=CH_2$ | H | O | $CH_3$ | cyclopropyl | $CH_3$ | $CH_3$ | N | |
| $CH_2CH=CH_2$ | H | O | $CH_3$ | cyclopropyl | $CH_3$ | $OCH_3$ | N | |
| $CH_2CH=CH_2$ | H | O | $CH_3$ | cyclopropyl | $OCH_3$ | $OCH_3$ | N | |
| $CH_2CH=CH_2$ | H | O | $CH_3$ | cyclopropyl | Cl | $OCH_3$ | CH | |
| $CH_2CH=CH_2$ | H | O | $CH_3$ | cyclobutyl | $CH_3$ | $CH_3$ | CH | |
| $CH_2CH=CH_2$ | H | O | $CH_3$ | cyclobutyl | $CH_3$ | $OCH_3$ | CH | |
| $CH_2CH=CH_2$ | H | O | $CH_3$ | cyclobutyl | $OCH_3$ | $OCH_3$ | CH | |
| $CH_2CH=CH_2$ | H | O | $CH_3$ | cyclobutyl | $CH_3$ | $CH_3$ | N | |
| $CH_2CH=CH_2$ | H | O | $CH_3$ | cyclobutyl | $CH_3$ | $OCH_3$ | N | |
| $CH_2CH=CH_2$ | H | O | $CH_3$ | cyclobutyl | $OCH_3$ | $OCH_3$ | N | |
| $CH_2CH=CH_2$ | H | O | $CH_3$ | cyclobutyl | Cl | $OCH_3$ | CH | |
| $CH_2CH=CH_2$ | H | O | $CH_3$ | cyclopentyl | $CH_3$ | $CH_3$ | CH | |
| $CH_2CH=CH_2$ | H | O | $CH_3$ | cyclopentyl | $CH_3$ | $OCH_3$ | CH | |
| $CH_2CH=CH_2$ | H | O | $CH_3$ | cyclopentyl | $OCH_3$ | $OCH_3$ | CH | |
| $CH_2CH=CH_2$ | H | O | $CH_3$ | cyclopentyl | $CH_3$ | $CH_3$ | N | |
| $CH_2CH=CH_2$ | H | O | $CH_3$ | cyclopentyl | $CH_3$ | $OCH_3$ | N | |
| $CH_2CH=CH_2$ | H | O | $CH_3$ | cyclopentyl | $OCH_3$ | $OCH_3$ | N | |
| $CH_2CH=CH_2$ | H | O | $CH_3$ | cyclopentyl | Cl | $OCH_3$ | CH | |
| $CH_2CH=CH_2$ | H | O | $CH_3$ | cyclopropyl | $OCH_3$ | $OCH_2CH_3$ | CH | |
| $CH_2CH=CH_2$ | H | O | $CH_3$ | cyclopropyl | cyclopropyl | $OCH_3$ | CH | |
| $CH_2CH=CH_2$ | H | O | $CH_3$ | cyclopropyl | $OCH_3$ | $CH(OCH_3)_2$ | CH | |
| $CH_2CH=CH_2$ | H | O | $CH_3$ | cyclopropyl | $NHCH_3$ | $OCH_2CH_3$ | N | |
| $CH_2CH=CH_2$ | H | O | $CH_3$ | cyclopropyl | $NHCH_3$ | $OCH_3$ | N | |
| $CH_2CH=CH_2$ | H | O | $CH_3$ | cyclopropyl | $OCH_3$ | $OCH_2CH_3$ | N | |
| $CH_2CH=CH_2$ | H | O | $CH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_2Cl$ | H | O | $CH_3$ | cyclopropyl | $CH_3$ | $CH_3$ | CH | |
| $CH_2Cl$ | H | O | $CH_3$ | cyclopropyl | $CH_3$ | $OCH_3$ | CH | |
| $CH_2Cl$ | H | O | $CH_3$ | cyclopropyl | $OCH_3$ | $OCH_3$ | CH | |
| $CH_2Cl$ | H | O | $CH_3$ | cyclopropyl | $CH_3$ | $CH_3$ | N | |
| $CH_2Cl$ | H | O | $CH_3$ | cyclopropyl | $CH_3$ | $OCH_3$ | N | |
| $CH_2Cl$ | H | O | $CH_3$ | cyclopropyl | $OCH_3$ | $OCH_3$ | N | |
| $CH_2Cl$ | H | O | $CH_3$ | cyclopropyl | Cl | $OCH_3$ | CH | |
| $CH_2Cl$ | H | O | $CH_3$ | cyclobutyl | $CH_3$ | $CH_3$ | CH | |
| $CH_2Cl$ | H | O | $CH_3$ | cyclobutyl | $CH_3$ | $OCH_3$ | CH | |
| $CH_2Cl$ | H | O | $CH_3$ | cyclobutyl | $OCH_3$ | $OCH_3$ | CH | |
| $CH_2Cl$ | H | O | $CH_3$ | cyclobutyl | $CH_3$ | $CH_3$ | N | |
| $CH_2Cl$ | H | O | $CH_3$ | cyclobutyl | $CH_3$ | $OCH_3$ | N | |
| $CH_2Cl$ | H | O | $CH_3$ | cyclobutyl | $OCH_3$ | $OCH_3$ | N | |
| $CH_2Cl$ | H | O | $CH_3$ | cyclobutyl | Cl | $OCH_3$ | CH | |
| $CH_2Cl$ | H | O | $CH_3$ | cyclopentyl | $CH_3$ | $CH_3$ | CH | |
| $CH_2Cl$ | H | O | $CH_3$ | cyclopentyl | $CH_3$ | $OCH_3$ | CH | |
| $CH_2Cl$ | H | O | $CH_3$ | cyclopentyl | $OCH_3$ | $OCH_3$ | CH | |
| $CH_2Cl$ | H | O | $CH_3$ | cyclopentyl | $CH_3$ | $CH_3$ | N | |
| $CH_2Cl$ | H | O | $CH_3$ | cyclopentyl | $CH_3$ | $OCH_3$ | N | |
| $CH_2Cl$ | H | O | $CH_3$ | cyclopentyl | $OCH_3$ | $OCH_3$ | N | |
| $CH_2Cl$ | H | O | $CH_3$ | cyclopentyl | Cl | $OCH_3$ | CH | |
| $CH_2Cl$ | H | O | $CH_3$ | cyclopropyl | $OCH_3$ | $OCH_2CH_3$ | CH | |
| $CH_2Cl$ | H | O | $CH_3$ | cyclopropyl | cyclopropyl | $OCH_3$ | CH | |
| $CH_2Cl$ | H | O | $CH_3$ | cyclopropyl | $OCH_3$ | $CH(OCH_3)_2$ | CH | |
| $CH_2Cl$ | H | O | $CH_3$ | cyclopropyl | $NHCH_3$ | $OCH_2CH_3$ | N | |
| $CH_2Cl$ | H | O | $CH_3$ | cyclopropyl | $NHCH_3$ | $OCH_3$ | N | |
| $CH_2Cl$ | H | O | $CH_3$ | cyclopropyl | $OCH_3$ | $OCH_2CH_3$ | N | |
| $CH_2Cl$ | H | O | $CH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_2C{\equiv}CH$ | H | O | $CH_3$ | cyclopropyl | $CH_3$ | $CH_3$ | CH | |
| $CH_2C{\equiv}CH$ | H | O | $CH_3$ | cyclopropyl | $CH_3$ | $OCH_3$ | CH | |
| $CH_2C{\equiv}CH$ | H | O | $CH_3$ | cyclopropyl | $OCH_3$ | $OCH_3$ | CH | |
| $CH_2C{\equiv}CH$ | H | O | $CH_3$ | cyclopropyl | $CH_3$ | $CH_3$ | N | |
| $CH_2C{\equiv}CH$ | H | O | $CH_3$ | cyclopropyl | $CH_3$ | $OCH_3$ | N | |
| $CH_2C{\equiv}CH$ | H | O | $CH_3$ | cyclopropyl | $OCH_3$ | $OCH_3$ | N | |
| $CH_2C{\equiv}CH$ | H | O | $CH_3$ | cyclopropyl | Cl | $OCH_3$ | CH | |
| $CH_2C{\equiv}CH$ | H | O | $CH_3$ | cyclobutyl | $CH_3$ | $CH_3$ | CH | |
| $CH_2C{\equiv}CH$ | H | O | $CH_3$ | cyclobutyl | $CH_3$ | $OCH_3$ | CH | |
| $CH_2C{\equiv}CH$ | H | O | $CH_3$ | cyclobutyl | $OCH_3$ | $OCH_3$ | CH | |
| $CH_2C{\equiv}CH$ | H | O | $CH_3$ | cyclobutyl | $CH_3$ | $CH_3$ | N | |
| $CH_2C{\equiv}CH$ | H | O | $CH_3$ | cyclobutyl | $CH_3$ | $OCH_3$ | N | |
| $CH_2C{\equiv}CH$ | H | O | $CH_3$ | cyclobutyl | $OCH_3$ | $OCH_3$ | N | |
| $CH_2C{\equiv}CH$ | H | O | $CH_3$ | cyclobutyl | Cl | $OCH_3$ | CH | |
| $CH_2C{\equiv}CH$ | H | O | $CH_3$ | cyclopentyl | $CH_3$ | $CH_3$ | CH | |
| $CH_2C{\equiv}CH$ | H | O | $CH_3$ | cyclopentyl | $CH_3$ | $OCH_3$ | CH | |
| $CH_2C{\equiv}CH$ | H | O | $CH_3$ | cyclopentyl | $OCH_3$ | $OCH_3$ | CH | |
| $CH_2C{\equiv}CH$ | H | O | $CH_3$ | cyclopentyl | $CH_3$ | $CH_3$ | N | |
| $CH_2C{\equiv}CH$ | H | O | $CH_3$ | cyclopentyl | $CH_3$ | $OCH_3$ | N | |
| $CH_2C{\equiv}CH$ | H | O | $CH_3$ | cyclopentyl | $OCH_3$ | $OCH_3$ | N | |
| $CH_2C{\equiv}CH$ | H | O | $CH_3$ | cyclopentyl | Cl | $OCH_3$ | CH | |
| $CH_2C{\equiv}CH$ | H | O | $CH_3$ | cyclopropyl | $OCH_3$ | $OCH_2CH_3$ | CH | |
| $CH_2C{\equiv}CH$ | H | O | $CH_3$ | cyclopropyl | cyclopropyl | $OCH_3$ | CH | |
| $CH_2C{\equiv}CH$ | H | O | $CH_3$ | cyclopropyl | $OCH_3$ | $CH(OCH_3)_2$ | CH | |
| $CH_2C{\equiv}CH$ | H | O | $CH_3$ | cyclopropyl | $NHCH_3$ | $OCH_2CH_3$ | N | |
| $CH_2C{\equiv}CH$ | H | O | $CH_3$ | cyclopropyl | $NHCH_3$ | $OCH_3$ | N | |
| $CH_2C{\equiv}CH$ | H | O | $CH_3$ | cyclopropyl | $OCH_3$ | $OCH_2CH_3$ | N | |
| $CH_2C{\equiv}CH$ | H | O | $CH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 1 | H | cyclopropyl | $CH_3$ | $CH_3$ | CH | |
| $CH_3$ | H | 1 | H | cyclopropyl | $CH_3$ | $OCH_3$ | CH | |
| $CH_3$ | H | 1 | H | cyclopropyl | $OCH_3$ | $OCH_3$ | CH | |
| $CH_3$ | H | 1 | H | cyclopropyl | $CH_3$ | $CH_3$ | N | |
| $CH_3$ | H | 1 | H | cyclopropyl | $CH_3$ | $OCH_3$ | N | |
| $CH_3$ | H | 1 | H | cyclopropyl | $OCH_3$ | $OCH_3$ | N | |
| $CH_3$ | H | 1 | H | cyclopropyl | Cl | $OCH_3$ | CH | |
| $CH_3$ | H | 1 | H | cyclobutyl | $CH_3$ | $CH_3$ | CH | |
| $CH_3$ | H | 1 | H | cyclobutyl | $CH_3$ | $OCH_3$ | CH | |
| $CH_3$ | H | 1 | H | cyclobutyl | $OCH_3$ | $OCH_3$ | CH | |
| $CH_3$ | H | 1 | H | cyclobutyl | $CH_3$ | $CH_3$ | N | |
| $CH_3$ | H | 1 | H | cyclobutyl | $CH_3$ | $OCH_3$ | N | |
| $CH_3$ | H | 1 | H | cyclobutyl | $OCH_3$ | $OCH_3$ | N | |
| $CH_3$ | H | 1 | H | cyclobutyl | Cl | $OCH_3$ | CH | |
| $CH_3$ | H | 1 | H | cyclopentyl | $CH_3$ | $CH_3$ | CH | |
| $CH_3$ | H | 1 | H | cyclopentyl | $CH_3$ | $OCH_3$ | CH | |
| $CH_3$ | H | 1 | H | cyclopentyl | $OCH_3$ | $OCH_3$ | CH | |
| $CH_3$ | H | 1 | H | cyclopentyl | $CH_3$ | $CH_3$ | N | |
| $CH_3$ | H | 1 | H | cyclopentyl | $CH_3$ | $OCH_3$ | N | |
| $CH_3$ | H | 1 | H | cyclopentyl | $OCH_3$ | $OCH_3$ | N | |
| $CH_3$ | H | 1 | H | cyclopentyl | Cl | $OCH_3$ | CH | |
| $CH_3$ | H | 1 | H | cyclopropyl | $OCH_3$ | $OCH_2CH_3$ | CH | |
| $CH_3$ | H | 1 | H | cyclopropyl | cyclopropyl | $OCH_3$ | CH | |
| $CH_3$ | H | 1 | H | cyclopropyl | $OCH_3$ | $CH(OCH_3)_2$ | CH | |
| $CH_3$ | H | 1 | H | cyclopropyl | $NHCH_3$ | $OCH_2CH_3$ | N | |
| $CH_3$ | H | 1 | H | cyclopropyl | $NHCH_3$ | $OCH_3$ | N | |
| $CH_3$ | H | 1 | H | cyclopropyl | $OCH_3$ | $OCH_2CH_3$ | N | |
| $CH_3$ | H | 1 | H | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 1 | Cl | cyclopropyl | $CH_3$ | $CH_3$ | CH | |
| $CH_3$ | H | 1 | Cl | cyclopropyl | $CH_3$ | $OCH_3$ | CH | |
| $CH_3$ | H | 1 | Cl | cyclopropyl | $OCH_3$ | $OCH_3$ | CH | |
| $CH_3$ | H | 1 | Cl | cyclopropyl | $CH_3$ | $CH_3$ | N | |
| $CH_3$ | H | 1 | Cl | cyclopropyl | $CH_3$ | $OCH_3$ | N | |
| $CH_3$ | H | 1 | Cl | cyclopropyl | $OCH_3$ | $OCH_3$ | N | |
| $CH_3$ | H | 1 | Cl | cyclopropyl | Cl | $OCH_3$ | CH | |
| $CH_3$ | H | 1 | Cl | cyclobutyl | $CH_3$ | $CH_3$ | CH | |
| $CH_3$ | H | 1 | Cl | cyclobutyl | $CH_3$ | $OCH_3$ | CH | |
| $CH_3$ | H | 1 | Cl | cyclobutyl | $OCH_3$ | $OCH_3$ | CH | |
| $CH_3$ | H | 1 | Cl | cyclobutyl | $CH_3$ | $CH_3$ | N | |
| $CH_3$ | H | 1 | Cl | cyclobutyl | $CH_3$ | $OCH_3$ | N | |
| $CH_3$ | H | 1 | Cl | cyclobutyl | $OCH_3$ | $OCH_3$ | N | |
| $CH_3$ | H | 1 | Cl | cyclobutyl | Cl | $OCH_3$ | CH | |
| $CH_3$ | H | 1 | Cl | cyclopentyl | $CH_3$ | $CH_3$ | CH | |
| $CH_3$ | H | 1 | Cl | cyclopentyl | $CH_3$ | $OCH_3$ | CH | |
| $CH_3$ | H | 1 | Cl | cyclopentyl | $OCH_3$ | $OCH_3$ | CH | |
| $CH_3$ | H | 1 | Cl | cyclopentyl | $CH_3$ | $CH_3$ | N | |
| $CH_3$ | H | 1 | Cl | cyclopentyl | $CH_3$ | $OCH_3$ | N | |
| $CH_3$ | H | 1 | Cl | cyclopentyl | $OCH_3$ | $OCH_3$ | N | |
| $CH_3$ | H | 1 | Cl | cyclopentyl | Cl | $OCH_3$ | CH | |
| $CH_3$ | H | 1 | Cl | cyclopropyl | $OCH_3$ | $OCH_2CH_3$ | CH | |
| $CH_3$ | H | 1 | Cl | cyclopropyl | cyclopropyl | $OCH_3$ | CH | |
| $CH_3$ | H | 1 | Cl | cyclopropyl | $OCH_3$ | $CH(OCH_3)_2$ | CH | |
| $CH_3$ | H | 1 | Cl | cyclopropyl | $NHCH_3$ | $OCH_2CH_3$ | N | |
| $CH_3$ | H | 1 | Cl | cyclopropyl | $NHCH_3$ | $OCH_3$ | N | |
| $CH_3$ | H | 1 | Cl | cyclopropyl | $OCH_3$ | $OCH_2CH_3$ | N | |
| $CH_3$ | H | 1 | Cl | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 1 | $CH_3$ | cylopropyl | $CH_3$ | $CH_3$ | CH | |
| $CH_3$ | H | 1 | $CH_3$ | cyclopropyl | $CH_3$ | $OCH_3$ | CH | |
| $CH_3$ | H | 1 | $CH_3$ | cyclopropyl | $OCH_3$ | $OCH_3$ | CH | |
| $CH_3$ | H | 1 | $CH_3$ | cyclopropyl | $CH_3$ | $CH_3$ | N | |
| $CH_3$ | H | 1 | $CH_3$ | cyclopropyl | $CH_3$ | $OCH_3$ | N | |
| $CH_3$ | H | 1 | $CH_3$ | cyclopropyl | $OCH_3$ | $OCH_3$ | N | |
| $CH_3$ | H | 1 | $CH_3$ | cyclopropyl | Cl | $OCH_3$ | CH | |
| $CH_3$ | H | 1 | $CH_3$ | cyclobutyl | $CH_3$ | $CH_3$ | CH | |
| $CH_3$ | H | 1 | $CH_3$ | cyclobutyl | $CH_3$ | $OCH_3$ | CH | |
| $CH_3$ | H | 1 | $CH_3$ | cyclobutyl | $OCH_3$ | $OCH_3$ | CH | |
| $CH_3$ | H | 1 | $CH_3$ | cyclobutyl | $CH_3$ | $CH_3$ | N | |
| $CH_3$ | H | 1 | $CH_3$ | cyclobutyl | $CH_3$ | $OCH_3$ | N | |
| $CH_3$ | H | 1 | $CH_3$ | cyclobutyl | $OCH_3$ | $OCH_3$ | N | |
| $CH_3$ | H | 1 | $CH_3$ | cyclobutyl | Cl | $OCH_3$ | CH | |
| $CH_3$ | H | 1 | $CH_3$ | cyclopentyl | $CH_3$ | $CH_3$ | CH | |
| $CH_3$ | H | 1 | $CH_3$ | cyclopentyl | $CH_3$ | $OCH_3$ | CH | |
| $CH_3$ | H | 1 | $CH_3$ | cyclopentyl | $OCH_3$ | $OCH_3$ | CH | |
| $CH_3$ | H | 1 | $CH_3$ | cyclopentyl | $CH_3$ | $CH_3$ | N | |
| $CH_3$ | H | 1 | $CH_3$ | cyclopentyl | $CH_3$ | $OCH_3$ | N | |
| $CH_3$ | H | 1 | $CH_3$ | cyclopentyl | $OCH_3$ | $OCH_3$ | N | |
| $CH_3$ | H | 1 | $CH_3$ | cyclopentyl | Cl | $OCH_3$ | CH | |
| $CH_3$ | H | 1 | $CH_3$ | cyclopropyl | $OCH_3$ | $OCH_2CH_3$ | CH | |
| $CH_3$ | H | 1 | $CH_3$ | cyclopropyl | cyclopropyl | $OCH_3$ | CH | |
| $CH_3$ | H | 1 | $CH_3$ | cyclopropyl | $OCH_3$ | $CH(OCH_3)_2$ | CH | |
| $CH_3$ | H | 1 | $CH_3$ | cyclopropyl | $NHCH_3$ | $OCH_2CH_3$ | N | |
| $CH_3$ | H | 1 | $CH_3$ | cyclopropyl | $NHCH_3$ | $OCH_3$ | N | |
| $CH_3$ | H | 1 | $CH_3$ | cyclopropyl | $OCH_3$ | $OCH_2CH_3$ | N | |
| $CH_3$ | H | 1 | $CH_3$ | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | $CH_3$ | O | H | cylopropyl | $CH_3$ | $CH_3$ | CH | |
| $CH_3$ | $CH_3$ | O | H | cyclopropyl | $CH_3$ | $OCH_3$ | CH | |
| $CH_3$ | $CH_3$ | O | H | cyclopropyl | $OCH_3$ | $OCH_3$ | CH | |
| $CH_3$ | $CH_3$ | O | H | cyclopropyl | $CH_3$ | $CH_3$ | N | |
| $CH_3$ | $CH_3$ | O | H | cyclopropyl | $CH_3$ | $OCH_3$ | N | |
| $CH_3$ | $CH_3$ | O | H | cyclopropyl | $OCH_3$ | $OCH_3$ | N | |
| $CH_3$ | $CH_3$ | O | H | cyclopropyl | Cl | $OCH_3$ | CH | |
| $CH_3$ | $CH_3$ | O | H | cyclobutyl | $CH_3$ | $CH_3$ | CH | |
| $CH_3$ | $CH_3$ | O | H | cyclobutyl | $CH_3$ | $OCH_3$ | CH | |
| $CH_3$ | $CH_3$ | O | H | cyclobutyl | $OCH_3$ | $OCH_3$ | CH | |
| $CH_3$ | $CH_3$ | O | H | cyclobutyl | $CH_3$ | $CH_3$ | N | |
| $CH_3$ | $CH_3$ | O | H | cyclobutyl | $CH_3$ | $OCH_3$ | N | |
| $CH_3$ | $CH_3$ | O | H | cyclobutyl | $OCH_3$ | $OCH_3$ | N | |
| $CH_3$ | $CH_3$ | O | H | cyclobutyl | Cl | $OCH_3$ | CH | |
| $CH_3$ | $CH_3$ | O | H | cyclopentyl | $CH_3$ | $CH_3$ | CH | |
| $CH_3$ | $CH_3$ | O | H | cyclopentyl | $CH_3$ | $OCH_3$ | CH | |
| $CH_3$ | $CH_3$ | O | H | cyclopentyl | $OCH_3$ | $OCH_3$ | CH | |
| $CH_3$ | $CH_3$ | O | H | cyclopentyl | $CH_3$ | $CH_3$ | N | |
| $CH_3$ | $CH_3$ | O | H | cyclopentyl | $CH_3$ | $OCH_3$ | N | |
| $CH_3$ | $CH_3$ | O | H | cyclopentyl | $OCH_3$ | $OCH_3$ | N | |
| $CH_3$ | $CH_3$ | O | H | cyclopentyl | Cl | $OCH_3$ | CH | |
| $CH_3$ | $CH_3$ | O | H | cyclopropyl | $OCH_3$ | $OCH_2CH_3$ | CH | |
| $CH_3$ | $CH_3$ | O | H | cyclopropyl | cyclopropyl | $OCH_3$ | CH | |
| $CH_3$ | $CH_3$ | O | H | cyclopropyl | $OCH_3$ | $CH(OCH_3)_2$ | CH | |
| $CH_3$ | $CH_3$ | O | H | cyclopropyl | $NHCH_3$ | $OCH_2CH_3$ | N | |
| $CH_3$ | $CH_3$ | O | H | cyclopropyl | $NHCH_3$ | $OCH_3$ | N | |
| $CH_3$ | $CH_3$ | O | H | cyclopropyl | $OCH_3$ | $OCH_2CH_3$ | N | |
| $CH_3$ | $CH_3$ | O | H | cyclopropyl | $CH_2F$ | $CH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | O | H | cylopropyl | $CH_2CH_3$ | $CH_3$ | CH | |
| $CH_3$ | H | O | H | cyclopropyl | $CH_2CH_3$ | $OCH_3$ | CH | |
| $CH_3$ | H | O | H | cyclopropyl | $CH_2F$ | $CH_3$ | CH | |
| $CH_3$ | H | O | H | cyclopropyl | $OCF_2H$ | $CH_3$ | CH | |
| $CH_3$ | H | O | H | cyclopropyl | $OCF_2H$ | $OCH_3$ | CH | |
| $CH_3$ | H | O | H | cyclopropyl | $OCH_2CF_3$ | $OCH_3$ | CH | |
| $CH_3$ | H | O | H | cyclopropyl | $CH_2F$ | $OCH_3$ | CH | |
| $CH_3$ | H | O | H | cyclopropyl | $CH_2Cl$ | $OCH_3$ | N | |
| $CH_3$ | H | O | H | cyclopropyl | $SCH_3$ | $OCH_3$ | CH | |
| $CH_3$ | H | O | H | cyclopropyl | $SCH_2F$ | $OCH_3$ | CH | |
| $CH_3$ | H | O | H | cyclopropyl | Br | $OCH_3$ | CH | |
| $CH_3$ | H | O | H | cyclopropyl | $CH_2OCH_3$ | $OCH_3$ | N | |
| $CH_3$ | H | O | H | cyclopropyl | $OCH_2OCH_3$ | $OCH_3$ | CH | |
| $CH_3$ | H | O | H | cyclopropyl | $OCH_2OCH_2CH_3$ | $CH_3$ | CH | |
| $CH_3$ | H | O | H | cyclopropyl | $N(CH_3)_2$ | $OCH_3$ | N | |
| $CH_3$ | H | O | H | cyclopropyl | $NHCH_2CH_3$ | $CH_3$ | N | |
| $CH_3$ | H | O | H | cyclopropyl | $NHCH_3$ | $OCH_2CH_3$ | N | |
| $CH_3$ | H | O | H | cyclopropyl | $CH_3$ | $CH_2SCH_3$ | CH | |
| $CH_3$ | H | O | H | cyclopropyl | $OCH_3$ | $CH_2SO_2CH_3$ | CH | |
| $CH_3$ | H | O | H | cyclopropyl | $NH_2$ | $OCH_2CH_3$ | N | |
| $CH_3$ | H | O | H | cyclopropyl | $CH_3$ | $OCH_2CH{=}CH_2$ | CH | |
| $CH_3$ | H | O | H | cyclopropyl | $CH_2CH_3$ | $OCF_2H$ | CH | |
| $CH_3$ | H | O | H | cyclopropyl | $OCF_2H$ | $OCF_2H$ | CH | |
| $CH_3$ | H | O | H | cyclopropyl | $OCH_3$ | $OCH{=}CH_2$ | CH | |
| $CH_3$ | H | O | H | cyclopropyl | $OCH_3$ | $C(O)CH_3$ | N | |
| $CH_3$ | H | O | H | cyclopropyl | $CH_3$ | $N(OCH_3)CH_3$ | N | |
| $CH_3$ | H | O | H | cyclopropyl | $OCH(CH_3)_2$ | $OCF_2H$ | CH | |

Table I (cont.)

| R | $R_1$ | n | $R_2$ | R' | X | Y | Z | m.p.(°C) |
|---|---|---|---|---|---|---|---|---|
| $CH_3$ | H | 0 | H | $CH_3$ | $CH_3$ | $CH_3$ | CH | 185-188 |
| $CH_3$ | H | 0 | H | $CH_3$ | $CH_3$ | $OCH_3$ | CH | 180-183 |
| $CH_3$ | H | 0 | H | $CH_3$ | $OCH_3$ | $OCH_3$ | CH | 179-182 |
| $CH_3$ | H | 0 | H | $CH_3$ | $CH_3$ | $CH_3$ | N | |
| $CH_3$ | H | 0 | H | $CH_3$ | $CH_3$ | $OCH_3$ | N | 110-112 |
| $CH_3$ | H | 0 | H | $CH_3$ | $OCH_3$ | $OCH_3$ | N | 163-165 |
| $CH_3$ | H | 0 | H | $CH_3$ | Cl | $OCH_3$ | CH | 199-201 |
| $CH_3$ | H | 0 | H | $CH_2CH_3$ | $CH_3$ | $CH_3$ | CH | 157-161 |
| $CH_3$ | H | 0 | H | $CH_2CH_3$ | $CH_3$ | $OCH_3$ | CH | 151-154 |
| $CH_3$ | H | 0 | H | $CH_2CH_3$ | $OCH_3$ | $OCH_3$ | CH | 143-146 |
| $CH_3$ | H | 0 | H | $CH_2CH_3$ | $CH_3$ | $CH_3$ | N | |
| $CH_3$ | H | 0 | H | $CH_2CH_3$ | $CH_3$ | $OCH_3$ | N | 145-147 |
| $CH_3$ | H | 0 | H | $CH_2CH_3$ | $OCH_3$ | $OCH_3$ | N | 122-137 |
| $CH_3$ | H | 0 | H | $CH_2CH_3$ | Cl | $OCH_3$ | CH | 180-182 |
| $CH_3$ | H | 0 | H | $CH_2CH_2CH_3$ | $CH_3$ | $CH_3$ | CH | 163-165 |
| $CH_3$ | H | 0 | H | $CH_2CH_2CH_3$ | $CH_3$ | $OCH_3$ | CH | 173-175 |
| $CH_3$ | H | 0 | H | $CH_2CH_2CH_3$ | $OCH_3$ | $OCH_3$ | CH | 180-184 |
| $CH_3$ | H | 0 | H | $CH_2CH_2CH_3$ | $CH_3$ | $CH_3$ | N | |
| $CH_3$ | H | 0 | H | $CH_2CH_2CH_3$ | $CH_3$ | $OCH_3$ | N | 174-176 |
| $CH_3$ | H | 0 | H | $CH_2CH_2CH_3$ | $OCH_3$ | $OCH_3$ | N | 154-156 |
| $CH_3$ | H | 0 | H | $CH_2CH_2CH_3$ | Cl | $OCH_3$ | CH | 203-205 |
| $CH_3$ | H | 0 | H | $CH_2CH_2CH_2CH_3$ | $CH_3$ | $CH_3$ | CH | |
| $CH_3$ | H | 0 | H | $CH_2CH_2CH_2CH_3$ | $CH_3$ | $OCH_3$ | CH | |
| $CH_3$ | H | 0 | H | $CH_2CH_2CH_2CH_3$ | $OCH_3$ | $OCH_3$ | CH | |
| $CH_3$ | H | 0 | H | $CH_2CH_2CH_2CH_3$ | $CH_3$ | $CH_3$ | N | |
| $CH_3$ | H | 0 | H | $CH_2CH_2CH_2CH_3$ | $CH_3$ | $OCH_3$ | N | |
| $CH_3$ | H | 0 | H | $CH_2CH_2CH_2CH_3$ | $OCH_3$ | $OCH_3$ | N | |
| $CH_3$ | H | 0 | H | $CH_2CH_2CH_2CH_3$ | Cl | $OCH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 0 | H | $CH_2CH_2CH_2CH_2CH_3$ | $CH_3$ | $CH_3$ | CH | |
| $CH_3$ | H | 0 | H | $CH_2CH_2CH_2CH_2CH_3$ | $CH_3$ | $OCH_3$ | CH | |
| $CH_3$ | H | 0 | H | $CH_2CH_2CH_2CH_2CH_3$ | $OCH_3$ | $OCH_3$ | CH | |
| $CH_3$ | H | 0 | H | $CH_2CH_2CH_2CH_2CH_3$ | $CH_3$ | $CH_3$ | N | |
| $CH_3$ | H | 0 | H | $CH_2CH_2CH_2CH_2CH_3$ | $CH_3$ | $OCH_3$ | N | |
| $CH_3$ | H | 0 | H | $CH_2CH_2CH_2CH_2CH_3$ | $OCH_3$ | $OCH_3$ | N | |
| $CH_3$ | H | 0 | H | $CH_2CH_2CH_2CH_2CH_3$ | Cl | $OCH_3$ | CH | |
| $CH_3$ | H | 0 | H | $CH(CH_3)_2$ | $CH_3$ | $CH_3$ | CH | 148-150 |
| $CH_3$ | H | 0 | H | $CH(CH_3)_2$ | $CH_3$ | $OCH_3$ | CH | 178-180 |
| $CH_3$ | H | 0 | H | $CH(CH_3)_2$ | $OCH_3$ | $OCH_3$ | CH | 181-183 |
| $CH_3$ | H | 0 | H | $CH(CH_3)_2$ | $CH_3$ | $CH_3$ | N | |
| $CH_3$ | H | 0 | H | $CH(CH_3)_2$ | $CH_3$ | $OCH_3$ | N | 152-154 |
| $CH_3$ | H | 0 | H | $CH(CH_3)_2$ | $OCH_3$ | $OCH_3$ | N | 159-161 |
| $CH_3$ | H | 0 | H | $CH(CH_3)_2$ | Cl | $OCH_3$ | CH | 194-196 |
| $CH_3$ | H | 0 | H | $CH_2CH(CH_3)_2$ | $CH_3$ | $CH_3$ | CH | |
| $CH_3$ | H | 0 | H | $CH_2CH(CH_3)_2$ | $CH_3$ | $OCH_3$ | CH | |
| $CH_3$ | H | 0 | H | $CH_2CH(CH_3)_2$ | $OCH_3$ | $OCH_3$ | CH | |
| $CH_3$ | H | 0 | H | $CH_2CH(CH_3)_2$ | $CH_3$ | $CH_3$ | N | |
| $CH_3$ | H | 0 | H | $CH_2CH(CH_3)_2$ | $CH_3$ | $OCH_3$ | N | |
| $CH_3$ | H | 0 | H | $CH_2CH(CH_3)_2$ | $OCH_3$ | $OCH_3$ | N | |
| $CH_3$ | H | 0 | H | $CH_2CH(CH_3)_2$ | Cl | $OCH_3$ | CH | |
| $CH_3$ | H | 0 | H | $CH(CH_3)CH_2CH_3$ | $CH_3$ | $CH_3$ | CH | |
| $CH_3$ | H | 0 | H | $CH(CH_3)CH_2CH_3$ | $CH_3$ | $OCH_3$ | CH | |
| $CH_3$ | H | 0 | H | $CH(CH_3)CH_2CH_3$ | $OCH_3$ | $OCH_3$ | CH | |
| $CH_3$ | H | 0 | H | $CH_2CH_2CH(CH_3)_2$ | $CH_3$ | $CH_3$ | N | |
| $CH_3$ | H | 0 | H | $CH_2CH_2CH(CH_3)_2$ | $CH_3$ | $OCH_3$ | N | |
| $CH_3$ | H | 0 | H | $CH_2CH_2CH(CH_3)_2$ | $OCH_3$ | $OCH_3$ | N | |
| $CH_3$ | H | 0 | H | $CH_2CH_2CH(CH_3)_2$ | Cl | $OCH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 0 | H | $CH_2OCH_3$ | $CH_3$ | $CH_3$ | CH | |
| $CH_3$ | H | 0 | H | $CH_2OCH_3$ | $CH_3$ | $OCH_3$ | CH | |
| $CH_3$ | H | 0 | H | $CH_2OCH_3$ | $OCH_3$ | $OCH_3$ | CH | |
| $CH_3$ | H | 0 | H | $CH_2OCH_3$ | $CH_3$ | $CH_3$ | N | |
| $CH_3$ | H | 0 | H | $CH_2OCH_3$ | $CH_3$ | $OCH_3$ | N | |
| $CH_3$ | H | 0 | H | $CH_2OCH_3$ | $OCH_3$ | $OCH_3$ | N | |
| $CH_3$ | H | 0 | H | $CH_2OCH_3$ | Cl | $OCH_3$ | CH | |
| $CH_3$ | H | 0 | H | $CH_2OCH_2CH_3$ | $CH_3$ | $CH_3$ | CH | |
| $CH_3$ | H | 0 | H | $CH_2OCH_2CH_3$ | $CH_3$ | $OCH_3$ | CH | |
| $CH_3$ | H | 0 | H | $CH_2OCH_2CH_3$ | $OCH_3$ | $OCH_3$ | CH | |
| $CH_3$ | H | 0 | H | $CH_2OCH_2CH_3$ | $CH_3$ | $CH_3$ | N | |
| $CH_3$ | H | 0 | H | $CH_2CH_2OCH_3$ | $CH_3$ | $OCH_3$ | N | |
| $CH_3$ | H | 0 | H | $CH_2CH_2OCH_3$ | $OCH_3$ | $OCH_3$ | N | |
| $CH_3$ | H | 0 | H | $CH_2CH_2OCH_3$ | Cl | $OCH_3$ | CH | |
| $CH_3$ | H | 0 | H | $CH_2CH_2OCH_3$ | $CH_3$ | $CH_3$ | CH | |
| $CH_3$ | H | 0 | H | $CH_2CH_2OCH_3$ | $CH_3$ | $OCH_3$ | CH | |
| $CH_3$ | H | 0 | H | $CH_2CH_2OCH_3$ | $OCH_3$ | $OCH_3$ | CH | |
| $CH_3$ | H | 0 | H | $CH_2CH_2OCH_3$ | $CH_3$ | $CH_3$ | N | |
| $CH_3$ | H | 0 | H | $CH(OCH_3)_2$ | $CH_3$ | $OCH_3$ | N | |
| $CH_3$ | H | 0 | H | $CH(OCH_3)_2$ | $OCH_3$ | $OCH_3$ | N | |
| $CH_3$ | H | 0 | H | $CH(OCH_3)_2$ | Cl | $OCH_3$ | CH | |
| $CH_3$ | H | 0 | H | $CH_2SCH_3$ | $CH_3$ | $CH_3$ | CH | |
| $CH_3$ | H | 0 | H | $CH_2SCH_3$ | $CH_3$ | $OCH_3$ | CH | |
| $CH_3$ | H | 0 | H | $CH_2SCH_3$ | $OCH_3$ | $OCH_3$ | CH | |
| $CH_3$ | H | 0 | H | $CH_2SCH_3$ | $CH_3$ | $CH_3$ | N | |
| $CH_3$ | H | 0 | H | $CH_2SCH_3$ | $CH_3$ | $OCH_3$ | N | |
| $CH_3$ | H | 0 | H | $CH_2SCH_3$ | $OCH_3$ | $OCH_3$ | N | |
| $CH_3$ | H | 0 | H | $CH_2SCH_3$ | Cl | $OCH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 0 | H | $CH_2N(CH_3)_2$ | $CH_3$ | $CH_3$ | CH | |
| $CH_3$ | H | 0 | H | $CH_2N(CH_3)_2$ | $CH_3$ | $OCH_3$ | CH | |
| $CH_3$ | H | 0 | H | $CH_2N(CH_3)_2$ | $OCH_3$ | $OCH_3$ | CH | |
| $CH_3$ | H | 0 | H | $CH_2N(CH_3)_2$ | $CH_3$ | $CH_3$ | N | |
| $CH_3$ | H | 0 | H | $CH_2N(CH_3)_2$ | $CH_3$ | $OCH_3$ | N | |
| $CH_3$ | H | 0 | H | $CH_2N(CH_3)_2$ | $OCH_3$ | $OCH_3$ | N | |
| $CH_3$ | H | 0 | H | $CH_2N(CH_3)_2$ | Cl | $OCH_3$ | CH | |
| $CH_3$ | H | 0 | H | $CH_2CH_2N(CH_3)_2$ | $CH_3$ | $CH_3$ | CH | |
| $CH_3$ | H | 0 | H | $CH_2CH_2N(CH_3)_2$ | $CH_3$ | $OCH_3$ | CH | |
| $CH_3$ | H | 0 | H | $CH_2CH_2N(CH_3)_2$ | $OCH_3$ | $OCH_3$ | CH | |
| $CH_3$ | H | 0 | H | $CH_2CH_2N(CH_3)_2$ | $CH_3$ | $CH_3$ | N | |
| $CH_3$ | H | 0 | H | $CH_2CH_2N(CH_3)_2$ | $CH_3$ | $OCH_3$ | N | |
| $CH_3$ | H | 0 | H | $CH_2CH_2N(CH_3)_2$ | $OCH_3$ | $OCH_3$ | N | |
| $CH_3$ | H | 0 | H | $CH_2CH_2N(CH_3)_2$ | Cl | $OCH_3$ | CH | |
| $CH_3$ | H | 0 | H | $CH_2CH=CH_2$ | $CH_3$ | $CH_3$ | CH | |
| $CH_3$ | H | 0 | H | $CH_2CH=CH_2$ | $CH_3$ | $OCH_3$ | CH | |
| $CH_3$ | H | 0 | H | $CH_2CH=CH_2$ | $OCH_3$ | $OCH_3$ | CH | |
| $CH_3$ | H | 0 | H | $CH_2CH=CH_2$ | $CH_3$ | $CH_3$ | N | |
| $CH_3$ | H | 0 | H | $CH_2CH=CH_2$ | $CH_3$ | $OCH_3$ | N | |
| $CH_3$ | H | 0 | H | $CH_2CH=CH_2$ | $OCH_3$ | $OCH_3$ | N | |
| $CH_3$ | H | 0 | H | $CH_2CH=CH_2$ | Cl | $OCH_3$ | CH | |
| $CH_3$ | H | 0 | H | $CH=CH_2$ | $CH_3$ | $CH_3$ | CH | |
| $CH_3$ | H | 0 | H | $CH=CH_2$ | $CH_3$ | $OCH_3$ | CH | |
| $CH_3$ | H | 0 | H | $CH=CH_2$ | $OCH_3$ | $OCH_3$ | CH | |
| $CH_3$ | H | 0 | H | $CH=CH_2$ | $CH_3$ | $CH_3$ | N | |
| $CH_3$ | H | 0 | H | $CH=CH_2$ | $CH_3$ | $OCH_3$ | N | |
| $CH_3$ | H | 0 | H | $CH=CH_2$ | $OCH_3$ | $OCH_3$ | N | |
| $CH_3$ | H | 0 | H | $CH=CH_2$ | Cl | $OCH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 0 | $CH_3$ | $CH_2OCH_3$ | $CH_3$ | $CH_3$ | CH | |
| $CH_3$ | H | 0 | $CH_3$ | $CH_2OCH_3$ | $CH_3$ | $OCH_3$ | CH | |
| $CH_3$ | H | 0 | $CH_3$ | $CH_2OCH_3$ | $OCH_3$ | $OCH_3$ | CH | |
| $CH_3$ | H | 0 | $CH_3$ | $CH_2OCH_3$ | $CH_3$ | $CH_3$ | N | |
| $CH_3$ | H | 0 | $CH_3$ | $CH_2OCH_3$ | $CH_3$ | $OCH_3$ | N | |
| $CH_3$ | H | 0 | $CH_3$ | $CH_2OCH_3$ | $OCH_3$ | $OCH_3$ | N | |
| $CH_3$ | H | 0 | $CH_3$ | $CH_2OCH_3$ | Cl | $OCH_3$ | CH | |
| $CH_3$ | H | 0 | $CH_3$ | $CH_2OCH_2CH_3$ | $CH_3$ | $CH_3$ | CH | |
| $CH_3$ | H | 0 | $CH_3$ | $CH_2OCH_2CH_3$ | $CH_3$ | $OCH_3$ | CH | |
| $CH_3$ | H | 0 | $CH_3$ | $CH_2OCH_2CH_3$ | $OCH_3$ | $OCH_3$ | CH | |
| $CH_3$ | H | 0 | $CH_3$ | $CH_2OCH_2CH_3$ | $CH_3$ | $CH_3$ | N | |
| $CH_3$ | H | 0 | $CH_3$ | $CH_2CH_2OCH_3$ | $CH_3$ | $OCH_3$ | N | |
| $CH_3$ | H | 0 | $CH_3$ | $CH_2CH_2OCH_3$ | $OCH_3$ | $OCH_3$ | N | |
| $CH_3$ | H | 0 | $CH_3$ | $CH_2CH_2OCH_3$ | Cl | $OCH_3$ | CH | |
| $CH_3$ | H | 0 | $CH_3$ | $CH_2CH_2OCH_3$ | $CH_3$ | $CH_3$ | CH | |
| $CH_3$ | H | 0 | $CH_3$ | $CH_2CH_2OCH_3$ | $CH_3$ | $OCH_3$ | CH | |
| $CH_3$ | H | 0 | $CH_3$ | $CH_2CH_2OCH_3$ | $OCH_3$ | $OCH_3$ | CH | |
| $CH_3$ | H | 0 | $CH_3$ | $CH_2CH_2OCH_3$ | $CH_3$ | $CH_3$ | N | |
| $CH_3$ | H | 0 | $CH_3$ | $CH(OCH_3)_2$ | $CH_3$ | $OCH_3$ | N | |
| $CH_3$ | H | 0 | $CH_3$ | $CH(OCH_3)_2$ | $OCH_3$ | $OCH_3$ | N | |
| $CH_3$ | H | 0 | $CH_3$ | $CH(OCH_3)_2$ | Cl | $OCH_3$ | CH | |
| $CH_3$ | H | 0 | $CH_3$ | $CH_2SCH_3$ | $CH_3$ | $CH_3$ | CH | |
| $CH_3$ | H | 0 | $CH_3$ | $CH_2SCH_3$ | $CH_3$ | $OCH_3$ | CH | |
| $CH_3$ | H | 0 | $CH_3$ | $CH_2SCH_3$ | $OCH_3$ | $OCH_3$ | CH | |
| $CH_3$ | H | 0 | $CH_3$ | $CH_2SCH_3$ | $CH_3$ | $CH_3$ | N | |
| $CH_3$ | H | 0 | $CH_3$ | $CH_2SCH_3$ | $CH_3$ | $OCH_3$ | N | |
| $CH_3$ | H | 0 | $CH_3$ | $CH_2SCH_3$ | $OCH_3$ | $OCH_3$ | N | |
| $CH_3$ | H | 0 | $CH_3$ | $CH_2SCH_3$ | Cl | $OCH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 0 | Cl | $CH_2OCH_2F$ | $CH_3$ | $CH_3$ | CH | |
| $CH_3$ | H | 0 | Cl | $CH_2OCH_2F$ | $CH_3$ | $OCH_3$ | CH | |
| $CH_3$ | H | 0 | Cl | $CH_2OCH_2F$ | $OCH_3$ | $OCH_3$ | CH | |
| $CH_3$ | H | 0 | Cl | $CH_2OCH_2F$ | $CH_3$ | $CH_3$ | N | |
| $CH_3$ | H | 0 | Cl | $CH_2OCH_2F$ | $CH_3$ | $OCH_3$ | N | |
| $CH_3$ | H | 0 | Cl | $CH_2OCH_2F$ | $OCH_3$ | $OCH_3$ | N | |
| $CH_3$ | H | 0 | Cl | $CH_2OCH_2F$ | Cl | $OCH_3$ | CH | |
| $CH_3$ | H | 0 | Cl | $CH_2SOCH_3$ | $CH_3$ | $CH_3$ | CH | |
| $CH_3$ | H | 0 | Cl | $CH_2SOCH_3$ | $CH_3$ | $OCH_3$ | CH | |
| $CH_3$ | H | 0 | Cl | $CH_2SOCH_3$ | $OCH_3$ | $OCH_3$ | CH | |
| $CH_3$ | H | 0 | Cl | $CH_2SOCH_3$ | $CH_3$ | $CH_3$ | N | |
| $CH_3$ | H | 0 | Cl | $CH_2SOCH_3$ | $CH_3$ | $OCH_3$ | N | |
| $CH_3$ | H | 0 | Cl | $CH_2SOCH_3$ | $OCH_3$ | $OCH_3$ | N | |
| $CH_3$ | H | 0 | Cl | $CH_2SOCH_3$ | Cl | $OCH_3$ | CH | |
| $CH_3$ | H | 0 | Cl | $CH_2SO_2CH_3$ | $CH_3$ | $CH_3$ | CH | |
| $CH_3$ | H | 0 | Cl | $CH_2SO_2CH_3$ | $CH_3$ | $OCH_3$ | CH | |
| $CH_3$ | H | 0 | Cl | $CH_2SO_2CH_3$ | $OCH_3$ | $OCH_3$ | CH | |
| $CH_3$ | H | 0 | Cl | $CH_2SO_2CH_3$ | $CH_3$ | $CH_3$ | N | |
| $CH_3$ | H | 0 | Cl | $CH_2SO_2CH_3$ | $CH_3$ | $OCH_3$ | N | |
| $CH_3$ | H | 0 | Cl | $CH_2SO_2CH_3$ | $OCH_3$ | $OCH_3$ | N | |
| $CH_3$ | H | 0 | Cl | $CH_2SO_2CH_3$ | Cl | $OCH_3$ | CH | |
| $CH_3$ | H | 0 | Cl | $CH_2CN$ | $CH_3$ | $CH_3$ | CH | |
| $CH_3$ | H | 0 | Cl | $CH_2CH_2CN$ | $CH_3$ | $OCH_3$ | CH | |
| $CH_3$ | H | 0 | Cl | $CH(CH_3)CN$ | $OCH_3$ | $OCH_3$ | CH | |
| $CH_3$ | H | 0 | Cl | $CH_2NO_2$ | $CH_3$ | $CH_3$ | N | |
| $CH_3$ | H | 0 | Cl | $CH_2CH_2NO_2$ | $CH_3$ | $OCH_3$ | N | |
| $CH_3$ | H | 0 | Cl | $CH_2CN$ | $OCH_3$ | $OCH_3$ | N | |
| $CH_3$ | H | 0 | Cl | $CH_2CN$ | Cl | $OCH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 0 | $CF_3$ | $CH_3$ | $CH_3$ | $CH_3$ | CH | |
| $CH_3$ | H | 0 | $CF_3$ | $CH_3$ | $CH_3$ | $OCH_3$ | CH | |
| $CH_3$ | H | 0 | $CF_3$ | $CH_3$ | $OCH_3$ | $OCH_3$ | CH | |
| $CH_3$ | H | 0 | $CF_3$ | $CH_3$ | $CH_3$ | $CH_3$ | N | |
| $CH_3$ | H | 0 | $CF_3$ | $CH_3$ | $CH_3$ | $OCH_3$ | N | |
| $CH_3$ | H | 0 | $CF_3$ | $CH_3$ | $OCH_3$ | $OCH_3$ | N | |
| $CH_3$ | H | 0 | $CF_3$ | $CH_3$ | Cl | $OCH_3$ | CH | |
| $CH_3$ | H | 0 | $CF_3$ | $CH_2CH_3$ | $CH_3$ | $CH_3$ | CH | |
| $CH_3$ | H | 0 | $CF_3$ | $CH_2CH_3$ | $CH_3$ | $OCH_3$ | CH | |
| $CH_3$ | H | 0 | $CF_3$ | $CH_2CH_3$ | $OCH_3$ | $OCH_3$ | CH | |
| $CH_3$ | H | 0 | $CF_3$ | $CH_2CH_3$ | $CH_3$ | $CH_3$ | N | |
| $CH_3$ | H | 0 | $CF_3$ | $CH_2CH_3$ | $CH_3$ | $OCH_3$ | N | |
| $CH_3$ | H | 0 | $CF_3$ | $CH_2CH_3$ | $OCH_3$ | $OCH_3$ | N | |
| $CH_3$ | H | 0 | $CF_3$ | $CH_2CH_3$ | Cl | $OCH_3$ | CH | |
| $CH_3$ | H | 0 | $CF_3$ | $CH_2CH_2CH_3$ | $CH_3$ | $CH_3$ | CH | |
| $CH_3$ | H | 0 | $CF_3$ | $CH_2CH_2CH_3$ | $CH_3$ | $OCH_3$ | CH | |
| $CH_3$ | H | 0 | $CF_3$ | $CH_2CH_2CH_3$ | $OCH_3$ | $OCH_3$ | CH | |
| $CH_3$ | H | 0 | $CF_3$ | $CH_2CH_2CH_3$ | $CH_3$ | $CH_3$ | N | |
| $CH_3$ | H | 0 | $CF_3$ | $CH_2CH_2CH_3$ | $CH_3$ | $OCH_3$ | N | |
| $CH_3$ | H | 0 | $CF_3$ | $CH_2CH_2CH_3$ | $OCH_3$ | $OCH_3$ | N | |
| $CH_3$ | H | 0 | $CF_3$ | $CH_2CH_2CH_3$ | Cl | $OCH_3$ | CH | |
| $CH_3$ | H | 0 | $CF_3$ | $CH_2CH_2CH_2CH_3$ | $CH_3$ | $CH_3$ | CH | |
| $CH_3$ | H | 0 | $CF_3$ | $CH_2CH_2CH_2CH_3$ | $CH_3$ | $OCH_3$ | CH | |
| $CH_3$ | H | 0 | $CF_3$ | $CH_2CH_2CH_2CH_3$ | $OCH_3$ | $OCH_3$ | CH | |
| $CH_3$ | H | 0 | $CF_3$ | $CH_2CH_2CH_2CH_3$ | $CH_3$ | $CH_3$ | N | |
| $CH_3$ | H | 0 | $CF_3$ | $CH_2CH_2CH_2CH_3$ | $CH_3$ | $OCH_3$ | N | |
| $CH_3$ | H | 0 | $CF_3$ | $CH_2CH_2CH_2CH_3$ | $OCH_3$ | $OCH_3$ | N | |
| $CH_3$ | H | 0 | $CF_3$ | $CH_2CH_2CH_2CH_3$ | Cl | $OCH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 0 | CN | $CH_3$ | $CH_3$ | $CH_3$ | CH | |
| $CH_3$ | H | 0 | CN | $CH_3$ | $CH_3$ | $OCH_3$ | CH | |
| $CH_3$ | H | 0 | CN | $CH_3$ | $OCH_3$ | $OCH_3$ | CH | |
| $CH_3$ | H | 0 | CN | $CH_3$ | $CH_3$ | $CH_3$ | N | |
| $CH_3$ | H | 0 | CN | $CH_3$ | $CH_3$ | $OCH_3$ | N | |
| $CH_3$ | H | 0 | CN | $CH_3$ | $OCH_3$ | $OCH_3$ | N | |
| $CH_3$ | H | 0 | CN | $CH_3$ | Cl | $OCH_3$ | CH | |
| $CH_3$ | H | 0 | CN | $CH_2CH_3$ | $CH_3$ | $CH_3$ | CH | |
| $CH_3$ | H | 0 | CN | $CH_2CH_3$ | $CH_3$ | $OCH_3$ | CH | |
| $CH_3$ | H | 0 | CN | $CH_2CH_3$ | $OCH_3$ | $OCH_3$ | CH | |
| $CH_3$ | H | 0 | CN | $CH_2CH_3$ | $CH_3$ | $CH_3$ | N | |
| $CH_3$ | H | 0 | CN | $CH_2CH_3$ | $CH_3$ | $OCH_3$ | N | |
| $CH_3$ | H | 0 | CN | $CH_2CH_3$ | $OCH_3$ | $OCH_3$ | N | |
| $CH_3$ | H | 0 | CN | $CH_2CH_3$ | Cl | $OCH_3$ | CH | |
| $CH_3$ | H | 0 | CN | $CH_2CH_2CH_3$ | $CH_3$ | $CH_3$ | CH | |
| $CH_3$ | H | 0 | CN | $CH_2CH_2CH_3$ | $CH_3$ | $OCH_3$ | CH | |
| $CH_3$ | H | 0 | CN | $CH_2CH_2CH_3$ | $OCH_3$ | $OCH_3$ | CH | |
| $CH_3$ | H | 0 | CN | $CH_2CH_2CH_3$ | $CH_3$ | $CH_3$ | N | |
| $CH_3$ | H | 0 | CN | $CH_2CH_2CH_3$ | $CH_3$ | $OCH_3$ | N | |
| $CH_3$ | H | 0 | CN | $CH_2CH_2CH_3$ | $OCH_3$ | $OCH_3$ | N | |
| $CH_3$ | H | 0 | CN | $CH_2CH_2CH_3$ | Cl | $OCH_3$ | CH | |
| $CH_3$ | H | 0 | CN | $CH_2CH_2CH_2CH_3$ | $CH_3$ | $CH_3$ | CH | |
| $CH_3$ | H | 0 | CN | $CH_2CH_2CH_2CH_3$ | $CH_3$ | $OCH_3$ | CH | |
| $CH_3$ | H | 0 | CN | $CH_2CH_2CH_2CH_3$ | $OCH_3$ | $OCH_3$ | CH | |
| $CH_3$ | H | 0 | CN | $CH_2CH_2CH_2CH_3$ | $CH_3$ | $CH_3$ | N | |
| $CH_3$ | H | 0 | CN | $CH_2CH_2CH_2CH_3$ | $CH_3$ | $OCH_3$ | N | |
| $CH_3$ | H | 0 | CN | $CH_2CH_2CH_2CH_3$ | $OCH_3$ | $OCH_3$ | N | |
| $CH_3$ | H | 0 | CN | $CH_2CH_2CH_2CH_3$ | Cl | $OCH_3$ | CH | |

Table I (cont.)

| R | $R_1$ | n | $R_2$ | R' | X | Y | Z | m.p.(°C) |
|---|---|---|---|---|---|---|---|---|
| $CH_2CH_3$ | H | 0 | H | $CH_2CH_2CH_2CH_2CH_3$ | $CH_3$ | $CH_3$ | CH | |
| $CH_2CH_3$ | H | 0 | H | $CH_2CH_2CH_2CH_2CH_3$ | $CH_3$ | $OCH_3$ | CH | |
| $CH_2CH_3$ | H | 0 | H | $CH_2CH_2CH_2CH_2CH_3$ | $OCH_3$ | $OCH_3$ | CH | |
| $CH_2CH_3$ | H | 0 | H | $CH_2CH_2CH_2CH_2CH_3$ | $CH_3$ | $CH_3$ | N | |
| $CH_2CH_3$ | H | 0 | H | $CH_2CH_2CH_2CH_2CH_3$ | $CH_3$ | $OCH_3$ | N | |
| $CH_2CH_3$ | H | 0 | H | $CH_2CH_2CH_2CH_2CH_3$ | $OCH_3$ | $OCH_3$ | N | |
| $CH_2CH_3$ | H | 0 | H | $CH_2CH_2CH_2CH_2CH_3$ | Cl | $OCH_3$ | CH | |
| $CH_2CH_3$ | H | 0 | H | $CH(CH_3)_2$ | $CH_3$ | $CH_3$ | CH | |
| $CH_2CH_3$ | H | 0 | H | $CH(CH_3)_2$ | $CH_3$ | $OCH_3$ | CH | |
| $CH_2CH_3$ | H | 0 | H | $CH(CH_3)_2$ | $OCH_3$ | $OCH_3$ | CH | |
| $CH_2CH_3$ | H | 0 | H | $CH(CH_3)_2$ | $CH_3$ | $CH_3$ | N | |
| $CH_2CH_3$ | H | 0 | H | $CH(CH_3)_2$ | $CH_3$ | $OCH_3$ | N | |
| $CH_2CH_3$ | H | 0 | H | $CH(CH_3)_2$ | $OCH_3$ | $OCH_3$ | N | |
| $CH_2CH_3$ | H | 0 | H | $CH(CH_3)_2$ | Cl | $OCH_3$ | CH | |
| $CH_2CH_3$ | H | 0 | H | $CH_2CH(CH_3)_2$ | $CH_3$ | $CH_3$ | CH | |
| $CH_2CH_3$ | H | 0 | H | $CH_2CH(CH_3)_2$ | $CH_3$ | $OCH_3$ | CH | |
| $CH_2CH_3$ | H | 0 | H | $CH_2CH(CH_3)_2$ | $OCH_3$ | $OCH_3$ | CH | |
| $CH_2CH_3$ | H | 0 | H | $CH_2CH(CH_3)_2$ | $CH_3$ | $CH_3$ | N | |
| $CH_2CH_3$ | H | 0 | H | $CH_2CH(CH_3)_2$ | $CH_3$ | $OCH_3$ | N | |
| $CH_2CH_3$ | H | 0 | H | $CH_2CH(CH_3)_2$ | $OCH_3$ | $OCH_3$ | N | |
| $CH_2CH_3$ | H | 0 | H | $CH_2CH(CH_3)_2$ | Cl | $OCH_3$ | CH | |
| $CH_2CH_3$ | H | 0 | H | $CH(CH_3)CH_2CH_3$ | $CH_3$ | $CH_3$ | CH | |
| $CH_2CH_3$ | H | 0 | H | $CH(CH_3)CH_2CH_3$ | $CH_3$ | $OCH_3$ | CH | |
| $CH_2CH_3$ | H | 0 | H | $CH(CH_3)CH_2CH_3$ | $OCH_3$ | $OCH_3$ | CH | |
| $CH_2CH_3$ | H | 0 | H | $CH_2CH_2CH(CH_3)_2$ | $CH_3$ | $CH_3$ | N | |
| $CH_2CH_3$ | H | 0 | H | $CH_2CH_2CH(CH_3)_2$ | $CH_3$ | $OCH_3$ | N | |
| $CH_2CH_3$ | H | 0 | H | $CH_2CH_2CH(CH_3)_2$ | $OCH_3$ | $OCH_3$ | N | |
| $CH_2CH_3$ | H | 0 | H | $CH_2CH_2CH(CH_3)_2$ | Cl | $OCH_3$ | CH | |

Table I (cont.)

| R | $R_1$ | n | $R_2$ | R' | X | Y | Z | m.p.(°C) |
|---|---|---|---|---|---|---|---|---|
| $CH_2OCH_3$ | H | 0 | H | $CH_2CH_2CH_2CH_2CH_3$ | $CH_3$ | $CH_3$ | CH | |
| $CH_2OCH_3$ | H | 0 | H | $CH_2CH_2CH_2CH_2CH_3$ | $CH_3$ | $OCH_3$ | CH | |
| $CH_2OCH_3$ | H | 0 | H | $CH_2CH_2CH_2CH_2CH_3$ | $OCH_3$ | $OCH_3$ | CH | |
| $CH_2OCH_3$ | H | 0 | H | $CH_2CH_2CH_2CH_2CH_3$ | $CH_3$ | $CH_3$ | N | |
| $CH_2OCH_3$ | H | 0 | H | $CH_2CH_2CH_2CH_2CH_3$ | $CH_3$ | $OCH_3$ | N | |
| $CH_2OCH_3$ | H | 0 | H | $CH_2CH_2CH_2CH_2CH_3$ | $OCH_3$ | $OCH_3$ | N | |
| $CH_2OCH_3$ | H | 0 | H | $CH_2CH_2CH_2CH_2CH_3$ | Cl | $OCH_3$ | CH | |
| $CH_2OCH_3$ | H | 0 | H | $CH(CH_3)_2$ | $CH_3$ | $CH_3$ | CH | |
| $CH_2OCH_3$ | H | 0 | H | $CH(CH_3)_2$ | $CH_3$ | $OCH_3$ | CH | |
| $CH_2OCH_3$ | H | 0 | H | $CH(CH_3)_2$ | $OCH_3$ | $OCH_3$ | CH | |
| $CH_2OCH_3$ | H | 0 | H | $CH(CH_3)_2$ | $CH_3$ | $CH_3$ | N | |
| $CH_2OCH_3$ | H | 0 | H | $CH(CH_3)_2$ | $CH_3$ | $OCH_3$ | N | |
| $CH_2OCH_3$ | H | 0 | H | $CH(CH_3)_2$ | $OCH_3$ | $OCH_3$ | N | |
| $CH_2OCH_3$ | H | 0 | H | $CH(CH_3)_2$ | Cl | $OCH_3$ | CH | |
| $CH_2OCH_3$ | H | 0 | H | $CH_2CH(CH_3)_2$ | $CH_3$ | $CH_3$ | CH | |
| $CH_2OCH_3$ | H | 0 | H | $CH_2CH(CH_3)_2$ | $CH_3$ | $OCH_3$ | CH | |
| $CH_2OCH_3$ | H | 0 | H | $CH_2CH(CH_3)_2$ | $OCH_3$ | $OCH_3$ | CH | |
| $CH_2OCH_3$ | H | 0 | H | $CH_2CH(CH_3)_2$ | $CH_3$ | $CH_3$ | N | |
| $CH_2OCH_3$ | H | 0 | H | $CH_2CH(CH_3)_2$ | $CH_3$ | $OCH_3$ | N | |
| $CH_2OCH_3$ | H | 0 | H | $CH_2CH(CH_3)_2$ | $OCH_3$ | $OCH_3$ | N | |
| $CH_2OCH_3$ | H | 0 | H | $CH_2CH(CH_3)_2$ | Cl | $OCH_3$ | CH | |
| $CH_2OCH_3$ | H | 0 | H | $CH(CH_3)CH_2CH_3$ | $CH_3$ | $CH_3$ | CH | |
| $CH_2OCH_3$ | H | 0 | H | $CH(CH_3)CH_2CH_3$ | $CH_3$ | $OCH_3$ | CH | |
| $CH_2OCH_3$ | H | 0 | H | $CH(CH_3)CH_2CH_3$ | $OCH_3$ | $OCH_3$ | CH | |
| $CH_2OCH_3$ | H | 0 | H | $CH_2CH_2CH(CH_3)_2$ | $CH_3$ | $CH_3$ | N | |
| $CH_2OCH_3$ | H | 0 | H | $CH_2CH_2CH(CH_3)_2$ | $CH_3$ | $OCH_3$ | N | |
| $CH_2OCH_3$ | H | 0 | H | $CH_2CH_2CH(CH_3)_2$ | $OCH_3$ | $OCH_3$ | N | |
| $CH_2OCH_3$ | H | 0 | H | $CH_2CH_2CH(CH_3)_2$ | Cl | $OCH_3$ | CH | |

Table I (cont.)

| R | $R_1$ | n | $R_2$ | R' | X | Y | Z | m.p.(°C) |
|---|---|---|---|---|---|---|---|---|
| $CH_3$ | H | 0 | H | $CH_3$ | H | $CH_3$ | CH | |
| $CH_3$ | H | 0 | H | $CH_3$ | H | $OCH_3$ | CH | |
| $CH_3$ | H | 0 | H | $CH_3$ | $CH_3$ | $OCH_2CH_3$ | CH | |
| $CH_3$ | H | 0 | H | $CH_3$ | $CH_3$ | $OCH(CH_3)_2$ | CH | |
| $CH_3$ | H | 0 | H | $CH_3$ | H | $OCH_3$ | N | |
| $CH_3$ | H | 0 | H | $CH_3$ | $OCH_3$ | $OCH_2CH_3$ | CH | |
| $CH_3$ | H | 0 | H | $CH_3$ | $CH_2F$ | $CH_3$ | CH | |
| $CH_3$ | H | 0 | H | $CH_3$ | $CH_2F$ | $OCH_3$ | CH | |
| $CH_3$ | H | 0 | H | $CH_3$ | $OCF_2H$ | $CH_3$ | CH | |
| $CH_3$ | H | 0 | H | $CH_3$ | $OCF_2H$ | $OCH_3$ | CH | |
| $CH_3$ | H | 0 | H | $CH_3$ | $OCH_2CF_3$ | $OCH_3$ | N | |
| $CH_3$ | H | 0 | H | $CH_3$ | $SCH_3$ | $OCH_3$ | CH | |
| $CH_3$ | H | 0 | H | $CH_3$ | $OCH_3$ | $NHCH_3$ | N | |
| $CH_3$ | H | 0 | H | $CH_3$ | $OCH_2CH_3$ | $NHCH_3$ | N | 195-197 |
| $CH_3$ | H | 0 | H | $CH_3$ | $OCH_3$ | $OCH_2CH_3$ | N | |
| $CH_3$ | H | 0 | H | $CH_3$ | $SCF_2H$ | $OCH_3$ | CH | |
| $CH_3$ | H | 0 | H | $CH_3$ | Br | $OCH_3$ | CH | |
| $CH_3$ | H | 0 | H | $CH_3$ | $CH_2OCH_3$ | $OCH_3$ | CH | |
| $CH_3$ | H | 0 | H | $CH_3$ | $CH_2OCH_3$ | $CH_3$ | N | |
| $CH_3$ | H | 0 | H | $CH_3$ | $NH_2$ | $OCH_3$ | N | |
| $CH_3$ | H | 0 | H | $CH_3$ | $N(CH_3)_2$ | $OCH_3$ | N | |
| $CH_3$ | H | 0 | H | $CH_3$ | $NHCH_3$ | $NHCH_3$ | N | |
| $CH_3$ | H | 0 | H | $CH_3$ | cyclopropyl | $OCH_3$ | CH | |
| $CH_3$ | H | 0 | H | $CH_3$ | cyclopropyl | $CH_3$ | N | |
| $CH_3$ | H | 0 | H | $CH_3$ | cyclopropyl | $CH_3$ | CH | |
| $CH_3$ | H | 0 | H | $CH_3$ | cyclopropyl | $OCH_3$ | N | |
| $CH_3$ | H | 0 | H | $CH_3$ | $CH_3$ | $CH(OCH_3)_2$ | CH | |
| $CH_3$ | H | 0 | H | $CH_3$ | $CF_3$ | $OCH_3$ | CH | |
| $CH_3$ | H | 0 | H | $CH_3$ | $OCH_3$ | $C\equiv CH$ | CH | |

Table I (cont.)

E is $CH_2$

| R | $R_1$ | n | $R_2$ | R' | X | Y | Z | m.p.(°C) |
|---|---|---|---|---|---|---|---|---|
| $CH_3$ | H | 0 | H | $CH_3$ | $CH_3$ | $CH_3$ | CH | |
| $CH_3$ | H | 0 | H | $CH_3$ | $CH_3$ | $OCH_3$ | CH | |
| $CH_3$ | H | 0 | H | $CH_3$ | $OCH_3$ | $OCH_3$ | CH | |
| $CH_3$ | H | 0 | H | $CH_3$ | $CH_3$ | $CH_3$ | N | |
| $CH_3$ | H | 0 | H | $CH_3$ | $CH_3$ | $OCH_3$ | N | |
| $CH_3$ | H | 0 | H | $CH_3$ | $OCH_3$ | $OCH_3$ | N | |
| $CH_3$ | H | 0 | H | $CH_3$ | Cl | $OCH_3$ | CH | |
| $CH_3$ | H | 0 | H | $CH_2CH_3$ | $CH_3$ | $CH_3$ | CH | |
| $CH_3$ | H | 0 | H | $CH_2CH_3$ | $CH_3$ | $OCH_3$ | CH | |
| $CH_3$ | H | 0 | H | $CH_2CH_3$ | $OCH_3$ | $OCH_3$ | CH | |
| $CH_3$ | H | 0 | H | $CH_2CH_3$ | $CH_3$ | $CH_3$ | N | |
| $CH_3$ | H | 0 | H | $CH_2CH_3$ | $CH_3$ | $OCH_3$ | N | |
| $CH_3$ | H | 0 | H | $CH_2CH_3$ | $OCH_3$ | $OCH_3$ | N | |
| $CH_3$ | H | 0 | H | $CH_2CH_3$ | Cl | $OCH_3$ | CH | |
| $CH_3$ | H | 0 | H | $CH_2CH_2CH_3$ | $CH_3$ | $CH_3$ | CH | |
| $CH_3$ | H | 0 | H | $CH_2CH_2CH_3$ | $CH_3$ | $OCH_3$ | CH | |
| $CH_3$ | H | 0 | H | $CH_2CH_2CH_3$ | $OCH_3$ | $OCH_3$ | CH | |
| $CH_3$ | H | 0 | H | $CH_2CH_2CH_3$ | $CH_3$ | $CH_3$ | N | |
| $CH_3$ | H | 0 | H | $CH_2CH_2CH_3$ | $CH_3$ | $OCH_3$ | N | |
| $CH_3$ | H | 0 | H | $CH_2CH_2CH_3$ | $OCH_3$ | $OCH_3$ | N | |
| $CH_3$ | H | 0 | H | $CH_2CH_2CH_3$ | Cl | $OCH_3$ | CH | |
| $CH_3$ | H | 0 | H | $CH_2CH_2CH_2CH_3$ | $CH_3$ | $CH_3$ | CH | |
| $CH_3$ | H | 0 | H | $CH_2CH_2CH_2CH_3$ | $CH_3$ | $OCH_3$ | CH | |
| $CH_3$ | H | 0 | H | $CH_2CH_2CH_2CH_3$ | $OCH_3$ | $OCH_3$ | CH | |
| $CH_3$ | H | 0 | H | $CH_2CH_2CH_2CH_3$ | $CH_3$ | $CH_3$ | N | |
| $CH_3$ | H | 0 | H | $CH_2CH_2CH_2CH_3$ | $CH_3$ | $OCH_3$ | N | |
| $CH_3$ | H | 0 | H | $CH_2CH_2CH_2CH_3$ | $OCH_3$ | $OCH_3$ | N | |
| $CH_3$ | H | 0 | H | $CH_2CH_2CH_2CH_3$ | Cl | $OCH_3$ | CH | |

Table I (cont.)

W = S

| R | $R_1$ | n | $R_2$ | R' | X | Y | Z | m.p.(°C) |
|---|---|---|---|---|---|---|---|---|
| $CH_3$ | H | 0 | H | $CH_3$ | $CH_3$ | $CH_3$ | CH | |
| $CH_3$ | H | 0 | H | $CH_3$ | $CH_3$ | $OCH_3$ | CH | |
| $CH_3$ | H | 0 | H | $CH_3$ | $OCH_3$ | $OCH_3$ | CH | |
| $CH_3$ | H | 0 | H | $CH_3$ | $CH_3$ | $CH_3$ | N | |
| $CH_3$ | H | 0 | H | $CH_3$ | $CH_3$ | $OCH_3$ | N | |
| $CH_3$ | H | 0 | H | $CH_3$ | $OCH_3$ | $OCH_3$ | N | |
| $CH_3$ | H | 0 | H | $CH_3$ | Cl | $OCH_3$ | CH | |
| $CH_3$ | H | 0 | H | $CH_2CH_3$ | $CH_3$ | $CH_3$ | CH | |
| $CH_3$ | H | 0 | H | $CH_2CH_3$ | $CH_3$ | $OCH_3$ | CH | |
| $CH_3$ | H | 0 | H | $CH_2CH_3$ | $OCH_3$ | $OCH_3$ | CH | |
| $CH_3$ | H | 0 | H | $CH_2CH_3$ | $CH_3$ | $CH_3$ | N | |
| $CH_3$ | H | 0 | H | $CH_2CH_3$ | $CH_3$ | $OCH_3$ | N | |
| $CH_3$ | H | 0 | H | $CH_2CH_3$ | $OCH_3$ | $OCH_3$ | N | |
| $CH_3$ | H | 0 | H | $CH_2CH_3$ | Cl | $OCH_3$ | CH | |
| $CH_3$ | H | 0 | H | $CH_2CH_2CH_3$ | $CH_3$ | $CH_3$ | CH | |
| $CH_3$ | H | 0 | H | $CH_2CH_2CH_3$ | $CH_3$ | $OCH_3$ | CH | |
| $CH_3$ | H | 0 | H | $CH_2CH_2CH_3$ | $OCH_3$ | $OCH_3$ | CH | |
| $CH_3$ | H | 0 | H | $CH_2CH_2CH_3$ | $CH_3$ | $CH_3$ | N | |
| $CH_3$ | H | 0 | H | $CH_2CH_2CH_3$ | $CH_3$ | $OCH_3$ | N | |
| $CH_3$ | H | 0 | H | $CH_2CH_2CH_3$ | $OCH_3$ | $OCH_3$ | N | |
| $CH_3$ | H | 0 | H | $CH_2CH_2CH_3$ | Cl | $OCH_3$ | CH | |
| $CH_3$ | H | 0 | H | $CH_2CH_2CH_2CH_3$ | $CH_3$ | $CH_3$ | CH | |
| $CH_3$ | H | 0 | H | $CH_2CH_2CH_2CH_3$ | $CH_3$ | $OCH_3$ | CH | |
| $CH_3$ | H | 0 | H | $CH_2CH_2CH_2CH_3$ | $OCH_3$ | $OCH_3$ | CH | |
| $CH_3$ | H | 0 | H | $CH_2CH_2CH_2CH_3$ | $CH_3$ | $CH_3$ | N | |
| $CH_3$ | H | 0 | H | $CH_2CH_2CH_2CH_3$ | $CH_3$ | $OCH_3$ | N | |
| $CH_3$ | H | 0 | H | $CH_2CH_2CH_2CH_3$ | $OCH_3$ | $OCH_3$ | N | |
| $CH_3$ | H | 0 | H | $CH_2CH_2CH_2CH_3$ | Cl | $OCH_3$ | CH | |

Table II

| R | $R_1$ | n | $R_2$ | R' | X | Y | Z | m.p.(°C) |
|---|---|---|---|---|---|---|---|---|
| $CH_3$ | H | O | H | cyclopropyl | $CH_3$ | $CH_3$ | CH | |
| $CH_3$ | H | O | H | cyclopropyl | $CH_3$ | $OCH_3$ | CH | |
| $CH_3$ | H | O | H | cyclopropyl | $OCH_3$ | $OCH_3$ | CH | |
| $CH_3$ | H | O | H | cyclopropyl | $CH_3$ | $CH_3$ | N | |
| $CH_3$ | H | O | H | cyclopropyl | $CH_3$ | $OCH_3$ | N | |
| $CH_3$ | H | O | H | cyclopropyl | $OCH_3$ | $OCH_3$ | N | |
| $CH_3$ | H | O | H | cyclopropyl | Cl | $OCH_3$ | CH | |
| $CH_3$ | H | O | H | cyclobutyl | $CH_3$ | $CH_3$ | CH | |
| $CH_3$ | H | O | H | cyclobutyl | $CH_3$ | $OCH_3$ | CH | |
| $CH_3$ | H | O | H | cyclobutyl | $OCH_3$ | $OCH_3$ | CH | |
| $CH_3$ | H | O | H | cyclobutyl | $CH_3$ | $CH_3$ | N | |
| $CH_3$ | H | O | H | cyclobutyl | $CH_3$ | $OCH_3$ | N | |
| $CH_3$ | H | O | H | cyclobutyl | $OCH_3$ | $OCH_3$ | N | |
| $CH_3$ | H | O | H | cyclobutyl | Cl | $OCH_3$ | CH | |
| $CH_3$ | H | O | H | cyclopentyl | $CH_3$ | $CH_3$ | CH | |
| $CH_3$ | H | O | H | cyclopentyl | $CH_3$ | $OCH_3$ | CH | |
| $CH_3$ | H | O | H | cyclopentyl | $OCH_3$ | $OCH_3$ | CH | |
| $CH_3$ | H | O | H | cyclopentyl | $CH_3$ | $CH_3$ | N | |
| $CH_3$ | H | O | H | cyclopentyl | $CH_3$ | $OCH_3$ | N | |
| $CH_3$ | H | O | H | cyclopentyl | $OCH_3$ | $OCH_3$ | N | |
| $CH_3$ | H | O | H | cyclopentyl | Cl | $OCH_3$ | CH | |
| $CH_3$ | H | O | H | cyclopropyl | $OCH_3$ | $OCH_2CH_3$ | CH | |
| $CH_3$ | H | O | H | cyclopropyl | cyclopropyl | $OCH_3$ | CH | |
| $CH_3$ | H | O | H | cyclopropyl | $OCH_3$ | $CH(OCH_3)_2$ | CH | |
| $CH_3$ | H | O | H | cyclopropyl | $NHCH_3$ | $OCH_2CH_3$ | N | |
| $CH_3$ | H | O | H | cyclopropyl | $NHCH_3$ | $OCH_3$ | N | |
| $CH_3$ | H | O | H | cyclopropyl | $OCH_3$ | $OCH_2CH_3$ | N | |
| $CH_3$ | H | O | H | cyclopropyl | $CH_2F$ | $CH_3$ | CH | |

Table II (cont.)

| R | $R_1$ | n | $R_2$ | R' | X | Y | Z | m.p.(°C) |
|---|---|---|---|---|---|---|---|---|
| $CH_3$ | H | O | $CH_2F$ | cyclopropyl | $CH_3$ | $CH_3$ | CH | |
| $CH_3$ | H | O | $CH_2F$ | cyclopropyl | $CH_3$ | $OCH_3$ | CH | |
| $CH_3$ | H | O | $CH_2F$ | cyclopropyl | $OCH_3$ | $OCH_3$ | CH | |
| $CH_3$ | H | O | $CH_2F$ | cyclopropyl | $CH_3$ | $CH_3$ | N | |
| $CH_3$ | H | O | $CH_2F$ | cyclopropyl | $CH_3$ | $OCH_3$ | N | |
| $CH_3$ | H | O | $CH_2F$ | cyclopropyl | $OCH_3$ | $OCH_3$ | N | |
| $CH_3$ | H | O | $CH_2F$ | cyclopropyl | Cl | $OCH_3$ | CH | |
| $CH_3$ | H | O | $CH_2F$ | cyclobutyl | $CH_3$ | $CH_3$ | CH | |
| $CH_3$ | H | O | $CH_2F$ | cyclobutyl | $CH_3$ | $OCH_3$ | CH | |
| $CH_3$ | H | O | $CH_2F$ | cyclobutyl | $OCH_3$ | $OCH_3$ | CH | |
| $CH_3$ | H | O | $CH_2F$ | cyclobutyl | $CH_3$ | $CH_3$ | N | |
| $CH_3$ | H | O | $CH_2F$ | cyclobutyl | $CH_3$ | $OCH_3$ | N | |
| $CH_3$ | H | O | $CH_2F$ | cyclobutyl | $OCH_3$ | $OCH_3$ | N | |
| $CH_3$ | H | O | $CH_2F$ | cyclobutyl | Cl | $OCH_3$ | CH | |
| $CH_3$ | H | O | $CH_2F$ | cyclopentyl | $CH_3$ | $CH_3$ | CH | |
| $CH_3$ | H | O | $CH_2F$ | cyclopentyl | $CH_3$ | $OCH_3$ | CH | |
| $CH_3$ | H | O | $CH_2F$ | cyclopentyl | $OCH_3$ | $OCH_3$ | CH | |
| $CH_3$ | H | O | $CH_2F$ | cyclopentyl | $CH_3$ | $CH_3$ | N | |
| $CH_3$ | H | O | $CH_2F$ | cyclopentyl | $CH_3$ | $OCH_3$ | N | |
| $CH_3$ | H | O | $CH_2F$ | cyclopentyl | $OCH_3$ | $OCH_3$ | N | |
| $CH_3$ | H | O | $CH_2F$ | cyclopentyl | Cl | $OCH_3$ | CH | |
| $CH_3$ | H | O | $CH_2F$ | cyclopropyl | $OCH_3$ | $OCH_2CH_3$ | CH | |
| $CH_3$ | H | O | $CH_2F$ | cyclopropyl | cyclopropyl | $OCH_3$ | CH | |
| $CH_3$ | H | O | $CH_2F$ | cyclopropyl | $OCH_3$ | $CH(OCH_3)_2$ | CH | |
| $CH_3$ | H | O | $CH_2F$ | cyclopropyl | $NHCH_3$ | $OCH_2CH_3$ | N | |
| $CH_3$ | H | O | $CH_2F$ | cyclopropyl | $NHCH_3$ | $OCH_3$ | N | |
| $CH_3$ | H | O | $CH_2F$ | cyclopropyl | $OCH_3$ | $OCH_2CH_3$ | N | |
| $CH_3$ | H | O | $CH_2F$ | cyclopropyl | $CH_2F$ | $CH_3$ | CH | |

Table II (cont.)

| R | $R_1$ | n | $R_2$ | R' | X | Y | Z | m.p.(°C) |
|---|---|---|---|---|---|---|---|---|
| $CH_3$ | H | O | CN | cyclopropyl | $CH_3$ | $CH_3$ | CH | |
| $CH_3$ | H | O | CN | cyclopropyl | $CH_3$ | $OCH_3$ | CH | |
| $CH_3$ | H | O | CN | cyclopropyl | $OCH_3$ | $OCH_3$ | CH | |
| $CH_3$ | H | O | CN | cyclopropyl | $CH_3$ | $CH_3$ | N | |
| $CH_3$ | H | O | CN | cyclopropyl | $CH_3$ | $OCH_3$ | N | |
| $CH_3$ | H | O | CN | cyclopropyl | $OCH_3$ | $OCH_3$ | N | |
| $CH_3$ | H | O | CN | cyclopropyl | Cl | $OCH_3$ | CH | |
| $CH_3$ | H | O | CN | cyclobutyl | $CH_3$ | $CH_3$ | CH | |
| $CH_3$ | H | O | CN | cyclobutyl | $CH_3$ | $OCH_3$ | CH | |
| $CH_3$ | H | O | CN | cyclobutyl | $OCH_3$ | $OCH_3$ | CH | |
| $CH_3$ | H | O | CN | cyclobutyl | $CH_3$ | $CH_3$ | N | |
| $CH_3$ | H | O | CN | cyclobutyl | $CH_3$ | $OCH_3$ | N | |
| $CH_3$ | H | O | CN | cyclobutyl | $OCH_3$ | $OCH_3$ | N | |
| $CH_3$ | H | O | CN | cyclobutyl | Cl | $OCH_3$ | CH | |
| $CH_3$ | H | O | CN | cyclopentyl | $CH_3$ | $CH_3$ | CH | |
| $CH_3$ | H | O | CN | cyclopentyl | $CH_3$ | $OCH_3$ | CH | |
| $CH_3$ | H | O | CN | cyclopentyl | $OCH_3$ | $OCH_3$ | CH | |
| $CH_3$ | H | O | CN | cyclopentyl | $CH_3$ | $CH_3$ | N | |
| $CH_3$ | H | O | CN | cyclopentyl | $CH_3$ | $OCH_3$ | N | |
| $CH_3$ | H | O | CN | cyclopentyl | $OCH_3$ | $OCH_3$ | N | |
| $CH_3$ | H | O | CN | cyclopentyl | Cl | $OCH_3$ | CH | |
| $CH_3$ | H | O | CN | cyclopropyl | $OCH_3$ | $OCH_2CH_3$ | CH | |
| $CH_3$ | H | O | CN | cyclopropyl | cyclopropyl | $OCH_3$ | CH | |
| $CH_3$ | H | O | CN | cyclopropyl | $OCH_3$ | $CH(OCH_3)_2$ | CH | |
| $CH_3$ | H | O | CN | cyclopropyl | $NHCH_3$ | $OCH_2CH_3$ | N | |
| $CH_3$ | H | O | CN | cyclopropyl | $NHCH_3$ | $OCH_3$ | N | |
| $CH_3$ | H | O | CN | cyclopropyl | $OCH_3$ | $OCH_2CH_3$ | N | |
| $CH_3$ | H | O | CN | cyclopropyl | $CH_2F$ | $CH_3$ | CH | |

Table II (cont.)

| R | $R_1$ | n | $R_2$ | R' | X | Y | Z | m.p.(°C) |
|---|---|---|---|---|---|---|---|---|
| $CH_2CH_3$ | H | O | H | cyclopropyl | $CH_3$ | $CH_3$ | CH | |
| $CH_2CH_3$ | H | O | H | cyclopropyl | $CH_3$ | $OCH_3$ | CH | |
| $CH_2CH_3$ | H | O | H | cyclopropyl | $OCH_3$ | $OCH_3$ | CH | |
| $CH_2CH_3$ | H | O | H | cyclopropyl | $CH_3$ | $CH_3$ | N | |
| $CH_2CH_3$ | H | O | H | cyclopropyl | $CH_3$ | $OCH_3$ | N | |
| $CH_2CH_3$ | H | O | H | cyclopropyl | $OCH_3$ | $OCH_3$ | N | |
| $CH_2CH_3$ | H | O | H | cyclopropyl | Cl | $OCH_3$ | CH | |
| $CH_2CH_3$ | H | O | H | cyclobutyl | $CH_3$ | $CH_3$ | CH | |
| $CH_2CH_3$ | H | O | H | cyclobutyl | $CH_3$ | $OCH_3$ | CH | |
| $CH_2CH_3$ | H | O | H | cyclobutyl | $OCH_3$ | $OCH_3$ | CH | |
| $CH_2CH_3$ | H | O | H | cyclobutyl | $CH_3$ | $CH_3$ | N | |
| $CH_2CH_3$ | H | O | H | cyclobutyl | $CH_3$ | $OCH_3$ | N | |
| $CH_2CH_3$ | H | O | H | cyclobutyl | $OCH_3$ | $OCH_3$ | N | |
| $CH_2CH_3$ | H | O | H | cyclobutyl | Cl | $OCH_3$ | CH | |
| $CH_2CH_3$ | H | O | H | cyclopentyl | $CH_3$ | $CH_3$ | CH | |
| $CH_2CH_3$ | H | O | H | cyclopentyl | $CH_3$ | $OCH_3$ | CH | |
| $CH_2CH_3$ | H | O | H | cyclopentyl | $OCH_3$ | $OCH_3$ | CH | |
| $CH_2CH_3$ | H | O | H | cyclopentyl | $CH_3$ | $CH_3$ | N | |
| $CH_2CH_3$ | H | O | H | cyclopentyl | $CH_3$ | $OCH_3$ | N | |
| $CH_2CH_3$ | H | O | H | cyclopentyl | $OCH_3$ | $OCH_3$ | N | |
| $CH_2CH_3$ | H | O | H | cyclopentyl | Cl | $OCH_3$ | CH | |
| $CH_2CH_3$ | H | O | H | cyclopropyl | $OCH_3$ | $OCH_2CH_3$ | CH | |
| $CH_2CH_3$ | H | O | H | cyclopropyl | cyclopropyl | $OCH_3$ | CH | |
| $CH_2CH_3$ | H | O | H | cyclopropyl | $OCH_3$ | $CH(OCH_3)_2$ | CH | |
| $CH_2CH_3$ | H | O | H | cyclopropyl | $NHCH_3$ | $OCH_2CH_3$ | N | |
| $CH_2CH_3$ | H | O | H | cyclopropyl | $NHCH_3$ | $OCH_3$ | N | |
| $CH_2CH_3$ | H | O | H | cyclopropyl | $OCH_3$ | $OCH_2CH_3$ | N | |
| $CH_2CH_3$ | H | O | H | cyclopropyl | $CH_2F$ | $CH_3$ | CH | |

Table II (cont.)

| R | $R_1$ | n | $R_2$ | R' | X | Y | Z | m.p.(°C) |
|---|---|---|---|---|---|---|---|---|
| $CH_2C\equiv CH$ | H | 0 | H | cyclopropyl | $CH_3$ | $CH_3$ | CH | |
| $CH_2C\equiv CH$ | H | 0 | H | cyclopropyl | $CH_3$ | $OCH_3$ | CH | |
| $CH_2C\equiv CH$ | H | 0 | H | cyclopropyl | $OCH_3$ | $OCH_3$ | CH | |
| $CH_2C\equiv CH$ | H | 0 | H | cyclopropyl | $CH_3$ | $CH_3$ | N | |
| $CH_2C\equiv CH$ | H | 0 | H | cyclopropyl | $CH_3$ | $OCH_3$ | N | |
| $CH_2C\equiv CH$ | H | 0 | H | cyclopropyl | $OCH_3$ | $OCH_3$ | N | |
| $CH_2C\equiv CH$ | H | 0 | H | cyclopropyl | Cl | $OCH_3$ | CH | |
| $CH_2C\equiv CH$ | H | 0 | H | cyclobutyl | $CH_3$ | $CH_3$ | CH | |
| $CH_2C\equiv CH$ | H | 0 | H | cyclobutyl | $CH_3$ | $OCH_3$ | CH | |
| $CH_2C\equiv CH$ | H | 0 | H | cyclobutyl | $OCH_3$ | $OCH_3$ | CH | |
| $CH_2C\equiv CH$ | H | 0 | H | cyclobutyl | $CH_3$ | $CH_3$ | N | |
| $CH_2C\equiv CH$ | H | 0 | H | cyclobutyl | $CH_3$ | $OCH_3$ | N | |
| $CH_2C\equiv CH$ | H | 0 | H | cyclobutyl | $OCH_3$ | $OCH_3$ | N | |
| $CH_2C\equiv CH$ | H | 0 | H | cyclobutyl | Cl | $OCH_3$ | CH | |
| $CH_2\equiv CH$ | H | 0 | H | cyclopentyl | $CH_3$ | $CH_3$ | CH | |
| $CH_2C\equiv CH$ | H | 0 | H | cyclopentyl | $CH_3$ | $OCH_3$ | CH | |
| $CH_2C\equiv CH$ | H | 0 | H | cyclopentyl | $OCH_3$ | $OCH_3$ | CH | |
| $CH_2C\equiv CH$ | H | 0 | H | cyclopentyl | $CH_3$ | $CH_3$ | N | |
| $CH_2C\equiv CH$ | H | 0 | H | cyclopentyl | $CH_3$ | $OCH_3$ | N | |
| $CH_2C\equiv CH$ | H | 0 | H | cyclopentyl | $OCH_3$ | $OCH_3$ | N | |
| $CH_2C\equiv CH$ | H | 0 | H | cyclopentyl | Cl | $OCH_3$ | CH | |
| $CH_2C\equiv CH$ | H | 0 | H | cyclopropyl | $OCH_3$ | $OCH_2CH_3$ | CH | |
| $CH_2C\equiv CH$ | H | 0 | H | cyclopropyl | cyclopropyl | $OCH_3$ | CH | |
| $CH_2C\equiv CH$ | H | 0 | H | cyclopropyl | $OCH_3$ | $CH(OCH_3)_2$ | CH | |
| $CH_2C\equiv CH$ | H | 0 | H | cyclopropyl | $NHCH_3$ | $OCH_2CH_3$ | N | |
| $CH_2C\equiv CH$ | H | 0 | H | cyclopropyl | $NHCH_3$ | $OCH_3$ | N | |
| $CH_2C\equiv CH$ | H | 0 | H | cyclopropyl | $OCH_3$ | $OCH_2CH_3$ | N | |
| $CH_2C\equiv CH$ | H | 0 | H | cyclopropyl | $CH_2F$ | $CH_3$ | CH | |

Table II (cont.)

| R | $R_1$ | n | $R_2$ | R' | X | Y | Z | m.p.(°C) |
|---|---|---|---|---|---|---|---|---|
| H | H | 0 | Cl | cyclopropyl | $CH_3$ | $CH_3$ | CH | |
| H | H | 0 | Cl | cyclopropyl | $CH_3$ | $OCH_3$ | CH | |
| H | H | 0 | Cl | cyclopropyl | $OCH_3$ | $OCH_3$ | CH | |
| H | H | 0 | Cl | cyclopropyl | $CH_3$ | $CH_3$ | N | |
| H | H | 0 | Cl | cyclopropyl | $CH_3$ | $OCH_3$ | N | |
| H | H | 0 | Cl | cyclopropyl | $OCH_3$ | $OCH_3$ | N | |
| H | H | 0 | Cl | cyclopropyl | Cl | $OCH_3$ | CH | |
| H | H | 0 | Cl | cyclobutyl | $CH_3$ | $CH_3$ | CH | |
| H | H | 0 | Cl | cyclobutyl | $CH_3$ | $OCH_3$ | CH | |
| H | H | 0 | Cl | cyclobutyl | $OCH_3$ | $OCH_3$ | CH | |
| H | H | 0 | Cl | cyclobutyl | $CH_3$ | $CH_3$ | N | |
| H | H | 0 | Cl | cyclobutyl | $CH_3$ | $OCH_3$ | N | |
| H | H | 0 | Cl | cyclobutyl | $OCH_3$ | $OCH_3$ | N | |
| H | H | 0 | Cl | cyclobutyl | Cl | $OCH_3$ | CH | |
| H | H | 0 | Cl | cyclopentyl | $CH_3$ | $CH_3$ | CH | |
| H | H | 0 | Cl | cyclopentyl | $CH_3$ | $OCH_3$ | CH | |
| H | H | 0 | Cl | cyclopentyl | $OCH_3$ | $OCH_3$ | CH | |
| H | H | 0 | Cl | cyclopentyl | $CH_3$ | $CH_3$ | N | |
| H | H | 0 | Cl | cyclopentyl | $CH_3$ | $OCH_3$ | N | |
| H | H | 0 | Cl | cyclopentyl | $OCH_3$ | $OCH_3$ | N | |
| H | H | 0 | Cl | cyclopentyl | Cl | $OCH_3$ | CH | |
| H | H | 0 | Cl | cyclopropyl | $OCH_3$ | $OCH_2CH_3$ | CH | |
| H | H | 0 | Cl | cyclopropyl | cyclopropyl | $OCH_3$ | CH | |
| H | H | 0 | Cl | cyclopropyl | $OCH_3$ | $CH(OCH_3)_2$ | CH | |
| H | H | 0 | Cl | cyclopropyl | $NHCH_3$ | $OCH_2CH_3$ | N | |
| H | H | 0 | Cl | cyclopropyl | $NHCH_3$ | $OCH_3$ | N | |
| H | H | 0 | Cl | cyclopropyl | $OCH_3$ | $OCH_2CH_3$ | N | |
| H | H | 0 | Cl | cyclopropyl | $CH_2F$ | $CH_3$ | CH | |

Table II (cont.)

| R | $R_1$ | n | $R_2$ | R' | X | Y | Z | m.p.(°C) |
|---|---|---|---|---|---|---|---|---|
| H | H | O | $CH_3$ | cyclopropyl | $CH_3$ | $CH_3$ | CH | |
| H | H | O | $CH_3$ | cyclopropyl | $CH_3$ | $OCH_3$ | CH | |
| H | H | O | $CH_3$ | cyclopropyl | $OCH_3$ | $OCH_3$ | CH | |
| H | H | O | $CH_3$ | cyclopropyl | $CH_3$ | $CH_3$ | N | |
| H | H | O | $CH_3$ | cyclopropyl | $CH_3$ | $OCH_3$ | N | |
| H | H | O | $CH_3$ | cyclopropyl | $OCH_3$ | $OCH_3$ | N | |
| H | H | O | $CH_3$ | cyclopropyl | Cl | $OCH_3$ | CH | |
| H | H | O | $CH_3$ | cyclobutyl | $CH_3$ | $CH_3$ | CH | |
| H | H | O | $CH_3$ | cyclobutyl | $CH_3$ | $OCH_3$ | CH | |
| H | H | O | $CH_3$ | cyclobutyl | $OCH_3$ | $OCH_3$ | CH | |
| H | H | O | $CH_3$ | cyclobutyl | $CH_3$ | $CH_3$ | N | |
| H | H | O | $CH_3$ | cyclobutyl | $CH_3$ | $OCH_3$ | N | |
| H | H | O | $CH_3$ | cyclobutyl | $OCH_3$ | $OCH_3$ | N | |
| H | H | O | $CH_3$ | cyclobutyl | Cl | $OCH_3$ | CH | |
| H | H | O | $CH_3$ | cyclopentyl | $CH_3$ | $CH_3$ | CH | |
| H | H | O | $CH_3$ | cyclopentyl | $CH_3$ | $OCH_3$ | CH | |
| H | H | O | $CH_3$ | cyclopentyl | $OCH_3$ | $OCH_3$ | CH | |
| H | H | O | $CH_3$ | cyclopentyl | $CH_3$ | $CH_3$ | N | |
| H | H | O | $CH_3$ | cyclopentyl | $CH_3$ | $OCH_3$ | N | |
| H | H | O | $CH_3$ | cyclopentyl | $OCH_3$ | $OCH_3$ | N | |
| H | H | O | $CH_3$ | cyclopentyl | Cl | $OCH_3$ | CH | |
| H | H | O | $CH_3$ | cyclopropyl | $OCH_3$ | $OCH_2CH_3$ | CH | |
| H | H | O | $CH_3$ | cyclopropyl | cyclopropyl | $OCH_3$ | CH | |
| H | H | O | $CH_3$ | cyclopropyl | $OCH_3$ | $CH(OCH_3)_2$ | CH | |
| H | H | O | $CH_3$ | cyclopropyl | $NHCH_3$ | $OCH_2CH_3$ | N | |
| H | H | O | $CH_3$ | cyclopropyl | $NHCH_3$ | $OCH_3$ | N | |
| H | H | O | $CH_3$ | cyclopropyl | $OCH_3$ | $OCH_2CH_3$ | N | |
| H | H | O | $CH_3$ | cyclopropyl | $CH_2F$ | $CH_3$ | CH | |

Table II (cont.)

| R | $R_1$ | n | $R_2$ | R' | X | Y | Z | m.p.(°C) |
|---|---|---|---|---|---|---|---|---|
| $CH_3$ | H | 0 | H | cylobutyl | $CH_2CH_3$ | $CH_3$ | CH | |
| $CH_3$ | H | 0 | H | cyclobutyl | $CH_2CH_3$ | $OCH_3$ | CH | |
| $CH_3$ | H | 0 | H | cyclobutyl | $CH_2F$ | $CH_3$ | CH | |
| $CH_3$ | H | 0 | H | cyclobutyl | $OCF_2H$ | $CH_3$ | CH | |
| $CH_3$ | H | 0 | H | cyclobutyl | $OCF_2H$ | $OCH_3$ | CH | |
| $CH_3$ | H | 0 | H | cyclobutyl | $OCH_2CF_3$ | $OCH_3$ | CH | |
| $CH_3$ | H | 0 | H | cyclobutyl | $CH_2F$ | $OCH_3$ | CH | |
| $CH_3$ | H | 0 | H | cyclobutyl | $CH_2Cl$ | $OCH_3$ | N | |
| $CH_3$ | H | 0 | H | cyclobutyl | $SCH_3$ | $OCH_3$ | CH | |
| $CH_3$ | H | 0 | H | cyclobutyl | $SCH_2F$ | $OCH_3$ | CH | |
| $CH_3$ | H | 0 | H | cyclobutyl | Br | $OCH_3$ | CH | |
| $CH_3$ | H | 0 | H | cyclobutyl | $CH_2OCH_3$ | $OCH_3$ | N | |
| $CH_3$ | H | 0 | H | cyclobutyl | $OCH_2OCH_3$ | $OCH_3$ | CH | |
| $CH_3$ | H | 0 | H | cyclobutyl | $OCH_2OCH_2CH_3$ | $CH_3$ | CH | |
| $CH_3$ | H | 0 | H | cyclobutyl | $N(CH_3)_2$ | $OCH_3$ | N | |
| $CH_3$ | H | 0 | H | cyclobutyl | $NHCH_2CH_3$ | $CH_3$ | N | |
| $CH_3$ | H | 0 | H | cyclobutyl | $NHCH_3$ | $OCH_2CH_3$ | N | |
| $CH_3$ | H | 0 | H | cyclobutyl | $CH_3$ | $CH_2SCH_3$ | CH | |
| $CH_3$ | H | 0 | H | cyclobutyl | $OCH_3$ | $CH_2SO_2CH_3$ | CH | |
| $CH_3$ | H | 0 | H | cyclobutyl | $NH_2$ | $OCH_2CH_3$ | N | |
| $CH_3$ | H | 0 | H | cyclobutyl | $CH_3$ | $OCH_2CH=CH_2$ | CH | |
| $CH_3$ | H | 0 | H | cyclobutyl | $CH_2CH_3$ | $OCF_2H$ | CH | |
| $CH_3$ | H | 0 | H | cyclobutyl | $OCF_2H$ | $OCF_2H$ | CH | |
| $CH_3$ | H | 0 | H | cyclobutyl | $OCH_3$ | $OCH=CH_2$ | CH | |
| $CH_3$ | H | 0 | H | cyclobutyl | $OCH_3$ | $C(O)CH_3$ | N | |
| $CH_3$ | H | 0 | H | cyclobutyl | $CH_3$ | $N(OCH_3)CH_3$ | N | |
| $CH_3$ | H | 0 | H | cyclobutyl | $OCH(CH_3)_2$ | $OCF_2H$ | CH | |

Table II (cont.)

| R | $R_1$ | n | $R_2$ | R' | X | Y | Z | m.p.(°C) |
|---|---|---|---|---|---|---|---|---|
| $CH_3$ | H | 0 | H | $CH_3$ | $CH_3$ | $CH_3$ | CH | |
| $CH_3$ | H | 0 | H | $CH_3$ | $CH_3$ | $OCH_3$ | CH | |
| $CH_3$ | H | 0 | H | $CH_3$ | $OCH_3$ | $OCH_3$ | CH | |
| $CH_3$ | H | 0 | H | $CH_3$ | $CH_3$ | $CH_3$ | N | |
| $CH_3$ | H | 0 | H | $CH_3$ | $CH_3$ | $OCH_3$ | N | |
| $CH_3$ | H | 0 | H | $CH_3$ | $OCH_3$ | $OCH_3$ | N | |
| $CH_3$ | H | 0 | H | $CH_3$ | Cl | $OCH_3$ | CH | |
| $CH_3$ | H | 0 | H | $CH_2CH_3$ | $CH_3$ | $CH_3$ | CH | |
| $CH_3$ | H | 0 | H | $CH_2CH_3$ | $CH_3$ | $OCH_3$ | CH | |
| $CH_3$ | H | 0 | H | $CH_2CH_3$ | $OCH_3$ | $OCH_3$ | CH | |
| $CH_3$ | H | 0 | H | $CH_2CH_3$ | $CH_3$ | $CH_3$ | N | |
| $CH_3$ | H | 0 | H | $CH_2CH_3$ | $CH_3$ | $OCH_3$ | N | |
| $CH_3$ | H | 0 | H | $CH_2CH_3$ | $OCH_3$ | $OCH_3$ | N | |
| $CH_3$ | H | 0 | H | $CH_2CH_3$ | Cl | $OCH_3$ | CH | |
| $CH_3$ | H | 0 | H | $CH_2CH_2CH_3$ | $CH_3$ | $CH_3$ | CH | |
| $CH_3$ | H | 0 | H | $CH_2CH_2CH_3$ | $CH_3$ | $OCH_3$ | CH | |
| $CH_3$ | H | 0 | H | $CH_2CH_2CH_3$ | $OCH_3$ | $OCH_3$ | CH | |
| $CH_3$ | H | 0 | H | $CH_2CH_2CH_3$ | $CH_3$ | $CH_3$ | N | |
| $CH_3$ | H | 0 | H | $CH_2CH_2CH_3$ | $CH_3$ | $OCH_3$ | N | |
| $CH_3$ | H | 0 | H | $CH_2CH_2CH_3$ | $OCH_3$ | $OCH_3$ | N | |
| $CH_3$ | H | 0 | H | $CH_2CH_2CH_3$ | Cl | $OCH_3$ | CH | |
| $CH_3$ | H | 0 | H | $CH_2CH_2CH_2CH_3$ | $CH_3$ | $CH_3$ | CH | |
| $CH_3$ | H | 0 | H | $CH_2CH_2CH_2CH_3$ | $CH_3$ | $OCH_3$ | CH | |
| $CH_3$ | H | 0 | H | $CH_2CH_2CH_2CH_3$ | $OCH_3$ | $OCH_3$ | CH | |
| $CH_3$ | H | 0 | H | $CH_2CH_2CH_2CH_3$ | $CH_3$ | $CH_3$ | N | |
| $CH_3$ | H | 0 | H | $CH_2CH_2CH_2CH_3$ | $CH_3$ | $OCH_3$ | N | |
| $CH_3$ | H | 0 | H | $CH_2CH_2CH_2CH_3$ | $OCH_3$ | $OCH_3$ | N | |
| $CH_3$ | H | 0 | H | $CH_2CH_2CH_2CH_3$ | Cl | $OCH_3$ | CH | |

Table II (cont.)

| R | $R_1$ | n | $R_2$ | R' | X | Y | Z | m.p.(°C) |
|---|---|---|---|---|---|---|---|---|
| $CH_3$ | H | 0 | H | $CH_2OH$ | $CH_3$ | $CH_3$ | CH | |
| $CH_3$ | H | 0 | H | $CH_2OH$ | $CH_3$ | $OCH_3$ | CH | |
| $CH_3$ | H | 0 | H | $CH_2OH$ | $OCH_3$ | $OCH_3$ | CH | |
| $CH_3$ | H | 0 | H | $CH_2OH$ | $CH_3$ | $CH_3$ | N | |
| $CH_3$ | H | 0 | H | $CH_2OH$ | $CH_3$ | $OCH_3$ | N | |
| $CH_3$ | H | 0 | H | $CH_2OH$ | $OCH_3$ | $OCH_3$ | N | |
| $CH_3$ | H | 0 | H | $CH_2OH$ | Cl | $OCH_3$ | CH | |
| $CH_3$ | H | 0 | H | $CH(CH_3)OH$ | $CH_3$ | $CH_3$ | CH | |
| $CH_3$ | H | 0 | H | $CH(CH_3)OH$ | $CH_3$ | $OCH_3$ | CH | |
| $CH_3$ | H | 0 | H | $CH(CH_3)OH$ | $OCH_3$ | $OCH_3$ | CH | |
| $CH_3$ | H | 0 | H | $CH(CH_3)OH$ | $CH_3$ | $CH_3$ | N | |
| $CH_3$ | H | 0 | H | $CH(CH_3)OH$ | $CH_3$ | $OCH_3$ | N | |
| $CH_3$ | H | 0 | H | $CH(CH_3)OH$ | $OCH_3$ | $OCH_3$ | N | |
| $CH_3$ | H | 0 | H | $CH(CH_3)OH$ | Cl | $OCH_3$ | CH | |
| $CH_3$ | H | 0 | H | $CH_2OSi(CH_3)_3$ | $CH_3$ | $CH_3$ | CH | |
| $CH_3$ | H | 0 | H | $CH_2OSi(CH_3)_3$ | $CH_3$ | $OCH_3$ | CH | |
| $CH_3$ | H | 0 | H | $CH_2OSi(CH_3)_3$ | $OCH_3$ | $OCH_3$ | CH | |
| $CH_3$ | H | 0 | H | $CH_2OSi(CH_3)_3$ | $CH_3$ | $CH_3$ | N | |
| $CH_3$ | H | 0 | H | $CH_2OSi(CH_3)_3$ | $CH_3$ | $OCH_3$ | N | |
| $CH_3$ | H | 0 | H | $CH_2OSi(CH_3)_3$ | $OCH_3$ | $OCH_3$ | N | |
| $CH_3$ | H | 0 | H | $CH_2OSi(CH_3)_3$ | Cl | $OCH_3$ | CH | |
| $CH_3$ | H | 0 | H | $CH_2COCH_3$ | $CH_3$ | $CH_3$ | CH | |
| $CH_3$ | H | 0 | H | $CH_2COCH_3$ | $CH_3$ | $OCH_3$ | CH | |
| $CH_3$ | H | 0 | H | $CH_2COCH_3$ | $OCH_3$ | $OCH_3$ | CH | |
| $CH_3$ | H | 0 | H | $CH_2COCH_3$ | $CH_3$ | $CH_3$ | N | |
| $CH_3$ | H | 0 | H | $CH_2COCH_3$ | $CH_3$ | $OCH_3$ | N | |
| $CH_3$ | H | 0 | H | $CH_2CO_2CH_3$ | $OCH_3$ | $OCH_3$ | N | |
| $CH_3$ | H | 0 | H | $CH_2CO_2CH_3$ | Cl | $OCH_3$ | CH | |

Table II (cont.)

| R | $R_1$ | n | $R_2$ | R' | X | Y | Z | m.p.(°C) |
|---|---|---|---|---|---|---|---|---|
| $CH_3$ | H | 0 | $CH_2F$ | $CH_3$ | $CH_3$ | $CH_3$ | CH | |
| $CH_3$ | H | 0 | $CH_2F$ | $CH_3$ | $CH_3$ | $OCH_3$ | CH | |
| $CH_3$ | H | 0 | $CH_2F$ | $CH_3$ | $OCH_3$ | $OCH_3$ | CH | |
| $CH_3$ | H | 0 | $CH_2F$ | $CH_3$ | $CH_3$ | $CH_3$ | N | |
| $CH_3$ | H | 0 | $CH_2F$ | $CH_3$ | $CH_3$ | $OCH_3$ | N | |
| $CH_3$ | H | 0 | $CH_2F$ | $CH_3$ | $OCH_3$ | $OCH_3$ | N | |
| $CH_3$ | H | 0 | $CH_2F$ | $CH_3$ | Cl | $OCH_3$ | CH | |
| $CH_3$ | H | 0 | $CH_2F$ | $CH_2CH_3$ | $CH_3$ | $CH_3$ | CH | |
| $CH_3$ | H | 0 | $CH_2F$ | $CH_2CH_3$ | $CH_3$ | $OCH_3$ | CH | |
| $CH_3$ | H | 0 | $CH_2F$ | $CH_2CH_3$ | $OCH_3$ | $OCH_3$ | CH | |
| $CH_3$ | H | 0 | $CH_2F$ | $CH_2CH_3$ | $CH_3$ | $CH_3$ | N | |
| $CH_3$ | H | 0 | $CH_2F$ | $CH_2CH_3$ | $CH_3$ | $OCH_3$ | N | |
| $CH_3$ | H | 0 | $CH_2F$ | $CH_2CH_3$ | $OCH_3$ | $OCH_3$ | N | |
| $CH_3$ | H | 0 | $CH_2F$ | $CH_2CH_3$ | Cl | $OCH_3$ | CH | |
| $CH_3$ | H | 0 | $CH_2F$ | $CH_2CH_2CH_3$ | $CH_3$ | $CH_3$ | CH | |
| $CH_3$ | H | 0 | $CH_2F$ | $CH_2CH_2CH_3$ | $CH_3$ | $OCH_3$ | CH | |
| $CH_3$ | H | 0 | $CH_2F$ | $CH_2CH_2CH_3$ | $OCH_3$ | $OCH_3$ | CH | |
| $CH_3$ | H | 0 | $CH_2F$ | $CH_2CH_2CH_3$ | $CH_3$ | $CH_3$ | N | |
| $CH_3$ | H | 0 | $CH_2F$ | $CH_2CH_2CH_3$ | $CH_3$ | $OCH_3$ | N | |
| $CH_3$ | H | 0 | $CH_2F$ | $CH_2CH_2CH_3$ | $OCH_3$ | $OCH_3$ | N | |
| $CH_3$ | H | 0 | $CH_2F$ | $CH_2CH_2CH_3$ | Cl | $OCH_3$ | CH | |
| $CH_3$ | H | 0 | $CH_2F$ | $CH_2CH_2CH_2CH_3$ | $CH_3$ | $CH_3$ | CH | |
| $CH_3$ | H | 0 | $CH_2F$ | $CH_2CH_2CH_2CH_3$ | $CH_3$ | $OCH_3$ | CH | |
| $CH_3$ | H | 0 | $CH_2F$ | $CH_2CH_2CH_2CH_3$ | $OCH_3$ | $OCH_3$ | CH | |
| $CH_3$ | H | 0 | $CH_2F$ | $CH_2CH_2CH_2CH_3$ | $CH_3$ | $CH_3$ | N | |
| $CH_3$ | H | 0 | $CH_2F$ | $CH_2CH_2CH_2CH_3$ | $CH_3$ | $OCH_3$ | N | |
| $CH_3$ | H | 0 | $CH_2F$ | $CH_2CH_2CH_2CH_3$ | $OCH_3$ | $OCH_3$ | N | |
| $CH_3$ | H | 0 | $CH_2F$ | $CH_2CH_2CH_2CH_3$ | Cl | $OCH_3$ | CH | |

Table II (cont.)

| R | $R_1$ | n | $R_2$ | R' | X | Y | Z | m.p.(°C) |
|---|---|---|---|---|---|---|---|---|
| $CH_2OCH_3$ | H | 0 | H | $CH_2CH_2CH_2CH_2CH_3$ | $CH_3$ | $CH_3$ | CH | |
| $CH_2OCH_3$ | H | 0 | H | $CH_2CH_2CH_2CH_2CH_3$ | $CH_3$ | $OCH_3$ | CH | |
| $CH_2OCH_3$ | H | 0 | H | $CH_2CH_2CH_2CH_2CH_3$ | $OCH_3$ | $OCH_3$ | CH | |
| $CH_2OCH_3$ | H | 0 | H | $CH_2CH_2CH_2CH_2CH_3$ | $CH_3$ | $CH_3$ | N | |
| $CH_2OCH_3$ | H | 0 | H | $CH_2CH_2CH_2CH_2CH_3$ | $CH_3$ | $OCH_3$ | N | |
| $CH_2OCH_3$ | H | 0 | H | $CH_2CH_2CH_2CH_2CH_3$ | $OCH_3$ | $OCH_3$ | N | |
| $CH_2OCH_3$ | H | 0 | H | $CH_2CH_2CH_2CH_2CH_3$ | Cl | $OCH_3$ | CH | |
| $CH_2OCH_3$ | H | 0 | H | $CH(CH_3)_2$ | $CH_3$ | $CH_3$ | CH | |
| $CH_2OCH_3$ | H | 0 | H | $CH(CH_3)_2$ | $CH_3$ | $OCH_3$ | CH | |
| $CH_2OCH_3$ | H | 0 | H | $CH(CH_3)_2$ | $OCH_3$ | $OCH_3$ | CH | |
| $CH_2OCH_3$ | H | 0 | H | $CH(CH_3)_2$ | $CH_3$ | $CH_3$ | N | |
| $CH_2OCH_3$ | H | 0 | H | $CH(CH_3)_2$ | $CH_3$ | $OCH_3$ | N | |
| $CH_2OCH_3$ | H | 0 | H | $CH(CH_3)_2$ | $OCH_3$ | $OCH_3$ | N | |
| $CH_2OCH_3$ | H | 0 | H | $CH(CH_3)_2$ | Cl | $OCH_3$ | CH | |
| $CH_2OCH_3$ | H | 0 | H | $CH_2CH(CH_3)_2$ | $CH_3$ | $CH_3$ | CH | |
| $CH_2OCH_3$ | H | 0 | H | $CH_2CH(CH_3)_2$ | $CH_3$ | $OCH_3$ | CH | |
| $CH_2OCH_3$ | H | 0 | H | $CH_2CH(CH_3)_2$ | $OCH_3$ | $OCH_3$ | CH | |
| $CH_2OCH_3$ | H | 0 | H | $CH_2CH(CH_3)_2$ | $CH_3$ | $CH_3$ | N | |
| $CH_2OCH_3$ | H | 0 | H | $CH_2CH(CH_3)_2$ | $CH_3$ | $OCH_3$ | N | |
| $CH_2OCH_3$ | H | 0 | H | $CH_2CH(CH_3)_2$ | $OCH_3$ | $OCH_3$ | N | |
| $CH_2OCH_3$ | H | 0 | H | $CH_2CH(CH_3)_2$ | Cl | $OCH_3$ | CH | |
| $CH_2OCH_3$ | H | 0 | H | $CH(CH_3)CH_2CH_3$ | $CH_3$ | $CH_3$ | CH | |
| $CH_2OCH_3$ | H | 0 | H | $CH(CH_3)CH_2CH_3$ | $CH_3$ | $OCH_3$ | CH | |
| $CH_2OCH_3$ | H | 0 | H | $CH(CH_3)CH_2CH_3$ | $OCH_3$ | $OCH_3$ | CH | |
| $CH_2OCH_3$ | H | 0 | H | $CH_2CH_2CH(CH_3)_2$ | $CH_3$ | $CH_3$ | N | |
| $CH_2OCH_3$ | H | 0 | H | $CH_2CH_2CH(CH_3)_2$ | $CH_3$ | $OCH_3$ | N | |
| $CH_2OCH_3$ | H | 0 | H | $CH_2CH_2CH(CH_3)_2$ | $OCH_3$ | $OCH_3$ | N | |
| $CH_2OCH_3$ | H | 0 | H | $CH_2CH_2CH(CH_3)_2$ | Cl | $OCH_3$ | CH | |

Table III

| R | $R_1$ | n | $R_2$ | R' | X | Y | Z | m.p.(°C) |
|---|---|---|---|---|---|---|---|---|
| $CH_3$ | H | O | H | cyclopropyl | $CH_3$ | $CH_3$ | CH | 157-159 |
| $CH_3$ | H | O | H | cyclopropyl | $CH_3$ | $OCH_3$ | CH | 179-182 |
| $CH_3$ | H | O | H | cyclopropyl | $OCH_3$ | $OCH_3$ | CH | 159-161 |
| $CH_3$ | H | O | H | cyclopropyl | $CH_3$ | $CH_3$ | N | |
| $CH_3$ | H | O | H | cyclopropyl | $CH_3$ | $OCH_3$ | N | 185-187 |
| $CH_3$ | H | O | H | cyclopropyl | $OCH_3$ | $OCH_3$ | N | 196-198 |
| $CH_3$ | H | O | H | cyclopropyl | Cl | $OCH_3$ | CH | 202-205 |
| $CH_3$ | H | O | H | cyclobutyl | $CH_3$ | $CH_3$ | CH | |
| $CH_3$ | H | O | H | cyclobutyl | $CH_3$ | $OCH_3$ | CH | |
| $CH_3$ | H | O | H | cyclobutyl | $OCH_3$ | $OCH_3$ | CH | |
| $CH_3$ | H | O | H | cyclobutyl | $CH_3$ | $CH_3$ | N | |
| $CH_3$ | H | O | H | cyclobutyl | $CH_3$ | $OCH_3$ | N | |
| $CH_3$ | H | O | H | cyclobutyl | $OCH_3$ | $OCH_3$ | N | |
| $CH_3$ | H | O | H | cyclobutyl | Cl | $OCH_3$ | CH | |
| $CH_3$ | H | O | H | cyclopentyl | $CH_3$ | $CH_3$ | CH | |
| $CH_3$ | H | O | H | cyclopentyl | $CH_3$ | $OCH_3$ | CH | |
| $CH_3$ | H | O | H | cyclopentyl | $OCH_3$ | $OCH_3$ | CH | |
| $CH_3$ | H | O | H | cyclopentyl | $CH_3$ | $CH_3$ | N | |
| $CH_3$ | H | O | H | cyclopentyl | $CH_3$ | $OCH_3$ | N | |
| $CH_3$ | H | O | H | cyclopentyl | $OCH_3$ | $OCH_3$ | N | |
| $CH_3$ | H | O | H | cyclopentyl | Cl | $OCH_3$ | CH | |
| $CH_3$ | H | O | H | cyclopropyl | $OCH_3$ | $OCH_2CH_3$ | CH | |
| $CH_3$ | H | O | H | cyclopropyl | cyclopropyl | $OCH_3$ | CH | |
| $CH_3$ | H | O | H | cyclopropyl | $OCH_3$ | $CH(OCH_3)_2$ | CH | |
| $CH_3$ | H | O | H | cyclopropyl | $NHCH_3$ | $OCH_2CH_3$ | N | |
| $CH_3$ | H | O | H | cyclopropyl | $NHCH_3$ | $OCH_3$ | N | |
| $CH_3$ | H | O | H | cyclopropyl | $OCH_3$ | $OCH_2CH_3$ | N | |
| $CH_3$ | H | O | H | cyclopropyl | $CH_2F$ | $CH_3$ | CH | |

Table III (cont.)

| R | $R_1$ | n | $R_2$ | R' | X | Y | Z | m.p.(°C) |
|---|---|---|---|---|---|---|---|---|
| $CH_3$ | H | O | Br | cyclopropyl | $CH_3$ | $CH_3$ | CH | |
| $CH_3$ | H | O | Br | cyclopropyl | $CH_3$ | $OCH_3$ | CH | |
| $CH_3$ | H | O | Br | cyclopropyl | $OCH_3$ | $OCH_3$ | CH | |
| $CH_3$ | H | O | Br | cyclopropyl | $CH_3$ | $CH_3$ | N | |
| $CH_3$ | H | O | Br | cyclopropyl | $CH_3$ | $OCH_3$ | N | |
| $CH_3$ | H | O | Br | cyclopropyl | $OCH_3$ | $OCH_3$ | N | |
| $CH_3$ | H | O | Br | cyclopropyl | Cl | $OCH_3$ | CH | |
| $CH_3$ | H | O | Br | cyclobutyl | $CH_3$ | $CH_3$ | CH | |
| $CH_3$ | H | O | Br | cyclobutyl | $CH_3$ | $OCH_3$ | CH | |
| $CH_3$ | H | O | Br | cyclobutyl | $OCH_3$ | $OCH_3$ | CH | |
| $CH_3$ | H | O | Br | cyclobutyl | $CH_3$ | $CH_3$ | N | |
| $CH_3$ | H | O | Br | cyclobutyl | $CH_3$ | $OCH_3$ | N | |
| $CH_3$ | H | O | Br | cyclobutyl | $OCH_3$ | $OCH_3$ | N | |
| $CH_3$ | H | O | Br | cyclobutyl | Cl | $OCH_3$ | CH | |
| $CH_3$ | H | O | Br | cyclopentyl | $CH_3$ | $CH_3$ | CH | |
| $CH_3$ | H | O | Br | cyclopentyl | $CH_3$ | $OCH_3$ | CH | |
| $CH_3$ | H | O | Br | cyclopentyl | $OCH_3$ | $OCH_3$ | CH | |
| $CH_3$ | H | O | Br | cyclopentyl | $CH_3$ | $CH_3$ | N | |
| $CH_3$ | H | O | Br | cyclopentyl | $CH_3$ | $OCH_3$ | N | |
| $CH_3$ | H | O | Br | cyclopentyl | $OCH_3$ | $OCH_3$ | N | |
| $CH_3$ | H | O | Br | cyclopentyl | Cl | $OCH_3$ | CH | |
| $CH_3$ | H | O | Br | cyclopropyl | $OCH_3$ | $OCH_2CH_3$ | CH | |
| $CH_3$ | H | O | Br | cyclopropyl | cyclopropyl | $OCH_3$ | CH | |
| $CH_3$ | H | O | Br | cyclopropyl | $OCH_3$ | $CH(OCH_3)_2$ | CH | |
| $CH_3$ | H | O | Br | cyclopropyl | $NHCH_3$ | $OCH_2CH_3$ | N | |
| $CH_3$ | H | O | Br | cyclopropyl | $NHCH_3$ | $OCH_3$ | N | |
| $CH_3$ | H | O | Br | cyclopropyl | $OCH_3$ | $OCH_2CH_3$ | N | |
| $CH_3$ | H | O | Br | cyclopropyl | $CH_2F$ | $CH_3$ | CH | |

Table III (cont.)

| R | $R_1$ | n | $R_2$ | R' | X | Y | Z | m.p.(°C) |
|---|---|---|---|---|---|---|---|---|
| $CH_3$ | H | O | $SO_2CH_3$ | cyclopropyl | $CH_3$ | $CH_3$ | CH | |
| $CH_3$ | H | O | $SO_2CH_3$ | cyclopropyl | $CH_3$ | $OCH_3$ | CH | |
| $CH_3$ | H | O | $SO_2CH_3$ | cyclopropyl | $OCH_3$ | $OCH_3$ | CH | |
| $CH_3$ | H | O | $SO_2CH_3$ | cyclopropyl | $CH_3$ | $CH_3$ | N | |
| $CH_3$ | H | O | $SO_2CH_3$ | cyclopropyl | $CH_3$ | $OCH_3$ | N | |
| $CH_3$ | H | O | $SO_2CH_3$ | cyclopropyl | $OCH_3$ | $OCH_3$ | N | |
| $CH_3$ | H | O | $SO_2CH_3$ | cyclopropyl | Cl | $OCH_3$ | CH | |
| $CH_3$ | H | O | $SO_2CH_3$ | cyclobutyl | $CH_3$ | $CH_3$ | CH | |
| $CH_3$ | H | O | $SO_2CH_3$ | cyclobutyl | $CH_3$ | $OCH_3$ | CH | |
| $CH_3$ | H | O | $SO_2CH_3$ | cyclobutyl | $OCH_3$ | $OCH_3$ | CH | |
| $CH_3$ | H | O | $SO_2CH_3$ | cyclobutyl | $CH_3$ | $CH_3$ | N | |
| $CH_3$ | H | O | $SO_2CH_3$ | cyclobutyl | $CH_3$ | $OCH_3$ | N | |
| $CH_3$ | H | O | $SO_2CH_3$ | cyclobutyl | $OCH_3$ | $OCH_3$ | N | |
| $CH_3$ | H | O | $SO_2CH_3$ | cyclobutyl | Cl | $OCH_3$ | CH | |
| $CH_3$ | H | O | $SO_2CH_3$ | cyclopentyl | $CH_3$ | $CH_3$ | CH | |
| $CH_3$ | H | O | $SO_2CH_3$ | cyclopentyl | $CH_3$ | $OCH_3$ | CH | |
| $CH_3$ | H | O | $SO_2CH_3$ | cyclopentyl | $OCH_3$ | $OCH_3$ | CH | |
| $CH_3$ | H | O | $SO_2CH_3$ | cyclopentyl | $CH_3$ | $CH_3$ | N | |
| $CH_3$ | H | O | $SO_2CH_3$ | cyclopentyl | $CH_3$ | $OCH_3$ | N | |
| $CH_3$ | H | O | $SO_2CH_3$ | cyclopentyl | $OCH_3$ | $OCH_3$ | N | |
| $CH_3$ | H | O | $SO_2CH_3$ | cyclopentyl | Cl | $OCH_3$ | CH | |
| $CH_3$ | H | O | $SO_2CH_3$ | cyclopropyl | $OCH_3$ | $OCH_2CH_3$ | CH | |
| $CH_3$ | H | O | $SO_2CH_3$ | cyclopropyl | cyclopropyl | $OCH_3$ | CH | |
| $CH_3$ | H | O | $SO_2CH_3$ | cyclopropyl | $OCH_3$ | $CH(OCH_3)_2$ | CH | |
| $CH_3$ | H | O | $SO_2CH_3$ | cyclopropyl | $NHCH_3$ | $OCH_2CH_3$ | N | |
| $CH_3$ | H | O | $SO_2CH_3$ | cyclopropyl | $NHCH_3$ | $OCH_3$ | N | |
| $CH_3$ | H | O | $SO_2CH_3$ | cyclopropyl | $OCH_3$ | $OCH_2CH_3$ | N | |
| $CH_3$ | H | O | $SO_2CH_3$ | cyclopropyl | $CH_2F$ | $CH_3$ | CH | |

Table III (cont.)

| R | $R_1$ | n | $R_2$ | R' | X | Y | Z | m.p.(°C) |
|---|---|---|---|---|---|---|---|---|
| $CH_3$ | H | O | $CH(OH)CH_3$ | cyclopropyl | $CH_3$ | $CH_3$ | CH | |
| $CH_3$ | H | O | $CH(OH)CH_3$ | cyclopropyl | $CH_3$ | $OCH_3$ | CH | |
| $CH_3$ | H | O | $CH(OH)CH_3$ | cyclopropyl | $OCH_3$ | $OCH_3$ | CH | |
| $CH_3$ | H | O | $CH(OH)CH_3$ | cyclopropyl | $CH_3$ | $CH_3$ | N | |
| $CH_3$ | H | O | $CH(OH)CH_3$ | cyclopropyl | $CH_3$ | $OCH_3$ | N | |
| $CH_3$ | H | O | $CH(OH)CH_3$ | cyclopropyl | $OCH_3$ | $OCH_3$ | N | |
| $CH_3$ | H | O | $CH(OH)CH_3$ | cyclopropyl | Cl | $OCH_3$ | CH | |
| $CH_3$ | H | O | $CH(OH)CH_3$ | cyclobutyl | $CH_3$ | $CH_3$ | CH | |
| $CH_3$ | H | O | $CH(OH)CH_3$ | cyclobutyl | $CH_3$ | $OCH_3$ | CH | |
| $CH_3$ | H | O | $CH(OH)CH_3$ | cyclobutyl | $OCH_3$ | $OCH_3$ | CH | |
| $CH_3$ | H | O | $CH(OH)CH_3$ | cyclobutyl | $CH_3$ | $CH_3$ | N | |
| $CH_3$ | H | O | $CH(OH)CH_3$ | cyclobutyl | $CH_3$ | $OCH_3$ | N | |
| $CH_3$ | H | O | $CH(OH)CH_3$ | cyclobutyl | $OCH_3$ | $OCH_3$ | N | |
| $CH_3$ | H | O | $CH(OH)CH_3$ | cyclobutyl | Cl | $OCH_3$ | CH | |
| $CH_3$ | H | O | $CH(OH)CH_3$ | cyclopentyl | $CH_3$ | $CH_3$ | CH | |
| $CH_3$ | H | O | $CH(OH)CH_3$ | cyclopentyl | $CH_3$ | $OCH_3$ | CH | |
| $CH_3$ | H | O | $CH(OH)CH_3$ | cyclopentyl | $OCH_3$ | $OCH_3$ | CH | |
| $CH_3$ | H | O | $CH(OH)CH_3$ | cyclopentyl | $CH_3$ | $CH_3$ | N | |
| $CH_3$ | H | O | $CH(OH)CH_3$ | cyclopentyl | $CH_3$ | $OCH_3$ | N | |
| $CH_3$ | H | O | $CH(OH)CH_3$ | cyclopentyl | $OCH_3$ | $OCH_3$ | N | |
| $CH_3$ | H | O | $CH(OH)CH_3$ | cyclopentyl | Cl | $OCH_3$ | CH | |
| $CH_3$ | H | O | $CH(OH)CH_3$ | cyclopropyl | $OCH_3$ | $OCH_2CH_3$ | CH | |
| $CH_3$ | H | O | $CH(OH)CH_3$ | cyclopropyl | cyclopropyl | $OCH_3$ | CH | |
| $CH_3$ | H | O | $CH(OH)CH_3$ | cyclopropyl | $OCH_3$ | $CH(OCH_3)_2$ | CH | |
| $CH_3$ | H | O | $CH(OH)CH_3$ | cyclopropyl | $NHCH_3$ | $OCH_2CH_3$ | N | |
| $CH_3$ | H | O | $CH(OH)CH_3$ | cyclopropyl | $NHCH_3$ | $OCH_3$ | N | |
| $CH_3$ | H | O | $CH(OH)CH_3$ | cyclopropyl | $OCH_3$ | $OCH_2CH_3$ | N | |
| $CH_3$ | H | O | $CH(OH)CH_3$ | cyclopropyl | $CH_2F$ | $CH_3$ | CH | |

Table III (cont.)

| R | $R_1$ | n | $R_2$ | R' | X | Y | Z | m.p.(°C) |
|---|---|---|---|---|---|---|---|---|
| $CH_2CH=CH_2$ | H | 0 | H | cyclopropyl | $CH_3$ | $CH_3$ | CH | |
| $CH_2CH=CH_2$ | H | 0 | H | cyclopropyl | $CH_3$ | $OCH_3$ | CH | |
| $CH_2CH=CH_2$ | H | 0 | H | cyclopropyl | $OCH_3$ | $OCH_3$ | CH | |
| $CH_2CH=CH_2$ | H | 0 | H | cyclopropyl | $CH_3$ | $CH_3$ | N | |
| $CH_2CH=CH_2$ | H | 0 | H | cyclopropyl | $CH_3$ | $OCH_3$ | N | |
| $CH_2CH=CH_2$ | H | 0 | H | cyclopropyl | $OCH_3$ | $OCH_3$ | N | |
| $CH_2CH=CH_2$ | H | 0 | H | cyclopropyl | Cl | $OCH_3$ | CH | |
| $CH_2CH=CH_2$ | H | 0 | H | cyclobutyl | $CH_3$ | $CH_3$ | CH | |
| $CH_2CH=CH_2$ | H | 0 | H | cyclobutyl | $CH_3$ | $OCH_3$ | CH | |
| $CH_2CH=CH_2$ | H | 0 | H | cyclobutyl | $OCH_3$ | $OCH_3$ | CH | |
| $CH_2CH=CH_2$ | H | 0 | H | cyclobutyl | $CH_3$ | $CH_3$ | N | |
| $CH_2CH=CH_2$ | H | 0 | H | cyclobutyl | $CH_3$ | $OCH_3$ | N | |
| $CH_2CH=CH_2$ | H | 0 | H | cyclobutyl | $OCH_3$ | $OCH_3$ | N | |
| $CH_2CH=CH_2$ | H | 0 | H | cyclobutyl | Cl | $OCH_3$ | CH | |
| $CH_2CH=CH_2$ | H | 0 | H | cyclopentyl | $CH_3$ | $CH_3$ | CH | |
| $CH_2CH=CH_2$ | H | 0 | H | cyclopentyl | $CH_3$ | $OCH_3$ | CH | |
| $CH_2CH=CH_2$ | H | 0 | H | cyclopentyl | $OCH_3$ | $OCH_3$ | CH | |
| $CH_2CH=CH_2$ | H | 0 | H | cyclopentyl | $CH_3$ | $CH_3$ | N | |
| $CH_2CH=CH_2$ | H | 0 | H | cyclopentyl | $CH_3$ | $OCH_3$ | N | |
| $CH_2CH=CH_2$ | H | 0 | H | cyclopentyl | $OCH_3$ | $OCH_3$ | N | |
| $CH_2CH=CH_2$ | H | 0 | H | cyclopentyl | Cl | $OCH_3$ | CH | |
| $CH_2CH=CH_2$ | H | 0 | H | cyclopropyl | $OCH_3$ | $OCH_2CH_3$ | CH | |
| $CH_2CH=CH_2$ | H | 0 | H | cyclopropyl | cyclopropyl | $OCH_3$ | CH | |
| $CH_2CH=CH_2$ | H | 0 | H | cyclopropyl | $OCH_3$ | $CH(OCH_3)_2$ | CH | |
| $CH_2CH=CH_2$ | H | 0 | H | cyclopropyl | $NHCH_3$ | $OCH_2CH_3$ | N | |
| $CH_2CH=CH_2$ | H | 0 | H | cyclopropyl | $NHCH_3$ | $OCH_3$ | N | |
| $CH_2CH=CH_2$ | H | 0 | H | cyclopropyl | $OCH_3$ | $OCH_2CH_3$ | N | |
| $CH_2CH=CH_2$ | H | 0 | H | cyclopropyl | $CH_2F$ | $CH_3$ | CH | |

Table III (cont.)

| R | $R_1$ | n | $R_2$ | R' | X | Y | Z | m.p.(°C) |
|---|---|---|---|---|---|---|---|---|
| Ph | H | O | Cl | cyclopropyl | $CH_3$ | $CH_3$ | CH | |
| Ph | H | O | Cl | cyclopropyl | $CH_3$ | $OCH_3$ | CH | |
| Ph | H | O | Cl | cyclopropyl | $OCH_3$ | $OCH_3$ | CH | |
| Ph | H | O | Cl | cyclopropyl | $CH_3$ | $CH_3$ | N | |
| Ph | H | O | Cl | cyclopropyl | $CH_3$ | $OCH_3$ | N | |
| Ph | H | O | Cl | cyclopropyl | $OCH_3$ | $OCH_3$ | N | |
| Ph | H | O | Cl | cyclopropyl | Cl | $OCH_3$ | CH | |
| Ph | H | O | Cl | cyclobutyl | $CH_3$ | $CH_3$ | CH | |
| Ph | H | O | Cl | cyclobutyl | $CH_3$ | $OCH_3$ | CH | |
| Ph | H | O | Cl | cyclobutyl | $OCH_3$ | $OCH_3$ | CH | |
| Ph | H | O | Cl | cyclobutyl | $CH_3$ | $CH_3$ | N | |
| Ph | H | O | Cl | cyclobutyl | $CH_3$ | $OCH_3$ | N | |
| Ph | H | O | Cl | cyclobutyl | $OCH_3$ | $OCH_3$ | N | |
| Ph | H | O | Cl | cyclobutyl | Cl | $OCH_3$ | CH | |
| Ph | H | O | Cl | cyclopentyl | $CH_3$ | $CH_3$ | CH | |
| Ph | H | O | Cl | cyclopentyl | $CH_3$ | $OCH_3$ | CH | |
| Ph | H | O | Cl | cyclopentyl | $OCH_3$ | $OCH_3$ | CH | |
| Ph | H | O | Cl | cyclopentyl | $CH_3$ | $CH_3$ | N | |
| Ph | H | O | Cl | cyclopentyl | $CH_3$ | $OCH_3$ | N | |
| Ph | H | O | Cl | cyclopentyl | $OCH_3$ | $OCH_3$ | N | |
| Ph | H | O | Cl | cyclopentyl | Cl | $OCH_3$ | CH | |
| Ph | H | O | Cl | cyclopropyl | $OCH_3$ | $OCH_2CH_3$ | CH | |
| Ph | H | O | Cl | cyclopropyl | cyclopropyl | $OCH_3$ | CH | |
| Ph | H | O | Cl | cyclopropyl | $OCH_3$ | $CH(OCH_3)_2$ | CH | |
| Ph | H | O | Cl | cyclopropyl | $NHCH_3$ | $OCH_2CH_3$ | N | |
| Ph | H | O | Cl | cyclopropyl | $NHCH_3$ | $OCH_3$ | N | |
| Ph | H | O | Cl | cyclopropyl | $OCH_3$ | $OCH_2CH_3$ | N | |
| Ph | H | O | Cl | cyclopropyl | $CH_2F$ | $CH_3$ | CH | |

Table III (cont.)

| R | $R_1$ | n | $R_2$ | R' | X | Y | Z | m.p.(°C) |
|---|---|---|---|---|---|---|---|---|
| $CH_2C{\equiv}CH$ | H | O | $CH_3$ | cyclopropyl | $CH_3$ | $CH_3$ | CH | |
| $CH_2C{\equiv}CH$ | H | O | $CH_3$ | cyclopropyl | $CH_3$ | $OCH_3$ | CH | |
| $CH_2C{\equiv}CH$ | H | O | $CH_3$ | cyclopropyl | $OCH_3$ | $OCH_3$ | CH | |
| $CH_2C{\equiv}CH$ | H | O | $CH_3$ | cyclopropyl | $CH_3$ | $CH_3$ | N | |
| $CH_2C{\equiv}CH$ | H | O | $CH_3$ | cyclopropyl | $CH_3$ | $OCH_3$ | N | |
| $CH_2C{\equiv}CH$ | H | O | $CH_3$ | cyclopropyl | $OCH_3$ | $OCH_3$ | N | |
| $CH_2C{\equiv}CH$ | H | O | $CH_3$ | cyclopropyl | Cl | $OCH_3$ | CH | |
| $CH_2C{\equiv}CH$ | H | O | $CH_3$ | cyclobutyl | $CH_3$ | $CH_3$ | CH | |
| $CH_2C{\equiv}CH$ | H | O | $CH_3$ | cyclobutyl | $CH_3$ | $OCH_3$ | CH | |
| $CH_2C{\equiv}CH$ | H | O | $CH_3$ | cyclobutyl | $OCH_3$ | $OCH_3$ | CH | |
| $CH_2C{\equiv}CH$ | H | O | $CH_3$ | cyclobutyl | $CH_3$ | $CH_3$ | N | |
| $CH_2C{\equiv}CH$ | H | O | $CH_3$ | cyclobutyl | $CH_3$ | $OCH_3$ | N | |
| $CH_2C{\equiv}CH$ | H | O | $CH_3$ | cyclobutyl | $OCH_3$ | $OCH_3$ | N | |
| $CH_2C{\equiv}CH$ | H | O | $CH_3$ | cyclobutyl | Cl | $OCH_3$ | CH | |
| $CH_2C{\equiv}CH$ | H | O | $CH_3$ | cyclopentyl | $CH_3$ | $CH_3$ | CH | |
| $CH_2C{\equiv}CH$ | H | O | $CH_3$ | cyclopentyl | $CH_3$ | $OCH_3$ | CH | |
| $CH_2C{\equiv}CH$ | H | O | $CH_3$ | cyclopentyl | $OCH_3$ | $OCH_3$ | CH | |
| $CH_2C{\equiv}CH$ | H | O | $CH_3$ | cyclopentyl | $CH_3$ | $CH_3$ | N | |
| $CH_2C{\equiv}CH$ | H | O | $CH_3$ | cyclopentyl | $CH_3$ | $OCH_3$ | N | |
| $CH_2C{\equiv}CH$ | H | O | $CH_3$ | cyclopentyl | $OCH_3$ | $OCH_3$ | N | |
| $CH_2C{\equiv}CH$ | H | O | $CH_3$ | cyclopentyl | Cl | $OCH_3$ | CH | |
| $CH_2C{\equiv}CH$ | H | O | $CH_3$ | cyclopropyl | $OCH_3$ | $OCH_2CH_3$ | CH | |
| $CH_2C{\equiv}CH$ | H | O | $CH_3$ | cyclopropyl | cyclopropyl | $OCH_3$ | CH | |
| $CH_2C{\equiv}CH$ | H | O | $CH_3$ | cyclopropyl | $OCH_3$ | $CH(OCH_3)_2$ | CH | |
| $CH_2C{\equiv}CH$ | H | O | $CH_3$ | cyclopropyl | $NHCH_3$ | $OCH_2CH_3$ | N | |
| $CH_2C{\equiv}CH$ | H | O | $CH_3$ | cyclopropyl | $NHCH_3$ | $OCH_3$ | N | |
| $CH_2C{\equiv}CH$ | H | O | $CH_3$ | cyclopropyl | $OCH_3$ | $OCH_2CH_3$ | N | |
| $CH_2C{\equiv}CH$ | H | O | $CH_3$ | cyclopropyl | $CH_2F$ | $CH_3$ | CH | |

Table III (cont.)

| R | $R_1$ | n | $R_2$ | R' | X | Y | Z | m.p.(°C) |
|---|---|---|---|---|---|---|---|---|
| $CH_3$ | H | O | H | cylopropyl | $CH_2CH_3$ | $CH_3$ | CH | |
| $CH_3$ | H | O | H | cyclopropyl | $CH_2CH_3$ | $OCH_3$ | CH | |
| $CH_3$ | H | O | H | cyclopropyl | $CH_2F$ | $CH_3$ | CH | |
| $CH_3$ | H | O | H | cyclopropyl | $OCF_2H$ | $CH_3$ | CH | |
| $CH_3$ | H | O | H | cyclopropyl | $OCF_2H$ | $OCH_3$ | CH | |
| $CH_3$ | H | O | H | cyclopropyl | $OCH_2CF_3$ | $OCH_3$ | CH | |
| $CH_3$ | H | O | H | cyclopropyl | $CH_2F$ | $OCH_3$ | CH | |
| $CH_3$ | H | O | H | cyclopropyl | $CH_2Cl$ | $OCH_3$ | N | |
| $CH_3$ | H | O | H | cyclopropyl | $SCH_3$ | $OCH_3$ | CH | |
| $CH_3$ | H | O | H | cyclopropyl | $SCH_2F$ | $OCH_3$ | CH | |
| $CH_3$ | H | O | H | cyclopropyl | Br | $OCH_3$ | CH | |
| $CH_3$ | H | O | H | cyclopropyl | $CH_2OCH_3$ | $OCH_3$ | N | |
| $CH_3$ | H | O | H | cyclopropyl | $OCH_2OCH_3$ | $OCH_3$ | CH | |
| $CH_3$ | H | O | H | cyclopropyl | $OCH_2OCH_2CH_3$ | $CH_3$ | CH | |
| $CH_3$ | H | O | H | cyclopropyl | $N(CH_3)_2$ | $OCH_3$ | N | |
| $CH_3$ | H | O | H | cyclopropyl | $NHCH_2CH_3$ | $CH_3$ | N | |
| $CH_3$ | H | O | H | cyclopropyl | $NHCH_3$ | $OCH_2CH_3$ | N | |
| $CH_3$ | H | O | H | cyclopropyl | $CH_3$ | $CH_2SCH_3$ | CH | |
| $CH_3$ | H | O | H | cyclopropyl | $OCH_3$ | $CH_2SO_2CH_3$ | CH | |
| $CH_3$ | H | O | H | cyclopropyl | $NH_2$ | $OCH_2CH_3$ | N | |
| $CH_3$ | H | O | H | cyclopropyl | $CH_3$ | $OCH_2CH{=}CH_2$ | CH | |
| $CH_3$ | H | O | H | cyclopropyl | $CH_2CH_3$ | $OCF_2H$ | CH | |
| $CH_3$ | H | O | H | cyclopropyl | $OCF_2H$ | $OCF_2H$ | CH | |
| $CH_3$ | H | O | H | cyclopropyl | $OCH_3$ | $OCH{=}CH_2$ | CH | |
| $CH_3$ | H | O | H | cyclopropyl | $OCH_3$ | $C(O)CH_3$ | N | |
| $CH_3$ | H | O | H | cyclopropyl | $CH_3$ | $N(OCH_3)CH_3$ | N | |
| $CH_3$ | H | O | H | cyclopropyl | $OCH(CH_3)_2$ | $OCF_2H$ | CH | |

**Table III (cont.)**

| R | $R_1$ | n | $R_2$ | R' | X | Y | Z | m.p.(°C) |
|---|---|---|---|---|---|---|---|---|
| $CH_3$ | H | 0 | H | $CH_3$ | $CH_3$ | $CH_3$ | CH | |
| $CH_3$ | H | 0 | H | $CH_3$ | $CH_3$ | $OCH_3$ | CH | |
| $CH_3$ | H | 0 | H | $CH_3$ | $OCH_3$ | $OCH_3$ | CH | |
| $CH_3$ | H | 0 | H | $CH_3$ | $CH_3$ | $CH_3$ | N | |
| $CH_3$ | H | 0 | H | $CH_3$ | $CH_3$ | $OCH_3$ | N | |
| $CH_3$ | H | 0 | H | $CH_3$ | $OCH_3$ | $OCH_3$ | N | |
| $CH_3$ | H | 0 | H | $CH_3$ | Cl | $OCH_3$ | CH | |
| $CH_3$ | H | 0 | H | $CH_2CH_3$ | $CH_3$ | $CH_3$ | CH | |
| $CH_3$ | H | 0 | H | $CH_2CH_3$ | $CH_3$ | $OCH_3$ | CH | |
| $CH_3$ | H | 0 | H | $CH_2CH_3$ | $OCH_3$ | $OCH_3$ | CH | |
| $CH_3$ | H | 0 | H | $CH_2CH_3$ | $CH_3$ | $CH_3$ | N | |
| $CH_3$ | H | 0 | H | $CH_2CH_3$ | $CH_3$ | $OCH_3$ | N | |
| $CH_3$ | H | 0 | H | $CH_2CH_3$ | $OCH_3$ | $OCH_3$ | N | |
| $CH_3$ | H | 0 | H | $CH_2CH_3$ | Cl | $OCH_3$ | CH | |
| $CH_3$ | H | 0 | H | $CH_2CH_2CH_3$ | $CH_3$ | $CH_3$ | CH | |
| $CH_3$ | H | 0 | H | $CH_2CH_2CH_3$ | $CH_3$ | $OCH_3$ | CH | |
| $CH_3$ | H | 0 | H | $CH_2CH_2CH_3$ | $OCH_3$ | $OCH_3$ | CH | |
| $CH_3$ | H | 0 | H | $CH_2CH_2CH_3$ | $CH_3$ | $CH_3$ | N | |
| $CH_3$ | H | 0 | H | $CH_2CH_2CH_3$ | $CH_3$ | $OCH_3$ | N | |
| $CH_3$ | H | 0 | H | $CH_2CH_2CH_3$ | $OCH_3$ | $OCH_3$ | N | |
| $CH_3$ | H | 0 | H | $CH_2CH_2CH_3$ | Cl | $OCH_3$ | CH | |
| $CH_3$ | H | 0 | H | $CH_2CH_2CH_2CH_3$ | $CH_3$ | $CH_3$ | CH | |
| $CH_3$ | H | 0 | H | $CH_2CH_2CH_2CH_3$ | $CH_3$ | $OCH_3$ | CH | |
| $CH_3$ | H | 0 | H | $CH_2CH_2CH_2CH_3$ | $OCH_3$ | $OCH_3$ | CH | |
| $CH_3$ | H | 0 | H | $CH_2CH_2CH_2CH_3$ | $CH_3$ | $CH_3$ | N | |
| $CH_3$ | H | 0 | H | $CH_2CH_2CH_2CH_3$ | $CH_3$ | $OCH_3$ | N | |
| $CH_3$ | H | 0 | H | $CH_2CH_2CH_2CH_3$ | $OCH_3$ | $OCH_3$ | N | |
| $CH_3$ | H | 0 | H | $CH_2CH_2CH_2CH_3$ | Cl | $OCH_3$ | CH | |

Table III (cont.)

| R | $R_1$ | n | $R_2$ | R' | X | Y | Z | m.p.(°C) |
|---|---|---|---|---|---|---|---|---|
| $CH_3$ | H | 0 | $CH_3$ | $CH_2OCH_2F$ | $CH_3$ | $CH_3$ | CH | |
| $CH_3$ | H | 0 | $CH_3$ | $CH_2OCH_2F$ | $CH_3$ | $OCH_3$ | CH | |
| $CH_3$ | H | 0 | $CH_3$ | $CH_2OCH_2F$ | $OCH_3$ | $OCH_3$ | CH | |
| $CH_3$ | H | 0 | $CH_3$ | $CH_2OCH_2F$ | $CH_3$ | $CH_3$ | N | |
| $CH_3$ | H | 0 | $CH_3$ | $CH_2OCH_2F$ | $CH_3$ | $OCH_3$ | N | |
| $CH_3$ | H | 0 | $CH_3$ | $CH_2OCH_2F$ | $OCH_3$ | $OCH_3$ | N | |
| $CH_3$ | H | 0 | $CH_3$ | $CH_2OCH_2F$ | Cl | $OCH_3$ | CH | |
| $CH_3$ | H | 0 | $CH_3$ | $CH_2SOCH_3$ | $CH_3$ | $CH_3$ | CH | |
| $CH_3$ | H | 0 | $CH_3$ | $CH_2SOCH_3$ | $CH_3$ | $OCH_3$ | CH | |
| $CH_3$ | H | 0 | $CH_3$ | $CH_2SOCH_3$ | $OCH_3$ | $OCH_3$ | CH | |
| $CH_3$ | H | 0 | $CH_3$ | $CH_2SOCH_3$ | $CH_3$ | $CH_3$ | N | |
| $CH_3$ | H | 0 | $CH_3$ | $CH_2SOCH_3$ | $CH_3$ | $OCH_3$ | N | |
| $CH_3$ | H | 0 | $CH_3$ | $CH_2SOCH_3$ | $OCH_3$ | $OCH_3$ | N | |
| $CH_3$ | H | 0 | $CH_3$ | $CH_2SOCH_3$ | Cl | $OCH_3$ | CH | |
| $CH_3$ | H | 0 | $CH_3$ | $CH_2SO_2CH_3$ | $CH_3$ | $CH_3$ | CH | |
| $CH_3$ | H | 0 | $CH_3$ | $CH_2SO_2CH_3$ | $CH_3$ | $OCH_3$ | CH | |
| $CH_3$ | H | 0 | $CH_3$ | $CH_2SO_2CH_3$ | $OCH_3$ | $OCH_3$ | CH | |
| $CH_3$ | H | 0 | $CH_3$ | $CH_2SO_2CH_3$ | $CH_3$ | $CH_3$ | N | |
| $CH_3$ | H | 0 | $CH_3$ | $CH_2SO_2CH_3$ | $CH_3$ | $OCH_3$ | N | |
| $CH_3$ | H | 0 | $CH_3$ | $CH_2SO_2CH_3$ | $OCH_3$ | $OCH_3$ | N | |
| $CH_3$ | H | 0 | $CH_3$ | $CH_2SO_2CH_3$ | Cl | $OCH_3$ | CH | |
| $CH_3$ | H | 0 | $CH_3$ | $CH_2CN$ | $CH_3$ | $CH_3$ | CH | |
| $CH_3$ | H | 0 | $CH_3$ | $CH_2CH_2CN$ | $CH_3$ | $OCH_3$ | CH | |
| $CH_3$ | H | 0 | $CH_3$ | $CH_2(CH_3)CN$ | $OCH_3$ | $OCH_3$ | CH | |
| $CH_3$ | H | 0 | $CH_3$ | $CH_2NO_2$ | $CH_3$ | $CH_3$ | N | |
| $CH_3$ | H | 0 | $CH_3$ | $CH_2CH_2NO_2$ | $CH_3$ | $OCH_3$ | N | |
| $CH_3$ | H | 0 | $CH_3$ | $CH_2CN$ | $OCH_3$ | $OCH_3$ | N | |
| $CH_3$ | H | 0 | $CH_3$ | $CH_2CN$ | Cl | $OCH_3$ | CH | |

Table III (cont.)

| R | $R_1$ | n | $R_2$ | R' | X | Y | Z | m.p.(°C) |
|---|---|---|---|---|---|---|---|---|
| $CH_3$ | H | 0 | Br | $CH_3$ | $CH_3$ | $CH_3$ | CH | |
| $CH_3$ | H | 0 | Br | $CH_3$ | $CH_3$ | $OCH_3$ | CH | |
| $CH_3$ | H | 0 | Br | $CH_3$ | $OCH_3$ | $OCH_3$ | CH | |
| $CH_3$ | H | 0 | Br | $CH_3$ | $CH_3$ | $CH_3$ | N | |
| $CH_3$ | H | 0 | Br | $CH_3$ | $CH_3$ | $OCH_3$ | N | |
| $CH_3$ | H | 0 | Br | $CH_3$ | $OCH_3$ | $OCH_3$ | N | |
| $CH_3$ | H | 0 | Br | $CH_3$ | Cl | $OCH_3$ | CH | |
| $CH_3$ | H | 0 | Br | $CH_2CH_3$ | $CH_3$ | $CH_3$ | CH | |
| $CH_3$ | H | 0 | Br | $CH_2CH_3$ | $CH_3$ | $OCH_3$ | CH | |
| $CH_3$ | H | 0 | Br | $CH_2CH_3$ | $OCH_3$ | $OCH_3$ | CH | |
| $CH_3$ | H | 0 | Br | $CH_2CH_3$ | $CH_3$ | $CH_3$ | N | |
| $CH_3$ | H | 0 | Br | $CH_2CH_3$ | $CH_3$ | $OCH_3$ | N | |
| $CH_3$ | H | 0 | Br | $CH_2CH_3$ | $OCH_3$ | $OCH_3$ | N | |
| $CH_3$ | H | 0 | Br | $CH_2CH_3$ | Cl | $OCH_3$ | CH | |
| $CH_3$ | H | 0 | Br | $CH_2CH_2CH_3$ | $CH_3$ | $CH_3$ | CH | |
| $CH_3$ | H | 0 | Br | $CH_2CH_2CH_3$ | $CH_3$ | $OCH_3$ | CH | |
| $CH_3$ | H | 0 | Br | $CH_2CH_2CH_3$ | $OCH_3$ | $OCH_3$ | CH | |
| $CH_3$ | H | 0 | Br | $CH_2CH_2CH_3$ | $CH_3$ | $CH_3$ | N | |
| $CH_3$ | H | 0 | Br | $CH_2CH_2CH_3$ | $CH_3$ | $OCH_3$ | N | |
| $CH_3$ | H | 0 | Br | $CH_2CH_2CH_3$ | $OCH_3$ | $OCH_3$ | N | |
| $CH_3$ | H | 0 | Br | $CH_2CH_2CH_3$ | Cl | $OCH_3$ | CH | |
| $CH_3$ | H | 0 | Br | $CH_2CH_2CH_2CH_3$ | $CH_3$ | $CH_3$ | CH | |
| $CH_3$ | H | 0 | Br | $CH_2CH_2CH_2CH_3$ | $CH_3$ | $OCH_3$ | CH | |
| $CH_3$ | H | 0 | Br | $CH_2CH_2CH_2CH_3$ | $OCH_3$ | $OCH_3$ | CH | |
| $CH_3$ | H | 0 | Br | $CH_2CH_2CH_2CH_3$ | $CH_3$ | $CH_3$ | N | |
| $CH_3$ | H | 0 | Br | $CH_2CH_2CH_2CH_3$ | $CH_3$ | $OCH_3$ | N | |
| $CH_3$ | H | 0 | Br | $CH_2CH_2CH_2CH_3$ | $OCH_3$ | $OCH_3$ | N | |
| $CH_3$ | H | 0 | Br | $CH_2CH_2CH_2CH_3$ | Cl | $OCH_3$ | CH | |

Table IV

| R | $R_1$ | n | $R_2$ | R' | X | Y | Z | m.p.(°C) |
|---|---|---|---|---|---|---|---|---|
| $CH_3$ | H | O | H | cyclopropyl | $CH_3$ | $CH_3$ | CH | |
| $CH_3$ | H | O | H | cyclopropyl | $CH_3$ | $OCH_3$ | CH | |
| $CH_3$ | H | O | H | cyclopropyl | $OCH_3$ | $OCH_3$ | CH | |
| $CH_3$ | H | O | H | cyclopropyl | $CH_3$ | $CH_3$ | N | |
| $CH_3$ | H | O | H | cyclopropyl | $CH_3$ | $OCH_3$ | N | |
| $CH_3$ | H | O | H | cyclopropyl | $OCH_3$ | $OCH_3$ | N | |
| $CH_3$ | H | O | H | cyclopropyl | Cl | $OCH_3$ | CH | |
| $CH_3$ | H | O | H | cyclobutyl | $CH_3$ | $CH_3$ | CH | |
| $CH_3$ | H | O | H | cyclobutyl | $CH_3$ | $OCH_3$ | CH | |
| $CH_3$ | H | O | H | cyclobutyl | $OCH_3$ | $OCH_3$ | CH | |
| $CH_3$ | H | O | H | cyclobutyl | $CH_3$ | $CH_3$ | N | |
| $CH_3$ | H | O | H | cyclobutyl | $CH_3$ | $OCH_3$ | N | |
| $CH_3$ | H | O | H | cyclobutyl | $OCH_3$ | $OCH_3$ | N | |
| $CH_3$ | H | O | H | cyclobutyl | Cl | $OCH_3$ | CH | |
| $CH_3$ | H | O | H | cyclopentyl | $CH_3$ | $CH_3$ | CH | |
| $CH_3$ | H | O | H | cyclopentyl | $CH_3$ | $OCH_3$ | CH | |
| $CH_3$ | H | O | H | cyclopentyl | $OCH_3$ | $OCH_3$ | CH | |
| $CH_3$ | H | O | H | cyclopentyl | $CH_3$ | $CH_3$ | N | |
| $CH_3$ | H | O | H | cyclopentyl | $CH_3$ | $OCH_3$ | N | |
| $CH_3$ | H | O | H | cyclopentyl | $OCH_3$ | $OCH_3$ | N | |
| $CH_3$ | H | O | H | cyclopentyl | Cl | $OCH_3$ | CH | |
| $CH_3$ | H | O | H | cyclopropyl | $OCH_3$ | $OCH_2CH_3$ | CH | |
| $CH_3$ | H | O | H | cyclopropyl | cyclopropyl | $OCH_3$ | CH | |
| $CH_3$ | H | O | H | cyclopropyl | $OCH_3$ | $CH(OCH_3)_2$ | CH | |
| $CH_3$ | H | O | H | cyclopropyl | $NHCH_3$ | $OCH_2CH_3$ | N | |
| $CH_3$ | H | O | H | cyclopropyl | $NHCH_3$ | $OCH_3$ | N | |
| $CH_3$ | H | O | H | cyclopropyl | $OCH_3$ | $OCH_2CH_3$ | N | |
| $CH_3$ | H | O | H | cyclopropyl | $CH_2F$ | $CH_3$ | CH | |

Table IV (cont.)

| R | $R_1$ | n | $R_2$ | R' | X | Y | Z | m.p.(°C) |
|---|---|---|---|---|---|---|---|---|
| $CH_3$ | H | 0 | $CH_2F$ | cyclopropyl | $CH_3$ | $CH_3$ | CH | |
| $CH_3$ | H | 0 | $CH_2F$ | cyclopropyl | $CH_3$ | $OCH_3$ | CH | |
| $CH_3$ | H | 0 | $CH_2F$ | cyclopropyl | $OCH_3$ | $OCH_3$ | CH | |
| $CH_3$ | H | 0 | $CH_2F$ | cyclopropyl | $CH_3$ | $CH_3$ | N | |
| $CH_3$ | H | 0 | $CH_2F$ | cyclopropyl | $CH_3$ | $OCH_3$ | N | |
| $CH_3$ | H | 0 | $CH_2F$ | cyclopropyl | $OCH_3$ | $OCH_3$ | N | |
| $CH_3$ | H | 0 | $CH_2F$ | cyclopropyl | Cl | $OCH_3$ | CH | |
| $CH_3$ | H | 0 | $CH_2F$ | cyclobutyl | $CH_3$ | $CH_3$ | CH | |
| $CH_3$ | H | 0 | $CH_2F$ | cyclobutyl | $CH_3$ | $OCH_3$ | CH | |
| $CH_3$ | H | 0 | $CH_2F$ | cyclobutyl | $OCH_3$ | $OCH_3$ | CH | |
| $CH_3$ | H | 0 | $CH_2F$ | cyclobutyl | $CH_3$ | $CH_3$ | N | |
| $CH_3$ | H | 0 | $CH_2F$ | cyclobutyl | $CH_3$ | $OCH_3$ | N | |
| $CH_3$ | H | 0 | $CH_2F$ | cyclobutyl | $OCH_3$ | $OCH_3$ | N | |
| $CH_3$ | H | 0 | $CH_2F$ | cyclobutyl | Cl | $OCH_3$ | CH | |
| $CH_3$ | H | 0 | $CH_2F$ | cyclopentyl | $CH_3$ | $CH_3$ | CH | |
| $CH_3$ | H | 0 | $CH_2F$ | cyclopentyl | $CH_3$ | $OCH_3$ | CH | |
| $CH_3$ | H | 0 | $CH_2F$ | cyclopentyl | $OCH_3$ | $OCH_3$ | CH | |
| $CH_3$ | H | 0 | $CH_2F$ | cyclopentyl | $CH_3$ | $CH_3$ | N | |
| $CH_3$ | H | 0 | $CH_2F$ | cyclopentyl | $CH_3$ | $OCH_3$ | N | |
| $CH_3$ | H | 0 | $CH_2F$ | cyclopentyl | $OCH_3$ | $OCH_3$ | N | |
| $CH_3$ | H | 0 | $CH_2F$ | cyclopentyl | Cl | $OCH_3$ | CH | |
| $CH_3$ | H | 0 | $CH_2F$ | cyclopropyl | $OCH_3$ | $OCH_2CH_3$ | CH | |
| $CH_3$ | H | 0 | $CH_2F$ | cyclopropyl | cyclopropyl | $OCH_3$ | CH | |
| $CH_3$ | H | 0 | $CH_2F$ | cyclopropyl | $OCH_3$ | $CH(OCH_3)_2$ | CH | |
| $CH_3$ | H | 0 | $CH_2F$ | cyclopropyl | $NHCH_3$ | $OCH_2CH_3$ | N | |
| $CH_3$ | H | 0 | $CH_2F$ | cyclopropyl | $NHCH_3$ | $OCH_3$ | N | |
| $CH_3$ | H | 0 | $CH_2F$ | cyclopropyl | $OCH_3$ | $OCH_2CH_3$ | N | |
| $CH_3$ | H | 0 | $CH_2F$ | cyclopropyl | $CH_2F$ | $CH_3$ | CH | |

Table IV (cont.)

| R | $R_1$ | n | $R_2$ | R' | X | Y | Z | m.p.(°C) |
|---|---|---|---|---|---|---|---|---|
| $CH_3$ | H | O | CN | cyclopropyl | $CH_3$ | $CH_3$ | CH | |
| $CH_3$ | H | O | CN | cyclopropyl | $CH_3$ | $OCH_3$ | CH | |
| $CH_3$ | H | O | CN | cyclopropyl | $OCH_3$ | $OCH_3$ | CH | |
| $CH_3$ | H | O | CN | cyclopropyl | $CH_3$ | $CH_3$ | N | |
| $CH_3$ | H | O | CN | cyclopropyl | $CH_3$ | $OCH_3$ | N | |
| $CH_3$ | H | O | CN | cyclopropyl | $OCH_3$ | $OCH_3$ | N | |
| $CH_3$ | H | O | CN | cyclopropyl | Cl | $OCH_3$ | CH | |
| $CH_3$ | H | O | CN | cyclobutyl | $CH_3$ | $CH_3$ | CH | |
| $CH_3$ | H | O | CN | cyclobutyl | $CH_3$ | $OCH_3$ | CH | |
| $CH_3$ | H | O | CN | cyclobutyl | $OCH_3$ | $OCH_3$ | CH | |
| $CH_3$ | H | O | CN | cyclobutyl | $CH_3$ | $CH_3$ | N | |
| $CH_3$ | H | O | CN | cyclobutyl | $CH_3$ | $OCH_3$ | N | |
| $CH_3$ | H | O | CN | cyclobutyl | $OCH_3$ | $OCH_3$ | N | |
| $CH_3$ | H | O | CN | cyclobutyl | Cl | $OCH_3$ | CH | |
| $CH_3$ | H | O | CN | cyclopentyl | $CH_3$ | $CH_3$ | CH | |
| $CH_3$ | H | O | CN | cyclopentyl | $CH_3$ | $OCH_3$ | CH | |
| $CH_3$ | H | O | CN | cyclopentyl | $OCH_3$ | $OCH_3$ | CH | |
| $CH_3$ | H | O | CN | cyclopentyl | $CH_3$ | $CH_3$ | N | |
| $CH_3$ | H | O | CN | cyclopentyl | $CH_3$ | $OCH_3$ | N | |
| $CH_3$ | H | O | CN | cyclopentyl | $OCH_3$ | $OCH_3$ | N | |
| $CH_3$ | H | O | CN | cyclopentyl | Cl | $OCH_3$ | CH | |
| $CH_3$ | H | O | CN | cyclopropyl | $OCH_3$ | $OCH_2CH_3$ | CH | |
| $CH_3$ | H | O | CN | cyclopropyl | cyclopropyl | $OCH_3$ | CH | |
| $CH_3$ | H | O | CN | cyclopropyl | $OCH_3$ | $CH(OCH_3)_2$ | CH | |
| $CH_3$ | H | O | CN | cyclopropyl | $NHCH_3$ | $OCH_2CH_3$ | N | |
| $CH_3$ | H | O | CN | cyclopropyl | $NHCH_3$ | $OCH_3$ | N | |
| $CH_3$ | H | O | CN | cyclopropyl | $OCH_3$ | $OCH_2CH_3$ | N | |
| $CH_3$ | H | O | CN | cyclopropyl | $CH_2F$ | $CH_3$ | CH | |

## Table IV (cont.)

| R | $R_1$ | n | $R_2$ | R' | X | Y | Z | m.p.(°C) |
|---|---|---|---|---|---|---|---|---|
| $CH_2CH_3$ | H | 0 | H | cyclopropyl | $CH_3$ | $CH_3$ | CH | |
| $CH_2CH_3$ | H | 0 | H | cyclopropyl | $CH_3$ | $OCH_3$ | CH | |
| $CH_2CH_3$ | H | 0 | H | cyclopropyl | $OCH_3$ | $OCH_3$ | CH | |
| $CH_2CH_3$ | H | 0 | H | cyclopropyl | $CH_3$ | $CH_3$ | N | |
| $CH_2CH_3$ | H | 0 | H | cyclopropyl | $CH_3$ | $OCH_3$ | N | |
| $CH_2CH_3$ | H | 0 | H | cyclopropyl | $OCH_3$ | $OCH_3$ | N | |
| $CH_2CH_3$ | H | 0 | H | cyclopropyl | Cl | $OCH_3$ | CH | |
| $CH_2CH_3$ | H | 0 | H | cyclobutyl | $CH_3$ | $CH_3$ | CH | |
| $CH_2CH_3$ | H | 0 | H | cyclobutyl | $CH_3$ | $OCH_3$ | CH | |
| $CH_2CH_3$ | H | 0 | H | cyclobutyl | $OCH_3$ | $OCH_3$ | CH | |
| $CH_2CH_3$ | H | 0 | H | cyclobutyl | $CH_3$ | $CH_3$ | N | |
| $CH_2CH_3$ | H | 0 | H | cyclobutyl | $CH_3$ | $OCH_3$ | N | |
| $CH_2CH_3$ | H | 0 | H | cyclobutyl | $OCH_3$ | $OCH_3$ | N | |
| $CH_2CH_3$ | H | 0 | H | cyclobutyl | Cl | $OCH_3$ | CH | |
| $CH_2CH_3$ | H | 0 | H | cyclopentyl | $CH_3$ | $CH_3$ | CH | |
| $CH_2CH_3$ | H | 0 | H | cyclopentyl | $CH_3$ | $OCH_3$ | CH | |
| $CH_2CH_3$ | H | 0 | H | cyclopentyl | $OCH_3$ | $OCH_3$ | CH | |
| $CH_2CH_3$ | H | 0 | H | cyclopentyl | $CH_3$ | $CH_3$ | N | |
| $CH_2CH_3$ | H | 0 | H | cyclopentyl | $CH_3$ | $OCH_3$ | N | |
| $CH_2CH_3$ | H | 0 | H | cyclopentyl | $OCH_3$ | $OCH_3$ | N | |
| $CH_2CH_3$ | H | 0 | H | cyclopentyl | Cl | $OCH_3$ | CH | |
| $CH_2CH_3$ | H | 0 | H | cyclopropyl | $OCH_3$ | $OCH_2CH_3$ | CH | |
| $CH_2CH_3$ | H | 0 | H | cyclopropyl | cyclopropyl | $OCH_3$ | CH | |
| $CH_2CH_3$ | H | 0 | H | cyclopropyl | $OCH_3$ | $CH(OCH_3)_2$ | CH | |
| $CH_2CH_3$ | H | 0 | H | cyclopropyl | $NHCH_3$ | $OCH_2CH_3$ | N | |
| $CH_2CH_3$ | H | 0 | H | cyclopropyl | $NHCH_3$ | $OCH_3$ | N | |
| $CH_2CH_3$ | H | 0 | H | cyclopropyl | $OCH_3$ | $OCH_2CH_3$ | N | |
| $CH_2CH_3$ | H | 0 | H | cyclopropyl | $CH_2F$ | $CH_3$ | CH | |

Table IV (cont.)

| R | $R_1$ | n | $R_2$ | R' | X | Y | Z | m.p.(°C) |
|---|---|---|---|---|---|---|---|---|
| $CH_2C{\equiv}CH$ | H | O | H | cyclopropyl | $CH_3$ | $CH_3$ | CH | |
| $CH_2C{\equiv}CH$ | H | O | H | cyclopropyl | $CH_3$ | $OCH_3$ | CH | |
| $CH_2C{\equiv}CH$ | H | O | H | cyclopropyl | $OCH_3$ | $OCH_3$ | CH | |
| $CH_2C{\equiv}CH$ | H | O | H | cyclopropyl | $CH_3$ | $CH_3$ | N | |
| $CH_2C{\equiv}CH$ | H | O | H | cyclopropyl | $CH_3$ | $OCH_3$ | N | |
| $CH_2C{\equiv}CH$ | H | O | H | cyclopropyl | $OCH_3$ | $OCH_3$ | N | |
| $CH_2C{\equiv}CH$ | H | O | H | cyclopropyl | Cl | $OCH_3$ | CH | |
| $CH_2C{\equiv}CH$ | H | O | H | cyclobutyl | $CH_3$ | $CH_3$ | CH | |
| $CH_2C{\equiv}CH$ | H | O | H | cyclobutyl | $CH_3$ | $OCH_3$ | CH | |
| $CH_2C{\equiv}CH$ | H | O | H | cyclobutyl | $OCH_3$ | $OCH_3$ | CH | |
| $CH_2C{\equiv}CH$ | H | O | H | cyclobutyl | $CH_3$ | $CH_3$ | N | |
| $CH_2C{\equiv}CH$ | H | O | H | cyclobutyl | $CH_3$ | $OCH_3$ | N | |
| $CH_2C{\equiv}CH$ | H | O | H | cyclobutyl | $OCH_3$ | $OCH_3$ | N | |
| $CH_2C{\equiv}CH$ | H | O | H | cyclobutyl | Cl | $OCH_3$ | CH | |
| $CH_2{\equiv}CH$ | H | O | H | cyclopentyl | $CH_3$ | $CH_3$ | CH | |
| $CH_2C{\equiv}CH$ | H | O | H | cyclopentyl | $CH_3$ | $OCH_3$ | CH | |
| $CH_2C{\equiv}CH$ | H | O | H | cyclopentyl | $OCH_3$ | $OCH_3$ | CH | |
| $CH_2C{\equiv}CH$ | H | O | H | cyclopentyl | $CH_3$ | $CH_3$ | N | |
| $CH_2C{\equiv}CH$ | H | O | H | cyclopentyl | $CH_3$ | $OCH_3$ | N | |
| $CH_2C{\equiv}CH$ | H | O | H | cyclopentyl | $OCH_3$ | $OCH_3$ | N | |
| $CH_2C{\equiv}CH$ | H | O | H | cyclopentyl | Cl | $OCH_3$ | CH | |
| $CH_2C{\equiv}CH$ | H | O | H | cyclopropyl | $OCH_3$ | $OCH_2CH_3$ | CH | |
| $CH_2C{\equiv}CH$ | H | O | H | cyclopropyl | cyclopropyl | $OCH_3$ | CH | |
| $CH_2C{\equiv}CH$ | H | O | H | cyclopropyl | $OCH_3$ | $CH(OCH_3)_2$ | CH | |
| $CH_2C{\equiv}CH$ | H | O | H | cyclopropyl | $NHCH_3$ | $OCH_2CH_3$ | N | |
| $CH_2C{\equiv}CH$ | H | O | H | cyclopropyl | $NHCH_3$ | $OCH_3$ | N | |
| $CH_2C{\equiv}CH$ | H | O | H | cyclopropyl | $OCH_3$ | $OCH_2CH_3$ | N | |
| $CH_2C{\equiv}CH$ | H | O | H | cyclopropyl | $CH_2F$ | $CH_3$ | CH | |

Table IV (cont.)

| R | $R_1$ | n | $R_2$ | R' | X | Y | Z | m.p.(°C) |
|---|---|---|---|---|---|---|---|---|
| H | H | O | Cl | cyclopropyl | $CH_3$ | $CH_3$ | CH | |
| H | H | O | Cl | cyclopropyl | $CH_3$ | $OCH_3$ | CH | |
| H | H | O | Cl | cyclopropyl | $OCH_3$ | $OCH_3$ | CH | |
| H | H | O | Cl | cyclopropyl | $CH_3$ | $CH_3$ | N | |
| H | H | O | Cl | cyclopropyl | $CH_3$ | $OCH_3$ | N | |
| H | H | O | Cl | cyclopropyl | $OCH_3$ | $OCH_3$ | N | |
| H | H | O | Cl | cyclopropyl | Cl | $OCH_3$ | CH | |
| H | H | O | Cl | cyclobutyl | $CH_3$ | $CH_3$ | CH | |
| H | H | O | Cl | cyclobutyl | $CH_3$ | $OCH_3$ | CH | |
| H | H | O | Cl | cyclobutyl | $OCH_3$ | $OCH_3$ | CH | |
| H | H | O | Cl | cyclobutyl | $CH_3$ | $CH_3$ | N | |
| H | H | O | Cl | cyclobutyl | $CH_3$ | $OCH_3$ | N | |
| H | H | O | Cl | cyclobutyl | $OCH_3$ | $OCH_3$ | N | |
| H | H | O | Cl | cyclobutyl | Cl | $OCH_3$ | CH | |
| H | H | O | Cl | cyclopentyl | $CH_3$ | $CH_3$ | CH | |
| H | H | O | Cl | cyclopentyl | $CH_3$ | $OCH_3$ | CH | |
| H | H | O | Cl | cyclopentyl | $OCH_3$ | $OCH_3$ | CH | |
| H | H | O | Cl | cyclopentyl | $CH_3$ | $CH_3$ | N | |
| H | H | O | Cl | cyclopentyl | $CH_3$ | $OCH_3$ | N | |
| H | H | O | Cl | cyclopentyl | $OCH_3$ | $OCH_3$ | N | |
| H | H | O | Cl | cyclopentyl | Cl | $OCH_3$ | CH | |
| H | H | O | Cl | cyclopropyl | $OCH_3$ | $OCH_2CH_3$ | CH | |
| H | H | O | Cl | cyclopropyl | cyclopropyl | $OCH_3$ | CH | |
| H | H | O | Cl | cyclopropyl | $OCH_3$ | $CH(OCH_3)_2$ | CH | |
| H | H | O | Cl | cyclopropyl | $NHCH_3$ | $OCH_2CH_3$ | N | |
| H | H | O | Cl | cyclopropyl | $NHCH_3$ | $OCH_3$ | N | |
| H | H | O | Cl | cyclopropyl | $OCH_3$ | $OCH_2CH_3$ | N | |
| H | H | O | Cl | cyclopropyl | $CH_2F$ | $CH_3$ | CH | |

Table IV (cont.)

| R | $R_1$ | n | $R_2$ | R' | X | Y | Z | m.p.(°C) |
|---|---|---|---|---|---|---|---|---|
| H | H | 0 | $CH_3$ | cyclopropyl | $CH_3$ | $CH_3$ | CH | |
| H | H | 0 | $CH_3$ | cyclopropyl | $CH_3$ | $OCH_3$ | CH | |
| H | H | 0 | $CH_3$ | cyclopropyl | $OCH_3$ | $OCH_3$ | CH | |
| H | H | 0 | $CH_3$ | cyclopropyl | $CH_3$ | $CH_3$ | N | |
| H | H | 0 | $CH_3$ | cyclopropyl | $CH_3$ | $OCH_3$ | N | |
| H | H | 0 | $CH_3$ | cyclopropyl | $OCH_3$ | $OCH_3$ | N | |
| H | H | 0 | $CH_3$ | cyclopropyl | Cl | $OCH_3$ | CH | |
| H | H | 0 | $CH_3$ | cyclobutyl | $CH_3$ | $CH_3$ | CH | |
| H | H | 0 | $CH_3$ | cyclobutyl | $CH_3$ | $OCH_3$ | CH | |
| H | H | 0 | $CH_3$ | cyclobutyl | $OCH_3$ | $OCH_3$ | CH | |
| H | H | 0 | $CH_3$ | cyclobutyl | $CH_3$ | $CH_3$ | N | |
| H | H | 0 | $CH_3$ | cyclobutyl | $CH_3$ | $OCH_3$ | N | |
| H | H | 0 | $CH_3$ | cyclobutyl | $OCH_3$ | $OCH_3$ | N | |
| H | H | 0 | $CH_3$ | cyclobutyl | Cl | $OCH_3$ | CH | |
| H | H | 0 | $CH_3$ | cyclopentyl | $CH_3$ | $CH_3$ | CH | |
| H | H | 0 | $CH_3$ | cyclopentyl | $CH_3$ | $OCH_3$ | CH | |
| H | H | 0 | $CH_3$ | cyclopentyl | $OCH_3$ | $OCH_3$ | CH | |
| H | H | 0 | $CH_3$ | cyclopentyl | $CH_3$ | $CH_3$ | N | |
| H | H | 0 | $CH_3$ | cyclopentyl | $CH_3$ | $OCH_3$ | N | |
| H | H | 0 | $CH_3$ | cyclopentyl | $OCH_3$ | $OCH_3$ | N | |
| H | H | 0 | $CH_3$ | cyclopentyl | Cl | $OCH_3$ | CH | |
| H | H | 0 | $CH_3$ | cyclopropyl | $OCH_3$ | $OCH_2CH_3$ | CH | |
| H | H | 0 | $CH_3$ | cyclopropyl | cyclopropyl | $OCH_3$ | CH | |
| H | H | 0 | $CH_3$ | cyclopropyl | $OCH_3$ | $CH(OCH_3)_2$ | CH | |
| H | H | 0 | $CH_3$ | cyclopropyl | $NHCH_3$ | $OCH_2CH_3$ | N | |
| H | H | 0 | $CH_3$ | cyclopropyl | $NHCH_3$ | $OCH_3$ | N | |
| H | H | 0 | $CH_3$ | cyclopropyl | $OCH_3$ | $OCH_2CH_3$ | N | |
| H | H | 0 | $CH_3$ | cyclopropyl | $CH_2F$ | $CH_3$ | CH | |

Table IV (cont.)

| R | $R_1$ | n | $R_2$ | R' | X | Y | Z | m.p.(°C) |
|---|---|---|---|---|---|---|---|---|
| $CH_3$ | H | O | H | cyclobutyl | $CH_2CH_3$ | $CH_3$ | CH | |
| $CH_3$ | H | O | H | cyclobutyl | $CH_2CH_3$ | $OCH_3$ | CH | |
| $CH_3$ | H | O | H | cyclobutyl | $CH_2F$ | $CH_3$ | CH | |
| $CH_3$ | H | O | H | cyclobutyl | $OCF_2H$ | $CH_3$ | CH | |
| $CH_3$ | H | O | H | cyclobutyl | $OCF_2H$ | $OCH_3$ | CH | |
| $CH_3$ | H | O | H | cyclobutyl | $OCH_2CF_3$ | $OCH_3$ | CH | |
| $CH_3$ | H | O | H | cyclobutyl | $CH_2F$ | $OCH_3$ | CH | |
| $CH_3$ | H | O | H | cyclobutyl | $CH_2Cl$ | $OCH_3$ | N | |
| $CH_3$ | H | O | H | cyclobutyl | $SCH_3$ | $OCH_3$ | CH | |
| $CH_3$ | H | O | H | cyclobutyl | $SCH_2F$ | $OCH_3$ | CH | |
| $CH_3$ | H | O | H | cyclobutyl | Br | $OCH_3$ | CH | |
| $CH_3$ | H | O | H | cyclobutyl | $CH_2OCH_3$ | $OCH_3$ | N | |
| $CH_3$ | H | O | H | cyclobutyl | $OCH_2OCH_3$ | $OCH_3$ | CH | |
| $CH_3$ | H | O | H | cyclobutyl | $OCH_2OCH_2CH_3$ | $CH_3$ | CH | |
| $CH_3$ | H | O | H | cyclobutyl | $N(CH_3)_2$ | $OCH_3$ | N | |
| $CH_3$ | H | O | H | cyclobutyl | $NHCH_2CH_3$ | $CH_3$ | N | |
| $CH_3$ | H | O | H | cyclobutyl | $NHCH_3$ | $OCH_2CH_3$ | N | |
| $CH_3$ | H | O | H | cyclobutyl | $CH_3$ | $CH_2SCH_3$ | CH | |
| $CH_3$ | H | O | H | cyclobutyl | $OCH_3$ | $CH_2SO_2CH_3$ | CH | |
| $CH_3$ | H | O | H | cyclobutyl | $NH_2$ | $OCH_2CH_3$ | N | |
| $CH_3$ | H | O | H | cyclobutyl | $CH_3$ | $OCH_2CH=CH_2$ | CH | |
| $CH_3$ | H | O | H | cyclobutyl | $CH_2CH_3$ | $OCF_2H$ | CH | |
| $CH_3$ | H | O | H | cyclobutyl | $OCF_2H$ | $OCF_2H$ | CH | |
| $CH_3$ | H | O | H | cyclobutyl | $OCH_3$ | $OCH=CH_2$ | CH | |
| $CH_3$ | H | O | H | cyclobutyl | $OCH_3$ | $C(O)CH_3$ | N | |
| $CH_3$ | H | O | H | cyclobutyl | $CH_3$ | $N(OCH_3)CH_3$ | N | |
| $CH_3$ | H | O | H | cyclobutyl | $OCH(CH_3)_2$ | $OCF_2H$ | CH | |

Table IV (cont.)

| R | $R_1$ | n | $R_2$ | R' | X | Y | Z | m.p.(°C) |
|---|---|---|---|---|---|---|---|---|
| $CH_3$ | H | 0 | H | $CH_3$ | $CH_3$ | $CH_3$ | CH | |
| $CH_3$ | H | 0 | H | $CH_3$ | $CH_3$ | $OCH_3$ | CH | |
| $CH_3$ | H | 0 | H | $CH_3$ | $OCH_3$ | $OCH_3$ | CH | |
| $CH_3$ | H | 0 | H | $CH_3$ | $CH_3$ | $CH_3$ | N | |
| $CH_3$ | H | 0 | H | $CH_3$ | $CH_3$ | $OCH_3$ | N | |
| $CH_3$ | H | 0 | H | $CH_3$ | $OCH_3$ | $OCH_3$ | N | |
| $CH_3$ | H | 0 | H | $CH_3$ | Cl | $OCH_3$ | CH | |
| $CH_3$ | H | 0 | H | $CH_2CH_3$ | $CH_3$ | $CH_3$ | CH | |
| $CH_3$ | H | 0 | H | $CH_2CH_3$ | $CH_3$ | $OCH_3$ | CH | |
| $CH_3$ | H | 0 | H | $CH_2CH_3$ | $OCH_3$ | $OCH_3$ | CH | |
| $CH_3$ | H | 0 | H | $CH_2CH_3$ | $CH_3$ | $CH_3$ | N | |
| $CH_3$ | H | 0 | H | $CH_2CH_3$ | $CH_3$ | $OCH_3$ | N | |
| $CH_3$ | H | 0 | H | $CH_2CH_3$ | $OCH_3$ | $OCH_3$ | N | |
| $CH_3$ | H | 0 | H | $CH_2CH_3$ | Cl | $OCH_3$ | CH | |
| $CH_3$ | H | 0 | H | $CH_2CH_2CH_3$ | $CH_3$ | $CH_3$ | CH | |
| $CH_3$ | H | 0 | H | $CH_2CH_2CH_3$ | $CH_3$ | $OCH_3$ | CH | |
| $CH_3$ | H | 0 | H | $CH_2CH_2CH_3$ | $OCH_3$ | $OCH_3$ | CH | |
| $CH_3$ | H | 0 | H | $CH_2CH_2CH_3$ | $CH_3$ | $CH_3$ | N | |
| $CH_3$ | H | 0 | H | $CH_2CH_2CH_3$ | $CH_3$ | $OCH_3$ | N | |
| $CH_3$ | H | 0 | H | $CH_2CH_2CH_3$ | $OCH_3$ | $OCH_3$ | N | |
| $CH_3$ | H | 0 | H | $CH_2CH_2CH_3$ | Cl | $OCH_3$ | CH | |
| $CH_3$ | H | 0 | H | $CH_2CH_2CH_2CH_3$ | $CH_3$ | $CH_3$ | CH | |
| $CH_3$ | H | 0 | H | $CH_2CH_2CH_2CH_3$ | $CH_3$ | $OCH_3$ | CH | |
| $CH_3$ | H | 0 | H | $CH_2CH_2CH_2CH_3$ | $OCH_3$ | $OCH_3$ | CH | |
| $CH_3$ | H | 0 | H | $CH_2CH_2CH_2CH_3$ | $CH_3$ | $CH_3$ | N | |
| $CH_3$ | H | 0 | H | $CH_2CH_2CH_2CH_3$ | $CH_3$ | $OCH_3$ | N | |
| $CH_3$ | H | 0 | H | $CH_2CH_2CH_2CH_3$ | $OCH_3$ | $OCH_3$ | N | |
| $CH_3$ | H | 0 | H | $CH_2CH_2CH_2CH_3$ | Cl | $OCH_3$ | CH | |

Table IV (cont.)

| R | $R_1$ | n | $R_2$ | R' | X | Y | Z | m.p.(°C) |
|---|---|---|---|---|---|---|---|---|
| $CH_3$ | H | 0 | Cl | $C(CH_3)=CH_2$ | $CH_3$ | $CH_3$ | CH | |
| $CH_3$ | H | 0 | Cl | $C(CH_3)=CH_2$ | $CH_3$ | $OCH_3$ | CH | |
| $CH_3$ | H | 0 | Cl | $C(CH_3)=CH_2$ | $OCH_3$ | $OCH_3$ | CH | |
| $CH_3$ | H | 0 | Cl | $C(CH_3)=CH_2$ | $CH_3$ | $CH_3$ | N | |
| $CH_3$ | H | 0 | Cl | $C(CH_3)=CH_2$ | $CH_3$ | $OCH_3$ | N | |
| $CH_3$ | H | 0 | Cl | $C(CH_3)=CH_2$ | $OCH_3$ | $OCH_3$ | N | |
| $CH_3$ | H | 0 | Cl | $C(CH_3)=CH_2$ | Cl | $OCH_3$ | CH | |
| $CH_3$ | H | 0 | Cl | $CH_2CH=CHCH_3$ | $CH_3$ | $CH_3$ | CH | |
| $CH_3$ | H | 0 | Cl | $CH=CH-CH_2CH_3$ | $CH_3$ | $OCH_3$ | CH | |
| $CH_3$ | H | 0 | Cl | $CH=CH-CH_2CH_3$ | $OCH_3$ | $OCH_3$ | CH | |
| $CH_3$ | H | 0 | Cl | $CH=CH-CH_2CH_3$ | $CH_3$ | $CH_3$ | N | |
| $CH_3$ | H | 0 | Cl | $CH=CH-CH_2CH_3$ | $CH_3$ | $OCH_3$ | N | |
| $CH_3$ | H | 0 | Cl | $CH=CH-CH_2CH_3$ | $OCH_3$ | $OCH_3$ | N | |
| $CH_3$ | H | 0 | Cl | $CH=CH-CH_2CH_3$ | Cl | $OCH_3$ | CH | |
| $CH_3$ | H | 0 | Cl | $CH=CHCH_2F$ | $CH_3$ | $CH_3$ | CH | |
| $CH_3$ | H | 0 | Cl | $CH=CHCH_2F$ | $CH_3$ | $OCH_3$ | CH | |
| $CH_3$ | H | 0 | Cl | $CH=CHCH_2F$ | $OCH_3$ | $OCH_3$ | CH | |
| $CH_3$ | H | 0 | Cl | $CH=CHCH_2F$ | $CH_3$ | $CH_3$ | N | |
| $CH_3$ | H | 0 | Cl | $CH=CHCH_2F$ | $CH_3$ | $OCH_3$ | N | |
| $CH_3$ | H | 0 | Cl | $CH=CHCH_2F$ | $OCH_3$ | $OCH_3$ | N | |
| $CH_3$ | H | 0 | Cl | $CH=CHCH_2F$ | Cl | $OCH_3$ | CH | |
| $CH_3$ | H | 0 | Cl | CH=CHF | $CH_3$ | $CH_3$ | CH | |
| $CH_3$ | H | 0 | Cl | CH=CHF | $CH_3$ | $OCH_3$ | CH | |
| $CH_3$ | H | 0 | Cl | CH=CHF | $OCH_3$ | $OCH_3$ | CH | |
| $CH_3$ | H | 0 | Cl | CH=CHF | $CH_3$ | $CH_3$ | N | |
| $CH_3$ | H | 0 | Cl | CH=CHF | $CH_3$ | $OCH_3$ | N | |
| $CH_3$ | H | 0 | Cl | CH=CHF | $OCH_3$ | $OCH_3$ | N | |
| $CH_3$ | H | 0 | Cl | CH=CHF | Cl | $OCH_3$ | CH | |

Table IV (cont.)

| R | $R_1$ | n | $R_2$ | R' | X | Y | Z | m.p.(°C) |
|---|---|---|---|---|---|---|---|---|
| $CH_3$ | H | 0 | $CO_2CH_3$ | $CH_3$ | $CH_3$ | $CH_3$ | CH | |
| $CH_3$ | H | 0 | $CO_2CH_3$ | $CH_3$ | $CH_3$ | $OCH_3$ | CH | |
| $CH_3$ | H | 0 | $CO_2CH_3$ | $CH_3$ | $OCH_3$ | $OCH_3$ | CH | |
| $CH_3$ | H | 0 | $CO_2CH_3$ | $CH_3$ | $CH_3$ | $CH_3$ | N | |
| $CH_3$ | H | 0 | $CO_2CH_3$ | $CH_3$ | $CH_3$ | $OCH_3$ | N | |
| $CH_3$ | H | 0 | $CO_2CH_3$ | $CH_3$ | $OCH_3$ | $OCH_3$ | N | |
| $CH_3$ | H | 0 | $CO_2CH_3$ | $CH_3$ | Cl | $OCH_3$ | CH | |
| $CH_3$ | H | 0 | $CO_2CH_3$ | $CH_2CH_3$ | $CH_3$ | $CH_3$ | CH | |
| $CH_3$ | H | 0 | $CO_2CH_3$ | $CH_2CH_3$ | $CH_3$ | $OCH_3$ | CH | |
| $CH_3$ | H | 0 | $CO_2CH_3$ | $CH_2CH_3$ | $OCH_3$ | $OCH_3$ | CH | |
| $CH_3$ | H | 0 | $CO_2CH_3$ | $CH_2CH_3$ | $CH_3$ | $CH_3$ | N | |
| $CH_3$ | H | 0 | $CO_2CH_3$ | $CH_2CH_3$ | $CH_3$ | $OCH_3$ | N | |
| $CH_3$ | H | 0 | $CO_2CH_3$ | $CH_2CH_3$ | $OCH_3$ | $OCH_3$ | N | |
| $CH_3$ | H | 0 | $CO_2CH_3$ | $CH_2CH_3$ | Cl | $OCH_3$ | CH | |
| $CH_3$ | H | 0 | $CO_2CH_3$ | $CH_2CH_2CH_3$ | $CH_3$ | $CH_3$ | CH | |
| $CH_3$ | H | 0 | $CO_2CH_3$ | $CH_2CH_2CH_3$ | $CH_3$ | $OCH_3$ | CH | |
| $CH_3$ | H | 0 | $CO_2CH_3$ | $CH_2CH_2CH_3$ | $OCH_3$ | $OCH_3$ | CH | |
| $CH_3$ | H | 0 | $CO_2CH_3$ | $CH_2CH_2CH_3$ | $CH_3$ | $CH_3$ | N | |
| $CH_3$ | H | 0 | $CO_2CH_3$ | $CH_2CH_2CH_3$ | $CH_3$ | $OCH_3$ | N | |
| $CH_3$ | H | 0 | $CO_2CH_3$ | $CH_2CH_2CH_3$ | $OCH_3$ | $OCH_3$ | N | |
| $CH_3$ | H | 0 | $CO_2CH_3$ | $CH_2CH_2CH_3$ | Cl | $OCH_3$ | CH | |
| $CH_3$ | H | 0 | $CO_2CH_3$ | $CH_2CH_2CH_2CH_3$ | $CH_3$ | $CH_3$ | CH | |
| $CH_3$ | H | 0 | $CO_2CH_3$ | $CH_2CH_2CH_2CH_3$ | $CH_3$ | $OCH_3$ | CH | |
| $CH_3$ | H | 0 | $CO_2CH_3$ | $CH_2CH_2CH_2CH_3$ | $OCH_3$ | $OCH_3$ | CH | |
| $CH_3$ | H | 0 | $CO_2CH_3$ | $CH_2CH_2CH_2CH_3$ | $CH_3$ | $CH_3$ | N | |
| $CH_3$ | H | 0 | $CO_2CH_3$ | $CH_2CH_2CH_2CH_3$ | $CH_3$ | $OCH_3$ | N | |
| $CH_3$ | H | 0 | $CO_2CH_3$ | $CH_2CH_2CH_2CH_3$ | $OCH_3$ | $OCH_3$ | N | |
| $CH_3$ | H | 0 | $CO_2CH_3$ | $CH_2CH_2CH_2CH_3$ | Cl | $OCH_3$ | CH | |

Table IV (cont.)

| R | $R_1$ | n | $R_2$ | R' | X | Y | Z | m.p.(°C) |
|---|---|---|---|---|---|---|---|---|
| $CH_2CN$ | H | 0 | H | $CH_2F$ | $CH_3$ | $CH_3$ | CH | |
| $CH_2CN$ | H | 0 | H | $CH_2F$ | $CH_3$ | $OCH_3$ | CH | |
| $CH_2CN$ | H | 0 | H | $CH_2F$ | $OCH_3$ | $OCH_3$ | CH | |
| $CH_2CN$ | H | 0 | H | $CH_2F$ | $CH_3$ | $CH_3$ | N | |
| $CH_2CN$ | H | 0 | H | $CH_2F$ | $CH_3$ | $OCH_3$ | N | |
| $CH_2CN$ | H | 0 | H | $CH_2F$ | $OCH_3$ | $OCH_3$ | N | |
| $CH_2CN$ | H | 0 | H | $CH_2F$ | Cl | $OCH_3$ | CH | |
| $CH_2CN$ | H | 0 | H | $CH_2CH_2F$ | $CH_3$ | $CH_3$ | CH | |
| $CH_2CN$ | H | 0 | H | $CH_2CH_2F$ | $CH_3$ | $OCH_3$ | CH | |
| $CH_2CN$ | H | 0 | H | $CH_2CH_2F$ | $OCH_3$ | $OCH_3$ | CH | |
| $CH_2CN$ | H | 0 | H | $CH_2CH_2F$ | $CH_3$ | $CH_3$ | N | |
| $CH_2CN$ | H | 0 | H | $CH_2CH_2F$ | $CH_3$ | $OCH_3$ | N | |
| $CH_2CN$ | H | 0 | H | $CH_2CH_2F$ | $OCH_3$ | $OCH_3$ | N | |
| $CH_2CN$ | H | 0 | H | $CH_2CH_2F$ | Cl | $OCH_3$ | CH | |
| $CH_2CN$ | H | 0 | H | $CHF_2$ | $CH_3$ | $CH_3$ | CH | |
| $CH_2CN$ | H | 0 | H | $CHF_2$ | $CH_3$ | $OCH_3$ | CH | |
| $CH_2CN$ | H | 0 | H | $CHF_2$ | $OCH_3$ | $OCH_3$ | CH | |
| $CH_2CN$ | H | 0 | H | $CHF_2$ | $CH_3$ | $CH_3$ | N | |
| $CH_2CN$ | H | 0 | H | $CHF_2$ | $CH_3$ | $OCH_3$ | N | |
| $CH_2CN$ | H | 0 | H | $CHF_2$ | $OCH_3$ | $OCH_3$ | N | |
| $CH_2CN$ | H | 0 | H | $CHF_2$ | Cl | $OCH_3$ | CH | |
| $CH_2CN$ | H | 0 | H | $CH_2CF_3$ | $CH_3$ | $CH_3$ | CH | |
| $CH_2CN$ | H | 0 | H | $CH_2CF_3$ | $CH_3$ | $OCH_3$ | CH | |
| $CH_2CN$ | H | 0 | H | $CH_2CF_3$ | $OCH_3$ | $OCH_3$ | CH | |
| $CH_2CN$ | H | 0 | H | $CH_2Cl$ | $CH_3$ | $CH_3$ | N | |
| $CH_2CN$ | H | 0 | H | $CH_2Cl$ | $CH_3$ | $OCH_3$ | N | |
| $CH_2CN$ | H | 0 | H | $CH_2Cl$ | $OCH_3$ | $OCH_3$ | N | |
| $CH_2CN$ | H | 0 | H | $CH_2Cl$ | Cl | $OCH_3$ | CH | |

## Table V

| R | $R_1$ | $R_2$ | R' | n | A | $X_1$ | $Y_1$ | $X_2$ | $Y_2$ | $X_3$ | $Y_3$ | m.p. (°C) |
|---|---|---|---|---|---|---|---|---|---|---|---|---|
| $CH_3$ | H | H | cyclopropyl | 0 | A-2 | $CH_3$ | O | - | - | - | - | |
| $CH_3$ | H | H | cyclopropyl | 0 | A-2 | $OCH_3$ | O | - | - | - | - | |
| $CH_3$ | H | H | cyclopropyl | 0 | A-2 | $OCH_3$ | $CH_2$ | - | - | - | - | |
| $CH_3$ | H | H | cyclopropyl | 0 | A-2 | $OCH_2CH_3$ | O | - | - | - | - | |
| $CH_3$ | H | H | cyclopropyl | 0 | A-3 | $CH_3$ | - | - | - | - | - | |
| $CH_3$ | H | H | cyclopropyl | 0 | A-3 | $OCH_3$ | - | - | - | - | - | |
| $CH_3$ | H | H | cyclopropyl | 0 | A-3 | $OCH_2CH_3$ | - | - | - | - | - | |
| $CH_3$ | H | H | cyclopropyl | 0 | A-3 | $OCF_2H$ | - | - | - | - | - | |
| $CH_3$ | H | H | cyclopropyl | 0 | A-4 | $CH_3$ | - | - | - | - | H | |
| $CH_3$ | H | H | cyclopropyl | 0 | A-4 | $CH_3$ | - | - | - | - | $CH_3$ | |
| $CH_3$ | H | H | cyclopropyl | 0 | A-4 | $OCH_3$ | - | - | - | - | H | |
| $CH_3$ | H | H | cyclopropyl | 0 | A-4 | $OCH_3$ | - | - | - | - | $CH_3$ | |
| $CH_3$ | H | H | cyclopropyl | 0 | A-4 | $OCF_2H$ | - | - | - | - | H | |
| $CH_3$ | H | H | cyclopropyl | 0 | A-5 | - | - | $CH_3$ | $OCH_3$ | - | - | |
| $CH_3$ | H | H | cyclopropyl | 0 | A-5 | - | - | $CH_3$ | $OCH_2CH_3$ | - | - | |
| $CH_3$ | H | H | cyclopropyl | 0 | A-5 | - | - | $CH_3$ | $SCH_3$ | - | - | |
| $CH_3$ | H | H | cyclopropyl | 0 | A-5 | - | - | $CH_2CH_3$ | $OCH_3$ | - | - | |
| $CH_3$ | H | H | cyclopropyl | 0 | A-6 | - | - | - | - | $CH_3$ | - | |
| $CH_3$ | H | H | cyclopropyl | 0 | A-6 | - | - | - | - | $OCH_3$ | - | |

| R | $R_1$ | $R_2$ | R' | n | A | $X_4$ | $Y_4$ | $Z_1$ | m.p. (°C) |
|---|---|---|---|---|---|---|---|---|---|
| $CH_3$ | H | H | cyclopropyl | 0 | A-7 | $CH_3$ | $CH_3$ | CH | |

Table V (cont.)

| R | $R_1$ | $R_2$ | R' | n | A | $X_1$ | $Y_1$ | $X_2$ | $Y_2$ | $X_3$ | $Y_3$ | m.p. (°C) |
|---|---|---|---|---|---|---|---|---|---|---|---|---|
| $CH_3$ | H | $CH_3$ | cyclopropyl | O | A-2 | $CH_3$ | O | - | - | - | - | |
| $CH_3$ | H | $CH_3$ | cyclopropyl | O | A-2 | $OCH_3$ | O | - | - | - | - | |
| $CH_3$ | H | $CH_3$ | cyclopropyl | O | A-2 | $OCH_3$ | $CH_2$ | - | - | - | - | |
| $CH_3$ | H | $CH_3$ | cyclopropyl | O | A-2 | $OCH_2CH_3$ | O | - | - | - | - | |
| $CH_3$ | H | $CH_3$ | cyclopropyl | O | A-3 | $CH_3$ | - | - | - | - | - | |
| $CH_3$ | H | $CH_3$ | cyclopropyl | O | A-3 | $OCH_3$ | - | - | - | - | - | |
| $CH_3$ | H | $CH_3$ | cyclopropyl | O | A-3 | $OCH_2CH_3$ | - | - | - | - | - | |
| $CH_3$ | H | $CH_3$ | cyclopropyl | O | A-3 | $OCF_2H$ | - | - | - | - | - | |
| $CH_3$ | H | $CH_3$ | cyclopropyl | O | A-4 | $CH_3$ | - | - | - | - | H | |
| $CH_3$ | H | $CH_3$ | cyclopropyl | O | A-4 | $CH_3$ | - | - | - | - | $CH_3$ | |
| $CH_3$ | H | $CH_3$ | cyclopropyl | O | A-4 | $OCH_3$ | - | - | - | - | H | |
| $CH_3$ | H | $CH_3$ | cyclopropyl | O | A-4 | $OCH_3$ | - | - | - | - | $CH_3$ | |
| $CH_3$ | H | $CH_3$ | cyclopropyl | O | A-4 | $OCF_2H$ | - | - | - | - | H | |
| $CH_3$ | H | $CH_3$ | cyclopropyl | O | A-5 | - | - | $CH_3$ | $OCH_3$ | - | - | |
| $CH_3$ | H | $CH_3$ | cyclopropyl | O | A-5 | - | - | $CH_3$ | $OCH_2CH_3$ | - | - | |
| $CH_3$ | H | $CH_3$ | cyclopropyl | O | A-5 | - | - | $CH_3$ | $SCH_3$ | - | - | |
| $CH_3$ | H | $CH_3$ | cyclopropyl | O | A-5 | - | - | $CH_2CH_3$ | $OCH_3$ | - | - | |
| $CH_3$ | H | $CH_3$ | cyclopropyl | O | A-6 | - | - | - | - | $CH_3$ | - | |
| $CH_3$ | H | $CH_3$ | cyclopropyl | O | A-6 | - | - | - | - | $OCH_3$ | - | |

Table Va

Where n' = o

| J | R | R' | A | $X_1$ | $Y_1$ | m.p.(°C) |
|---|---|---|---|---|---|---|
| J-1 | $CH_3$ | $CH_3$ | A-2 | $CH_3$ | O | |
| J-1 | $CH_3$ | $CH_3$ | A-2 | $OCH_3$ | O | |
| J-1 | $CH_3$ | $CH_3$ | A-2 | $OCH_2CH_3$ | O | |
| J-1 | $CH_3$ | $CH_3$ | A-2 | $OCF_2H$ | O | |
| J-1 | $CH_3$ | $CH_3$ | A-2 | $CH_3$ | $CH_2$ | |
| J-1 | $CH_3$ | $CH_3$ | A-2 | $OCH_3$ | $CH_2$ | |
| J-5 | – | cyclopropyl | A-2 | $CH_3$ | O | |
| J-5 | – | cyclopropyl | A-2 | $OCH_3$ | O | |
| J-5 | – | cyclopropyl | A-2 | $OCH_2CH_3$ | O | |
| J-5 | – | cyclopropyl | A-2 | $OCF_2H$ | O | |
| J-5 | – | cyclopropyl | A-2 | $CH_3$ | $CH_2$ | |
| J-5 | – | cyclopropyl | A-2 | $OCH_3$ | $CH_2$ | |
| J-8 | – | cyclopropyl | A-2 | $CH_3$ | O | |
| J-8 | – | cyclopropyl | A-2 | $OCH_3$ | O | |
| J-8 | – | cyclopropyl | A-2 | $OCH_2CH_3$ | O | |
| J-8 | – | cyclopropyl | A-2 | $OCF_2H$ | O | |
| J-8 | – | cyclopropyl | A-2 | $CH_3$ | $CH_2$ | |
| J-8 | – | cyclopropyl | A-2 | $OCH_3$ | $CH_2$ | |

| J | R | R' | A | $X_1$ | m.p.(°C) |
|---|---|---|---|---|---|
| J-1 | $CH_3$ | $CH_3$ | A-3 | $CH_3$ | |
| J-1 | $CH_3$ | $CH_3$ | A-3 | $OCH_3$ | |
| J-1 | $CH_3$ | $CH_3$ | A-3 | $OCH_2CH_3$ | |
| J-1 | $CH_3$ | $CH_3$ | A-3 | $OCF_2H$ | |
| J-5 | – | cyclopropyl | A-3 | $CH_3$ | |
| J-5 | – | cyclopropyl | A-3 | $OCH_3$ | |
| J-5 | – | cyclopropyl | A-3 | $OCH_2CH_3$ | |
| J-5 | – | cyclopropyl | A-3 | $OCF_2H$ | |
| J-8 | – | cyclopropyl | A-3 | $CH_3$ | |
| J-8 | – | cyclopropyl | A-3 | $OCH_3$ | |
| J-8 | – | cyclopropyl | A-3 | $OCH_2CH_3$ | |
| J-8 | – | cyclopropyl | A-3 | $OCF_2H$ | |

Table Va (cont.)

| J | R | R' | A | $X_1$ | $Y_3$ | m.p.(°C) |
|---|---|---|---|---|---|---|
| J-1 | $CH_3$ | $CH_3$ | A-4 | $CH_3$ | H | |
| J-1 | $CH_3$ | $CH_3$ | A-4 | $OCH_3$ | H | |
| J-1 | $CH_3$ | $CH_3$ | A-4 | $OCH_2CH_3$ | H | |
| J-1 | $CH_3$ | $CH_3$ | A-4 | $OCF_2H$ | H | |
| J-1 | $CH_3$ | $CH_3$ | A-4 | $CH_3$ | $CH_3$ | |
| J-1 | $CH_3$ | $CH_3$ | A-4 | $OCH_3$ | $CH_3$ | |
| J-5 | - | cyclopropyl | A-4 | $CH_3$ | H | |
| J-5 | - | cyclopropyl | A-4 | $OCH_3$ | H | |
| J-5 | - | cyclopropyl | A-4 | $OCH_2CH_3$ | H | |
| J-5 | - | cyclopropyl | A-4 | $OCF_2H$ | H | |
| J-5 | - | cyclopropyl | A-4 | $CH_3$ | $CH_3$ | |
| J-5 | - | cyclopropyl | A-4 | $OCH_3$ | $CH_3$ | |
| J-8 | - | cyclopropyl | A-4 | $CH_3$ | H | |
| J-8 | - | cyclopropyl | A-4 | $OCH_3$ | H | |
| J-8 | - | cyclopropyl | A-4 | $OCH_2CH_3$ | H | |
| J-8 | - | cyclopropyl | A-4 | $OCF_2H$ | H | |
| J-8 | - | cyclopropyl | A-4 | $CH_3$ | $CH_3$ | |
| J-8 | - | cyclopropyl | A-4 | $OCH_3$ | $CH_3$ | |

| J | R | R' | A | $X_2$ | $Y_2$ | m.p.(°C) |
|---|---|---|---|---|---|---|
| J-1 | $CH_3$ | $CH_3$ | A-5 | $CH_3$ | $OCH_3$ | |
| J-1 | $CH_3$ | $CH_3$ | A-5 | $CH_3$ | $OCH_2CH_3$ | |
| J-1 | $CH_3$ | $CH_3$ | A-5 | $CH_3$ | $SCH_3$ | |
| J-1 | $CH_3$ | $CH_3$ | A-5 | $CH_3$ | $CH_3$ | |
| J-1 | $CH_3$ | $CH_3$ | A-5 | $CH_3$ | $CH_2CH_3$ | |
| J-1 | $CH_3$ | $CH_3$ | A-5 | $CH_2CH_3$ | $OCH_3$ | |
| J-1 | $CH_3$ | $CH_3$ | A-5 | $CH_2CF_3$ | $CH_3$ | |
| J-5 | - | cyclopropyl | A-5 | $CH_3$ | $OCH_3$ | |
| J-5 | - | cyclopropyl | A-5 | $CH_3$ | $OCH_2CH_3$ | |

Table Va (cont.)

| J | R | R' | A | $X_2$ | $Y_2$ | m.p.(°C) |
|---|---|---|---|---|---|---|
| J-5 | - | cyclopropyl | A-5 | $CH_3$ | $SCH_3$ | |
| J-5 | - | cyclopropyl | A-5 | $CH_3$ | $CH_3$ | |
| J-5 | - | cyclopropyl | A-5 | $CH_3$ | $CH_2CH_3$ | |
| J-5 | - | cyclopropyl | A-5 | $CH_2CH_3$ | $OCH_3$ | |
| J-5 | - | cyclopropyl | A-5 | $CH_2CF_3$ | $CH_3$ | |
| J-8 | - | cyclopropyl | A-5 | $CH_3$ | $OCH_3$ | |
| J-8 | - | cyclopropyl | A-5 | $CH_3$ | $OCH_2CH_3$ | |
| J-8 | - | cyclopropyl | A-5 | $CH_3$ | $SCH_3$ | |
| J-8 | - | cyclopropyl | A-5 | $CH_3$ | $CH_3$ | |
| J-8 | - | cyclopropyl | A-5 | $CH_3$ | $CH_2CH_3$ | |
| J-8 | - | cyclopropyl | A-5 | $CH_2CH_3$ | $OCH_3$ | |
| J-8 | - | cyclopropyl | A-5 | $CH_2CF_3$ | $CH_3$ | |

| J | R | R' | A | $X_3$ | m.p.(°C) |
|---|---|---|---|---|---|
| J-1 | $CH_3$ | $CH_3$ | A-6 | $CH_3$ | |
| J-1 | $CH_3$ | $CH_3$ | A-6 | $OCH_3$ | |
| J-5 | - | cyclopropyl | A-6 | $CH_3$ | |
| J-5 | - | cyclopropyl | A-6 | $OCH_3$ | |
| J-8 | - | cyclopropyl | A-6 | $CH_3$ | |
| J-8 | - | cyclopropyl | A-6 | $OCH_3$ | |

| J | R | R' | A | $X_4$ | $Y_4$ | m.p.(°C) |
|---|---|---|---|---|---|---|
| J-1 | $CH_3$ | $CH_3$ | A-7 | $CH_3$ | $OCH_3$ | |
| J-1 | $CH_3$ | $CH_3$ | A-7 | $OCH_3$ | $OCH_3$ | |
| J-5 | - | cyclopropyl | A-7 | $CH_3$ | $OCH_3$ | |
| J-5 | - | cyclopropyl | A-7 | $OCH_3$ | $OCH_3$ | |
| J-8 | - | cyclopropyl | A-7 | $CH_3$ | $OCH_3$ | |
| J-8 | - | cyclopropyl | A-7 | $OCH_3$ | $OCH_3$ | |

Table VI

| R | $R_1$ | n | $R_2$ | R' | X | Y | Z | m.p. (°C) |
|---|---|---|---|---|---|---|---|---|
| $CH_3$ | H | O | H | cyclopropyl | $CH_3$ | $CH_3$ | CH | |
| $CH_3$ | H | O | H | cyclopropyl | $CH_3$ | $OCH_3$ | CH | |
| $CH_3$ | H | O | H | cyclopropyl | $OCH_3$ | $OCH_3$ | CH | |
| $CH_3$ | H | O | H | cyclopropyl | $CH_3$ | $CH_3$ | N | |
| $CH_3$ | H | O | H | cyclopropyl | $CH_3$ | $OCH_3$ | N | |
| $CH_3$ | H | O | H | cyclopropyl | $OCH_3$ | $OCH_3$ | N | |
| $CH_3$ | H | O | H | cyclopropyl | Cl | $OCH_3$ | CH | |
| $CH_3$ | H | O | H | cyclobutyl | $CH_3$ | $CH_3$ | CH | |
| $CH_3$ | H | O | H | cyclobutyl | $CH_3$ | $OCH_3$ | CH | |
| $CH_3$ | H | O | H | cyclobutyl | $OCH_3$ | $OCH_3$ | CH | |
| $CH_3$ | H | O | H | cyclobutyl | $CH_3$ | $CH_3$ | N | |
| $CH_3$ | H | O | H | cyclobutyl | $CH_3$ | $OCH_3$ | N | |
| $CH_3$ | H | O | H | cyclobutyl | $OCH_3$ | $OCH_3$ | N | |
| $CH_3$ | H | O | H | cyclobutyl | Cl | $OCH_3$ | CH | |
| $CH_3$ | H | O | H | cyclopentyl | $CH_3$ | $CH_3$ | CH | |
| $CH_3$ | H | O | H | cyclopentyl | $CH_3$ | $OCH_3$ | CH | |
| $CH_3$ | H | O | H | cyclopentyl | $OCH_3$ | $OCH_3$ | CH | |
| $CH_3$ | H | O | H | cyclopentyl | $CH_3$ | $CH_3$ | N | |
| $CH_3$ | H | O | H | cyclopentyl | $CH_3$ | $OCH_3$ | N | |
| $CH_3$ | H | O | H | cyclopentyl | $OCH_3$ | $OCH_3$ | N | |
| $CH_3$ | H | O | H | cyclopentyl | Cl | $OCH_3$ | CH | |
| $CH_3$ | H | O | H | cyclopropyl | $OCH_3$ | $OCH_2CH_3$ | CH | |
| $CH_3$ | H | O | H | cyclopropyl | cyclopropyl | $OCH_3$ | CH | |
| $CH_3$ | H | O | H | cyclopropyl | $OCH_3$ | $CH(OCH_3)_2$ | CH | |
| $CH_3$ | H | O | H | cyclopropyl | $NHCH_3$ | $OCH_2CH_3$ | N | |
| $CH_3$ | H | O | H | cyclopropyl | $NHCH_3$ | $OCH_3$ | N | |
| $CH_3$ | H | O | H | cyclopropyl | $OCH_3$ | $OCH_2CH_3$ | N | |
| $CH_3$ | H | O | H | cyclopropyl | $CH_2F$ | $CH_3$ | CH | |

Table VII

| R | $R_1$ | n | $R_2$ | R' | X | Y | Z | m.p. (°C) |
|---|---|---|---|---|---|---|---|---|
| $CH_3$ | H | O | H | cyclopropyl | $CH_3$ | $CH_3$ | CH | |
| $CH_3$ | H | O | H | cyclopropyl | $CH_3$ | $OCH_3$ | CH | |
| $CH_3$ | H | O | H | cyclopropyl | $OCH_3$ | $OCH_3$ | CH | |
| $CH_3$ | H | O | H | cyclopropyl | $CH_3$ | $CH_3$ | N | |
| $CH_3$ | H | O | H | cyclopropyl | $CH_3$ | $OCH_3$ | N | |
| $CH_3$ | H | O | H | cyclopropyl | $OCH_3$ | $OCH_3$ | N | |
| $CH_3$ | H | O | H | cyclopropyl | Cl | $OCH_3$ | CH | |
| $CH_3$ | H | O | H | cyclobutyl | $CH_3$ | $CH_3$ | CH | |
| $CH_3$ | H | O | H | cyclobutyl | $CH_3$ | $OCH_3$ | CH | |
| $CH_3$ | H | O | H | cyclobutyl | $OCH_3$ | $OCH_3$ | CH | |
| $CH_3$ | H | O | H | cyclobutyl | $CH_3$ | $CH_3$ | N | |
| $CH_3$ | H | O | H | cyclobutyl | $CH_3$ | $OCH_3$ | N | |
| $CH_3$ | H | O | H | cyclobutyl | $OCH_3$ | $OCH_3$ | N | |
| $CH_3$ | H | O | H | cyclobutyl | Cl | $OCH_3$ | CH | |
| $CH_3$ | H | O | H | cyclopentyl | $CH_3$ | $CH_3$ | CH | |
| $CH_3$ | H | O | H | cyclopentyl | $CH_3$ | $OCH_3$ | CH | |
| $CH_3$ | H | O | H | cyclopentyl | $OCH_3$ | $OCH_3$ | CH | |
| $CH_3$ | H | O | H | cyclopentyl | $CH_3$ | $CH_3$ | N | |
| $CH_3$ | H | O | H | cyclopentyl | $CH_3$ | $OCH_3$ | N | |
| $CH_3$ | H | O | H | cyclopentyl | $OCH_3$ | $OCH_3$ | N | |
| $CH_3$ | H | O | H | cyclopentyl | Cl | $OCH_3$ | CH | |
| $CH_3$ | H | O | H | cyclopropyl | $OCH_3$ | $OCH_2CH_3$ | CH | |
| $CH_3$ | H | O | H | cyclopropyl | cyclopropyl | $OCH_3$ | CH | |
| $CH_3$ | H | O | H | cyclopropyl | $OCH_3$ | $CH(OCH_3)_2$ | CH | |
| $CH_3$ | H | O | H | cyclopropyl | $NHCH_3$ | $OCH_2CH_3$ | N | |
| $CH_3$ | H | O | H | cyclopropyl | $NHCH_3$ | $OCH_3$ | N | |
| $CH_3$ | H | O | H | cyclopropyl | $OCH_3$ | $OCH_2CH_3$ | N | |
| $CH_3$ | H | O | H | cyclopropyl | $CH_2F$ | $CH_3$ | CH | |

## Table VIII

Compounds of Formula I where
R is $CH_3$ and n' is 0

| J | $R_1$ | $R_2$ | n | R' | X | Y | Z | m.p.(°C) |
|---|---|---|---|---|---|---|---|---|
| J-8 | H | H | 1 | $CH_3$ | $CH_3$ | $CH_3$ | CH | |
| J-8 | H | H | 1 | $CH_3$ | $CH_3$ | $OCH_3$ | CH | |
| J-8 | H | H | 1 | $CH_3$ | $OCH_3$ | $OCH_3$ | CH | |
| J-8 | $CH_3$ | H | 1 | $CH_3$ | $OCH_3$ | $OCH_3$ | CH | |
| J-8 | H | H | 1 | $CH_3$ | $OCH_3$ | $OCH_3$ | N | |
| J-8 | H | H | 1 | $CH_3$ | $OCH_3$ | $CH_3$ | N | |
| J-8 | H | H | 1 | $CH_2CH_3$ | $CH_3$ | $CH_3$ | CH | |
| J-8 | H | H | 1 | $CH_2CH_3$ | $OCH_3$ | $CH_3$ | CH | |
| J-8 | H | H | 1 | $CH_2CH_3$ | $OCH_3$ | $OCH_3$ | CH | |
| J-8 | H | H | 1 | $CH_2CH_3$ | $OCH_3$ | $OCH_3$ | N | |
| J-8 | H | H | 1 | $CH_2CH_3$ | $OCH_3$ | $CH_3$ | N | |
| J-9 | H | H | 1 | $CH_2CH_2CH_3$ | $OCH_3$ | $OCH_3$ | CH | |
| J-1 | H | H | - | $CH_3$ | $OCH_3$ | $OCH_3$ | CH | |
| J-6 | H | H | - | $CH_3$ | $OCH_3$ | $OCH_3$ | CH | |

Table IX

| $R_1$ | $R_2$ | R' | X | Y | Z | m.p.(°C) |
|---|---|---|---|---|---|---|
| H | H | cyclopropyl | $CH_3$ | $CH_3$ | CH | 154-157 |
| H | H | cyclopropyl | $CH_3$ | $OCH_3$ | CH | 167-171 |
| H | H | cyclopropyl | $OCH_3$ | $OCH_3$ | CH | 165-168 |
| H | H | cyclopropyl | $CH_3$ | $CH_3$ | N | |
| H | H | cyclopropyl | $CH_3$ | $OCH_3$ | N | 146-148 |
| H | H | cyclopropyl | $OCH_3$ | $OCH_3$ | N | 146-149 |
| H | H | cyclopropyl | Cl | $OCH_3$ | CH | 122-125 |
| H | H | cyclobutyl | $CH_3$ | $CH_3$ | CH | |
| H | H | cyclobutyl | $CH_3$ | $OCH_3$ | CH | |
| H | H | cyclobutyl | $OCH_3$ | $OCH_3$ | CH | |
| H | H | cyclobutyl | $CH_3$ | $CH_3$ | N | |
| H | H | cyclobutyl | $CH_3$ | $OCH_3$ | N | |
| H | H | cyclobutyl | $OCH_3$ | $OCH_3$ | N | |
| H | H | cyclobutyl | Cl | $OCH_3$ | CH | |
| H | H | 2-fluorocyclopropyl | $CH_3$ | $CH_3$ | CH | |
| H | H | 2-fluorocyclopropyl | $CH_3$ | $OCH_3$ | CH | |
| H | H | 2-fluorocyclopropyl | $OCH_3$ | $OCH_3$ | CH | |
| H | H | 2-fluorocyclopropyl | $CH_3$ | $CH_3$ | N | |
| H | H | 2-fluorocyclopropyl | $CH_3$ | $OCH_3$ | N | |
| H | H | 2-fluorocyclopropyl | $OCH_3$ | $OCH_3$ | N | |
| H | H | 2-fluorocyclopropyl | Cl | $OCH_3$ | CH | |
| H | H | 2,2-difluorocyclopropyl | $CH_3$ | $CH_3$ | CH | |
| H | H | 2,2-difluorocyclopropyl | $CH_3$ | $OCH_3$ | CH | |
| H | H | 2,2-difluorocyclopropyl | $OCH_3$ | $OCH_3$ | CH | |
| H | H | 2,2-difluorocyclopropyl | $CH_3$ | $CH_3$ | N | |
| H | H | 2,2-difluorocyclopropyl | $CH_3$ | $OCH_3$ | N | |
| H | H | 2,2-difluorocyclopropyl | $OCH_3$ | $OCH_3$ | N | |
| H | H | 2,2-difluorocyclopropyl | Cl | $OCH_3$ | CH | |

Table IX (cont.)

| $R_1$ | $R_2$ | R' | X | Y | Z | m.p. (°C) |
|---|---|---|---|---|---|---|
| H | F | cyclopropyl | $CH_3$ | $CH_3$ | CH | |
| H | F | cyclopropyl | $CH_3$ | $OCH_3$ | CH | |
| H | F | cyclopropyl | $OCH_3$ | $OCH_3$ | CH | |
| H | F | cyclopropyl | $CH_3$ | $CH_3$ | N | |
| H | F | cyclopropyl | $CH_3$ | $OCH_3$ | N | |
| H | F | cyclopropyl | $OCH_3$ | $OCH_3$ | N | |
| H | F | cyclopropyl | Cl | $OCH_3$ | CH | |
| H | F | cyclobutyl | $CH_3$ | $CH_3$ | CH | |
| H | F | cyclobutyl | $CH_3$ | $OCH_3$ | CH | |
| H | F | cyclobutyl | $OCH_3$ | $OCH_3$ | CH | |
| H | F | cyclobutyl | $CH_3$ | $CH_3$ | N | |
| H | F | cyclobutyl | $CH_3$ | $OCH_3$ | N | |
| H | F | cyclobutyl | $OCH_3$ | $OCH_3$ | N | |
| H | F | cyclobutyl | Cl | $OCH_3$ | CH | |
| H | F | 2-fluorocyclopropyl | $CH_3$ | $CH_3$ | CH | |
| H | F | 2-fluorocyclopropyl | $CH_3$ | $OCH_3$ | CH | |
| H | F | 2-fluorocyclopropyl | $OCH_3$ | $OCH_3$ | CH | |
| H | F | 2-fluorocyclopropyl | $CH_3$ | $CH_3$ | N | |
| H | F | 2-fluorocyclopropyl | $CH_3$ | $OCH_3$ | N | |
| H | F | 2-fluorocyclopropyl | $OCH_3$ | $OCH_3$ | N | |
| H | F | 2-fluorocyclopropyl | Cl | $OCH_3$ | CH | |
| H | F | 2,2-difluorocyclopropyl | $CH_3$ | $CH_3$ | CH | |
| H | F | 2,2-difluorocyclopropyl | $CH_3$ | $OCH_3$ | CH | |
| H | F | 2,2-difluorocyclopropyl | $OCH_3$ | $OCH_3$ | CH | |
| H | F | 2,2-difluorocyclopropyl | $CH_3$ | $CH_3$ | N | |
| H | F | 2,2-difluorocyclopropyl | $CH_3$ | $OCH_3$ | N | |
| H | F | 2,2-difluorocyclopropyl | $OCH_3$ | $OCH_3$ | N | |
| H | F | 2,2-difluorocyclopropyl | Cl | $OCH_3$ | CH | |

Table IX (cont.)

| $R_1$ | $R_2$ | R' | X | Y | Z | m.p. (°C) |
|---|---|---|---|---|---|---|
| H | $CO_2CH_3$ | cyclopropyl | $CH_3$ | $CH_3$ | CH | |
| H | $CO_2CH_3$ | cyclopropyl | $CH_3$ | $OCH_3$ | CH | |
| H | $CO_2CH_3$ | cyclopropyl | $OCH_3$ | $OCH_3$ | CH | |
| H | $CO_2CH_3$ | cyclopropyl | $CH_3$ | $CH_3$ | N | |
| H | $CO_2CH_3$ | cyclopropyl | $CH_3$ | $OCH_3$ | N | |
| H | $CO_2CH_3$ | cyclopropyl | $OCH_3$ | $OCH_3$ | N | |
| H | $CO_2CH_3$ | cyclopropyl | Cl | $OCH_3$ | CH | |
| H | $CO_2CH_3$ | cyclobutyl | $CH_3$ | $CH_3$ | CH | |
| H | $CO_2CH_3$ | cyclobutyl | $CH_3$ | $OCH_3$ | CH | |
| H | $CO_2CH_3$ | cyclobutyl | $OCH_3$ | $OCH_3$ | CH | |
| H | $CO_2CH_3$ | cyclobutyl | $CH_3$ | $CH_3$ | N | |
| H | $CO_2CH_3$ | cyclobutyl | $CH_3$ | $OCH_3$ | N | |
| H | $CO_2CH_3$ | cyclobutyl | $OCH_3$ | $OCH_3$ | N | |
| H | $CO_2CH_3$ | cyclobutyl | Cl | $OCH_3$ | CH | |
| H | $CO_2CH_3$ | 2-fluorocyclopropyl | $CH_3$ | $CH_3$ | CH | |
| H | $CO_2CH_3$ | 2-fluorocyclopropyl | $CH_3$ | $OCH_3$ | CH | |
| H | $CO_2CH_3$ | 2-fluorocyclopropyl | $OCH_3$ | $OCH_3$ | CH | |
| H | $CO_2CH_3$ | 2-fluorocyclopropyl | $CH_3$ | $CH_3$ | N | |
| H | $CO_2CH_3$ | 2-fluorocyclopropyl | $CH_3$ | $OCH_3$ | N | |
| H | $CO_2CH_3$ | 2-fluorocyclopropyl | $OCH_3$ | $OCH_3$ | N | |
| H | $CO_2CH_3$ | 2-fluorocyclopropyl | Cl | $OCH_3$ | CH | |
| H | $CO_2CH_3$ | 2,2-difluorocyclopropyl | $CH_3$ | $CH_3$ | CH | |
| H | $CO_2CH_3$ | 2,2-difluorocyclopropyl | $CH_3$ | $OCH_3$ | CH | |
| H | $CO_2CH_3$ | 2,2-difluorocyclopropyl | $OCH_3$ | $OCH_3$ | CH | |
| H | $CO_2CH_3$ | 2,2-difluorocyclopropyl | $CH_3$ | $CH_3$ | N | |
| H | $CO_2CH_3$ | 2,2-difluorocyclopropyl | $CH_3$ | $OCH_3$ | N | |
| H | $CO_2CH_3$ | 2,2-difluorocyclopropyl | $OCH_3$ | $OCH_3$ | N | |
| H | $CO_2CH_3$ | 2,2-difluorocyclopropyl | Cl | $OCH_3$ | CH | |

Table IX (cont.)

| $R_1$ | $R_2$ | R' | X | Y | Z | m.p. (°C) |
|---|---|---|---|---|---|---|
| H | H | cyclopropyl | H | $CH_3$ | CH | |
| H | H | cyclopropyl | H | $OCH_3$ | CH | |
| H | H | cyclopropyl | $CH_3$ | $OCH_2CH_3$ | CH | |
| H | H | cyclopropyl | $CH_3$ | $OCH(CH_3)_2$ | CH | |
| H | H | cyclopropyl | H | $OCH_3$ | N | |
| H | H | cyclopropyl | $OCH_3$ | $OCH_2CH_3$ | CH | |
| H | H | cyclopropyl | $CH_2F$ | $CH_3$ | CH | |
| H | H | cyclopropyl | $CH_2F$ | $OCH_3$ | CH | |
| H | H | cyclopropyl | $OCF_2H$ | $CH_3$ | CH | |
| H | H | cyclopropyl | $OCF_2H$ | $OCH_3$ | CH | |
| H | H | cyclopropyl | $OCH_2CF_3$ | $OCH_3$ | N | |
| H | H | cyclopropyl | $SCH_3$ | $OCH_3$ | CH | |
| H | H | cyclopropyl | $OCH_3$ | $NHCH_3$ | N | |
| H | H | cyclopropyl | $OCH_2CH_3$ | $NHCH_3$ | N | |
| H | H | cyclopropyl | $OCH_3$ | $OCH_2CH_3$ | N | |
| H | H | cyclopropyl | $SCF_2H$ | $OCH_3$ | CH | |
| H | H | cyclopropyl | Br | $OCH_3$ | CH | |
| H | H | cyclopropyl | $CH_2OCH_3$ | $OCH_3$ | CH | |
| H | H | cyclopropyl | $CH_2OCH_3$ | $CH_3$ | N | |
| H | H | cyclopropyl | $NH_2$ | $OCH_3$ | N | |
| H | H | cyclopropyl | $N(CH_3)_2$ | $OCH_3$ | N | |
| H | H | cyclopropyl | $NHCH_3$ | $NHCH_3$ | N | |
| H | H | cyclopropyl | cyclopropyl | $OCH_3$ | CH | |
| H | H | cyclopropyl | cyclopropyl | $CH_3$ | N | |
| H | H | cyclopropyl | cyclopropyl | $CH_3$ | CH | |
| H | H | cyclopropyl | cyclopropyl | $OCH_3$ | N | |
| H | H | cyclopropyl | $CH_3$ | $CH(OCH_3)_2$ | CH | |
| H | H | cyclopropyl | $CF_3$ | $OCH_3$ | CH | |
| H | H | cyclopropyl | $OCH_3$ | $C\equiv CH$ | CH | |

Table X

| $R_1$ | $R_2$ | R' | X | Y | Z | m.p. (°C) |
|---|---|---|---|---|---|---|
| H | H | cyclopropyl | $CH_3$ | $CH_3$ | CH | |
| H | H | cyclopropyl | $CH_3$ | $OCH_3$ | CH | |
| H | H | cyclopropyl | $OCH_3$ | $OCH_3$ | CH | |
| H | H | cyclopropyl | $CH_3$ | $CH_3$ | N | |
| H | H | cyclopropyl | $CH_3$ | $OCH_3$ | N | |
| H | H | cyclopropyl | $OCH_3$ | $OCH_3$ | N | |
| H | H | cyclopropyl | Cl | $OCH_3$ | CH | |
| H | H | cyclobutyl | $CH_3$ | $CH_3$ | CH | |
| H | H | cyclobutyl | $CH_3$ | $OCH_3$ | CH | |
| H | H | cyclobutyl | $OCH_3$ | $OCH_3$ | CH | |
| H | H | cyclobutyl | $CH_3$ | $CH_3$ | N | |
| H | H | cyclobutyl | $CH_3$ | $OCH_3$ | N | |
| H | H | cyclobutyl | $OCH_3$ | $OCH_3$ | N | |
| H | H | cyclobutyl | Cl | $OCH_3$ | CH | |
| H | H | 2-fluorocyclopropyl | $CH_3$ | $CH_3$ | CH | |
| H | H | 2-fluorocyclopropyl | $CH_3$ | $OCH_3$ | CH | |
| H | H | 2-fluorocyclopropyl | $OCH_3$ | $OCH_3$ | CH | |
| H | H | 2-fluorocyclopropyl | $CH_3$ | $CH_3$ | N | |
| H | H | 2-fluorocyclopropyl | $CH_3$ | $OCH_3$ | N | |
| H | H | 2-fluorocyclopropyl | $OCH_3$ | $OCH_3$ | N | |
| H | H | 2-fluorocyclopropyl | Cl | $OCH_3$ | CH | |
| H | H | 2,2-difluorocyclopropyl | $CH_3$ | $CH_3$ | CH | |
| H | H | 2,2-difluorocyclopropyl | $CH_3$ | $OCH_3$ | CH | |
| H | H | 2,2-difluorocyclopropyl | $OCH_3$ | $OCH_3$ | CH | |
| H | H | 2,2-difluorocyclopropyl | $CH_3$ | $CH_3$ | N | |
| H | H | 2,2-difluorocyclopropyl | $CH_3$ | $OCH_3$ | N | |
| H | H | 2,2-difluorocyclopropyl | $OCH_3$ | $OCH_3$ | N | |
| H | H | 2,2-difluorocyclopropyl | Cl | $OCH_3$ | CH | |

Table X (cont.)

| $R_1$ | $R_2$ | R' | X | Y | Z | m.p. (°C) |
|---|---|---|---|---|---|---|
| H | $CO_2CH_3$ | cyclopropyl | $CH_3$ | $CH_3$ | CH | |
| H | $CO_2CH_3$ | cyclopropyl | $CH_3$ | $OCH_3$ | CH | |
| H | $CO_2CH_3$ | cyclopropyl | $OCH_3$ | $OCH_3$ | CH | |
| H | $CO_2CH_3$ | cyclopropyl | $CH_3$ | $CH_3$ | N | |
| H | $CO_2CH_3$ | cyclopropyl | $CH_3$ | $OCH_3$ | N | |
| H | $CO_2CH_3$ | cyclopropyl | $OCH_3$ | $OCH_3$ | N | |
| H | $CO_2CH_3$ | cyclopropyl | Cl | $OCH_3$ | CH | |
| H | $CO_2CH_3$ | cyclobutyl | $CH_3$ | $CH_3$ | CH | |
| H | $CO_2CH_3$ | cyclobutyl | $CH_3$ | $OCH_3$ | CH | |
| H | $CO_2CH_3$ | cyclobutyl | $OCH_3$ | $OCH_3$ | CH | |
| H | $CO_2CH_3$ | cyclobutyl | $CH_3$ | $CH_3$ | N | |
| H | $CO_2CH_3$ | cyclobutyl | $CH_3$ | $OCH_3$ | N | |
| H | $CO_2CH_3$ | cyclobutyl | $OCH_3$ | $OCH_3$ | N | |
| H | $CO_2CH_3$ | cyclobutyl | Cl | $OCH_3$ | CH | |
| H | $CO_2CH_3$ | 2-fluorocyclopropyl | $CH_3$ | $CH_3$ | CH | |
| H | $CO_2CH_3$ | 2-fluorocyclopropyl | $CH_3$ | $OCH_3$ | CH | |
| H | $CO_2CH_3$ | 2-fluorocyclopropyl | $OCH_3$ | $OCH_3$ | CH | |
| H | $CO_2CH_3$ | 2-fluorocyclopropyl | $CH_3$ | $CH_3$ | N | |
| H | $CO_2CH_3$ | 2-fluorocyclopropyl | $CH_3$ | $OCH_3$ | N | |
| H | $CO_2CH_3$ | 2-fluorocyclopropyl | $OCH_3$ | $OCH_3$ | N | |
| H | $CO_2CH_3$ | 2-fluorocyclopropyl | Cl | $OCH_3$ | CH | |
| H | $CO_2CH_3$ | 2,2-difluorocyclopropyl | $CH_3$ | $CH_3$ | CH | |
| H | $CO_2CH_3$ | 2,2-difluorocyclopropyl | $CH_3$ | $OCH_3$ | CH | |
| H | $CO_2CH_3$ | 2,2-difluorocyclopropyl | $OCH_3$ | $OCH_3$ | CH | |
| H | $CO_2CH_3$ | 2,2-difluorocyclopropyl | $CH_3$ | $CH_3$ | N | |
| H | $CO_2CH_3$ | 2,2-difluorocyclopropyl | $CH_3$ | $OCH_3$ | N | |
| H | $CO_2CH_3$ | 2,2-difluorocyclopropyl | $OCH_3$ | $OCH_3$ | N | |
| H | $CO_2CH_3$ | 2,2-difluorocyclopropyl | Cl | $OCH_3$ | CH | |

Table X (cont.)

| $R_1$ | $R_2$ | R' | X | Y | Z | m.p. (°C) |
|---|---|---|---|---|---|---|
| H | $CH_2F$ | cyclopropyl | $CH_3$ | $CH_3$ | CH | |
| H | $CH_2F$ | cyclopropyl | $CH_3$ | $OCH_3$ | CH | |
| H | $CH_2F$ | cyclopropyl | $OCH_3$ | $OCH_3$ | CH | |
| H | $CH_2F$ | cyclopropyl | $CH_3$ | $CH_3$ | N | |
| H | $CH_2F$ | cyclopropyl | $CH_3$ | $OCH_3$ | N | |
| H | $CH_2F$ | cyclopropyl | $OCH_3$ | $OCH_3$ | N | |
| H | $CH_2F$ | cyclopropyl | Cl | $OCH_3$ | CH | |
| H | $CH_2F$ | cyclobutyl | $CH_3$ | $CH_3$ | CH | |
| H | $CH_2F$ | cyclobutyl | $CH_3$ | $OCH_3$ | CH | |
| H | $CH_2F$ | cyclobutyl | $OCH_3$ | $OCH_3$ | CH | |
| H | $CH_2F$ | cyclobutyl | $CH_3$ | $CH_3$ | N | |
| H | $CH_2F$ | cyclobutyl | $CH_3$ | $OCH_3$ | N | |
| H | $CH_2F$ | cyclobutyl | $OCH_3$ | $OCH_3$ | N | |
| H | $CH_2F$ | cyclobutyl | Cl | $OCH_3$ | CH | |
| H | $CH_2F$ | 2-fluorocyclopropyl | $CH_3$ | $CH_3$ | CH | |
| H | $CH_2F$ | 2-fluorocyclopropyl | $CH_3$ | $OCH_3$ | CH | |
| H | $CH_2F$ | 2-fluorocyclopropyl | $OCH_3$ | $OCH_3$ | CH | |
| H | $CH_2F$ | 2-fluorocyclopropyl | $CH_3$ | $CH_3$ | N | |
| H | $CH_2F$ | 2-fluorocyclopropyl | $CH_3$ | $OCH_3$ | N | |
| H | $CH_2F$ | 2-fluorocyclopropyl | $OCH_3$ | $OCH_3$ | N | |
| H | $CH_2F$ | 2-fluorocyclopropyl | Cl | $OCH_3$ | CH | |
| H | $CH_2F$ | 2,2-difluorocyclopropyl | $CH_3$ | $CH_3$ | CH | |
| H | $CH_2F$ | 2,2-difluorocyclopropyl | $CH_3$ | $OCH_3$ | CH | |
| H | $CH_2F$ | 2,2-difluorocyclopropyl | $OCH_3$ | $OCH_3$ | CH | |
| H | $CH_2F$ | 2,2-difluorocyclopropyl | $CH_3$ | $CH_3$ | N | |
| H | $CH_2F$ | 2,2-difluorocyclopropyl | $CH_3$ | $OCH_3$ | N | |
| H | $CH_2F$ | 2,2-difluorocyclopropyl | $OCH_3$ | $OCH_3$ | N | |
| H | $CH_2F$ | 2,2-difluorocyclopropyl | Cl | $OCH_3$ | CH | |

Table XI

| $R_1$ | $R_2$ | R' | X | Y | Z | m.p. (°C) |
|---|---|---|---|---|---|---|
| H | H | cyclopropyl | $CH_3$ | $CH_3$ | CH | |
| H | H | cyclopropyl | $CH_3$ | $OCH_3$ | CH | |
| H | H | cyclopropyl | $OCH_3$ | $OCH_3$ | CH | |
| H | H | cyclopropyl | $CH_3$ | $CH_3$ | N | |
| H | H | cyclopropyl | $CH_3$ | $OCH_3$ | N | |
| H | H | cyclopropyl | $OCH_3$ | $OCH_3$ | N | |
| H | H | cyclopropyl | Cl | $OCH_3$ | CH | |
| H | H | cyclobutyl | $CH_3$ | $CH_3$ | CH | |
| H | H | cyclobutyl | $CH_3$ | $OCH_3$ | CH | |
| H | H | cyclobutyl | $OCH_3$ | $OCH_3$ | CH | |
| H | H | cyclobutyl | $CH_3$ | $CH_3$ | N | |
| H | H | cyclobutyl | $CH_3$ | $OCH_3$ | N | |
| H | H | cyclobutyl | $OCH_3$ | $OCH_3$ | N | |
| H | H | cyclobutyl | Cl | $OCH_3$ | CH | |
| H | H | 2-fluorocyclopropyl | $CH_3$ | $CH_3$ | CH | |
| H | H | 2-fluorocyclopropyl | $CH_3$ | $OCH_3$ | CH | |
| H | H | 2-fluorocyclopropyl | $OCH_3$ | $OCH_3$ | CH | |
| H | H | 2-fluorocyclopropyl | $CH_3$ | $CH_3$ | N | |
| H | H | 2-fluorocyclopropyl | $CH_3$ | $OCH_3$ | N | |
| H | H | 2-fluorocyclopropyl | $OCH_3$ | $OCH_3$ | N | |
| H | H | 2-fluorocyclopropyl | Cl | $OCH_3$ | CH | |
| H | H | 2,2-difluorocyclopropyl | $CH_3$ | $CH_3$ | CH | |
| H | H | 2,2-difluorocyclopropyl | $CH_3$ | $OCH_3$ | CH | |
| H | H | 2,2-difluorocyclopropyl | $OCH_3$ | $OCH_3$ | CH | |
| H | H | 2,2-difluorocyclopropyl | $CH_3$ | $CH_3$ | N | |
| H | H | 2,2-difluorocyclopropyl | $CH_3$ | $OCH_3$ | N | |
| H | H | 2,2-difluorocyclopropyl | $OCH_3$ | $OCH_3$ | N | |
| H | H | 2,2-difluorocyclopropyl | Cl | $OCH_3$ | CH | |

Table XI (cont.)

| $R_1$ | $R_2$ | R' | X | Y | Z | m.p. (°C) |
|---|---|---|---|---|---|---|
| H | $SO_2N(CH_3)_2$ | cyclopropyl | $CH_3$ | $CH_3$ | CH | |
| H | $SO_2N(CH_3)_2$ | cyclopropyl | $CH_3$ | $OCH_3$ | CH | |
| H | $SO_2N(CH_3)_2$ | cyclopropyl | $OCH_3$ | $OCH_3$ | CH | |
| H | $SO_2N(CH_3)_2$ | cyclopropyl | $CH_3$ | $CH_3$ | N | |
| H | $SO_2N(CH_3)_2$ | cyclopropyl | $CH_3$ | $OCH_3$ | N | |
| H | $SO_2N(CH_3)_2$ | cyclopropyl | $OCH_3$ | $OCH_3$ | N | |
| H | $SO_2N(CH_3)_2$ | cyclopropyl | Cl | $OCH_3$ | CH | |
| H | $SO_2N(CH_3)_2$ | cyclobutyl | $CH_3$ | $CH_3$ | CH | |
| H | $SO_2N(CH_3)_2$ | cyclobutyl | $CH_3$ | $OCH_3$ | CH | |
| H | $SO_2N(CH_3)_2$ | cyclobutyl | $OCH_3$ | $OCH_3$ | CH | |
| H | $SO_2N(CH_3)_2$ | cyclobutyl | $CH_3$ | $CH_3$ | N | |
| H | $SO_2N(CH_3)_2$ | cyclobutyl | $CH_3$ | $OCH_3$ | N | |
| H | $SO_2N(CH_3)_2$ | cyclobutyl | $OCH_3$ | $OCH_3$ | N | |
| H | $SO_2N(CH_3)_2$ | cyclobutyl | Cl | $OCH_3$ | CH | |
| H | $SO_2N(CH_3)_2$ | 2-fluorocyclopropyl | $CH_3$ | $CH_3$ | CH | |
| H | $SO_2N(CH_3)_2$ | 2-fluorocyclopropyl | $CH_3$ | $OCH_3$ | CH | |
| H | $SO_2N(CH_3)_2$ | 2-fluorocyclopropyl | $OCH_3$ | $OCH_3$ | CH | |
| H | $SO_2N(CH_3)_2$ | 2-fluorocyclopropyl | $CH_3$ | $CH_3$ | N | |
| H | $SO_2N(CH_3)_2$ | 2-fluorocyclopropyl | $CH_3$ | $OCH_3$ | N | |
| H | $SO_2N(CH_3)_2$ | 2-fluorocyclopropyl | $OCH_3$ | $OCH_3$ | N | |
| H | $SO_2N(CH_3)_2$ | 2-fluorocyclopropyl | Cl | $OCH_3$ | CH | |
| H | $SO_2N(CH_3)_2$ | 2,2-difluorocyclopropyl | $CH_3$ | $CH_3$ | CH | |
| H | $SO_2N(CH_3)_2$ | 2,2-difluorocyclopropyl | $CH_3$ | $OCH_3$ | CH | |
| H | $SO_2N(CH_3)_2$ | 2,2-difluorocyclopropyl | $OCH_3$ | $OCH_3$ | CH | |
| H | $SO_2N(CH_3)_2$ | 2,2-difluorocyclopropyl | $CH_3$ | $CH_3$ | N | |
| H | $SO_2N(CH_3)_2$ | 2,2-difluorocyclopropyl | $CH_3$ | $OCH_3$ | N | |
| H | $SO_2N(CH_3)_2$ | 2,2-difluorocyclopropyl | $OCH_3$ | $OCH_3$ | N | |
| H | $SO_2N(CH_3)_2$ | 2,2-difluorocyclopropyl | Cl | $OCH_3$ | CH | |

Table XI (cont.)

| $R_1$ | $R_2$ | R' | X | Y | Z | m.p. (°C) |
|---|---|---|---|---|---|---|
| H | $CH_2CN$ | cyclopropyl | $CH_3$ | $CH_3$ | CH | |
| H | $CH_2CN$ | cyclopropyl | $CH_3$ | $OCH_3$ | CH | |
| H | $CH_2CN$ | cyclopropyl | $OCH_3$ | $OCH_3$ | CH | |
| H | $CH_2CN$ | cyclopropyl | $CH_3$ | $CH_3$ | N | |
| H | $CH_2CN$ | cyclopropyl | $CH_3$ | $OCH_3$ | N | |
| H | $CH_2CN$ | cyclopropyl | $OCH_3$ | $OCH_3$ | N | |
| H | $CH_2CN$ | cyclopropyl | Cl | $OCH_3$ | CH | |
| H | $CH_2CN$ | cyclobutyl | $CH_3$ | $CH_3$ | CH | |
| H | $CH_2CN$ | cyclobutyl | $CH_3$ | $OCH_3$ | CH | |
| H | $CH_2CN$ | cyclobutyl | $OCH_3$ | $OCH_3$ | CH | |
| H | $CH_2CN$ | cyclobutyl | $CH_3$ | $CH_3$ | N | |
| H | $CH_2CN$ | cyclobutyl | $CH_3$ | $OCH_3$ | N | |
| H | $CH_2CN$ | cyclobutyl | $OCH_3$ | $OCH_3$ | N | |
| H | $CH_2CN$ | cyclobutyl | Cl | $OCH_3$ | CH | |
| H | $CH_2CN$ | 2-fluorocyclopropyl | $CH_3$ | $CH_3$ | CH | |
| H | $CH_2CN$ | 2-fluorocyclopropyl | $CH_3$ | $OCH_3$ | CH | |
| H | $CH_2CN$ | 2-fluorocyclopropyl | $OCH_3$ | $OCH_3$ | CH | |
| H | $CH_2CN$ | 2-fluorocyclopropyl | $CH_3$ | $CH_3$ | N | |
| H | $CH_2CN$ | 2-fluorocyclopropyl | $CH_3$ | $OCH_3$ | N | |
| H | $CH_2CN$ | 2-fluorocyclopropyl | $OCH_3$ | $OCH_3$ | N | |
| H | $CH_2CN$ | 2-fluorocyclopropyl | Cl | $OCH_3$ | CH | |
| H | $CH_2CN$ | 2,2-difluorocyclopropyl | $CH_3$ | $CH_3$ | CH | |
| H | $CH_2CN$ | 2,2-difluorocyclopropyl | $CH_3$ | $OCH_3$ | CH | |
| H | $CH_2CN$ | 2,2-difluorocyclopropyl | $OCH_3$ | $OCH_3$ | CH | |
| H | $CH_2CN$ | 2,2-difluorocyclopropyl | $CH_3$ | $CH_3$ | N | |
| H | $CH_2CN$ | 2,2-difluorocyclopropyl | $CH_3$ | $OCH_3$ | N | |
| H | $CH_2CN$ | 2,2-difluorocyclopropyl | $OCH_3$ | $OCH_3$ | N | |
| H | $CH_2CN$ | 2,2-difluorocyclopropyl | Cl | $OCH_3$ | CH | |

Table XII

| $R_1$ | n | $R_2$ | R' | X | Y | Z | m.p. (°C) |
|---|---|---|---|---|---|---|---|
| H | 0 | H | cyclopropyl | $CH_3$ | $CH_3$ | CH | |
| H | 0 | H | cyclopropyl | $CH_3$ | $OCH_3$ | CH | |
| H | 0 | H | cyclopropyl | $OCH_3$ | $OCH_3$ | CH | |
| H | 0 | H | cyclopropyl | $CH_3$ | $CH_3$ | N | |
| H | 0 | H | cyclopropyl | $CH_3$ | $OCH_3$ | N | |
| H | 0 | H | cyclopropyl | $OCH_3$ | $OCH_3$ | N | |
| H | 0 | H | cyclopropyl | Cl | $OCH_3$ | CH | |
| H | 0 | H | cyclobutyl | $CH_3$ | $CH_3$ | CH | |
| H | 0 | H | cyclobutyl | $CH_3$ | $OCH_3$ | CH | |
| H | 0 | H | cyclobutyl | $OCH_3$ | $OCH_3$ | CH | |
| H | 0 | H | cyclobutyl | $CH_3$ | $CH_3$ | N | |
| H | 0 | H | cyclobutyl | $CH_3$ | $OCH_3$ | N | |
| H | 0 | H | cyclobutyl | $OCH_3$ | $OCH_3$ | N | |
| H | 0 | H | cyclobutyl | Cl | $OCH_3$ | CH | |
| H | 0 | H | cyclopentyl | $CH_3$ | $CH_3$ | CH | |
| H | 0 | H | cyclopentyl | $CH_3$ | $OCH_3$ | CH | |
| H | 0 | H | cyclopentyl | $OCH_3$ | $OCH_3$ | CH | |
| H | 0 | H | cyclopentyl | $CH_3$ | $CH_3$ | N | |
| H | 0 | H | cyclopentyl | $CH_3$ | $OCH_3$ | N | |
| H | 0 | H | cyclopentyl | $OCH_3$ | $OCH_3$ | N | |
| H | 0 | H | cyclopentyl | Cl | $OCH_3$ | CH | |
| H | 0 | H | 2-fluorocyclopropyl | $CH_3$ | $CH_3$ | CH | |
| H | 0 | H | 2-fluorocyclopropyl | $CH_3$ | $OCH_3$ | CH | |
| H | 0 | H | 2-fluorocyclopropyl | $OCH_3$ | $OCH_3$ | CH | |
| H | 0 | H | 2-fluorocyclopropyl | $CH_3$ | $CH_3$ | N | |
| H | 0 | H | 2-fluorocyclopropyl | $CH_3$ | $OCH_3$ | N | |
| H | 0 | H | 2-fluorocyclopropyl | $OCH_3$ | $OCH_3$ | N | |
| H | 0 | H | 2-fluorocyclopropyl | Cl | $OCH_3$ | CH | |

Table XII (cont.)

| R1 | n | R2 | R' | X | Y | Z | m.p. (°C) |
|---|---|---|---|---|---|---|---|
| H | 0 | Cl | cyclopropyl | $CH_3$ | $CH_3$ | CH | |
| H | 0 | Cl | cyclopropyl | $CH_3$ | $OCH_3$ | CH | |
| H | 0 | Cl | cyclopropyl | $OCH_3$ | $OCH_3$ | CH | |
| H | 0 | Cl | cyclopropyl | $CH_3$ | $CH_3$ | N | |
| H | 0 | Cl | cyclopropyl | $CH_3$ | $OCH_3$ | N | |
| H | 0 | Cl | cyclopropyl | $OCH_3$ | $OCH_3$ | N | |
| H | 0 | Cl | cyclopropyl | Cl | $OCH_3$ | CH | |
| H | 0 | Cl | cyclobutyl | $CH_3$ | $CH_3$ | CH | |
| H | 0 | Cl | cyclobutyl | $CH_3$ | $OCH_3$ | CH | |
| H | 0 | Cl | cyclobutyl | $OCH_3$ | $OCH_3$ | CH | |
| H | 0 | Cl | cyclobutyl | $CH_3$ | $CH_3$ | N | |
| H | 0 | Cl | cyclobutyl | $CH_3$ | $OCH_3$ | N | |
| H | 0 | Cl | cyclobutyl | $OCH_3$ | $OCH_3$ | N | |
| H | 0 | Cl | cyclobutyl | Cl | $OCH_3$ | CH | |
| H | 0 | Cl | cyclopentyl | $CH_3$ | $CH_3$ | CH | |
| H | 0 | Cl | cyclopentyl | $CH_3$ | $OCH_3$ | CH | |
| H | 0 | Cl | cyclopentyl | $OCH_3$ | $OCH_3$ | CH | |
| H | 0 | Cl | cyclopentyl | $CH_3$ | $CH_3$ | N | |
| H | 0 | Cl | cyclopentyl | $CH_3$ | $OCH_3$ | N | |
| H | 0 | Cl | cyclopentyl | $OCH_3$ | $OCH_3$ | N | |
| H | 0 | Cl | cyclopentyl | Cl | $OCH_3$ | CH | |
| H | 0 | Cl | 2-fluorocyclopropyl | $CH_3$ | $CH_3$ | CH | |
| H | 0 | Cl | 2-fluorocyclopropyl | $CH_3$ | $OCH_3$ | CH | |
| H | 0 | Cl | 2-fluorocyclopropyl | $OCH_3$ | $OCH_3$ | CH | |
| H | 0 | Cl | 2-fluorocyclopropyl | $CH_3$ | $CH_3$ | N | |
| H | 0 | Cl | 2-fluorocyclopropyl | $CH_3$ | $OCH_3$ | N | |
| H | 0 | Cl | 2-fluorocyclopropyl | $OCH_3$ | $OCH_3$ | N | |
| H | 0 | Cl | 2-fluorocyclopropyl | Cl | $OCH_3$ | CH | |

Table XII (cont.)

| $R_1$ | n | $R_2$ | R' | X | Y | Z | m.p. (°C) |
|---|---|---|---|---|---|---|---|
| H | 0 | $CH_2CH_3$ | cyclopropyl | $CH_3$ | $CH_3$ | CH | |
| H | 0 | $CH_2CH_3$ | cyclopropyl | $CH_3$ | $OCH_3$ | CH | |
| H | 0 | $CH_2CH_3$ | cyclopropyl | $OCH_3$ | $OCH_3$ | CH | |
| H | 0 | $CH_2CH_3$ | cyclopropyl | $CH_3$ | $CH_3$ | N | |
| H | 0 | $CH_2CH_3$ | cyclopropyl | $CH_3$ | $OCH_3$ | N | |
| H | 0 | $CH_2CH_3$ | cyclopropyl | $OCH_3$ | $OCH_3$ | N | |
| H | 0 | $CH_2CH_3$ | cyclopropyl | Cl | $OCH_3$ | CH | |
| H | 0 | $CH_2CH_3$ | cyclobutyl | $CH_3$ | $CH_3$ | CH | |
| H | 0 | $CH_2CH_3$ | cyclobutyl | $CH_3$ | $OCH_3$ | CH | |
| H | 0 | $CH_2CH_3$ | cyclobutyl | $OCH_3$ | $OCH_3$ | CH | |
| H | 0 | $CH_2CH_3$ | cyclobutyl | $CH_3$ | $CH_3$ | N | |
| H | 0 | $CH_2CH_3$ | cyclobutyl | $CH_3$ | $OCH_3$ | N | |
| H | 0 | $CH_2CH_3$ | cyclobutyl | $OCH_3$ | $OCH_3$ | N | |
| H | 0 | $CH_2CH_3$ | cyclobutyl | Cl | $OCH_3$ | CH | |
| H | 0 | $CH_2CH_3$ | cyclopentyl | $CH_3$ | $CH_3$ | CH | |
| H | 0 | $CH_2CH_3$ | cyclopentyl | $CH_3$ | $OCH_3$ | CH | |
| H | 0 | $CH_2CH_3$ | cyclopentyl | $OCH_3$ | $OCH_3$ | CH | |
| H | 0 | $CH_2CH_3$ | cyclopentyl | $CH_3$ | $CH_3$ | N | |
| H | 0 | $CH_2CH_3$ | cyclopentyl | $CH_3$ | $OCH_3$ | N | |
| H | 0 | $CH_2CH_3$ | cyclopentyl | $OCH_3$ | $OCH_3$ | N | |
| H | 0 | $CH_2CH_3$ | cyclopentyl | Cl | $OCH_3$ | CH | |
| H | 0 | $CH_2CH_3$ | 2-fluorocyclopropyl | $CH_3$ | $CH_3$ | CH | |
| H | 0 | $CH_2CH_3$ | 2-fluorocyclopropyl | $CH_3$ | $OCH_3$ | CH | |
| H | 0 | $CH_2CH_3$ | 2-fluorocyclopropyl | $OCH_3$ | $OCH_3$ | CH | |
| H | 0 | $CH_2CH_3$ | 2-fluorocyclopropyl | $CH_3$ | $CH_3$ | N | |
| H | 0 | $CH_2CH_3$ | 2-fluorocyclopropyl | $CH_3$ | $OCH_3$ | N | |
| H | 0 | $CH_2CH_3$ | 2-fluorocyclopropyl | $OCH_3$ | $OCH_3$ | N | |
| H | 0 | $CH_2CH_3$ | 2-fluorocyclopropyl | Cl | $OCH_3$ | CH | |

Table XII (cont.)

| $R_1$ | n | $R_2$ | R' | X | Y | Z | m.p. (°C) |
|---|---|---|---|---|---|---|---|
| H | 0 | $OCHF_2$ | cyclopropyl | $CH_3$ | $CH_3$ | CH | |
| H | 0 | $OCHF_2$ | cyclopropyl | $CH_3$ | $OCH_3$ | CH | |
| H | 0 | $OCHF_2$ | cyclopropyl | $OCH_3$ | $OCH_3$ | CH | |
| H | 0 | $OCHF_2$ | cyclopropyl | $CH_3$ | $CH_3$ | N | |
| H | 0 | $OCHF_2$ | cyclopropyl | $CH_3$ | $OCH_3$ | N | |
| H | 0 | $OCHF_2$ | cyclopropyl | $OCH_3$ | $OCH_3$ | N | |
| H | 0 | $OCHF_2$ | cyclopropyl | Cl | $OCH_3$ | CH | |
| H | 0 | $OCHF_2$ | cyclobutyl | $CH_3$ | $CH_3$ | CH | |
| H | 0 | $OCHF_2$ | cyclobutyl | $CH_3$ | $OCH_3$ | CH | |
| H | 0 | $OCHF_2$ | cyclobutyl | $OCH_3$ | $OCH_3$ | CH | |
| H | 0 | $OCHF_2$ | cyclobutyl | $CH_3$ | $CH_3$ | N | |
| H | 0 | $OCHF_2$ | cyclobutyl | $CH_3$ | $OCH_3$ | N | |
| H | 0 | $OCHF_2$ | cyclobutyl | $OCH_3$ | $OCH_3$ | N | |
| H | 0 | $OCHF_2$ | cyclobutyl | Cl | $OCH_3$ | CH | |
| H | 0 | $OCHF_2$ | cyclopentyl | $CH_3$ | $CH_3$ | CH | |
| H | 0 | $OCHF_2$ | cyclopentyl | $CH_3$ | $OCH_3$ | CH | |
| H | 0 | $OCHF_2$ | cyclopentyl | $OCH_3$ | $OCH_3$ | CH | |
| H | 0 | $OCHF_2$ | cyclopentyl | $CH_3$ | $CH_3$ | N | |
| H | 0 | $OCHF_2$ | cyclopentyl | $CH_3$ | $OCH_3$ | N | |
| H | 0 | $OCHF_2$ | cyclopentyl | $OCH_3$ | $OCH_3$ | N | |
| H | 0 | $OCHF_2$ | cyclopentyl | Cl | $OCH_3$ | CH | |
| H | 0 | $OCHF_2$ | 2-fluorocyclopropyl | $CH_3$ | $CH_3$ | CH | |
| H | 0 | $OCHF_2$ | 2-fluorocyclopropyl | $CH_3$ | $OCH_3$ | CH | |
| H | 0 | $OCHF_2$ | 2-fluorocyclopropyl | $OCH_3$ | $OCH_3$ | CH | |
| H | 0 | $OCHF_2$ | 2-fluorocyclopropyl | $CH_3$ | $CH_3$ | N | |
| H | 0 | $OCHF_2$ | 2-fluorocyclopropyl | $CH_3$ | $OCH_3$ | N | |
| H | 0 | $OCHF_2$ | 2-fluorocyclopropyl | $OCH_3$ | $OCH_3$ | N | |
| H | 0 | $OCHF_2$ | 2-fluorocyclopropyl | Cl | $OCH_3$ | CH | |

Table XII (cont.)

| $R_1$ | n | $R_2$ | R' | X | Y | Z | m.p. (°C) |
|---|---|---|---|---|---|---|---|
| H | 0 | CN | cyclopropyl | $CH_3$ | $CH_3$ | CH | |
| H | 0 | CN | cyclopropyl | $CH_3$ | $OCH_3$ | CH | |
| H | 0 | CN | cyclopropyl | $OCH_3$ | $OCH_3$ | CH | |
| H | 0 | CN | cyclopropyl | $CH_3$ | $CH_3$ | N | |
| H | 0 | CN | cyclopropyl | $CH_3$ | $OCH_3$ | N | |
| H | 0 | CN | cyclopropyl | $OCH_3$ | $OCH_3$ | N | |
| H | 0 | CN | cyclopropyl | Cl | $OCH_3$ | CH | |
| H | 0 | CN | cyclobutyl | $CH_3$ | $CH_3$ | CH | |
| H | 0 | CN | cyclobutyl | $CH_3$ | $OCH_3$ | CH | |
| H | 0 | CN | cyclobutyl | $OCH_3$ | $OCH_3$ | CH | |
| H | 0 | CN | cyclobutyl | $CH_3$ | $CH_3$ | N | |
| H | 0 | CN | cyclobutyl | $CH_3$ | $OCH_3$ | N | |
| H | 0 | CN | cyclobutyl | $OCH_3$ | $OCH_3$ | N | |
| H | 0 | CN | cyclobutyl | Cl | $OCH_3$ | CH | |
| H | 0 | CN | cyclopentyl | $CH_3$ | $CH_3$ | CH | |
| H | 0 | CN | cyclopentyl | $CH_3$ | $OCH_3$ | CH | |
| H | 0 | CN | cyclopentyl | $OCH_3$ | $OCH_3$ | CH | |
| H | 0 | CN | cyclopentyl | $CH_3$ | $CH_3$ | N | |
| H | 0 | CN | cyclopentyl | $CH_3$ | $OCH_3$ | N | |
| H | 0 | CN | cyclopentyl | $OCH_3$ | $OCH_3$ | N | |
| H | 0 | CN | cyclopentyl | Cl | $OCH_3$ | CH | |
| H | 0 | CN | 2-fluorocyclopropyl | $CH_3$ | $CH_3$ | CH | |
| H | 0 | CN | 2-fluorocyclopropyl | $CH_3$ | $OCH_3$ | CH | |
| H | 0 | CN | 2-fluorocyclopropyl | $OCH_3$ | $OCH_3$ | CH | |
| H | 0 | CN | 2-fluorocyclopropyl | $CH_3$ | $CH_3$ | N | |
| H | 0 | CN | 2-fluorocyclopropyl | $CH_3$ | $OCH_3$ | N | |
| H | 0 | CN | 2-fluorocyclopropyl | $OCH_3$ | $OCH_3$ | N | |
| H | 0 | CN | 2-fluorocyclopropyl | Cl | $OCH_3$ | CH | |

Table XII (cont.)

| $R_1$ | n | $R_2$ | R' | X | Y | Z | m.p.(°C) |
|---|---|---|---|---|---|---|---|
| H | 0 | $CH_3$ | $CH_3$ | $CH_3$ | $CH_3$ | CH | |
| H | 0 | $CH_3$ | $CH_3$ | $CH_3$ | $OCH_3$ | CH | |
| H | 0 | $CH_3$ | $CH_3$ | $OCH_3$ | $OCH_3$ | CH | |
| H | 0 | $CH_3$ | $CH_3$ | $CH_3$ | $CH_3$ | N | |
| H | 0 | $CH_3$ | $CH_3$ | $CH_3$ | $OCH_3$ | N | |
| H | 0 | $CH_3$ | $CH_3$ | $OCH_3$ | $OCH_3$ | N | |
| H | 0 | $CH_3$ | $CH_3$ | Cl | $OCH_3$ | CH | |
| H | 0 | $CH_3$ | $CH_2CH_3$ | $CH_3$ | $CH_3$ | CH | |
| H | 0 | $CH_3$ | $CH_2CH_3$ | $CH_3$ | $OCH_3$ | CH | |
| H | 0 | $CH_3$ | $CH_2CH_3$ | $OCH_3$ | $OCH_3$ | CH | |
| H | 0 | $CH_3$ | $CH_2CH_3$ | $CH_3$ | $CH_3$ | N | |
| H | 0 | $CH_3$ | $CH_2CH_3$ | $CH_3$ | $OCH_3$ | N | |
| H | 0 | $CH_3$ | $CH_2CH_3$ | $OCH_3$ | $OCH_3$ | N | |
| H | 0 | $CH_3$ | $CH_2CH_3$ | Cl | $OCH_3$ | CH | |
| H | 0 | $CH_3$ | $CH_2CH_2CH_3$ | $CH_3$ | $CH_3$ | CH | |
| H | 0 | $CH_3$ | $CH_2CH_2CH_3$ | $CH_3$ | $OCH_3$ | CH | |
| H | 0 | $CH_3$ | $CH_2CH_2CH_3$ | $OCH_3$ | $OCH_3$ | CH | |
| H | 0 | $CH_3$ | $CH_2CH_2CH_3$ | $CH_3$ | $CH_3$ | N | |
| H | 0 | $CH_3$ | $CH_2CH_2CH_3$ | $CH_3$ | $OCH_3$ | N | |
| H | 0 | $CH_3$ | $CH_2CH_2CH_3$ | $OCH_3$ | $OCH_3$ | N | |
| H | 0 | $CH_3$ | $CH_2CH_2CH_3$ | Cl | $OCH_3$ | CH | |
| H | 0 | $CH_3$ | $CH_2CH_2CH_2CH_3$ | $CH_3$ | $CH_3$ | CH | |
| H | 0 | $CH_3$ | $CH_2CH_2CH_2CH_3$ | $CH_3$ | $OCH_3$ | CH | |
| H | 0 | $CH_3$ | $CH_2CH_2CH_2CH_3$ | $OCH_3$ | $OCH_3$ | CH | |
| H | 0 | $CH_3$ | $CH_2CH_2CH_2CH_3$ | $CH_3$ | $CH_3$ | N | |
| H | 0 | $CH_3$ | $CH_2CH_2CH_2CH_3$ | $CH_3$ | $OCH_3$ | N | |
| H | 0 | $CH_3$ | $CH_2CH_2CH_2CH_3$ | $OCH_3$ | $OCH_3$ | N | |
| H | 0 | $CH_3$ | $CH_2CH_2CH_2CH_3$ | Cl | $OCH_3$ | CH | |

Table XII (cont.)

| $R_1$ | n | $R_2$ | R' | X | Y | Z | m.p.(°C) |
|---|---|---|---|---|---|---|---|
| H | 0 | $OCH_3$ | $CH_3$ | $CH_3$ | $CH_3$ | CH | |
| H | 0 | $OCH_3$ | $CH_3$ | $CH_3$ | $OCH_3$ | CH | |
| H | 0 | $OCH_3$ | $CH_3$ | $OCH_3$ | $OCH_3$ | CH | |
| H | 0 | $OCH_3$ | $CH_3$ | $CH_3$ | $CH_3$ | N | |
| H | 0 | $OCH_3$ | $CH_3$ | $CH_3$ | $OCH_3$ | N | |
| H | 0 | $OCH_3$ | $CH_3$ | $OCH_3$ | $OCH_3$ | N | |
| H | 0 | $OCH_3$ | $CH_3$ | Cl | $OCH_3$ | CH | |
| H | 0 | $OCH_3$ | $CH_2CH_3$ | $CH_3$ | $CH_3$ | CH | |
| H | 0 | $OCH_3$ | $CH_2CH_3$ | $CH_3$ | $OCH_3$ | CH | |
| H | 0 | $OCH_3$ | $CH_2CH_3$ | $OCH_3$ | $OCH_3$ | CH | |
| H | 0 | $OCH_3$ | $CH_2CH_3$ | $CH_3$ | $CH_3$ | N | |
| H | 0 | $OCH_3$ | $CH_2CH_3$ | $CH_3$ | $OCH_3$ | N | |
| H | 0 | $OCH_3$ | $CH_2CH_3$ | $OCH_3$ | $OCH_3$ | N | |
| H | 0 | $OCH_3$ | $CH_2CH_3$ | Cl | $OCH_3$ | CH | |
| H | 0 | $OCH_3$ | $CH_2CH_2CH_3$ | $CH_3$ | $CH_3$ | CH | |
| H | 0 | $OCH_3$ | $CH_2CH_2CH_3$ | $CH_3$ | $OCH_3$ | CH | |
| H | 0 | $OCH_3$ | $CH_2CH_2CH_3$ | $OCH_3$ | $OCH_3$ | CH | |
| H | 0 | $OCH_3$ | $CH_2CH_2CH_3$ | $CH_3$ | $CH_3$ | N | |
| H | 0 | $OCH_3$ | $CH_2CH_2CH_3$ | $CH_3$ | $OCH_3$ | N | |
| H | 0 | $OCH_3$ | $CH_2CH_2CH_3$ | $OCH_3$ | $OCH_3$ | N | |
| H | 0 | $OCH_3$ | $CH_2CH_2CH_3$ | Cl | $OCH_3$ | CH | |
| H | 0 | $OCH_3$ | $CH_2CH_2CH_2CH_3$ | $CH_3$ | $CH_3$ | CH | |
| H | 0 | $OCH_3$ | $CH_2CH_2CH_2CH_3$ | $CH_3$ | $OCH_3$ | CH | |
| H | 0 | $OCH_3$ | $CH_2CH_2CH_2CH_3$ | $OCH_3$ | $OCH_3$ | CH | |
| H | 0 | $OCH_3$ | $CH_2CH_2CH_2CH_3$ | $CH_3$ | $CH_3$ | N | |
| H | 0 | $OCH_3$ | $CH_2CH_2CH_2CH_3$ | $CH_3$ | $OCH_3$ | N | |
| H | 0 | $OCH_3$ | $CH_2CH_2CH_2CH_3$ | $OCH_3$ | $OCH_3$ | N | |
| H | 0 | $OCH_3$ | $CH_2CH_2CH_2CH_3$ | Cl | $OCH_3$ | CH | |

Table XII (cont.)

| $R_1$ | n | $R_2$ | R' | X | Y | Z | m.p.(°C) |
|---|---|---|---|---|---|---|---|
| H | 0 | $SCH_3$ | $CH_3$ | $CH_3$ | $CH_3$ | CH | |
| H | 0 | $SCH_3$ | $CH_3$ | $CH_3$ | $OCH_3$ | CH | |
| H | 0 | $SCH_3$ | $CH_3$ | $OCH_3$ | $OCH_3$ | CH | |
| H | 0 | $SCH_3$ | $CH_3$ | $CH_3$ | $CH_3$ | N | |
| H | 0 | $SCH_3$ | $CH_3$ | $CH_3$ | $OCH_3$ | N | |
| H | 0 | $SCH_3$ | $CH_3$ | $OCH_3$ | $OCH_3$ | N | |
| H | 0 | $SCH_3$ | $CH_3$ | Cl | $OCH_3$ | CH | |
| H | 0 | $SCH_3$ | $CH_2CH_3$ | $CH_3$ | $CH_3$ | CH | |
| H | 0 | $SCH_3$ | $CH_2CH_3$ | $CH_3$ | $OCH_3$ | CH | |
| H | 0 | $SCH_3$ | $CH_2CH_3$ | $OCH_3$ | $OCH_3$ | CH | |
| H | 0 | $SCH_3$ | $CH_2CH_3$ | $CH_3$ | $CH_3$ | N | |
| H | 0 | $SCH_3$ | $CH_2CH_3$ | $CH_3$ | $OCH_3$ | N | |
| H | 0 | $SCH_3$ | $CH_2CH_3$ | $OCH_3$ | $OCH_3$ | N | |
| H | 0 | $SCH_3$ | $CH_2CH_3$ | Cl | $OCH_3$ | CH | |
| H | 0 | $SCH_3$ | $CH_2CH_2CH_3$ | $CH_3$ | $CH_3$ | CH | |
| H | 0 | $SCH_3$ | $CH_2CH_2CH_3$ | $CH_3$ | $OCH_3$ | CH | |
| H | 0 | $SCH_3$ | $CH_2CH_2CH_3$ | $OCH_3$ | $OCH_3$ | CH | |
| H | 0 | $SCH_3$ | $CH_2CH_2CH_3$ | $CH_3$ | $CH_3$ | N | |
| H | 0 | $SCH_3$ | $CH_2CH_2CH_3$ | $CH_3$ | $OCH_3$ | N | |
| H | 0 | $SCH_3$ | $CH_2CH_2CH_3$ | $OCH_3$ | $OCH_3$ | N | |
| H | 0 | $SCH_3$ | $CH_2CH_2CH_3$ | Cl | $OCH_3$ | CH | |
| H | 0 | $SCH_3$ | $CH_2CH_2CH_2CH_3$ | $CH_3$ | $CH_3$ | CH | |
| H | 0 | $SCH_3$ | $CH_2CH_2CH_2CH_3$ | $CH_3$ | $OCH_3$ | CH | |
| H | 0 | $SCH_3$ | $CH_2CH_2CH_2CH_3$ | $OCH_3$ | $OCH_3$ | CH | |
| H | 0 | $SCH_3$ | $CH_2CH_2CH_2CH_3$ | $CH_3$ | $CH_3$ | N | |
| H | 0 | $SCH_3$ | $CH_2CH_2CH_2CH_3$ | $CH_3$ | $OCH_3$ | N | |
| H | 0 | $SCH_3$ | $CH_2CH_2CH_2CH_3$ | $OCH_3$ | $OCH_3$ | N | |
| H | 0 | $SCH_3$ | $CH_2CH_2CH_2CH_3$ | Cl | $OCH_3$ | CH | |

Table XII (cont.)

| $R_1$ | n | $R_2$ | R' | X | Y | Z | m.p.(°C) |
|---|---|---|---|---|---|---|---|
| H | 0 | $N(CH_3)_2$ | $CH_3$ | $CH_3$ | $CH_3$ | CH | |
| H | 0 | $N(CH_3)_2$ | $CH_3$ | $CH_3$ | $OCH_3$ | CH | |
| H | 0 | $N(CH_3)_2$ | $CH_3$ | $OCH_3$ | $OCH_3$ | CH | |
| H | 0 | $N(CH_3)_2$ | $CH_3$ | $CH_3$ | $CH_3$ | N | |
| H | 0 | $N(CH_3)_2$ | $CH_3$ | $CH_3$ | $OCH_3$ | N | |
| H | 0 | $N(CH_3)_2$ | $CH_3$ | $OCH_3$ | $OCH_3$ | N | |
| H | 0 | $N(CH_3)_2$ | $CH_3$ | Cl | $OCH_3$ | CH | |
| H | 0 | $N(CH_3)_2$ | $CH_2CH_3$ | $CH_3$ | $CH_3$ | CH | |
| H | 0 | $N(CH_3)_2$ | $CH_2CH_3$ | $CH_3$ | $OCH_3$ | CH | |
| H | 0 | $N(CH_3)_2$ | $CH_2CH_3$ | $OCH_3$ | $OCH_3$ | CH | |
| H | 0 | $N(CH_3)_2$ | $CH_2CH_3$ | $CH_3$ | $CH_3$ | N | |
| H | 0 | $N(CH_3)_2$ | $CH_2CH_3$ | $CH_3$ | $OCH_3$ | N | |
| H | 0 | $N(CH_3)_2$ | $CH_2CH_3$ | $OCH_3$ | $OCH_3$ | N | |
| H | 0 | $N(CH_3)_2$ | $CH_2CH_3$ | Cl | $OCH_3$ | CH | |
| H | 0 | $N(CH_3)_2$ | $CH_2CH_2CH_3$ | $CH_3$ | $CH_3$ | CH | |
| H | 0 | $N(CH_3)_2$ | $CH_2CH_2CH_3$ | $CH_3$ | $OCH_3$ | CH | |
| H | 0 | $N(CH_3)_2$ | $CH_2CH_2CH_3$ | $OCH_3$ | $OCH_3$ | CH | |
| H | 0 | $N(CH_3)_2$ | $CH_2CH_2CH_3$ | $CH_3$ | $CH_3$ | N | |
| H | 0 | $N(CH_3)_2$ | $CH_2CH_2CH_3$ | $CH_3$ | $OCH_3$ | N | |
| H | 0 | $N(CH_3)_2$ | $CH_2CH_2CH_3$ | $OCH_3$ | $OCH_3$ | N | |
| H | 0 | $N(CH_3)_2$ | $CH_2CH_2CH_3$ | Cl | $OCH_3$ | CH | |
| H | 0 | $N(CH_3)_2$ | $CH_2CH_2CH_2CH_3$ | $CH_3$ | $CH_3$ | CH | |
| H | 0 | $N(CH_3)_2$ | $CH_2CH_2CH_2CH_3$ | $CH_3$ | $OCH_3$ | CH | |
| H | 0 | $N(CH_3)_2$ | $CH_2CH_2CH_2CH_3$ | $OCH_3$ | $OCH_3$ | CH | |
| H | 0 | $N(CH_3)_2$ | $CH_2CH_2CH_2CH_3$ | $CH_3$ | $CH_3$ | N | |
| H | 0 | $N(CH_3)_2$ | $CH_2CH_2CH_2CH_3$ | $CH_3$ | $OCH_3$ | N | |
| H | 0 | $N(CH_3)_2$ | $CH_2CH_2CH_2CH_3$ | $OCH_3$ | $OCH_3$ | N | |
| H | 0 | $N(CH_3)_2$ | $CH_2CH_2CH_2CH_3$ | Cl | $OCH_3$ | CH | |

Table XII (cont.)

| $R_1$ | n | $R_2$ | R' | X | Y | Z | m.p.(°C) |
|---|---|---|---|---|---|---|---|
| H | 0 | H | $CH_2CH_2CH_2CH_2CH_3$ | $CH_3$ | $CH_3$ | CH | |
| H | 0 | H | $CH_2CH_2CH_2CH_2CH_3$ | $CH_3$ | $OCH_3$ | CH | |
| H | 0 | H | $CH_2CH_2CH_2CH_2CH_3$ | $OCH_3$ | $OCH_3$ | CH | |
| H | 0 | H | $CH_2CH_2CH_2CH_2CH_3$ | $CH_3$ | $CH_3$ | N | |
| H | 0 | H | $CH_2CH_2CH_2CH_2CH_3$ | $CH_3$ | $OCH_3$ | N | |
| H | 0 | H | $CH_2CH_2CH_2CH_2CH_3$ | $OCH_3$ | $OCH_3$ | N | |
| H | 0 | H | $CH_2CH_2CH_2CH_2CH_3$ | Cl | $OCH_3$ | CH | |
| H | 0 | H | $CH(CH_3)_2$ | $CH_3$ | $CH_3$ | CH | |
| H | 0 | H | $CH(CH_3)_2$ | $CH_3$ | $OCH_3$ | CH | |
| H | 0 | H | $CH(CH_3)_2$ | $OCH_3$ | $OCH_3$ | CH | |
| H | 0 | H | $CH(CH_3)_2$ | $CH_3$ | $CH_3$ | N | |
| H | 0 | H | $CH(CH_3)_2$ | $CH_3$ | $OCH_3$ | N | |
| H | 0 | H | $CH(CH_3)_2$ | $OCH_3$ | $OCH_3$ | N | |
| H | 0 | H | $CH(CH_3)_2$ | Cl | $OCH_3$ | CH | |
| H | 0 | H | $CH_2CH(CH_3)_2$ | $CH_3$ | $CH_3$ | CH | |
| H | 0 | H | $CH_2CH(CH_3)_2$ | $CH_3$ | $OCH_3$ | CH | |
| H | 0 | H | $CH_2CH(CH_3)_2$ | $OCH_3$ | $OCH_3$ | CH | |
| H | 0 | H | $CH_2CH(CH_3)_2$ | $CH_3$ | $CH_3$ | N | |
| H | 0 | H | $CH_2CH(CH_3)_2$ | $CH_3$ | $OCH_3$ | N | |
| H | 0 | H | $CH_2CH(CH_3)_2$ | $OCH_3$ | $OCH_3$ | N | |
| H | 0 | H | $CH_2CH(CH_3)_2$ | Cl | $OCH_3$ | CH | |
| H | 0 | H | $CH(CH_3)CH_2CH_3$ | $CH_3$ | $CH_3$ | CH | |
| H | 0 | H | $CH(CH_3)CH_2CH_3$ | $CH_3$ | $OCH_3$ | CH | |
| H | 0 | H | $CH(CH_3)CH_2CH_3$ | $OCH_3$ | $OCH_3$ | CH | |
| H | 0 | H | $CH_2CH_2CH(CH_3)_2$ | $CH_3$ | $CH_3$ | N | |
| H | 0 | H | $CH_2CH_2CH(CH_3)_2$ | $CH_3$ | $OCH_3$ | N | |
| H | 0 | H | $CH_2CH_2CH(CH_3)_2$ | $OCH_3$ | $OCH_3$ | N | |
| H | 0 | H | $CH_2CH_2CH(CH_3)_2$ | Cl | $OCH_3$ | CH | |

Table XII (cont.)

| $R_1$ | n | $R_2$ | R' | X | Y | Z | m.p.(°C) |
|---|---|---|---|---|---|---|---|
| H | 0 | H | $CH_2Br$ | $CH_3$ | $CH_3$ | CH | |
| H | 0 | H | $CH_2Br$ | $CH_3$ | $OCH_3$ | CH | |
| H | 0 | H | $CH_2Br$ | $OCH_3$ | $OCH_3$ | CH | |
| H | 0 | H | $CH_2Br$ | $CH_3$ | $CH_3$ | N | |
| H | 0 | H | $CH_2CH_2Br$ | $CH_3$ | $OCH_3$ | N | |
| H | 0 | H | $CH_2CH_2Br$ | $OCH_3$ | $OCH_3$ | N | |
| H | 0 | H | $CH_2CH_2Br$ | Cl | $OCH_3$ | CH | |
| H | 0 | H | $CH(CH_3)CH_2F$ | $CH_3$ | $CH_3$ | CH | |
| H | 0 | H | $CH(CH_3)CH_2F$ | $CH_3$ | $OCH_3$ | CH | |
| H | 0 | H | $CH(CH_3)CH_2F$ | $OCH_3$ | $OCH_3$ | CH | |
| H | 0 | H | $CH(CH_3)CH_2F$ | $CH_3$ | $CH_3$ | N | |
| H | 0 | H | $CH(CH_3)CH_2F$ | $CH_3$ | $OCH_3$ | N | |
| H | 0 | H | $CH(CH_3)CH_2F$ | $OCH_3$ | $OCH_3$ | N | |
| H | 0 | H | $CH(CH_3)CH_2F$ | Cl | $OCH_3$ | CH | |
| H | 0 | H | $CH(CH_2F)_2$ | $CH_3$ | $CH_3$ | CH | |
| H | 0 | H | $CH(CH_2F)_2$ | $CH_3$ | $OCH_3$ | CH | |
| H | 0 | H | $CH(CH_2F)_2$ | $OCH_3$ | $OCH_3$ | CH | |
| H | 0 | H | $CH(CH_2F)_2$ | $CH_3$ | $CH_3$ | N | |
| H | 0 | H | $CH(CH_2F)_2$ | $CH_3$ | $OCH_3$ | N | |
| H | 0 | H | $CH(CH_2F)_2$ | $OCH_3$ | $OCH_3$ | N | |
| H | 0 | H | $CH(CH_2F)_2$ | Cl | $OCH_3$ | CH | |
| H | 0 | H | $CH_2I$ | $CH_3$ | $CH_3$ | CH | |
| H | 0 | H | $CH_2I$ | $CH_3$ | $OCH_3$ | CH | |
| H | 0 | H | $CH_2I$ | $OCH_3$ | $OCH_3$ | CH | |
| H | 0 | H | $CH_2I$ | $CH_3$ | $CH_3$ | N | |
| H | 0 | H | $CH_2CH_2CH_2F$ | $CH_3$ | $OCH_3$ | N | |
| H | 0 | H | $CH_2CH_2CH_2F$ | $OCH_3$ | $OCH_3$ | N | |
| H | 0 | H | $CH_2CH_2CH_2F$ | Cl | $OCH_3$ | CH | |

Table XII (cont.)

| $R_1$ | n | $R_2$ | R' | X | Y | Z | m.p.(°C) |
|---|---|---|---|---|---|---|---|
| H | 0 | H | $CH_2OCH_2F$ | $CH_3$ | $CH_3$ | CH | |
| H | 0 | H | $CH_2OCH_2F$ | $CH_3$ | $OCH_3$ | CH | |
| H | 0 | H | $CH_2OCH_2F$ | $OCH_3$ | $OCH_3$ | CH | |
| H | 0 | H | $CH_2OCH_2F$ | $CH_3$ | $CH_3$ | N | |
| H | 0 | H | $CH_2OCH_2F$ | $CH_3$ | $OCH_3$ | N | |
| H | 0 | H | $CH_2OCH_2F$ | $OCH_3$ | $OCH_3$ | N | |
| H | 0 | H | $CH_2OCH_2F$ | Cl | $OCH_3$ | CH | |
| H | 0 | H | $CH_2SOCH_3$ | $CH_3$ | $CH_3$ | CH | |
| H | 0 | H | $CH_2SOCH_3$ | $CH_3$ | $OCH_3$ | CH | |
| H | 0 | H | $CH_2SOCH_3$ | $OCH_3$ | $OCH_3$ | CH | |
| H | 0 | H | $CH_2SOCH_3$ | $CH_3$ | $CH_3$ | N | |
| H | 0 | H | $CH_2SOCH_3$ | $CH_3$ | $OCH_3$ | N | |
| H | 0 | H | $CH_2SOCH_3$ | $OCH_3$ | $OCH_3$ | N | |
| H | 0 | H | $CH_2SOCH_3$ | Cl | $OCH_3$ | CH | |
| H | 0 | H | $CH_2SO_2CH_3$ | $CH_3$ | $CH_3$ | CH | |
| H | 0 | H | $CH_2SO_2CH_3$ | $CH_3$ | $OCH_3$ | CH | |
| H | 0 | H | $CH_2SO_2CH_3$ | $OCH_3$ | $OCH_3$ | CH | |
| H | 0 | H | $CH_2SO_2CH_3$ | $CH_3$ | $CH_3$ | N | |
| H | 0 | H | $CH_2SO_2CH_3$ | $CH_3$ | $OCH_3$ | N | |
| H | 0 | H | $CH_2SO_2CH_3$ | $OCH_3$ | $OCH_3$ | N | |
| H | 0 | H | $CH_2SO_2CH_3$ | Cl | $OCH_3$ | CH | |
| H | 0 | H | $CH_2CN$ | $CH_3$ | $CH_3$ | CH | |
| H | 0 | H | $CH_2CH_2CN$ | $CH_3$ | $OCH_3$ | CH | |
| H | 0 | H | $CH_2(CH_3)CN$ | $OCH_3$ | $OCH_3$ | CH | |
| H | 0 | H | $CH_2NO_2$ | $CH_3$ | $CH_3$ | N | |
| H | 0 | H | $CH_2CH_2NO_2$ | $CH_3$ | $OCH_3$ | N | |
| H | 0 | H | $CH_2CN$ | $OCH_3$ | $OCH_3$ | N | |
| H | 0 | H | $CH_2CN$ | Cl | $OCH_3$ | CH | |

Table XII (cont.)

| $R_1$ | n | $R_2$ | R' | X | Y | Z | m.p.(°C) |
|---|---|---|---|---|---|---|---|
| H | 0 | H | $CH_2N(CH_3)_2$ | $CH_3$ | $CH_3$ | CH | |
| H | 0 | H | $CH_2N(CH_3)_2$ | $CH_3$ | $OCH_3$ | CH | |
| H | 0 | H | $CH_2N(CH_3)_2$ | $OCH_3$ | $OCH_3$ | CH | |
| H | 0 | H | $CH_2N(CH_3)_2$ | $CH_3$ | $CH_3$ | N | |
| H | 0 | H | $CH_2N(CH_3)_2$ | $CH_3$ | $OCH_3$ | N | |
| H | 0 | H | $CH_2N(CH_3)_2$ | $OCH_3$ | $OCH_3$ | N | |
| H | 0 | H | $CH_2N(CH_3)_2$ | Cl | $OCH_3$ | CH | |
| H | 0 | H | $CH_2CH_2N(CH_3)_2$ | $CH_3$ | $CH_3$ | CH | |
| H | 0 | H | $CH_2CH_2N(CH_3)_2$ | $CH_3$ | $OCH_3$ | CH | |
| H | 0 | H | $CH_2CH_2N(CH_3)_2$ | $OCH_3$ | $OCH_3$ | CH | |
| H | 0 | H | $CH_2CH_2N(CH_3)_2$ | $CH_3$ | $CH_3$ | N | |
| H | 0 | H | $CH_2CH_2N(CH_3)_2$ | $CH_3$ | $OCH_3$ | N | |
| H | 0 | H | $CH_2CH_2N(CH_3)_2$ | $OCH_3$ | $OCH_3$ | N | |
| H | 0 | H | $CH_2CH_2N(CH_3)_2$ | Cl | $OCH_3$ | CH | |
| H | 0 | H | $CH_2CH=CH_2$ | $CH_3$ | $CH_3$ | CH | |
| H | 0 | H | $CH_2CH=CH_2$ | $CH_3$ | $OCH_3$ | CH | |
| H | 0 | H | $CH_2CH=CH_2$ | $OCH_3$ | $OCH_3$ | CH | |
| H | 0 | H | $CH_2CH=CH_2$ | $CH_3$ | $CH_3$ | N | |
| H | 0 | H | $CH_2CH=CH_2$ | $CH_3$ | $OCH_3$ | N | |
| H | 0 | H | $CH_2CH=CH_2$ | $OCH_3$ | $OCH_3$ | N | |
| H | 0 | H | $CH_2CH=CH_2$ | Cl | $OCH_3$ | CH | |
| H | 0 | H | $CH=CH_2$ | $CH_3$ | $CH_3$ | CH | |
| H | 0 | H | $CH=CH_2$ | $CH_3$ | $OCH_3$ | CH | |
| H | 0 | H | $CH=CH_2$ | $OCH_3$ | $OCH_3$ | CH | |
| H | 0 | H | $CH=CH_2$ | $CH_3$ | $CH_3$ | N | |
| H | 0 | H | $CH=CH_2$ | $CH_3$ | $OCH_3$ | N | |
| H | 0 | H | $CH=CH_2$ | $OCH_3$ | $OCH_3$ | N | |
| H | 0 | H | $CH=CH_2$ | Cl | $OCH_3$ | CH | |

Table XII (cont.)

| $R_1$ | n | $R_2$ | R' | X | Y | Z | m.p.(°C) |
|---|---|---|---|---|---|---|---|
| H | 1 | H | $CH_3$ | $CH_3$ | $CH_3$ | CH | |
| H | 1 | H | $CH_3$ | $CH_3$ | $OCH_3$ | CH | |
| H | 1 | H | $CH_3$ | $OCH_3$ | $OCH_3$ | CH | |
| H | 1 | H | $CH_3$ | $CH_3$ | $CH_3$ | N | |
| H | 1 | H | $CH_3$ | $CH_3$ | $OCH_3$ | N | |
| H | 1 | H | $CH_3$ | $OCH_3$ | $OCH_3$ | N | |
| H | 1 | H | $CH_3$ | Cl | $OCH_3$ | CH | |
| H | 1 | H | $CH_2CH_3$ | $CH_3$ | $CH_3$ | CH | |
| H | 1 | H | $CH_2CH_3$ | $CH_3$ | $OCH_3$ | CH | |
| H | 1 | H | $CH_2CH_3$ | $OCH_3$ | $OCH_3$ | CH | |
| H | 1 | H | $CH_2CH_3$ | $CH_3$ | $CH_3$ | N | |
| H | 1 | H | $CH_2CH_3$ | $CH_3$ | $OCH_3$ | N | |
| H | 1 | H | $CH_2CH_3$ | $OCH_3$ | $OCH_3$ | N | |
| H | 1 | H | $CH_2CH_3$ | Cl | $OCH_3$ | CH | |
| H | 1 | H | $CH_2CH_2CH_3$ | $CH_3$ | $CH_3$ | CH | |
| H | 1 | H | $CH_2CH_2CH_3$ | $CH_3$ | $OCH_3$ | CH | |
| H | 1 | H | $CH_2CH_2CH_3$ | $OCH_3$ | $OCH_3$ | CH | |
| H | 1 | H | $CH_2CH_2CH_3$ | $CH_3$ | $CH_3$ | N | |
| H | 1 | H | $CH_2CH_2CH_3$ | $CH_3$ | $OCH_3$ | N | |
| H | 1 | H | $CH_2CH_2CH_3$ | $OCH_3$ | $OCH_3$ | N | |
| H | 1 | H | $CH_2CH_2CH_3$ | Cl | $OCH_3$ | CH | |
| H | 1 | H | $CH_2CH_2CH_2CH_3$ | $CH_3$ | $CH_3$ | CH | |
| H | 1 | H | $CH_2CH_2CH_2CH_3$ | $CH_3$ | $OCH_3$ | CH | |
| H | 1 | H | $CH_2CH_2CH_2CH_3$ | $OCH_3$ | $OCH_3$ | CH | |
| H | 1 | H | $CH_2CH_2CH_2CH_3$ | $CH_3$ | $CH_3$ | N | |
| H | 1 | H | $CH_2CH_2CH_2CH_3$ | $CH_3$ | $OCH_3$ | N | |
| H | 1 | H | $CH_2CH_2CH_2CH_3$ | $OCH_3$ | $OCH_3$ | N | |
| H | 1 | H | $CH_2CH_2CH_2CH_3$ | Cl | $OCH_3$ | CH | |

Table XIII

| $R_1$ | n | $R_2$ | R' | X | Y | Z | m.p. (°C) |
|---|---|---|---|---|---|---|---|
| H | 0 | H | cyclopropyl | $CH_3$ | $CH_3$ | CH | |
| H | 0 | H | cyclopropyl | $CH_3$ | $OCH_3$ | CH | |
| H | 0 | H | cyclopropyl | $OCH_3$ | $OCH_3$ | CH | |
| H | 0 | H | cyclopropyl | $CH_3$ | $CH_3$ | N | |
| H | 0 | H | cyclopropyl | $CH_3$ | $OCH_3$ | N | |
| H | 0 | H | cyclopropyl | $OCH_3$ | $OCH_3$ | N | |
| H | 0 | H | cyclopropyl | Cl | $OCH_3$ | CH | |
| H | 0 | H | cyclobutyl | $CH_3$ | $CH_3$ | CH | |
| H | 0 | H | cyclobutyl | $CH_3$ | $OCH_3$ | CH | |
| H | 0 | H | cyclobutyl | $OCH_3$ | $OCH_3$ | CH | |
| H | 0 | H | cyclobutyl | $CH_3$ | $CH_3$ | N | |
| H | 0 | H | cyclobutyl | $CH_3$ | $OCH_3$ | N | |
| H | 0 | H | cyclobutyl | $OCH_3$ | $OCH_3$ | N | |
| H | 0 | H | cyclobutyl | Cl | $OCH_3$ | CH | |
| H | 0 | H | cyclopentyl | $CH_3$ | $CH_3$ | CH | |
| H | 0 | H | cyclopentyl | $CH_3$ | $OCH_3$ | CH | |
| H | 0 | H | cyclopentyl | $OCH_3$ | $OCH_3$ | CH | |
| H | 0 | H | cyclopentyl | $CH_3$ | $CH_3$ | N | |
| H | 0 | H | cyclopentyl | $CH_3$ | $OCH_3$ | N | |
| H | 0 | H | cyclopentyl | $OCH_3$ | $OCH_3$ | N | |
| H | 0 | H | cyclopentyl | Cl | $OCH_3$ | CH | |
| H | 0 | H | 2-fluorocyclopropyl | $CH_3$ | $CH_3$ | CH | |
| H | 0 | H | 2-fluorocyclopropyl | $CH_3$ | $OCH_3$ | CH | |
| H | 0 | H | 2-fluorocyclopropyl | $OCH_3$ | $OCH_3$ | CH | |
| H | 0 | H | 2-fluorocyclopropyl | $CH_3$ | $CH_3$ | N | |
| H | 0 | H | 2-fluorocyclopropyl | $CH_3$ | $OCH_3$ | N | |
| H | 0 | H | 2-fluorocyclopropyl | $OCH_3$ | $OCH_3$ | N | |
| H | 0 | H | 2-fluorocyclopropyl | Cl | $OCH_3$ | CH | |

Table XIII (cont.)

| $R_1$ | n | $R_2$ | R' | X | Y | Z | m.p. (°C) |
|---|---|---|---|---|---|---|---|
| H | 0 | $OCHF_2$ | cyclopropyl | $CH_3$ | $CH_3$ | CH | |
| H | 0 | $OCHF_2$ | cyclopropyl | $CH_3$ | $OCH_3$ | CH | |
| H | 0 | $OCHF_2$ | cyclopropyl | $OCH_3$ | $OCH_3$ | CH | |
| H | 0 | $OCHF_2$ | cyclopropyl | $CH_3$ | $CH_3$ | N | |
| H | 0 | $OCHF_2$ | cyclopropyl | $CH_3$ | $OCH_3$ | N | |
| H | 0 | $OCHF_2$ | cyclopropyl | $OCH_3$ | $OCH_3$ | N | |
| H | 0 | $OCHF_2$ | cyclopropyl | Cl | $OCH_3$ | CH | |
| H | 0 | $OCHF_2$ | cyclobutyl | $CH_3$ | $CH_3$ | CH | |
| H | 0 | $OCHF_2$ | cyclobutyl | $CH_3$ | $OCH_3$ | CH | |
| H | 0 | $OCHF_2$ | cyclobutyl | $OCH_3$ | $OCH_3$ | CH | |
| H | 0 | $OCHF_2$ | cyclobutyl | $CH_3$ | $CH_3$ | N | |
| H | 0 | $OCHF_2$ | cyclobutyl | $CH_3$ | $OCH_3$ | N | |
| H | 0 | $OCHF_2$ | cyclobutyl | $OCH_3$ | $OCH_3$ | N | |
| H | 0 | $OCHF_2$ | cyclobutyl | Cl | $OCH_3$ | CH | |
| H | 0 | $OCHF_2$ | cyclopentyl | $CH_3$ | $CH_3$ | CH | |
| H | 0 | $OCHF_2$ | cyclopentyl | $CH_3$ | $OCH_3$ | CH | |
| H | 0 | $OCHF_2$ | cyclopentyl | $OCH_3$ | $OCH_3$ | CH | |
| H | 0 | $OCHF_2$ | cyclopentyl | $CH_3$ | $CH_3$ | N | |
| H | 0 | $OCHF_2$ | cyclopentyl | $CH_3$ | $OCH_3$ | N | |
| H | 0 | $OCHF_2$ | cyclopentyl | $OCH_3$ | $OCH_3$ | N | |
| H | 0 | $OCHF_2$ | cyclopentyl | Cl | $OCH_3$ | CH | |
| H | 0 | $OCHF_2$ | 2-fluorocyclopropyl | $CH_3$ | $CH_3$ | CH | |
| H | 0 | $OCHF_2$ | 2-fluorocyclopropyl | $CH_3$ | $OCH_3$ | CH | |
| H | 0 | $OCHF_2$ | 2-fluorocyclopropyl | $OCH_3$ | $OCH_3$ | CH | |
| H | 0 | $OCHF_2$ | 2-fluorocyclopropyl | $CH_3$ | $CH_3$ | N | |
| H | 0 | $OCHF_2$ | 2-fluorocyclopropyl | $CH_3$ | $OCH_3$ | N | |
| H | 0 | $OCHF_2$ | 2-fluorocyclopropyl | $OCH_3$ | $OCH_3$ | N | |
| H | 0 | $OCHF_2$ | 2-fluorocyclopropyl | Cl | $OCH_3$ | CH | |

Table XIII (cont.)

| $R_1$ | n | $R_2$ | R' | X | Y | Z | m.p.(°C) |
|---|---|---|---|---|---|---|---|
| H | 0 | H | $CH_2F$ | $CH_3$ | $CH_3$ | CH | |
| H | 0 | H | $CH_2F$ | $CH_3$ | $OCH_3$ | CH | |
| H | 0 | H | $CH_2F$ | $OCH_3$ | $OCH_3$ | CH | |
| H | 0 | H | $CH_2F$ | $CH_3$ | $CH_3$ | N | |
| H | 0 | H | $CH_2F$ | $CH_3$ | $OCH_3$ | N | |
| H | 0 | H | $CH_2F$ | $OCH_3$ | $OCH_3$ | N | |
| H | 0 | H | $CH_2F$ | Cl | $OCH_3$ | CH | |
| H | 0 | H | $CH_2CH_2F$ | $CH_3$ | $CH_3$ | CH | |
| H | 0 | H | $CH_2CH_2F$ | $CH_3$ | $OCH_3$ | CH | |
| H | 0 | H | $CH_2CH_2F$ | $OCH_3$ | $OCH_3$ | CH | |
| H | 0 | H | $CH_2CH_2F$ | $CH_3$ | $CH_3$ | N | |
| H | 0 | H | $CH_2CH_2F$ | $CH_3$ | $OCH_3$ | N | |
| H | 0 | H | $CH_2CH_2F$ | $OCH_3$ | $OCH_3$ | N | |
| H | 0 | H | $CH_2CH_2F$ | Cl | $OCH_3$ | CH | |
| H | 0 | H | $CHF_2$ | $CH_3$ | $CH_3$ | CH | |
| H | 0 | H | $CHF_2$ | $CH_3$ | $OCH_3$ | CH | |
| H | 0 | H | $CHF_2$ | $OCH_3$ | $OCH_3$ | CH | |
| H | 0 | H | $CHF_2$ | $CH_3$ | $CH_3$ | N | |
| H | 0 | H | $CHF_2$ | $CH_3$ | $OCH_3$ | N | |
| H | 0 | H | $CHF_2$ | $OCH_3$ | $OCH_3$ | N | |
| H | 0 | H | $CHF_2$ | Cl | $OCH_3$ | CH | |
| H | 0 | H | $CH_2CF_3$ | $CH_3$ | $CH_3$ | CH | |
| H | 0 | H | $CH_2CF_3$ | $CH_3$ | $OCH_3$ | CH | |
| H | 0 | H | $CH_2CF_3$ | $OCH_3$ | $OCH_3$ | CH | |
| H | 0 | H | $CH_2Cl$ | $CH_3$ | $CH_3$ | N | |
| H | 0 | H | $CH_2Cl$ | $CH_3$ | $OCH_3$ | N | |
| H | 0 | H | $CH_2Cl$ | $OCH_3$ | $OCH_3$ | N | |
| H | 0 | H | $CH_2Cl$ | Cl | $OCH_3$ | CH | |

Table XIV

| $R_1$ | n | $R_2$ | R' | X | Y | Z | m.p. (°C) |
|---|---|---|---|---|---|---|---|
| H | 0 | H | cyclopropyl | $CH_3$ | $CH_3$ | CH | |
| H | 0 | H | cyclopropyl | $CH_3$ | $OCH_3$ | CH | |
| H | 0 | H | cyclopropyl | $OCH_3$ | $OCH_3$ | CH | |
| H | 0 | H | cyclopropyl | $CH_3$ | $CH_3$ | N | |
| H | 0 | H | cyclopropyl | $CH_3$ | $OCH_3$ | N | |
| H | 0 | H | cyclopropyl | $OCH_3$ | $OCH_3$ | N | |
| H | 0 | H | cyclopropyl | Cl | $OCH_3$ | CH | |
| H | 0 | H | cyclobutyl | $CH_3$ | $CH_3$ | CH | |
| H | 0 | H | cyclobutyl | $CH_3$ | $OCH_3$ | CH | |
| H | 0 | H | cyclobutyl | $OCH_3$ | $OCH_3$ | CH | |
| H | 0 | H | cyclobutyl | $CH_3$ | $CH_3$ | N | |
| H | 0 | H | cyclobutyl | $CH_3$ | $OCH_3$ | N | |
| H | 0 | H | cyclobutyl | $OCH_3$ | $OCH_3$ | N | |
| H | 0 | H | cyclobutyl | Cl | $OCH_3$ | CH | |
| H | 0 | H | cyclopentyl | $CH_3$ | $CH_3$ | CH | |
| H | 0 | H | cyclopentyl | $CH_3$ | $OCH_3$ | CH | |
| H | 0 | H | cyclopentyl | $OCH_3$ | $OCH_3$ | CH | |
| H | 0 | H | cyclopentyl | $CH_3$ | $CH_3$ | N | |
| H | 0 | H | cyclopentyl | $CH_3$ | $OCH_3$ | N | |
| H | 0 | H | cyclopentyl | $OCH_3$ | $OCH_3$ | N | |
| H | 0 | H | cyclopentyl | Cl | $OCH_3$ | CH | |
| H | 0 | H | 2-fluorocyclopropyl | $CH_3$ | $CH_3$ | CH | |
| H | 0 | H | 2-fluorocyclopropyl | $CH_3$ | $OCH_3$ | CH | |
| H | 0 | H | 2-fluorocyclopropyl | $OCH_3$ | $OCH_3$ | CH | |
| H | 0 | H | 2-fluorocyclopropyl | $CH_3$ | $CH_3$ | N | |
| H | 0 | H | 2-fluorocyclopropyl | $CH_3$ | $OCH_3$ | N | |
| H | 0 | H | 2-fluorocyclopropyl | $OCH_3$ | $OCH_3$ | N | |
| H | 0 | H | 2-fluorocyclopropyl | Cl | $OCH_3$ | CH | |

Table XIV (cont.)

| $R_1$ | n | $R_2$ | R' | X | Y | Z | m.p.(°C) |
|---|---|---|---|---|---|---|---|
| H | 0 | $CH_3$ | $CH_3$ | $CH_3$ | $CH_3$ | CH | |
| H | 0 | $CH_3$ | $CH_3$ | $CH_3$ | $OCH_3$ | CH | |
| H | 0 | $CH_3$ | $CH_3$ | $OCH_3$ | $OCH_3$ | CH | |
| H | 0 | $CH_3$ | $CH_3$ | $CH_3$ | $CH_3$ | N | |
| H | 0 | $CH_3$ | $CH_3$ | $CH_3$ | $OCH_3$ | N | |
| H | 0 | $CH_3$ | $CH_3$ | $OCH_3$ | $OCH_3$ | N | |
| H | 0 | $CH_3$ | $CH_3$ | Cl | $OCH_3$ | CH | |
| H | 0 | $CH_3$ | $CH_2CH_3$ | $CH_3$ | $CH_3$ | CH | |
| H | 0 | $CH_3$ | $CH_2CH_3$ | $CH_3$ | $OCH_3$ | CH | |
| H | 0 | $CH_3$ | $CH_2CH_3$ | $OCH_3$ | $OCH_3$ | CH | |
| H | 0 | $CH_3$ | $CH_2CH_3$ | $CH_3$ | $CH_3$ | N | |
| H | 0 | $CH_3$ | $CH_2CH_3$ | $CH_3$ | $OCH_3$ | N | |
| H | 0 | $CH_3$ | $CH_2CH_3$ | $OCH_3$ | $OCH_3$ | N | |
| H | 0 | $CH_3$ | $CH_2CH_3$ | Cl | $OCH_3$ | CH | |
| H | 0 | $CH_3$ | $CH_2CH_2CH_3$ | $CH_3$ | $CH_3$ | CH | |
| H | 0 | $CH_3$ | $CH_2CH_2CH_3$ | $CH_3$ | $OCH_3$ | CH | |
| H | 0 | $CH_3$ | $CH_2CH_2CH_3$ | $OCH_3$ | $OCH_3$ | CH | |
| H | 0 | $CH_3$ | $CH_2CH_2CH_3$ | $CH_3$ | $CH_3$ | N | |
| H | 0 | $CH_3$ | $CH_2CH_2CH_3$ | $CH_3$ | $OCH_3$ | N | |
| H | 0 | $CH_3$ | $CH_2CH_2CH_3$ | $OCH_3$ | $OCH_3$ | N | |
| H | 0 | $CH_3$ | $CH_2CH_2CH_3$ | Cl | $OCH_3$ | CH | |
| H | 0 | $CH_3$ | $CH_2CH_2CH_2CH_3$ | $CH_3$ | $CH_3$ | CH | |
| H | 0 | $CH_3$ | $CH_2CH_2CH_2CH_3$ | $CH_3$ | $OCH_3$ | CH | |
| H | 0 | $CH_3$ | $CH_2CH_2CH_2CH_3$ | $OCH_3$ | $OCH_3$ | CH | |
| H | 0 | $CH_3$ | $CH_2CH_2CH_2CH_3$ | $CH_3$ | $CH_3$ | N | |
| H | 0 | $CH_3$ | $CH_2CH_2CH_2CH_3$ | $CH_3$ | $OCH_3$ | N | |
| H | 0 | $CH_3$ | $CH_2CH_2CH_2CH_3$ | $OCH_3$ | $OCH_3$ | N | |
| H | 0 | $CH_3$ | $CH_2CH_2CH_2CH_3$ | Cl | $OCH_3$ | CH | |

Table XIV (cont.)

| $R_1$ | n | $R_2$ | R' | X | Y | Z | m.p.(°C) |
|---|---|---|---|---|---|---|---|
| H | 0 | H | $CH_2F$ | $CH_3$ | $CH_3$ | CH | |
| H | 0 | H | $CH_2F$ | $CH_3$ | $OCH_3$ | CH | |
| H | 0 | H | $CH_2F$ | $OCH_3$ | $OCH_3$ | CH | |
| H | 0 | H | $CH_2F$ | $CH_3$ | $CH_3$ | N | |
| H | 0 | H | $CH_2F$ | $CH_3$ | $OCH_3$ | N | |
| H | 0 | H | $CH_2F$ | $OCH_3$ | $OCH_3$ | N | |
| H | 0 | H | $CH_2F$ | Cl | $OCH_3$ | CH | |
| H | 0 | H | $CH_2CH_2F$ | $CH_3$ | $CH_3$ | CH | |
| H | 0 | H | $CH_2CH_2F$ | $CH_3$ | $OCH_3$ | CH | |
| H | 0 | H | $CH_2CH_2F$ | $OCH_3$ | $OCH_3$ | CH | |
| H | 0 | H | $CH_2CH_2F$ | $CH_3$ | $CH_3$ | N | |
| H | 0 | H | $CH_2CH_2F$ | $CH_3$ | $OCH_3$ | N | |
| H | 0 | H | $CH_2CH_2F$ | $OCH_3$ | $OCH_3$ | N | |
| H | 0 | H | $CH_2CH_2F$ | Cl | $OCH_3$ | CH | |
| H | 0 | H | $CHF_2$ | $CH_3$ | $CH_3$ | CH | |
| H | 0 | H | $CHF_2$ | $CH_3$ | $OCH_3$ | CH | |
| H | 0 | H | $CHF_2$ | $OCH_3$ | $OCH_3$ | CH | |
| H | 0 | H | $CHF_2$ | $CH_3$ | $CH_3$ | N | |
| H | 0 | H | $CHF_2$ | $CH_3$ | $OCH_3$ | N | |
| H | 0 | H | $CHF_2$ | $OCH_3$ | $OCH_3$ | N | |
| H | 0 | H | $CHF_2$ | Cl | $OCH_3$ | CH | |
| H | 0 | H | $CH_2CF_3$ | $CH_3$ | $CH_3$ | CH | |
| H | 0 | H | $CH_2CF_3$ | $CH_3$ | $OCH_3$ | CH | |
| H | 0 | H | $CH_2CF_3$ | $OCH_3$ | $OCH_3$ | CH | |
| H | 0 | H | $CH_2Cl$ | $CH_3$ | $CH_3$ | N | |
| H | 0 | H | $CH_2Cl$ | $CH_3$ | $OCH_3$ | N | |
| H | 0 | H | $CH_2Cl$ | $OCH_3$ | $OCH_3$ | N | |
| H | 0 | H | $CH_2Cl$ | Cl | $OCH_3$ | CH | |

**Table XV**

| $R_1$ | n | $R_2$ | R' | X | Y | Z | m.p. (°C) |
|---|---|---|---|---|---|---|---|
| H | O | H | cyclopropyl | $CH_3$ | $CH_3$ | CH | |
| H | O | H | cyclopropyl | $CH_3$ | $OCH_3$ | CH | |
| H | O | H | cyclopropyl | $OCH_3$ | $OCH_3$ | CH | |
| H | O | H | cyclopropyl | $CH_3$ | $CH_3$ | N | |
| H | O | H | cyclopropyl | $CH_3$ | $OCH_3$ | N | |
| H | O | H | cyclopropyl | $OCH_3$ | $OCH_3$ | N | |
| H | O | H | cyclopropyl | Cl | $OCH_3$ | CH | |
| H | O | H | cyclobutyl | $CH_3$ | $CH_3$ | CH | |
| H | O | H | cyclobutyl | $CH_3$ | $OCH_3$ | CH | |
| H | O | H | cyclobutyl | $OCH_3$ | $OCH_3$ | CH | |
| H | O | H | cyclobutyl | $CH_3$ | $CH_3$ | N | |
| H | O | H | cyclobutyl | $CH_3$ | $OCH_3$ | N | |
| H | O | H | cyclobutyl | $OCH_3$ | $OCH_3$ | N | |
| H | O | H | cyclobutyl | Cl | $OCH_3$ | CH | |
| H | O | H | cyclopentyl | $CH_3$ | $CH_3$ | CH | |
| H | O | H | cyclopentyl | $CH_3$ | $OCH_3$ | CH | |
| H | O | H | cyclopentyl | $OCH_3$ | $OCH_3$ | CH | |
| H | O | H | cyclopentyl | $CH_3$ | $CH_3$ | N | |
| H | O | H | cyclopentyl | $CH_3$ | $OCH_3$ | N | |
| H | O | H | cyclopentyl | $OCH_3$ | $OCH_3$ | N | |
| H | O | H | cyclopentyl | Cl | $OCH_3$ | CH | |
| H | O | H | 2-fluorocyclopropyl | $CH_3$ | $CH_3$ | CH | |
| H | O | H | 2-fluorocyclopropyl | $CH_3$ | $OCH_3$ | CH | |
| H | O | H | 2-fluorocyclopropyl | $OCH_3$ | $OCH_3$ | CH | |
| H | O | H | 2-fluorocyclopropyl | $CH_3$ | $CH_3$ | N | |
| H | O | H | 2-fluorocyclopropyl | $CH_3$ | $OCH_3$ | N | |
| H | O | H | 2-fluorocyclopropyl | $OCH_3$ | $OCH_3$ | N | |
| H | O | H | 2-fluorocyclopropyl | Cl | $OCH_3$ | CH | |

Table XV (cont.)

| $R_1$ | n | $R_2$ | R' | X | Y | Z | m.p. (°C) |
|---|---|---|---|---|---|---|---|
| H | 1 | H | cyclopropyl | $CH_3$ | $CH_3$ | CH | |
| H | 1 | H | cyclopropyl | $CH_3$ | $OCH_3$ | CH | |
| H | 1 | H | cyclopropyl | $OCH_3$ | $OCH_3$ | CH | |
| H | 1 | H | cyclopropyl | $CH_3$ | $CH_3$ | N | |
| H | 1 | H | cyclopropyl | $CH_3$ | $OCH_3$ | N | |
| H | 1 | H | cyclopropyl | $OCH_3$ | $OCH_3$ | N | |
| H | 1 | H | cyclopropyl | Cl | $OCH_3$ | CH | |
| H | 1 | H | cyclobutyl | $CH_3$ | $CH_3$ | CH | |
| H | 1 | H | cyclobutyl | $CH_3$ | $OCH_3$ | CH | |
| H | 1 | H | cyclobutyl | $OCH_3$ | $OCH_3$ | CH | |
| H | 1 | H | cyclobutyl | $CH_3$ | $CH_3$ | N | |
| H | 1 | H | cyclobutyl | $CH_3$ | $OCH_3$ | N | |
| H | 1 | H | cyclobutyl | $OCH_3$ | $OCH_3$ | N | |
| H | 1 | H | cyclobutyl | Cl | $OCH_3$ | CH | |
| H | 1 | H | cyclopentyl | $CH_3$ | $CH_3$ | CH | |
| H | 1 | H | cyclopentyl | $CH_3$ | $OCH_3$ | CH | |
| H | 1 | H | cyclopentyl | $OCH_3$ | $OCH_3$ | CH | |
| H | 1 | H | cyclopentyl | $CH_3$ | $CH_3$ | N | |
| H | 1 | H | cyclopentyl | $CH_3$ | $OCH_3$ | N | |
| H | 1 | H | cyclopentyl | $OCH_3$ | $OCH_3$ | N | |
| H | 1 | H | cyclopentyl | Cl | $OCH_3$ | CH | |
| H | 1 | H | 2-fluorocyclopropyl | $CH_3$ | $CH_3$ | CH | |
| H | 1 | H | 2-fluorocyclopropyl | $CH_3$ | $OCH_3$ | CH | |
| H | 1 | H | 2-fluorocyclopropyl | $OCH_3$ | $OCH_3$ | CH | |
| H | 1 | H | 2-fluorocyclopropyl | $CH_3$ | $CH_3$ | N | |
| H | 1 | H | 2-fluorocyclopropyl | $CH_3$ | $OCH_3$ | N | |
| H | 1 | H | 2-fluorocyclopropyl | $OCH_3$ | $OCH_3$ | N | |
| H | 1 | H | 2-fluorocyclopropyl | Cl | $OCH_3$ | CH | |

Table XV (cont.)

| $R_1$ | n | $R_2$ | R' | X | Y | Z | m.p.(°C) |
|---|---|---|---|---|---|---|---|
| H | 0 | H | $CH_2OCH_3$ | $CH_3$ | $CH_3$ | CH | |
| H | 0 | H | $CH_2OCH_3$ | $CH_3$ | $OCH_3$ | CH | |
| H | 0 | H | $CH_2OCH_3$ | $OCH_3$ | $OCH_3$ | CH | |
| H | 0 | H | $CH_2OCH_3$ | $CH_3$ | $CH_3$ | N | |
| H | 0 | H | $CH_2OCH_3$ | $CH_3$ | $OCH_3$ | N | |
| H | 0 | H | $CH_2OCH_3$ | $OCH_3$ | $OCH_3$ | N | |
| H | 0 | H | $CH_2OCH_3$ | Cl | $OCH_3$ | CH | |
| H | 0 | H | $CH_2OCH_2CH_3$ | $CH_3$ | $CH_3$ | CH | |
| H | 0 | H | $CH_2OCH_2CH_3$ | $CH_3$ | $OCH_3$ | CH | |
| H | 0 | H | $CH_2OCH_2CH_3$ | $OCH_3$ | $OCH_3$ | CH | |
| H | 0 | H | $CH_2OCH_2CH_3$ | $CH_3$ | $CH_3$ | N | |
| H | 0 | H | $CH_2CH_2OCH_3$ | $CH_3$ | $OCH_3$ | N | |
| H | 0 | H | $CH_2CH_2OCH_3$ | $OCH_3$ | $OCH_3$ | N | |
| H | 0 | H | $CH_2CH_2OCH_3$ | Cl | $OCH_3$ | CH | |
| H | 0 | H | $CH_2CH_2OCH_3$ | $CH_3$ | $CH_3$ | CH | |
| H | 0 | H | $CH_2CH_2OCH_3$ | $CH_3$ | $OCH_3$ | CH | |
| H | 0 | H | $CH_2CH_2OCH_3$ | $OCH_3$ | $OCH_3$ | CH | |
| H | 0 | H | $CH_2CH_2OCH_3$ | $CH_3$ | $CH_3$ | N | |
| H | 0 | H | $CH(OCH_3)_2$ | $CH_3$ | $OCH_3$ | N | |
| H | 0 | H | $CH(OCH_3)_2$ | $OCH_3$ | $OCH_3$ | N | |
| H | 0 | H | $CH(OCH_3)_2$ | Cl | $OCH_3$ | CH | |
| H | 0 | H | $CH_2SCH_3$ | $CH_3$ | $CH_3$ | CH | |
| H | 0 | H | $CH_2SCH_3$ | $CH_3$ | $OCH_3$ | CH | |
| H | 0 | H | $CH_2SCH_3$ | $OCH_3$ | $OCH_3$ | CH | |
| H | 0 | H | $CH_2SCH_3$ | $CH_3$ | $CH_3$ | N | |
| H | 0 | H | $CH_2SCH_3$ | $CH_3$ | $OCH_3$ | N | |
| H | 0 | H | $CH_2SCH_3$ | $OCH_3$ | $OCH_3$ | N | |
| H | 0 | H | $CH_2SCH_3$ | Cl | $OCH_3$ | CH | |

## Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following

approximate proportions:

| | Weight Percent* | | |
|---|---|---|---|
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer-and S. A. Evans, "Weed Control Handbook", 5th Ed., Blackwell Scientific Publications, Oxford, 1968, pp. 101-103.

In the following Examples, all parts are by weight unless otherwise indicated.

Example 11

Wettable Powder

| | |
|---|---|
| 4-Acetyl-N-[(4,6-dimethoxypyrimidin-2-yl)aminocarbonyl]-1-methyl-1-H-pyrazole-5-sulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 12

Wettable Powder

| | |
|---|---|
| 4-Acetyl-N-[(4,6-dimethoxypyrimidin-2-yl)aminocarbonyl]-1-methyl-1-H-pyrazole-5-sulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 13

Granule

| | |
|---|---|
| Wettable Powder of Example 12 | 5% |
| attapulgite granules (U.S.S. 20 to 40 mesh; 0.84-0.42 mm) | 95% |

A slurry of wettable powder containing 25% solids is sprayed on the surface of attapulgite granules in a double-cone blender. The granules are dried and packaged.

Example 14

Extruded Pellet

| | |
|---|---|
| 4-Acetyl-N-[(4,6-dimethoxypyrimidin-2-yl)aminocarbonyl]-1-methyl-1-H-pyrazole-5-sulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 15

**Low Strength Granule**

| | |
|---|---|
| 4-Acetyl-N-[(4,6-dimethoxypyrimidin-2-yl)aminocarbonyl]-1-methyl-1-H-pyrazole-5-sulfonamide | 0.1% |
| attapulgite granules (U.S.S. 20 to 40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 16

**Granule**

| | |
|---|---|
| 4-Acetyl-N-[(4,6-dimethoxypyrimidin-2-yl)aminocarbonyl]-1-methyl-1-H-pyrazole-5-sulfonamide | 80% |
| wetting agent | 1% |
| crude ligninsulfonate salt (containing 5 to 20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14 to 100 mesh (1410 to 149 microns), and packaged for use.

Example 17

**Low Strength Granule**

| | |
|---|---|
| 4-Acetyl-N-[(4,6-dimethoxypyrimidin-2-yl)aminocarbonyl]-1-methyl-1-H-pyrazole-5-sulfonamide | 1% |
| N,N-dimethylformamide | 9% |
| attapulgite granules (U.S.S. 20 to 40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 18

Aqueous Suspension

| | |
|---|---|
| 4-Acetyl-N-[(4,6-dimethoxypyrimidin-2-yl)aminocarbonyl]-1-methyl-1-H-pyrazole-5-sulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 19

Solution

| | |
|---|---|
| 4-Acetyl-N-[(4,6-dimethoxypyrimidin-2-yl)aminocarbonyl]-1-methyl-1-H-pyrazole-5-sulfonamide, ammonium salt | 5% |
| water | 95% |

The salt is added directly to the water with stirring to produce the solution, which may then be packaged for use.

Example 20

High Strength Concentrate

| | |
|---|---|
| 4-Acetyl-N-[(4,6-dimethoxypyrimidin-2-yl)aminocarbonyl]-1-methyl-1-H-pyrazole-5-sulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). Thhe concentrate may be formulated further if necessary.

Example 21

Wettable Powder

| | |
|---|---|
| 4-Acetyl-N-[(4,6-dimethoxypyrimidin-2-yl)aminocarbon-yl)-methyl-1-H-pyrazole-5-sulfonamide | 90% |
| dioctyl sodium sulfosuccinate | 0.1% |
| synthetic fine silica | 9.9% |

The ingredients are blended and ground in a hammer-mill to produce particles essentially all below 100

microns. The material is sifted through a U.S.S. No. 50 screen (0.3 mm opening) and then packaged.

Example 22

**Wettable Powder**

| | |
|---|---|
| 4-Acetyl-N-[(4,6-dimethoxypyrimidin-2-yl)aminocarbonyl)-methyl-1-H-pyrazole-5-sulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 23

**Oil Suspension**

| | |
|---|---|
| 4-Acetyl-N-[(4.6-dimethoxypyrimidin-2-yl)aminocarbonyl]-1-methyl-1-H-pyrazole-5-sulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 24

**Dust**

| | |
|---|---|
| 4-Acetyl-N-[(4.6-dimethoxypyrimidin-2-yl)aminocarbonyl]-1-methyl-1-H-pyrazole-5-sulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Example 25

**Oil Suspension**

| | |
|---|---|
| 4-Acetyl-N-[(4.6-dimethoxypyrimidin-2-yl)aminocarbonyl]-1-methyl-1-H-pyrazole-5-sulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils

or emulsified in water.

Example 26

Wettable Powder

| | |
|---|---|
| 4'-Acetyl-N-[(4,6-dimethoxypyrimidin-2-yl)-[aminocarbonyl]-1-methyl-1-H-pyrazole-5-sulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 27

Wettable Powder

| | |
|---|---|
| 4-(Cyclopropylcarbonyl)-N-[(4,6-dimethoxypyrimidin 2-yl)aminocarbonyl]-1-methyl-1-H-pyrazole-5-sulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 28

Wettable Powder

| | |
|---|---|
| 4-(Cyclopropylcarbonyl)-N-[(4,6-dimethoxypyrimidin 2-yl)aminocarbonyl]-1-methyl-1-H-pyrazole-5-sulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 29

Granule

| | |
|---|---|
| Wettable Powder of Example 27 | 5% |
| attapulgite granules (U.S.S. 20 to 40 mesh; 0.84-0.42 mm) | 95% |

A slurry of wettable powder containing 25% solids is sprayed on the surface of attapulgite granules in a double-cone blender. The granules are dried and packaged.

Example 30

Low Strength Granule

| | |
|---|---|
| 4-(Cyclopropylcarbonyl)-N-[(4,6-dimethoxypyrimidin 2-yl)aminocarbonyl]-1-methyl-1-H-pyrazole-5-sulfonamide | 0.1% |
| attapulgite granules (U.S.S. 20 to 40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 31

Aqueous Suspension

| | |
|---|---|
| 4-(Cyclopropylcarbonyl)-N-[(4,6-dimethoxypyrimidin 2-yl)aminocarbonyl]-1-methyl-1-H-pyrazole-5-sulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 32

High Strength Concentrate

| | |
|---|---|
| 4-(Cyclopropylcarbonyl)-N-[(4,6-dimethoxypyrimidin 2-yl)aminocarbonyl]-1-methyl-1-H-pyrazole-5-sulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 33

Wettable Powder

| | |
|---|---|
| 4-(Cyclopropylcarbonyl)-N-[(4,6-dimethoxypyrimidin 2-yl)aminocarbonyl]-1-methyl-1-H-pyrazole-5-sulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 34

Oil Suspension

| | |
|---|---|
| 4-(Cyclopropylcarbonyl)-N-[(4,6-dimethoxypyrimidin 2-yl)aminocarbonyl]-1-methyl-1-H-pyrazole-5-sulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 35

**Dust**

| | |
|---|---|
| 4-(Cyclopropylcarbonyl)-N-[(4,6-dimethoxypyrimidin 2-yl)aminocarbonyl]-1-methyl-1-H-pyrazole-5-sulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Utility

Test results indicate that the compounds of the present invention are highly active preemergent or postemergent herbicides or plant growth regulants. Many of them have utility for broad-spectrum pre- and/or postemergence weed control in areas where complete control of all vegetation is desired, such as around industrial storage areas, parking lots, drive-in theaters, around billboards, highway and railroad structures. Some of the compounds have utility for selective weed control in crops such as wheat, barley, rice, soybeans and corn. Alternatively, the subject compounds are useful to modify plant growth.

The rates of application for the compounds of the invention are determined by a number of factors, including their use as plant growth modifiers or as herbicies, the crop species involved, the types of weeds to be controlled, weather and climate, formulations selected, mode of application, amount of foliage present, etc. In general terms, the subject compounds should be applied at levels of around 0.001 to 10 kg/ha, the lower rates being suggested for use on lighter soils and/or those having a low organic matter content, for plant growth modification or for situations where only short-term persistence is required.

The compounds of the invention may be used in combination with any other commercial herbicide, examples of which are those of the triazine, triazole, imidazolinone, uracil, urea, amide, diphenylether, carbamate and bipyridylium types as well as other sulfonylureas. They are particularly useful with the following herbicides.

| Common Name | Chemical Name |
|---|---|
| alachlor | 2-chloro-2',6'-diethyl-N-(methoxy-methyl)-acetanilide |
| atrazine | 2-chloro-4-(ethylamino)-6-(isopropyl-amino)-s-triazine |
| butylate | S-ethyl-diisobutylthiocarbamate |
| cyanazine | 2-[[4-chloro-6-(ethylamino)-s-triazin-2-yl]amino]-2-methylpropionitrile |
| dicamba | 3,6-dichloro-o-anisic acid |
| EPTC | S-ethyl dipropylthiocarbamate |
| linuron | 3-(3,4-dichlorophenyl)-1-methoxy-1-methylurea |
| metolachlor | 2-chloro-N-(2-ethyl-6-methylphenyl)-N-(2-methoxy-1-methylethyl)acetamide |
| metribuzin | 4-amino-6-tert-butyl-3-(methylthio)-as-triazine-5(4H)-one |
| tridiphane | 2-(3,5-dichlorophenyl)-2-(2,2,2-tri-chloroethyl)oxirane |
| 2,4-D | (2,4-dichlorophenoxy)acetic acid |
| thiobencarb | S-4-chlorobenzyldiethylthiocarbamate |
| molinate | S-ethyl N,N-hexamethylenethiocarbamate |
| butachlor | N-(butoxymethyl-2-chloro-2',6'-di-ethylacetanilide |
| naproanilide | N-phenyl-2-(1-naphthyloxy)propionamide |
| pyrazolate | 4-(2,4-dichlorobenzoyl)-1,3-dimethyl-pyrazol-5-yl-4-toluenesulfonate |
| pretilachlor | 2-chloro-2',6'-diethyl-N-(n-propoxy-ethyl)acetanilide |
| oxidiazon | 3-[2,4-dichloro-5-(1-methylethoxy)-phenyl]-5-(1,1-dimethylethyl)-1,3,4-oxadizol-2(3H)-one |

| Trade Name or Code Number | Chemical Name |
|---|---|
| Harmony® | 3-[[(4-methoxy-6-methyl-1,3,5-triazin-2-yl)aminocarbonyl]aminosulfonyl]-2-thiophenecarboxylic acid, methyl ester |
| Cinch® | exo-1-methyl-4-(1-methylethyl)-2-[(2-methylphenyl)methoxy]-7-oxabicyclo-[2.2.1]heptane |
| MY-93 | S-(1-methyl-1-phenethyl)piperidine-1-carbothioate |
| CH-83 | S-(2-methylpropyl)-hexanhydro-1H-azepine-1-carbothioic acid, ester |
| X-52 | 2,4-dichlorophenyl-3-methoxy-4-nitrophenyl ether |
| SC-2957 | S-benzyl-N-ethyl-N-propylthiocarbamate |
| HW-52 | N-(2,3-dichlorophenyl)-4-(ethoxymethoxy)benzamide |
| NTN-801 | 2-(benzothiazol-2-yl)-N-methyl-N-phenylacetamide |
| SL-49 | 2-[4-[(2,4-dichlorophenyl)carbonyl]-1,3-dimethyl-1H-pyrazol-5-yloxy]-1-phenylethanone |
| BAS-514 | 3,7-dichloro-8-quinoline carboxylic acid |

The herbicidal properties of the subject compounds were discovered in a number of greenhouse tests. The test procedures and results follow.

## Compounds

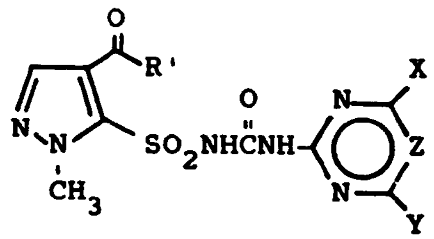

| Compound | R' | X | Y | Z |
|---|---|---|---|---|
| 1 | $CH_3$ | $OCH_3$ | $OCH_3$ | CH |
| 2 | $CH_2CH_2CH_3$ | $CH_3$ | $CH_3$ | CH |
| 3 | $CH_2CH_2CH_3$ | $OCH_3$ | $CH_3$ | CH |
| 4 | $CH_2CH_2CH_3$ | $OCH_3$ | $OCH_3$ | CH |
| 5 | $CH_2CH_2CH_3$ | $OCH_3$ | $CH_3$ | N |
| 6 | $CH_2CH_2CH_3$ | $OCH_3$ | $OCH_3$ | N |
| 7 | $CH_2CH_2CH_3$ | Cl | $OCH_3$ | CH |
| 8 | $CH_2CH_2CH_3$ | $OCH_3$ | $OCH_3$ | CH |
| 9 | $CH_2CH_3$ | $CH_3$ | $CH_3$ | CH |
| 10 | $CH_2CH_3$ | $CH_3$ | $OCH_3$ | CH |
| 11 | $CH_2CH_3$ | $CH_3$ | $OCH_3$ | N |
| 12 | $CH_2CH_3$ | $OCH_3$ | $OCH_3$ | N |
| 13 | $CH_2CH_3$ | Cl | $OCH_3$ | CH |
| 14 | $CH(CH_3)_2$ | $CH_3$ | $CH_3$ | CH |
| 15 | $CH(CH_3)_2$ | $CH_3$ | $OCH_3$ | CH |
| 16 | $CH(CH_3)_2$ | $OCH_3$ | $OCH_3$ | CH |
| 17 | $CH(CH_3)_2$ | $CH_3$ | $OCH_3$ | N |
| 18 | $CH(CH_3)_2$ | $OCH_3$ | $OCH_3$ | N |
| 19 | $CH(CH_3)_2$ | Cl | $OCH_3$ | CH |

Compounds (continued)

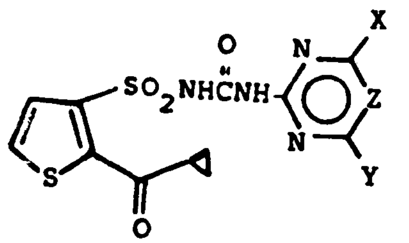

| Compound | X | Y | Z |
|---|---|---|---|
| 20 | $CH_3$ | $CH_3$ | CH |
| 21 | $CH_3$ | $OCH_3$ | CH |
| 22 | $OCH_3$ | $OCH_3$ | CH |
| 23 | $CH_3$ | $OCH_3$ | N |
| 24 | $OCH_3$ | $OCH_3$ | N |
| 25 | Cl | $OCH_3$ | CH |

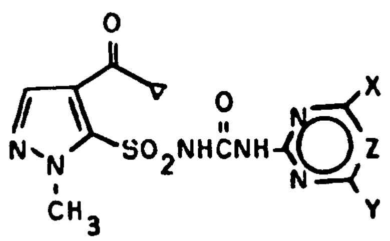

| Compound Number | X | Y | Z |
|---|---|---|---|
| 26 | $CH_3$ | $CH_3$ | CH |
| 27 | $CH_3$ | $OCH_3$ | CH |
| 28 | $OCH_3$ | $OCH_3$ | CH |
| 29 | $CH_3$ | $OCH_3$ | N |
| 30 | $OCH_3$ | $OCH_3$ | N |
| 31 | Cl | $OCH_3$ | CH |

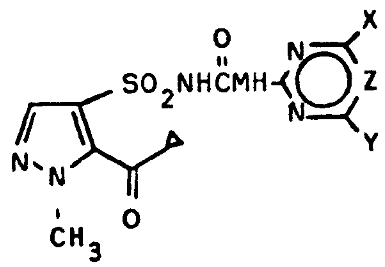

| Compound Number | X | Y | Z |
|---|---|---|---|
| 32 | $CH_3$ | $CH_3$ | CH |
| 33 | $CH_3$ | $OCH_3$ | CH |
| 34 | $OCH_3$ | $OCH_3$ | CH |
| 35 | $CH_3$ | $OCH_3$ | N |
| 36 | $OCH_3$ | $OCH_3$ | N |
| 37 | Cl | $OCH_3$ | CH |

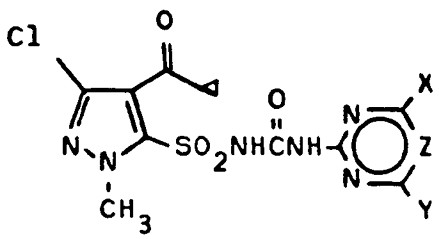

| Compound Number | X | Y | Z |
|---|---|---|---|
| 38 | $CH_3$ | $CH_3$ | CH |
| 39 | $OCH_3$ | $CH_3$ | CH |
| 40 | $OCH_3$ | $OCH_3$ | CH |

Test A

Seeds of crabgrass (Digitaria spp.), barnyardgrass (Echinochloa crusgalli), giant foxtail (Setaria faberi), wild oats (Avena fatua), cheatgrass (Bromus secalinus), velvetleaf (Abutilon theophrasti), morningglory (Ipomoea spp.), cocklebur (Xanthium pennsylvanicum), sorghum, corn soybean, sugarbeet, cotton, rice, wheat, barley and purple nutsedge (Cyperus rotundus) tubers were planted and treated preemergence with the test chemicals dissolved in a non-phytotoxic solvent. At the same time, these crop and weed species were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

C = chlorosis/necrosis
B = burn
D = defoliation
E = emergence inhibition
G = growth retardation
H = formative effect
U = unusual pigmentation
X = axillary stimulation
S = albinism
6Y = abscised buds or flowers

Table A

| | Compound 1 | | Compound 2 | |
|---|---|---|---|---|
| Rate g/ha | 0.05 | 0.01 | 0.05 | 0.01 |
| POSTEMERGENCE | | | | |
| Corn | 9H | 3C,5H | 0 | 0 |
| Wheat | 2G | 0 | 0 | 0 |
| Barley | 5G | 0 | 0 | 0 |
| Rice | 5C,9G | 8G | 4G | 0 |
| Soybean | 4C,9G | 6H | 0 | 0 |
| Cotton | 9G | 9H | 3C,8H | 3C,8H |
| Sugar beet | 9C | 9C | 3C,6G | 2C,3G |
| Crabgrass | 3C,8G | 2C,6G | 0 | 0 |
| Barnyardgrass | 9C | 5C,9H | 2C,6G | 0 |
| Nutsedge | 3C,9G | 9G | 7G | 0 |
| Giant Foxtail | 5C,9G | 3C,8G | 2G | 0 |
| Cheatgrass | 4C,9G | 8G | 6G | 0 |
| Wild Oats | 2C,5G | 0 | 0 | 0 |
| Cocklebur | 9C | 3C,9H | 4C,9G | 3C,9H |
| Morningglory | 9C | 3C,9H | 3C,8G | 2H |
| Velvetleaf | 9C | 3C,8H | 4C,9G | 3G |
| PREEMERGENCE | | | | |
| Corn | 3C,9H | 3C,7H | 0 | 0 |
| Wheat | 3G | 0 | 0 | 0 |
| Barley | 2C,5G | 2C,4G | 0 | 0 |
| Rice | 9H | 8H | 2C | 0 |
| Soybean | 3C,6H | 3C,4H | 0 | 0 |
| Cotton | 9G | 6H | 7G | 5G |
| Sugar beet | 9G | 4C,8G | 0 | 0 |
| Crabgrass | 5G | 2C,5G | 0 | 0 |
| Barnyardgrass | 9H | 9H | 2G | 0 |
| Nutsedge | 10E | 10E | 0 | 0 |
| Giant Foxtail | 7G | 3C,7H | 0 | 0 |
| Cheatgrass | 9H | 9H | 0 | 0 |
| Wild Oats | 3G | 2G | 0 | 0 |
| Cocklebur | 2C,7H | 5H | 8H | 8H |
| Morningglory | 8H | 8G | 2G | 0 |
| Velvetleaf | 7H | 0 | 5G | 0 |

Table A (continued)

| | Compound 3 | | Compound 4 | |
|---|---|---|---|---|
| Rate g/ha | 0.05 | 0.01 | 0.05 | 0.01 |
| POSTEMERGENCE | | | | |
| Corn | 3C,9H | 1C,4H | 3C,9H | 3C,9H |
| Wheat | 0 | 0 | 0 | 0 |
| Barley | 2C | 0 | 0 | 0 |
| Rice | 7G | 3G | 7G | 2G |
| Soybean | 2C,4H | 1H | 3C,8G,7X | 3H |
| Cotton | 10C | 4C,8H | 4C,9G | 4C,9G |
| Sugar beet | 4C,9H | 3C,6G | 4C,8G | 4C,8G |
| Crabgrass | 2G | 0 | 4G | 0 |
| Barnyardgrass | 4C,9H | 2C,5G | 5C,9H | 3C,5H |
| Nutsedge | 4C,9G | 2C,5G | 4C,9G | 4C,8G |
| Giant Foxtail | 3C,7G | 2G | 4C,8G | 4G |
| Cheatgrass | 8G | 5G | 8G | 2G |
| Wild Oats | 1C | 0 | 0 | 0 |
| Cocklebur | 9C | 4C,9G | 10C | 6C,9G |
| Morningglory | 9C | 3C,8G | 10C | 9C |
| Velvetleaf | 9C | 4C,9G | 10C | 4C,9G |
| PREEMERGENCE | | | | |
| Corn | 3C,8G | 2C,7G | 3C,8G | 2C,6G |
| Wheat | 0 | 0 | 0 | 0 |
| Barley | 2G | 0 | 0 | 0 |
| Rice | 2C,2G | 0 | 3G | 0 |
| Soybean | 1H | 0 | 3G | 0 |
| Cotton | 9G | 8G | 9G | 8G |
| Sugar beet | 8G | 5G | 9G | 6G |
| Crabgrass | 0 | 0 | 0 | 0 |
| Barnyardgrass | 3C,7G | 2G | 3C,8G | 2C,2G |
| Nutsedge | 7G | 8G | 9G | 7G |
| Giant Foxtail | 2C,4G | 0 | 3C,7G | 0 |
| Cheatgrass | 4G | 0 | 6G | 0 |
| Wild Oats | 0 | 0 | 0 | 0 |
| Cocklebur | 9H | 9H | 9H | 9H |
| Morningglory | 9G | 8G | 8G | 8G |
| Velvetleaf | 9C | 9G | 9C | 9G |

Table A (continued)

| | Compound 5 | | Compound 6 | |
|---|---|---|---|---|
| Rate g/ha | 0.05 | 0.01 | 0.05 | 0.01 |
| POSTEMERGENCE | | | | |
| Corn | 2C,6H | 0 | 6H | 0 |
| Wheat | 0 | 0 | 0 | 0 |
| Barley | 0 | 0 | 0 | 0 |
| Rice | 5G | 3G | 4C,9G | 3G |
| Soybean | 0 | 0 | 0 | 0 |
| Cotton | 8G | 0 | 4C,9G | 3G |
| Sugar beet | 1C | 2G | 3C,5G | 2H |
| Crabgrass | 0 | 0 | 0 | 0 |
| Barnyardgrass | 2C,5H | 2H | 9H | 0 |
| Nutsedge | 0 | 0 | 4G | 0 |
| Giant Foxtail | 2G | 0 | 2C,5G | 0 |
| Cheatgrass | 5G | 0 | 5G | 0 |
| Wild Oats | 0 | 0 | 0 | 0 |
| Cocklebur | 5C,9G | 4C,9H | 4C,9G | 4C,9H |
| Morningglory | 4C,9G | 3C,7G | 5C,9G | 3C,8H |
| Velvetleaf | 3C,8H | 2C,5G | 3C,8H | 6G |
| PREEMERGENCE | | | | |
| Corn | 3C,6G | 2C,4G | 2C,5G | 2C,5G |
| Wheat | 0 | 0 | 0 | 0 |
| Barley | 0 | 0 | 5G | 0 |
| Rice | 4G | 3G | 2C,6G | 2G |
| Soybean | 0 | 0 | 0 | 0 |
| Cotton | 2C,2G | 0 | 8G | 2C,2G |
| Sugar beet | 4H | 5G | 9G | 5G |
| Crabgrass | 0 | 0 | 0 | 0 |
| Barnyardgrass | 3G | 0 | 6G | 2C,2G |
| Nutsedge | 0 | 0 | 0 | 0 |
| Giant Foxtail | 4G | 0 | 0 | 0 |
| Cheatgrass | 0 | 0 | 5G | 0 |
| Wild Oats | 0 | 0 | 0 | 0 |
| Cocklebur | 7G | 7H | 9H | 3H |
| Morningglory | 6G | 5G | 7G | 5H |
| Velvetleaf | 9C | 8G | 8G | 3G |

Table A (continued)

| | Compound 7 | | Compound 8 | |
|---|---|---|---|---|
| Rate g/ha | 0.05 | 0.01 | 0.05 | 0.01 |
| POSTEMERGENCE | | | | |
| Corn | 0 | 0 | 9C | 4C,9G |
| Wheat | 0 | 0 | 0 | 0 |
| Barley | 0 | 0 | 0 | 0 |
| Rice | 2G | 0 | 5G | 0 |
| Soybean | 1C | 0 | 3C,8H | 3C,5H |
| Cotton | 7G | 0 | 10C | 10C |
| Sugar beet | 3C,6G | 0 | 10C | 10C |
| Crabgrass | 0 | 0 | 3C,7G | 2G |
| Barnyardgrass | 0 | 0 | 9C | 5C,9H |
| Nutsedge | 0 | 0 | 9C | 9C |
| Giant Foxtail | 0 | 0 | 10C | 9C |
| Cheatgrass | 0 | 0 | 9C | 7G |
| Wild Oats | 0 | 0 | 0 | 0 |
| Cocklebur | 3C,9G | 2C,8H | 10C | 10C |
| Morningglory | 3C,8G | 3G | 10C | 10C |
| Velvetleaf | 7G | 0 | 9C | 9C |
| Sorghum | - | - | 3C,8H | 3G |
| PREEMERGENCE | | | | |
| Corn | 2C,3G | 0 | 2C,9G | 2C,9G |
| Wheat | 0 | 0 | 2G | 0 |
| Barley | 0 | 0 | 3G | 0 |
| Rice | 2C,3G | 0 | 8H | 2G |
| Soybean | 0 | 0 | 6H | 6G |
| Cotton | 5G | 0 | 9G | 9G |
| Sugar beet | 8G | 0 | 9G | 8H |
| Crabgrass | 0 | 0 | 2G | 0 |
| Barnyardgrass | 3C,5G | 0 | 9H | 4G |
| Nutsedge | 0 | 0 | 10E | 10E |
| Giant Foxtail | 0 | 0 | 9H | 5G |
| Cheatgrass | 0 | 0 | 9G | 8G |
| Wild Oats | 0 | 0 | 1C | 0 |
| Cocklebur | 9H | - | 8H | 3C,6H |
| Morningglory | 8H | 0 | 9H | 9G |
| Velvetleaf | 7G | 0 | 8H | 6H |
| Sorghum | - | - | 9H | 7G |

Table A (continued)

| | CMPD 9 | | CMPD 10 | | CMPD 11 | | CMPD 12 | |
|---|---|---|---|---|---|---|---|---|
| RATE RATE=KG/HA | 0.01 | 0.05 | 0.01 | 0.05 | 0.01 | 0.05 | 0.01 | 0.05 |
| POSTEMERGENCE | | | | | | | | |
| COTTON | 10C | 9C | 10C | 10C | 7G | 10C | 6G | 10C |
| MORNING GLORY | 3C,8G | 9C | 5C,9G | 9C | 4C,8H | 10C | 3C,8H | 10C |
| COCKLEBUR | 10C | 10C | 10C | 10C | 5C,9G | 10C | 3C,7H | 9C |
| NUTSEDGE | 4C,9G | 9C | 5C,9G | 9C | 2G | 3C,8G | 0 | 0 |
| CRABGRASS | 0 | 3G | 3G | 9C | 0 | 4G | 0 | 0 |
| BARNYARD GRASS | 3C,9H | 9C | 4C,9H | 9C | 6H | 4C,9H | 3H | 4C,9H |
| WILD OATS | 0 | 3C,6G | 0 | 2C,6G | 0 | 0 | 0 | 1C |
| WHEAT | 0 | 7G | 0 | 7G | 0 | 0 | 0 | 0 |
| CORN | 4G | 2C,9H | 9H | 9C | 9H | 9G | 3H | 3C,9G |
| SOYBEAN | 3H | 6H | 2C,3H | 3C,7G | 0 | 2H | 0 | 7H |
| RICE | 8G | 9C | 4G | 2C,7G | 2G | 3C,9G | 5G | 6C,9G |
| SORGHUM | 3C,9G | 9C | 4C,9H | 9C | 2C,9H | 2C,9G | 3C,8H | 4C,9G |
| CHEATGRASS | 2C,8G | 9C | 7G | 9C | 5G | 5C,9G | 0 | 4C,9G |
| SUGAR BEETS | 9C | 10C | 5C,9G | 10C | 5G | 4C,8G | 2H | 6G |
| VELVETLEAF | 4C,9H | 10C | 9C | 10C | 2G | 3C,8H | 0 | 3C,7G |
| GIANT FOXTAIL | 2C,5G | 5C,9G | 3C,8G | 9C | 0 | 3C,7H | 0 | 4G |
| BARLEY | 6G | 3C,8G | 0 | 3C,7G | 0 | 0 | 0 | 0 |
| PREEMERGENCE | | | | | | | | |
| COTTON | 8H | 9G | 8G | 9G | 0 | 8H | 0 | 5G |
| MORNING GLORY | 7H | 9G | 9G | 9G | 0 | 9G | 0 | 7H |
| COCKLEBUR | 9H | 9H | 9H | | 1C | 2C,7H | 1H | 3C,3H |
| NUTSEDGE | 9G | 9G | 8G | 10E | 0 | 10E | 0 | 0 |
| CRABGRASS | 0 | 0 | 0 | 4G | 0 | 0 | 0 | 0 |
| BARNYARD GRASS | 7G | 9H | 6H | 9H | 0 | 7H | 0 | 3H |
| WILD OATS | 0 | 2G | 2C | 2C,4G | 0 | 0 | 0 | 2G |
| WHEAT | 0 | 4G | 0 | 3G | 0 | 0 | 0 | 2G |
| CORN | 0 | 2C,9G | 3C,9H | 9H | 2C,9H | 2C,9G | 4H | 2C,9H |
| SOYBEAN | 3G | 5G | 3G | 2C,7H | 0 | 3C,4G | 0 | 1C,1H |
| RICE | 8H | 9H | 7G | 3C,8H | 7H | 9H | 8G | 9H |
| SORGHUM | 9H | 10H | 9H | 10H | 9G | 9H | 2C,7H | 5C,9G |
| CHEATGRASS | 5G | 9G | 8G | 9G | 0 | 9H | 0 | 3G |
| SUGAR BEETS | 9G | 9G | 4C,9G | 3C,9G | 7H | 3C,8G | 4H | 5G |
| VELVETLEAF | 5H | 9H | 8H | 9H | 0 | 2H | 0 | 2C,2G |
| GIANT FOXTAIL | 0 | 2G | 2C,4G | 8G | 0 | 3G | 0 | 0 |
| BARLEY | 7G | 2C,8G | 0 | 7C | 0 | 6G | 2G | 5G |

Table A (continued)

| RATE RATE=KG/HA | CMPD 13 0.01 | CMPD 13 0.05 | CMPD 14 0.01 | CMPD 14 0.05 | CMPD 15 0.01 | CMPD 15 0.05 | CMPD 16 0.01 | CMPD 16 0.05 |
|---|---|---|---|---|---|---|---|---|
| POSTEMERGENCE | | | | | | | | |
| COTTON | 10C | 10C | 3C,8G | | 9H | | 5C,9G | |
| MORNING GLORY | 4C,9G | 10C | 3C,6G | | 3C,8G | | 9C | |
| COCKLEBUR | 9C | 10C | 3C,7G | | 4C,9G | | 10C | |
| NUTSEDGE | 4C,9G | 10C | 0 | | 3C,8G | | 5C,9G | |
| CRABGRASS | 0 | 4G | 0 | | 0 | | 2G | |
| BARNYARD GRASS | 7H | 5C,9H | 1C | | 0 | | 3C,5H | |
| WILD OATS | 0 | 0 | 0 | | 0 | | 0 | |
| WHEAT | 0 | 0 | 0 | | 0 | | 0 | |
| CORN | 7H | 3C,9G | 0 | | 7H | | 9G | |
| SOYBEAN | 0 | 3G | 2H | | 3C,5H | | 3H | |
| RICE | 2G | 7G | 3G | | 2G | | 1C | |
| SORGHUM | 5G | 3C,9H | 3C,5G | | 3C,8H | | 3C,8G | |
| CHEATGRASS | 5G | 5C,9G | 0 | | 2G | | 2C,6G | |
| SUGAR BEETS | 3C,7G | 9C | 3C,8G | | 5C,9G | | 5C,9G | |
| VELVETLEAF | 5G | 3C,7G | 3C,7G | | 4C,9G | | 9C | |
| GIANT FOXTAIL | 5G | 5C,9H | 1C | | 2G | | 4G | |
| BARLEY | 0 | 3G | 0 | | 0 | | 0 | |
| PREEMERGENCE | | | | | | | | |
| COTTON | 3G | 8G | 0 | | 2G | | 7G | |
| MORNING GLORY | 7G | 9G | 0 | | 3C,4H | | 9G | |
| COCKLEBUR | 3H | | 0 | | 3C,5G | | 3C,7H | |
| NUTSEDGE | 7G | 9G | 0 | | 10E | | 9G | |
| CRABGRASS | 0 | 3G | 0 | | 0 | | 8G | |
| BARNYARD GRASS | 2G | 9H | 0 | | 0 | | 2G | |
| WILD OATS | 0 | 0 | 0 | | 0 | | 0 | |
| WHEAT | 0 | 0 | 0 | | 2C | | 0 | |
| CORN | 2C,5G | 9G | 0 | | 1H | | 3C,4G | |
| SOYBEAN | 0 | 2G | 0 | | 0 | | 3G | |
| RICE | 0 | 8G | 0 | | 2C | | 2G | |
| SORGHUM | 3C,8H | 3C,9H | 0 | | 0 | | 5G | |
| CHEATGRASS | 5G | 9G | 0 | | 3G | | 4G | |
| SUGAR BEETS | 8G | 9G | 3G | | 2C,2H | | 8G | |
| VELVETLEAF | 3H | 7H | 0 | | 0 | | 6H | |
| GIANT FOXTAIL | 0 | 7G | 0 | | 0 | | 5G | |
| BARLEY | 0 | 2C,3G | 0 | | 0 | | 0 | |

Table A (continued)

| | CMPD 17 | | CMPD 18 | | CMPD 19 | | CMPD 20 | |
|---|---|---|---|---|---|---|---|---|
| RATE RATE=KG/HA | 0.01 | 0.05 | 0.01 | 0.05 | 0.01 | 0.05 | 0.01 | 0.05 |
| POSTEMERGENCE | | | | | | | | |
| COTTON | 3C,6G | | 3C,6H | | 2G | | 4C,9G | 5C,9G |
| MORNING GLORY | 3C,7H | | 3C,7G | | 2C,4H | | 3H | 10C |
| COCKLEBUR | 5C,9G | | 4C,8H | | 3C,7G | | 5C,9G | 9C |
| NUTSEDGE | 3C,5G | | 0 | | 0 | | 3C,8G | 4C,9G |
| CRABGRASS | 0 | | 0 | | 0 | | 0 | 0 |
| BARNYARD GRASS | 0 | | 0 | | 0 | | 3C,7H | 4C,9H |
| WILD OATS | 0 | | 0 | | 0 | | 3G | 3C,8G |
| WHEAT | 0 | | 0 | | 0 | | 3G | 2C,7G |
| CORN | 8H | | 3C,8H | | 0 | | 2G | 7H |
| SOYBEAN | 1H | | 1H | | 0 | | 5H | 3C,8G |
| RICE | 3G | | 7G | | 0 | | 8G | 5C,9G |
| SORGHUM | 3C,8G | | 3C,8G | | 3C,5G | | 4C,9H | 9C |
| CHEATGRASS | 3G | | 2G | | 0 | | 3C,7G | 3C,9G |
| SUGAR BEETS | 4C,8G | | 4C,9H | | 2C,3G | | 4C,8G | 4C,8G |
| VELVETLEAF | 2C,5G | | 3C,6G | | 1C | | 10C | 9C |
| GIANT FOXTAIL | 2C | | 1C | | 0 | | 2G | 3C,3G |
| BARLEY | 0 | | 0 | | 0 | | 3G | 9G |
| PREEMERGENCE | | | | | | | | |
| COTTON | 0 | | 0 | | 0 | | 2G | 8G |
| MORNING GLORY | 2C,5G | | 2C | | 2C | | 0 | 6G |
| COCKLEBUR | 2C | | 2C | | 1C | | 3G | 9H |
| NUTSEDGE | 0 | | 0 | | 0 | | 0 | 0 |
| CRABGRASS | 0 | | 4G | | 4G | | 0 | 0 |
| BARNYARD GRASS | 0 | | 0 | | 0 | | 7G | 8H |
| WILD OATS | 0 | | 0 | | 0 | | 2G | 7H |
| WHEAT | 0 | | 0 | | 0 | | 0 | 7G |
| CORN | 3C,6G | | 2C,5G | | 0 | | 0 | 7H |
| SOYBEAN | 0 | | 0 | | 0 | | 2G | 3C,3H |
| RICE | 0 | | 3G | | 0 | | 8G | 9H |
| SORGHUM | 3C,5G | | 2C,3G | | 2C,4G | | 9H | 10H |
| CHEATGRASS | 0 | | 0 | | 0 | | 5G | 8G |
| SUGAR BEETS | 7G | | 7H | | 4G | | 5H | 7G |
| VELVETLEAF | 2C | | 2G | | 0 | | 5G | 8H |
| GIANT FOXTAIL | 0 | | 2G | | 0 | | 0 | 0 |
| BARLEY | 0 | | 0 | | 0 | | 0 | 7G |

## Table A (continued)

| | CMPD 21 | | CMPD 22 | | CMPD 23 | | CMPD 24 | |
|---|---|---|---|---|---|---|---|---|
| RATE RATE=KG/HA | 0.01 | 0.05 | 0.01 | 0.05 | 0.01 | 0.05 | 0.01 | 0.05 |
| POSTEMERGENCE | | | | | | | | |
| COTTON | 5C,9G | 9C | 3C,9G | 4C,9G | 9C | 10C | 4C,9G | 4C,9G |
| MORNING GLORY | 10C | 10C | 4C,9G | 10C | 10C | 10C | 10C | 10C |
| COCKLEBUR | 9C | 10C | 10C | 10C | 10C | 10C | 10C | 10C |
| NUTSEDGE | 4C,8G | 9C | 9C | 10C | 0 | 0 | 0 | 2C,5G |
| CRABGRASS | 0 | 4G | 0 | 3G | 0 | 0 | 0 | 0 |
| BARNYARD GRASS | 3C,9H | 9C | 3C,5G | 3C,8H | 0 | 3C,7H | 0 | 5H |
| WILD OATS | 5G | 7G | 0 | 3G | 0 | 2G | 0 | 2G |
| WHEAT | 3G | 7G | 3G | 4G | 0 | 3G | 0 | 3G |
| CORN | 8H | 3C,9H | 2G | 3C,9H | 2C,3G | 9H | 0 | 4G |
| SOYBEAN | 5C,9G | 4C,9G | 3C,9G | 4C,9G | 9C | 5C,9G | 4C,9G | 4C,9G |
| RICE | 7G | 3C,9G | 4G | 2C,8G | 2G | 4C,8G | 1C | 4C,8G |
| SORGHUM | 9H | 10C | 7H | 3C,9H | 3G | 3C,8H | 2C,3G | 2C,6H |
| CHEATGRASS | 5C,9G | 5C,9G | 3C,5G | 9C | 0 | 2G | 0 | 2C,4G |
| SUGAR BEETS | 10C | 10C | 9C | 10C | 9C | 9C | 10C | 9C |
| VELVETLEAF | 10C | 10C | 4C,9G | 10C | 10C | 10C | 2C,4H | 5G |
| GIANT FOXTAIL | 3G | 3C,8G | 0 | 2C,4H | 0 | 3G | 0 | 3C,5G |
| BARLEY | 5G | 8G | 2G | 4G | 0 | 3G | 0 | 0 |
| PREEMERGENCE | | | | | | | | |
| COTTON | 7G | 8H | 0 | 5G | 8H | 9G | 5G | 3C,8G |
| MORNING GLORY | 5G | 9G | 2G | 7G | 9G | 9G | 9G | 4C,9G |
| COCKLEBUR | 4H | 3C,6H | 2G | 7G | 3C,7H | 9H | 3C,8H | 9H |
| NUTSEDGE | | 6G | 0 | 10E | 0 | 0 | 0 | 0 |
| CRABGRASS | 0 | 4G | 0 | 2G | 0 | 0 | 0 | 0 |
| BARNYARD GRASS | 7H | 9H | 0 | 7H | 1C | 2H | 0 | 0 |
| WILD OATS | 2G | 3C,8G | 0 | 0 | 0 | 0 | 0 | 0 |
| WHEAT | 2G | 7G | 0 | 0 | 0 | 0 | 0 | 0 |
| CORN | 2C,8H | 9G | 0 | 3C,7G | 3C,4G | 3C,7G | 1C | 2C,5G |
| SOYBEAN | 3C,5G | 3C,9H | 1C,1H | 3C,5H | 3C,7H | 9H | 3C,7H | 3C,9H |
| RICE | 8G | 3C,9H | 7G | 9G | 0 | 8G | 3G | 7G |
| SORGHUM | 3C,9H | 3C,9H | 8G | 3C,9H | 2C,3G | 8G | 2C,3G | 3C,6G |
| CHEATGRASS | 5G | 9G | 0 | 8G | 0 | 2G | 0 | 0 |
| SUGAR BEETS | 3C,8G | 5C,9G | 2G | 3C,7G | 9C | 9G | 4C,9G | 9C |
| VELVETLEAF | 6H | 9G | 0 | 4H | 7H | 4C,9G | 0 | 2G |
| GIANT FOXTAIL | 2G | 4H | 0 | 4G | 0 | 0 | 0 | 0 |
| BARLEY | 2G | 2C,8G | 0 | 1C | 0 | 2C,3G | 0 | 1 |

Table A (continued)

| | CMPD 25 | |
|---|---|---|
| RATE RATE=KG/HA | 0.01 | 0.05 |
| POSTEMERGENCE | | |
| COTTON | 9C | 9C |
| MORNING GLORY | 10C | 10C |
| COCKLEBUR | 9C | 10C |
| NUTSEDGE | 9C | 9C |
| CRABGRASS | 0 | 0 |
| BARNYARD GRASS | 0 | 2H |
| WILD OATS | 0 | 0 |
| WHEAT | 0 | 2G |
| CORN | 0 | 0 |
| SOYBEAN | 0 | 8G |
| RICE | 4H | 3G |
| SORGHUM | 0 | 3C,8G |
| CHEATGRASS | 0 | 3G |
| SUGAR BEETS | 4G | 10C |
| VELVETLEAF | 9C | 10C |
| GIANT FOXTAIL | 0 | 0 |
| BARLEY | 0 | 0 |
| PREEMERGENCE | | |
| COTTON | 0 | 2G |
| MORNING GLORY | 5G | 9H |
| COCKLEBUR | | 1C |
| NUTSEDGE | 5G | 9G |
| CRABGRASS | 0 | 0 |
| BARNYARD GRASS | 0 | 4H |
| WILD OATS | 0 | 0 |
| WHEAT | 0 | 0 |
| CORN | 0 | 2G |
| SOYBEAN | 0 | 1C |
| RICE | 0 | 7G |
| SORGHUM | 3G | 2C,7G |
| CHEATGRASS | 0 | 3G |
| SUGAR BEETS | 5G | 8G |
| VELVETLEAF | 3G | 6H |
| GIANT FOXTAIL | 0 | 0 |
| BARLEY | 0 | 0 |

Table A

| | Compound 26 | | Compound 27 | |
|---|---|---|---|---|
| Rate kg/ha | 0.05 | 0.01 | 0.05 | 0.01 |
| POSTEMERGENCE | | | | |
| Morningglory | 9C | 3C,8G | 10C | 10C |
| Cocklebur | 9C | 5C,9G | 10C | 10C |
| Velvetleaf | 10C | 9C | 10C | 10C |
| Nutsedge | 9G | 3C,8G | 5C,9G | 9G |
| Crabgrass | 3G | 0 | 2G | 0 |
| Barnyardgrass | 3H | 0 | 5C,9H | 3C,5H |
| Cheatgrass | 3C,7G | 0 | 3C,9G | 8G |
| Wild Oats | 0 | 0 | 2C,2G | 0 |
| Sicklepod | - | - | - | - |
| Wheat | 0 | 0 | 0 | 0 |
| Corn | 0 | 0 | 9H | 2C,6G |
| Soybean | 3H | 0 | 3C,6H | 3H |
| Rice | 3G | 0 | 3G | 0 |
| Sorghum | 9G | 3C,9H | 4C,9G | 4C,9G |
| Sugar beet | 9C | 10C | 9C | 10C |
| Cotton | 10C | 3C,9H | 10C | 10C |
| PREEMERGENCE | | | | |
| Morningglory | 8H | 0 | 3C,7H | 5G |
| Cocklebur | 7H | 2C,3H | 9H | 7H |
| Velvetleaf | 7H | 0 | 5C,9G | 5H |
| Nutsedge | 10E | 3C,8G | 10E | 10E |
| Crabgrass | 0 | 0 | 3C | 0 |
| Barnyardgrass | 2G | 0 | 3C,7G | 3G |
| Cheatgrass | 7G | 0 | 9G | 8G |
| Wild Oats | 0 | 0 | 1C | 0 |
| Sicklepod | - | - | - | - |
| Wheat | 0 | 2G | 0 | 0 |
| Corn | 3G | 0 | 2C,9G | 3C,7G |
| Soybean | 1H | 0 | 3C,6H | 2C,3H |
| Rice | 6G | 5G | 5G | 2G |
| Sorghum | 3C,9H | 3C,8G | 9G | 8H |
| Sugar beet | 3C,9G | 3C,7H | 4C,9G | 4C,9G |
| Cotton | 8G | 0 | 9G | 7H |

Table A (cont.)

| | Compound 28 | | Compound 29 | |
|---|---|---|---|---|
| Rate kg/ha | 0.05 | 0.01 | 0.05 | 0.01 |
| POSTEMERGENCE | | | | |
| Morningglory | 10C | 10C | 9C | 3C,8G |
| Cocklebur | 10C | 10C | 9C | 4C,9G |
| Velvetleaf | 10C | 10C | 9C | 3C,7H |
| Nutsedge | 9G | 9G | 9G | 2C,5G |
| Crabgrass | 2C,5G | 2G | 2C,5G | 0 |
| Barnyardgrass | 5C,9H | 6C,9H | 3C,8H | 0 |
| Cheatgrass | 2C,8G | 7G | 9G | 0 |
| Wild Oats | 0 | 0 | 0 | 0 |
| Sicklepod | - | - | - | - |
| Wheat | 2G | 0 | 3G | 0 |
| Corn | 9H | 9H | 3C,9H | 3C,7H |
| Soybean | 3C,5H | 5H | 3C,6H | 2H |
| Rice | 3G | 0 | 3G | 0 |
| Sorghum | 9H | 5H | 2C,9H | 2C,8G |
| Sugar beet | 9C | 9C | 5C,9H | 4C,8H |
| Cotton | 10C | 9C | 4C,9G | 8H |
| PREEMERGENCE | | | | |
| Morningglory | 9G | 7G | 9G | 7H |
| Cocklebur | 9H | - | 7H | - |
| Velvetleaf | 9G | 2H | 2C,5H | 2H |
| Nutsedge | 10E | 10E | 8G | 5G |
| Crabgrass | 3G | 0 | 2C,3G | 0 |
| Barnyardgrass | 9H | 7H | 4G | 2H |
| Cheatgrass | 9H | 7G | 7G | 0 |
| Wild Oats | 0 | 0 | 0 | 0 |
| Sicklepod | - | - | - | - |
| Wheat | 0 | 0 | 0 | 0 |
| Corn | 9G | 5G | 9H | 3C,9H |
| Soybean | 2C,5H | 0 | 3C,6H | 3C,5H |
| Rice | 2G | 0 | 6G | 1C |
| Sorghum | 2C,9H | 2G | 9H | 3C,9H |
| Sugar beet | 9G | 7G | 5C,9G | 8H |
| Cotton | 9G | 7G | 2C,7H | 2C,2G |

Table A (cont.)

| | Compound 30 | | Compound 31 | |
|---|---|---|---|---|
| Rate kg/ha | 0.05 | 0.01 | 0.05 | 0.01 |
| POSTEMERGENCE | | | | |
| Morningglory | 5C,9G | 3C,8H | 10C | 2C,4G |
| Cocklebur | 9H | 3C,9H | 9C | 2C,8H |
| Velvetleaf | 2G | 0 | 9C | 3C,7H |
| Nutsedge | 9G | 0 | 9G | 9G |
| Crabgrass | 4G | 0 | 0 | 0 |
| Barnyardgrass | 9H | 2H | 2C,5H | 0 |
| Cheatgrass | 3C,9G | 0 | 2C,5G | 0 |
| Wild Oats | 0 | 0 | 0 | 0 |
| Sicklepod | - | - | - | - |
| Wheat | 0 | 0 | 0 | 0 |
| Corn | 3C,9H | 2H | 2G | 0 |
| Soybean | 3C,6H | 3C,3H | 2C | 0 |
| Rice | 5C,9G | 0 | 0 | 0 |
| Sorghum | 4C,9G | 3C,7H | 3C,8H | 2G |
| Sugar beet | 4C,9H | 4C,8G | 5C,9G | 8G |
| Cotton | 10C | 5C,9G | 9C | 7G |
| PREEMERGENCE | | | | |
| Morningglory | 3C,8G | 2C,5H | 2C,5H | 0 |
| Cocklebur | 7H | 2C | 2C,5H | 0 |
| Velvetleaf | 0 | 0 | 3C,7G | 0 |
| Nutsedge | 9G | 9G | 10E | 0 |
| Crabgrass | 0 | 0 | 0 | 0 |
| Barnyardgrass | 3H | 0 | 5G | 0 |
| Cheatgrass | 5G | 0 | 3G | 0 |
| Wild Oats | 0 | 0 | 0 | 0 |
| Sicklepod | - | - | - | - |
| Wheat | 0 | 0 | 0 | 0 |
| Corn | 9H | 2C,8H | 2C,6G | 2G |
| Soybean | 3C,6H | 3C,3H | 0 | 0 |
| Rice | 3C,9H | 5G | 2G | 0 |
| Sorghum | 3C,9H | 2C,8G | 2C,7H | 0 |
| Sugar beet | 9G | 8H | 9G | 5G |
| Cotton | 3C,7H | 0 | 3G | 0 |

## Table A (cont.)

| | CMPD 32 | | CMPD 33 | | CMPD 34 | | CMPD 35 | |
|---|---|---|---|---|---|---|---|---|
| RATE RATE=KG/HA | 0.01 | 0.05 | 0.01 | 0.05 | 0.01 | 0.05 | 0.01 | 0.05 |
| POSTEMERGENCE | | | | | | | | |
| COTTON | 4C,9G | 10C | 5C,9G | 10C | 4C,9G | 10C | 2C,9H | 10C |
| MORNING GLORY | 5C,9G | 10C | 10C | 10C | 2C,6G | 10C | 3C,7H | 10C |
| COCKLEBUR | 3C,6G | 10C | 10C | 10C | 10C | 10C | 10C | 10C |
| NUTSEDGE | 3C,7G | 9C | 9C | 9C | 8G | 9C | 7G | 9C |
| CRABGRASS | 2G | 3C,7G | 7G | 4C,9G | 4G | 3C,9G | 3C,8G | 9C |
| BARNYARD GRASS | 4C,9H | 9C | 4C,9H | 9C | 9H | 9C | 9C | 9C |
| WILD OATS | 3C,5G | 3C,9G | 2C,3G | 4C,9G | 2C,5G | 2C,8G | 4C,8G | 4C,9G |
| WHEAT | 7G | 9G | 5G | 9G | 2G | 6G | 2C,8G | 5C,9G |
| CORN | 3C,7H | 3C,9G | 4C,9H | 6C,9G | 9G | 5C,9G | 5C,9G | 9C |
| SOYBEAN | 4C,9G | 5C,9G | 4C,9G | 9C | 9C | 5C,9G | 4C,9G | 9C |
| RICE | 9C | 9C | 4C,8G | 9C | 4C,9G | 5C,9G | 9C | 9C |
| SORGHUM | 3C,9G | 9C | 5C,9G | 9C | 9G | 5C,9G | 9C | 10C |
| CHEATGRASS | 9C | 10C | 10C | 9C | 9C | 10C | 9C | 5C,9G |
| SUGAR BEETS | 9C | 9C | 10C | 9C | 10C | 9C | 9C | 10C |
| VELVETLEAF | 4C,9H | 9C | 10C | 10C | 9C | 10C | 3C,7H | 6C,9H |
| GIANT FOXTAIL | 3C,6G | 4C,9G | 4C,8G | 5C,9G | 3C,7G | 5C,9G | 6C,9G | 9C |
| BARLEY | 3C,6G | 2C,8G | 3C,7G | 6C,9G | 2C,4G | 3C,7G | 2C,6G | 4C,9G |
| PREEMERGENCE | | | | | | | | |
| COTTON | 3C,6G | 9G | 3C,7H | 9G | 2G | 6G | 5G | 9G |
| MORNING GLORY | 7H | 9G | 3C,8H | 8H | 7G | 8G | 2C,5H | 9G |
| COCKLEBUR | | 9H | 3C,5G | 3C,7H | 1H | 8H | | |
| NUTSEDGE | 3C,8G | 10E | 10E | 10E | 9G | 10E | 4C,9G | 10E |
| CRABGRASS | 3G | 3G | 6G | 4C,9G | 3G | 6G | 4C,9G | 9H |
| BARNYARD GRASS | 3C,6G | 4C,9H | 3C,8G | 9H | 3C,7H | 9H | 4C,9H | 4C,9H |
| WILD OATS | 2C,4G | 4C,8H | 3C,3G | 3C,7G | 0 | 6G | 3C,7H | 4C,8H |
| WHEAT | 3G | 3C,8H | 2G | 7G | 0 | 2G | 7G | 2C,9H |
| CORN | 3C,4G | 4C,9H | 3C,7G | 3C,9H | 3C,7G | 8G | 2C,8G | 9G |
| SOYBEAN | 3C,5G | 3C,7H | 4C,8H | 9H | 2C,4G | 9H | 3C,7H | 9H |
| RICE | 9H | 10E | 9H | 10H | 9H | 9H | 5C,9H | 10E |
| SORGHUM | 3C,8H | 10E | 3C,9H | 10H | 3C,8G | 9H | 9H | 10H |
| CHEATGRASS | 3C,7G | 9G | 9H | 10E | 8G | 9H | 9H | 10E |
| SUGAR BEETS | 7G | 9G | 4C,9G | 4C,9G | 9G | 9C | 9G | 5C,9G |
| VELVETLEAF | 3C,6H | 3C,9G | 3C,5G | 3C,9G | 7H | 4C,9G | 7H | 3C,9G |
| GIANT FOXTAIL | 3C,5G | 4C,8H | 3C,8G | 9H | 3C,5G | 9H | 4C,9G | 4C,9H |
| BARLEY | 3C,7G | 9G | 3C,8G | 9G | 2C,4G | 7G | 9G | 4C,9H |

Table A (cont.)

| | CMPD 36 | | CMPD 37 | |
|---|---|---|---|---|
| RATE RATE=KG/HA | 0.01 | 0.05 | 0.01 | 0.05 |
| POSTEMERGENCE | | | | |
| COTTON | 5C,9H | 5C,9G | 4C,9G | 10C |
| MORNING GLORY | 3C,7H | 10C | 4C,8G | 9C |
| COCKLEBUR | 3C,8H | 10C | 10C | 10C |
| NUTSEDGE | 4G | 5G | 4C,9G | 5C,9G |
| CRABGRASS | 5C,8G | 9C | 2G | 2C,5G |
| BARNYARD GRASS | 5C,9H | 10C | 3C,7G | 4C,9H |
| WILD OATS | 6C,9G | 5C,9G | 0 | 2C,2G |
| WHEAT | 3C,9G | 6C,9G | 0 | 2C,4G |
| CORN | 9C | 9C | 2G | 3C,7H |
| SOYBEAN | 5C,9G | 6C,9G | 3C,5H | 4C,9G |
| RICE | 9C | 9C | 6G | 9C |
| SORGHUM | 9C | 9C | 3C,9H | 9G |
| CHEATGRASS | 6C,9G | 9C | 7G | 9C |
| SUGAR BEETS | 9C | 10C | 9C | 9C |
| VELVETLEAF | 6G | 7G | 9C | 10C |
| GIANT FOXTAIL | 5C,9G | 9C | 3C,3G | 4C,8H |
| BARLEY | 7G | 9C | 0 | 2C,3G |
| PREEMERGENCE | | | | |
| COTTON | 4H | 7H | 4G | 8G |
| MORNING GLORY | 3C,3H | 8G | 3C,5G | 5H |
| COCKLEBUR | 2C | 3C,7H | 0 | . |
| NUTSEDGE | 2C | 10E | 9G | 10E |
| CRABGRASS | 3C,8G | 4C,8G | 0 | 7G |
| BARNYARD GRASS | 3C,7H | 9H | 4H | 9H |
| WILD OATS | 3C,6G | 3C,7G | 0 | 3G |
| WHEAT | 7G | 9H | 0 | 3G |
| CORN | 3C,9G | 3C,9G | 2C | 3C,9G |
| SOYBEAN | 3C,7H | 9H | 3C,4H | 3C,7H |
| RICE | 9H | 10E | 8H | 9H |
| SORGHUM | 5C,9H | 10H | 3C,8H | 5C,9H |
| CHEATGRASS | 8H | 10E | 8G | 9H |
| SUGAR BEETS | 3G | 3C,8G | 8G | 8G |
| VELVETLEAF | 0 | 5H | 2H | 5H |
| GIANT FOXTAIL | 3C,8H | 4C,9H | 3G | 9H |
| BARLEY | 9G | 9G | 4G | 3C,5G |

Table A (cont.)

Compound 38

| RATE=KG/HA | 0.01 | 0.05 |
|---|---|---|
| POSTEMERGENCE | | |
| BARLEY | 0 | 2G |
| BARNYARD GRASS | 0 | 0 |
| CHEATGRASS | 3G | 8G |
| COCKLEBUR | 5C,9G | 10C |
| CORN | 0 | 0 |
| COTTON | 7G | 4C,9G |
| CRABGRASS | 0 | 0 |
| GIANT FOXTAIL | 0 | 3G |
| MORNING GLORY | 2C,5G | 3C,8G |
| NUTSEDGE | 3C,7G | 4C,9G |
| RICE | 0 | 5G |
| SORGHUM | 0 | 0 |
| SOYBEAN | 1H | 5H |
| SUGAR BEETS | 9C | 10C |
| VELVETLEAF | 4C,8H | 10C |
| WHEAT | 0 | 2G |
| WILD OATS | 0 | 2G |
| PREEMERGENCE | | |
| BARLEY | 0 | 2G |
| BARNYARD GRASS | 0 | 2C,2H |
| CHEATGRASS | 0 | 7G |
| COCKLEBUR | 3C,3H | - |
| CORN | 0 | 2G |
| COTTON | 0 | 7H |
| CRABGRASS | 0 | 0 |
| GIANT FOXTAIL | 0 | 2G |
| MORNING GLORY | 2G | 8G |
| NUTSEDGE | 0 | 9G |
| RICE | 0 | 0 |
| SORGHUM | 0 | 0 |
| SOYBEAN | 0 | 0 |
| SUGAR BEETS | 7G | 9G |
| VELVETLEAF | 0 | 3C,8H |
| WHEAT | 0 | 3G |
| WILD OATS | 0 | 3G |

Table A (cont.)

Compound 39

| RATE=KG/HA | 0.01 | 0.05 |
|---|---|---|
| POSTEMERGENCE | | |
| BARLEY | 0 | 3G |
| BARNYARD GRASS | 2H | 2C,5G |
| CHEATGRASS | 8G | 8G |
| COCKLEBUR | 10C | 10C |
| CORN | 0 | 0 |
| COTTON | 9C | 5C,9G |
| CRABGRASS | 0 | 0 |
| GIANT FOXTAIL | 0 | 3G |
| MORNING GLORY | 3C,8G | 9C |
| NUTSEDGE | 9G | 10C |
| RICE | 0 | 2G |
| SORGHUM | 0 | 2G |
| SOYBEAN | 2C,5H | 3C,7H |
| SUGAR BEETS | 4C,9G | 9C |
| VELVETLEAF | 9C | 10C |
| WHEAT | 0 | 5G |
| WILD OATS | 0 | 3C,7G |
| PREEMERGENCE | | |
| BARLEY | 0 | 0 |
| BARNYARD GRASS | 0 | 5G |
| CHEATGRASS | 0 | 8G |
| COCKLEBUR | 2C,3H | 9H |
| CORN | 0 | 3G |
| COTTON | 0 | 8G |
| CRABGRASS | 0 | 2G |
| GIANT FOXTAIL | 0 | 3G |
| MORNING GLORY | 5G | 9G |
| NUTSEDGE | 10E | 10E |
| RICE | 0 | 0 |
| SORGHUM | 0 | 0 |
| SOYBEAN | 0 | 6H |
| SUGAR BEETS | 7G | 9G |
| VELVETLEAF | 5H | 4C,9G |
| WHEAT | 0 | 4G |
| WILD OATS | 0 | 3G |

Table A (cont.)

Compound 40

| RATE=KG/HA | 0.01 | 0.05 |
|---|---|---|
| POSTEMERGENCE | | |
| BARLEY | 0 | 0 |
| BARNYARD GRASS | 3G | 2C,7G |
| CHEATGRASS | 6G | 4C,9G |
| COCKLEBUR | 10C | 10C |
| CORN | 0 | 2C,2H |
| COTTON | 5C,9G | 5C,9G |
| CRABGRASS | 2G | 3C,7G |
| GIANT FOXTAIL | 3G | 4C,7G |
| MORNING GLORY | 6G | 10C |
| NUTSEDGE | 4C,9G | 10C |
| RICE | 0 | 2G |
| SORGHUM | 0 | 0 |
| SOYBEAN | 3C,8H | 5C,9G |
| SUGAR BEETS | 10C | 10C |
| VELVETLEAF | 10C | 10C |
| WHEAT | 0 | 0 |
| WILD OATS | 0 | 0 |
| PREEMERGENCE | | |
| BARLEY | 0 | 2G |
| BARNYARD GRASS | 0 | 5G |
| CHEATGRASS | 4G | 9G |
| COCKLEBUR | 2C,4H | 7H |
| CORN | 0 | 2G |
| COTTON | - | 4H |
| CRABGRASS | 2C,3G | 2C,5G |
| GIANT FOXTAIL | 0 | 3G |
| MORNING GLORY | 7G | 8G |
| NUTSEDGE | 5G | 10E |
| RICE | 0 | 0 |
| SORGHUM | 0 | 0 |
| SOYBEAN | 0 | 6H |
| SUGAR BEETS | 9C | 9G |
| VELVETLEAF | 3H | 8G |
| WHEAT | 0 | 0 |
| WILD OATS | 0 | 0 |

Test B

Postemergence

Three round pans (25 cm diameter by 12.5 cm deep) were filled with Sassafras sandy loam soil. One pan was planted with nutsedge (Cyperus rotundus) tubers, crabgrass (Digitaria sanguinalis), sicklepod (Cassia obtusifolia), jimsonweed (Datura stramonium), velvetleaf (Abutilon theophrasti), lambsquarters (Chenopodium album), rice (Oryza sativa) and teaweed (Sida spinosa). The second pot was planted with green foxtail (Setaria viridis), cocklebur (Xantium pensylvanicum), morningglory (Ipomoea hederacea), cotton (Gossypium hirsutum), johnsongrass (Sorghum halepense), barnyardgrass (Echinochloa crusgalli), corn (Zea mays), soybean (Glycine max) and giant foxtail (Setaria faberi). The third pot was planted with wheat (Triticum aestivum), barley (Hordeum vulgare), wild buckwheat (Polgonum convolvulus L.), cheatgrass (Bromus secalinus L.), sugarbeet (Beta vulgaris), wild oats (Avena fatua), viola (Viola arvensis), blackgrass (Alopecurus myosuroides), and rape (Brassica napus). The plants were grown for approximately fourteen days, then sprayed postemergence with the chemicals dissolved in a non-phytotoxic solvent.

Preemergence

Three round pans (25 cm diameter by 12.5 cm deep) were filled with Sassafras sandy loam soil. One pan was planted with nutsedge tubers, crabgrass, sicklepod, jimsonweed, velvetleaf, lambsquarters, rice and teaweed. The second pot was planted with green foxtail, cocklebur, morningglory, cotton, johnsongrass, barnyardgrass, corn, soybean and giant foxtail. The third pot was planted with wheat, barley, wild buck-wheat, cheatgrass, sugarbeet, wild oat, viola, blackgrass and rape. The three pans were sprayed preemergence with the chemicals dissolved in a non-phytotoxic solvent.

Treated plants and controls were maintained in the greenhouse for 24 days, then all rated plants were compared to controls and visually rated for plant response.

Response ratings are based on a scale of 0 to 100 where 0 = no effect and 100 = complete control. A dash (-) response means no test.

Response ratings are contained in Table B.

Table B

Compound 3

| | POSTEMERGENCE | | | | PREEMERGENCE | | | |
|---|---|---|---|---|---|---|---|---|
| Rate g/ha | 62 | 16 | 4 | 1 | 250 | 62 | 16 | 4 |
| Corn | 20 | 0 | 0 | 0 | 40 | 0 | 0 | 0 |
| Wheat | 0 | 0 | 0 | 0 | 0 | 0 | 0 | 0 |
| Barley | 0 | 0 | 0 | 0 | 0 | 0 | 0 | 0 |
| Rice | 0 | 0 | 0 | 0 | 60 | 40 | 20 | 0 |
| Soybean | 0 | 0 | 0 | 0 | 20 | 0 | 0 | 0 |
| Cotton | 70 | 50 | 30 | 0 | 50 | 20 | 0 | 0 |
| Sugar beet | 100 | 70 | 50 | 30 | 100 | 90 | 80 | 70 |
| Rape | 90 | 70 | 50 | 30 | 90 | 80 | 70 | 50 |
| Crabgrass | 60 | 30 | 0 | 0 | 50 | 30 | 0 | 0 |
| Johnsongrass | 70 | 30 | 0 | 0 | 90 | 70 | 50 | 30 |
| Blackgrass | 30 | 0 | 0 | 0 | 70 | 30 | 0 | 0 |
| Barnyardgrass | 70 | 30 | 0 | 0 | 80 | 60 | 40 | 0 |
| Nutsedge | 80 | 60 | 30 | 0 | 0 | 0 | 0 | 0 |
| Giant Foxtail | 30 | 0 | 0 | 0 | 80 | 60 | 40 | 0 |
| Green Foxtail | 70 | 30 | 0 | 0 | 90 | 70 | 50 | 30 |
| Cheatgrass | 30 | 0 | 0 | 0 | 90 | 60 | 30 | 0 |
| Wild Oats | 0 | 0 | 0 | 0 | 0 | 0 | 0 | 0 |
| Wild Buckwheat | 70 | 50 | 30 | 0 | 90 | 70 | 50 | 30 |
| Viola | 90 | 70 | 50 | 30 | 100 | 90 | 70 | 50 |
| Lambsquarter | 100 | 70 | 50 | 30 | 100 | 90 | 70 | 50 |
| Cocklebur | 100 | 90 | 70 | 50 | 80 | 50 | 30 | 0 |
| Morningglory | 100 | 70 | 50 | 30 | 90 | 60 | 30 | 0 |
| Teaweed | 90 | 70 | 50 | 30 | 80 | 60 | 40 | 20 |
| Sicklepod | 60 | 30 | 0 | 0 | 100 | 70 | 50 | 30 |
| Jimsonweed | 70 | 50 | 30 | 0 | 90 | 70 | 50 | 30 |
| Velvetleaf | 90 | 70 | 50 | 30 | 90 | 70 | 50 | 30 |

Table B (cont.)

Compound 4

| | POSTEMERGENCE | | | | PREEMERGENCE | | | |
|---|---|---|---|---|---|---|---|---|
| Rate g/ha | 62 | 16 | 4 | 1 | 250 | 62 | 16 | 4 |
| Corn | 30 | 0 | 0 | 0 | 60 | 20 | 0 | 0 |
| Wheat | 0 | 0 | 0 | 0 | 30 | 0 | 0 | 0 |
| Barley | 0 | 0 | 0 | 0 | 30 | 0 | 0 | 0 |
| Rice | 0 | 0 | 0 | 0 | 70 | 30 | 0 | 0 |
| Soybean | 20 | 0 | 0 | 0 | 0 | 0 | 0 | 0 |
| Cotton | 90 | 40 | 0 | 0 | 20 | 0 | 0 | 0 |
| Sugar beet | 100 | 90 | 70 | 50 | 100 | 90 | 70 | 50 |
| Rape | 100 | 70 | 40 | 0 | 90 | 80 | 70 | 50 |
| Crabgrass | 70 | 50 | 30 | 0 | 50 | 30 | 0 | 0 |
| Johnsongrass | 50 | 30 | 0 | 0 | 70 | 50 | 30 | 0 |
| Blackgrass | 0 | 0 | 0 | 0 | 30 | 0 | 0 | 0 |
| Barnyardgrass | 90 | 60 | 30 | 0 | 70 | 30 | 0 | 0 |
| Nutsedge | 90 | 70 | 50 | 30 | 100 | 70 | 50 | 30 |
| Giant Foxtail | 80 | 60 | 30 | 0 | 70 | 50 | 30 | 0 |
| Green Foxtail | 70 | 50 | 30 | 0 | 90 | 70 | 50 | 30 |
| Cheatgrass | 30 | 0 | 0 | 0 | 70 | 30 | 0 | 0 |
| Wild Oats | 100 | 70 | 50 | 30 | 0 | 0 | 0 | 0 |
| Wild Buckwheat | 90 | 70 | 50 | 30 | 80 | 60 | 40 | 0 |
| Viola | 100 | 90 | 70 | 50 | 100 | 90 | 70 | 50 |
| Lambsquarter | 90 | 70 | 50 | 30 | 100 | 90 | 80 | 70 |
| Cocklebur | 100 | 90 | 70 | 50 | 90 | 70 | 50 | 30 |
| Morningglory | 100 | 90 | 70 | 50 | 90 | 80 | 60 | 40 |
| Teaweed | 90 | 70 | 50 | 30 | 90 | 70 | 50 | 30 |
| Sicklepod | 80 | 70 | 50 | 30 | 100 | 100 | 70 | 50 |
| Jimsonweed | 100 | 70 | 50 | 30 | 90 | 70 | 50 | 30 |
| Velvetleaf | 100 | 70 | 50 | 30 | 90 | 80 | 70 | 50 |

Table B (cont.)

Compound 8

| RATE RATE=G/HA | 0004 | 0016 | 0062 | 0250 |
|---|---|---|---|---|
| PREEMERGENCE | | | | |
| GIANT FOXTAIL | 40 | 90 | 100 | 100 |
| VELVETLEAF | 50 | 90 | 100 | 100 |
| SUGAR BEETS | 90 | 100 | 100 | 100 |
| CRABGRASS | 0 | 0 | 20 | 90 |
| TEAWEED | 30 | 80 | 90 | 100 |
| JIMSONWEED | 40 | 80 | 100 | 100 |
| RICE | 0 | 0 | 20 | 100 |
| COCKLEBUR | 40 | 50 | 90 | 100 |
| COTTON | 30 | 50 | 70 | 100 |
| SOYBEAN | 0 | 20 | 50 | 100 |
| BARNYARD GRASS | 50 | 90 | 100 | 100 |
| WILD OATS | 0 | 0 | 0 | 0 |
| MORNINGGLORY | 40 | 60 | 100 | 100 |
| WHEAT | 0 | 0 | 0 | 0 |
| CASSIA | 0 | 40 | 70 | 100 |
| JOHNSONGRASS | 0 | 40 | 60 | 90 |
| NUTSEDGE | 90 | 100 | 100 | 100 |
| CORN | 20 | 50 | 70 | 100 |
| WILD BUCKWHEAT | 80 | 90 | 100 | 100 |
| BLACK GRASS | | 40 | 90 | 90 |
| RAPESEED | 40 | 90 | 100 | 100 |
| BARLEY | 0 | 0 | 0 | 0 |
| GREEN FOXTAIL | 70 | 100 | 100 | 100 |
| CHEAT GRASS | 70 | 90 | 90 | 100 |
| LAMBSQUARTER | 90 | 100 | 100 | 100 |

## Table B (cont.)

### Compound 8

| RATE RATE=G/HA | 0001 | 0004 | 0016 | 0062 |
|---|---|---|---|---|
| POSTEMERGENCE | | | | |
| GIANT FOXTAIL | 20 | 60 | 90 | 100 |
| VELVETLEAF | 100 | 100 | 100 | 100 |
| SUGAR BEETS | 70 | 80 | 100 | 100 |
| CRABGRASS | 0 | 20 | 50 | 100 |
| TEAWEED | 0 | 30 | 70 | 100 |
| JIMSONWEED | 0 | 50 | 100 | 100 |
| RICE | 0 | 0 | 0 | 0 |
| COCKLEBUR | 40 | 70 | 100 | 100 |
| COTTON | 40 | 40 | 80 | 90 |
| SOYBEAN | 20 | 40 | 60 | 70 |
| BARNYARD GRASS | 40 | 40 | 80 | 100 |
| WILD OATS | 0 | 0 | 0 | 20 |
| MORNINGGLORY | 0 | 30 | 80 | 100 |
| WHEAT | 0 | 0 | 0 | 0 |
| CASSIA | 30 | 30 | 50 | 100 |
| JOHNSONGRASS | 0 | 0 | 0 | 0 |
| NUTSEDGE | 80 | 100 | 100 | 100 |
| CORN | 0 | 20 | 80 | 90 |
| WILD BUCKWHEAT | 30 | 50 | 100 | 100 |
| BLACK GRASS | 0 | 0 | 30 | 30 |
| RAPESEED | 100 | 100 | 100 | 100 |
| BARLEY | 0 | 0 | 0 | 0 |
| GREEN FOXTAIL | 0 | 30 | 100 | 100 |
| CHEAT GRASS | 0 | 30 | 50 | 80 |
| BUCKWHEAT | . | | | |
| VIOLA | | | | |
| LAMBSQUARTER | 100 | | | 100 |

Table B (cont.)

Compound 22

| RATE RATE=G/HA | 0001 | 0004 | 0016 | 0062 |
|---|---|---|---|---|
| POSTEMERGENCE | | | | |
| GIANT FOXTAIL | 0 | 0 | 30 | 50 |
| VELVETLEAF | 50 | 70 | 100 | 100 |
| SUGAR BEETS | 70 | 80 | 90 | 90 |
| CRABGRASS | 0 | 0 | 30 | 70 |
| TEAWEED | 30 | 50 | 70 | 90 |
| JIMSONWEED | 30 | 50 | 70 | 100 |
| RICE | 0 | 20 | 30 | 50 |
| COCKLEBUR | 50 | 80 | 100 | 100 |
| COTTON | 50 | 60 | 70 | 80 |
| SOYBEAN | 70 | 90 | 90 | 100 |
| BARNYARD GRASS | 0 | 0 | 20 | 40 |
| WILD OATS | 0 | 0 | 0 | 0 |
| MORNINGGLORY | 60 | 80 | 100 | 100 |
| WHEAT | 0 | 0 | 0 | 0 |
| CASSIA | 30 | 50 | 80 | 100 |
| JOHNSONGRASS | 0 | 0 | 30 | 70 |
| NUTSEDGE | 30 | 40 | 60 | 100 |
| CORN | 0 | 0 | 20 | 60 |
| WILD BUCKWHEAT | 30 | 50 | 70 | 80 |
| BLACK GRASS | 0 | 0 | 30 | 50 |
| RAPESEED | 100 | 100 | 100 | 100 |
| BARLEY | 0 | 0 | 0 | 20 |
| GREEN FOXTAIL | 0 | 0 | 30 | 50 |
| CHEAT GRASS | 0 | 0 | 30 | 60 |
| VIOLA | 70 | 90 | 100 | 100 |
| LAMBSQUARTER | 30 | 40 | 50 | 70 |

Table B (cont.)

Compound 22

| RATE RATE=G/HA | 0004 | 0016 | 0062 | 0250 |
|---|---|---|---|---|
| PREEMERGENCE | | | | |
| GIANT FOXTAIL | 0 | 30 | 50 | 70 |
| VELVETLEAF | 30 | 50 | 70 | 90 |
| SUGAR BEETS | 70 | 80 | 90 | 100 |
| CRABGRASS | 0 | 30 | 60 | 90 |
| TEAWEED | 50 | 70 | 80 | 90 |
| JIMSONWEED | 50 | 70 | 80 | 90 |
| RICE | 30 | 50 | 80 | 100 |
| COCKLEBUR | 60 | 70 | 80 | 90 |
| COTTON | 0 | 30 | 50 | 70 |
| SOYBEAN | 0 | 20 | 40 | 60 |
| BARNYARD GRASS | 30 | 50 | 70 | 90 |
| WILD OATS | 0 | 0 | 20 | 30 |
| MORNINGGLORY | 70 | 80 | 90 | 100 |
| WHEAT | 0 | 0 | 0 | 0 |
| CASSIA | 30 | 50 | 70 | 90 |
| JOHNSONGRASS | 30 | 50 | 70 | 90 |
| NUTSEDGE | 50 | 70 | 100 | 100 |
| CORN | 0 | 0 | 20 | 60 |
| WILD BUCKWHEAT | 70 | 80 | 90 | 100 |
| BLACK GRASS | 50 | 60 | 70 | 80 |
| RAPESEED | 80 | 90 | 100 | 100 |
| BARLEY | 0 | 0 | 0 | 0 |
| GREEN FOXTAIL | 0 | 30 | 60 | 90 |
| CHEAT GRASS | 0 | 30 | 50 | 80 |
| LAMBSQUARTER | 70 | 80 | 90 | 100 |

Table B (cont.)

Compound 28

| RATE RATE=G/HA | 0001. | 0004. | 0016. | 0062. |
|---|---|---|---|---|
| POSTEMERGENCE | | | | |
| GIANT FOXTAIL | 0 | 30 | 60 | 80 |
| VELVETLEAF | 60 | 100 | 100 | 100 |
| SUGAR BEETS | 100 | 100 | 100 | |
| CRABGRASS | 0 | 30 | 50 | 70 |
| TEAWEED | 30 | 50 | 70 | 90 |
| JIMSONWEED | 30 | 50 | 70 | 100 |
| RICE | 0 | 0 | 0 | 100 |
| COCKLEBUR | 100 | 100 | 100 | 100 |
| COTTON | 30 | 60 | 100 | 100 |
| SOYBEAN | 0 | 0 | 20 | |
| BARNYARD GRASS | 0 | 0 | 60 | 100 |
| WILD OATS | 0 | 0 | 0 | |
| MORNINGGLORY | 30 | 50 | 70 | 90 |
| WHEAT | 0 | 0 | 0 | |
| CASSIA | 0 | 30 | 50 | 70 |
| JOHNSONGRASS | 0 | 0 | 0 | 50 |
| NUTSEDGE | 100 | 100 | 100 | 100 |
| CORN | 0 | 30 | 50 | |
| WILD BUCKWHEAT | 30 | 50 | 70 | |
| BLACK GRASS | 0 | 30 | 50 | |
| RAPESEED | 100 | 100 | 100 | |
| BARLEY | 0 | 0 | 0 | |
| GREEN FOXTAIL | 0 | 0 | 30 | 60 |
| CHEAT GRASS | 0 | 0 | 30 | |
| BUCKWHEAT | | | | |
| VIOLA | 70 | 90 | 100 | |
| LAMBSQUARTER | 50 | 70 | 90 | 100 |

## Table B (cont.)

### Compound 28

| RATE RATE=G/HA | 0004. | 0016. | 0062. | 0.250 |
|---|---|---|---|---|
| PREEMERGENCE | | | | |
| GIANT FOXTAIL | 30 | 60 | 100 | 100 |
| VELVETLEAF | 50 | 70 | 80 | 100 |
| SUGAR BEETS | 60 | 70 | 80 | 90 |
| CRABGRASS | 30 | 50 | 80 | 100 |
| TEAWEED | 30 | 50 | 70 | 90 |
| JIMSONWEED | 40 | 70 | 80 | 90 |
| RICE | 0 | 0 | 30 | 80 |
| COCKLEBUR | 50 | 70 | 60 | 90 |
| COTTON | 0 | 30 | 60 | 80 |
| SOYBEAN | 0 | 20 | 50 | 70 |
| BARNYARD GRASS | 0 | 40 | 70 | 90 |
| WILD OATS | 0 | 0 | 0 | 0 |
| MORNINGGLORY | 30 | 70 | 80 | 90 |
| WHEAT | 0 | 0 | 0 | 0 |
| CASSIA | 30 | 50 | 60 | 80 |
| JOHNSONGRASS | 30 | 50 | 70 | 90 |
| NUTSEDGE | 100 | 100 | 100 | 100 |
| CORN | 0 | 20 | 60 | 80 |
| WILD BUCKWHEAT | 50 | 70 | 80 | 90 |
| BLACK GRASS | 0 | 30 | 60 | 90 |
| RAPESEED | 80 | 90 | 100 | 100 |
| BARLEY | 0 | 0 | 0 | 0 |
| GREEN FOXTAIL | 30 | 50 | 100 | 100 |
| CHEAT GRASS | 0 | 30 | 60 | 90 |
| BUCKWHEAT | | | | |
| VIOLA | 70 | 90 | 100 | 100 |
| LAMBSQUARTER | 80 | 100 | 100 | 100 |

Table B (cont.)

Compound 35

| RATE RATE=G/HA | 0001. | 0004. | 0016. | 0062. |
|---|---|---|---|---|
| PREEMERGENCE | | | | |
| GIANT FOXTAIL | 50 | 70 | 80 | 90 |
| VELVETLEAF | 30 | 50 | 70 | 90 |
| SUGAR BEETS | 70 | 90 | 100 | 100 |
| CRABGRASS | 50 | 70 | 80 | 100 |
| TEAWEED | 40 | 50 | 70 | 80 |
| JIMSONWEED | 50 | 70 | 80 | 90 |
| RICE | 90 | 100 | 100 | 100 |
| COCKLEBUR | 60 | 70 | 80 | 90 |
| COTTON | 20 | 40 | 60 | 80 |
| SOYBEAN | 20 | 40 | 70 | 90 |
| BARNYARD GRASS | 30 | 60 | 90 | 100 |
| WILD OATS | 40 | 50 | 70 | 90 |
| MORNINGGLORY | 30 | 50 | 60 | 70 |
| WHEAT | 20 | 30 | 60 | 100 |
| CASSIA | 80 | 90 | 100 | 100 |
| JOHNSONGRASS | 70 | 80 | 90 | 100 |
| NUTSEDGE | 0 | 30 | 60 | 90 |
| CORN | 0 | 60 | 80 | 100 |
| WILD BUCKWHEAT | 30 | 60 | 80 | 90 |
| BLACK GRASS | 50 | 70 | 80 | 100 |
| RAPESEED | 60 | 70 | 80 | 90 |
| BARLEY | 20 | 40 | 90 | 100 |
| GREEN FOXTAIL | 60 | 80 | 100 | 100 |
| CHEAT GRASS | 50 | 80 | 100 | 100 |
| BUCKWHEAT | | | | |
| VIOLA | 60 | 70 | 80 | 100 |
| LAMBSQUARTER | 70 | 80 | 90 | 100 |

## Table B (cont.)

### Compound 35

| RATE RATE=G/HA | 0.25 | 0001. | 0004. | 0016. |
|---|---|---|---|---|
| POSTEMERGENCE | | | | |
| GIANT FOXTAIL | 0 | 20 | 50 | 80 |
| VELVETLEAF | 30 | 40 | 60 | 80 |
| SUGAR BEETS | 70 | 80 | 90 | 100 |
| CRABGRASS | 0 | 0 | 60 | 60 |
| TEAWEED | 60 | 60 | 70 | 80 |
| JIMSONWEED | 0 | 40 | 70 | 80 |
| RICE | 30 | 50 | 80 | 100 |
| COCKLEBUR | 40 | 50 | 80 | 100 |
| COTTON | 0 | 20 | 80 | 80 |
| SOYBEAN | | 60 | 70 | 100 |
| BARNYARD GRASS | 20 | 60 | 70 | 100 |
| WILD OATS | 0 | 50 | 80 | 90 |
| MORNINGGLORY | 0 | 30 | 70 | 80 |
| WHEAT | 0 | 20 | 50 | 90 |
| CASSIA | 60 | | 80 | 90 |
| JOHNSONGRASS | 30 | 70 | 90 | 100 |
| NUTSEDGE | 20 | 40 | 60 | 60 |
| CORN | 0 | 40 | 90 | 90 |
| WILD BUCKWHEAT | 0 | 50 | 90 | 90 |
| BLACK GRASS | 20 | 70 | 80 | 100 |
| RAPESEED | 40 | 80 | 90 | 100 |
| BARLEY | 20 | 60 | 90 | 100 |
| GREEN FOXTAIL | 30 | 60 | 70 | 90 |
| CHEAT GRASS | 0 | 30 | 60 | 70 |
| BUCKWHEAT | | | | |
| VIOLA | 0 | 40 | 90 | 100 |
| LAMBSQUARTER | 40 | 70 | 90 | 100 |

Test C

Sixteen cm diameter Wagner pots, equipped with a stoppered drain opening near the bottom of the side wall, were partially filled with Woodstown sandy loam. About 1500 mls of water were added to each pot to bring the water level to a point 3 cm above the soil surface. Japonica and Indica rice seedlings were transplanted as described in Test E. Also, a number of barnyardgrass (Echinochola crusgalli) seeds were added to each pot. At the same time, seedlings or tubers of the following species were transplanted into the muddy soil: water plantain (Alisma trivale), Scirpus (Scirpus mucranatus), and Cyperus (Cyperus difformis). The weed species selected for this test are of economic importance in major rice-growing areas. The chemical treatments were applied directly to the paddy water after being formulated in a nonphytotoxic solvent within hours after transplanting of two additional species: water chestnuts (Eleocharis spp.) and arrowhead (Sagittaria latifolia). Shortly after treatment, the drain hole was opened to drop the water level by 2 cm. Water was then added to restore the water level to its original height. The following day the draining and refilling process was repeated. The pots were then maintained in the greenhouse. Rates of application and plant response ratings made 21 days after treatment are summarized in Table C.

In the subsequent tables, LS is used as an abbreviation for leaf stage.

Table C

Compound 8

| RATE RATE=G/HA | 0004 | 0008 | 0016 |
|---|---|---|---|
| SOIL | | | |
| BARNYARD GRASS | 50 | 67 | 70 |
| WATER CHESTNUT | 62 | 77 | 90 |
| ARROWHEAD | 0 | 57 | 80 |
| SCIRPUS (SEDGE) | 37 | 72 | 85 |
| CYPRESS (SEDGE) | 75 | 75 | 95 |
| WATER PLANTAIN | 75 | 55 | 90 |
| RICE JAP EFF | 0 | 0 | 10 |
| RICE INDICA EFF | 0 | 17 | 0 |

Table C (cont.)

Compound 28

| RATE RATE=G/HA | 0004. | 0008. | 0016. |
|---|---|---|---|
| SOIL | | | |
| BARNYARD GRASS | 45 | 60 | 65 |
| WATER CHESTNUT | 75 | 95 | 92 |
| ARROWHEAD | 85 | 90 | 95 |
| SCIRPUS (SEDGE) | 82 | 85 | 92 |
| CYPRESS (SEDGE) | 92 | 95 | 100 |
| WATER PLANTAIN | 87 | 100 | 100 |
| RICE JAP EFF | 0 | 5 | 10 |
| RICE INDICA EFF | 0 | 0 | 5 |

Test D

The soybeans were planted in large 25 cm-diameter pots of soil, 6 to 10 plants per pot. The other plant species were planted in 15 cm-diamter pots of soil. Carn, because of its importance as a rotational crop, was by itself in one container, 3 to 5 plants per pot. The weed species used in this test were all of major economic importance in soybean-growing regions. They were planted 3 to 4 species per pot, each confined to a separate quadrant of the soil surface. The following species were included in the screen:

| | |
|---|---|
| barnyardgrass | Echinochloa crus-galli |
| giant foxtail | Setaria faberi |
| green foxtail | Setaris virdis |
| johnsongrass | Soghum halepense |
| fall panicum | Panicum dichotomiflorum |
| purple nutsedge | Cyperus rotundus |

| | |
|---|---|
| signalgrass | Brachiaria platyphylla |
| crabgrass | Digitaria sanguinalis |
| velvetleaf | Abutilon theophrasti |
| jimsonweed | Datura stramonium |
| hemp sesbania | Sesbania exaltata |
| sicklepod | Cassia obtusifolia |
| cocklebur | Xanthium pensylvanicum |
| ivyleaf morningglory | Ipomoea hederacea |
| purslane | Portulaca oleracea |
| pigweed | Amaranthus retroflexus |
| lambsquarter | Chenopodium album |
| teaweed | Sida spinosa |
| bindweed | Convolvulus arvensis |

For the post-emergence phase of the test, crop and weed species were planted two to three weeks before application so that they were present as young plants at the time of treatment. Plantings for the pre-emergence phase were made on the day before, or on the day of treatment. Approximate planting depths were: corn and soybeans - 3 to 4 cm; morningglory, cocklebur and nutsedge - 2.5 to 3 cm; velvetleaf, sicklepod and sesbania - 2 cm; all other species - 0.5 cm.

The test chemicals were dissolved/suspended in a non-phytotoxic solvent in concentrations required to obtain the desirec rate of application. The solutions or suspensions were then applied as soil/foliage sprays to the young plants (post-emergence phase) and to the soil surfaces of the freshly planted containers (pre-emergence phase). Application was made utilizing an automatic spray machine at a spray volume of 500 liters per hectare. Immediately after treatment, the containers were transferred to a greenhouse and subsequently watered on a demand basis, taking care not to wet the foliage of the plants in the post-emergence phase of the test.

Table D

Compound 9

| RATE RATE GM/H | 0002 | 0004 | 0008 | 0016 | 0031 |
|---|---|---|---|---|---|
| POSTEMERGENCE | | | | | |
| SOYBEAN | 0 | 0 | 0 | 20 | 60 |
| CORN | 0 | 0 | 0 | 50 | 70 |
| VELVETLEAF | 50 | 65 | 90 | 100 | 100 |
| NIGHTSHADE | 0 | 20 | 30 | 75 | 85 |
| JIMSONWEED | 0 | 0 | 20 | 30 | 40 |
| SICKLEPOD | 0 | 0 | 0 | 20 | 50 |
| SESBANIA | 0 | 0 | 0 | 30 | 50 |
| COCKLEBUR | 40 | 85 | 100 | 100 | 100 |
| IVYLEAF M/G | 30 | 65 | 75 | 80 | 95 |
| PIGWEED | 40 | 60 | 75 | 85 | 90 |
| LAMBSQUARTER | 30 | 60 | 85 | 85 | 85 |
| PRICKLY SIDA | 0 | 30 | 50 | 75 | 85 |
| SMARTWEED | 40 | 50 | 80 | 80 | 90 |
| BARNYARDGRASS | 0 | 30 | 50 | 70 | 80 |
| GIANT FOXTAIL | 0 | 0 | 0 | 40 | 65 |
| GREEN FOXTAIL | 0 | 0 | 0 | 40 | 60 |
| JOHNSONGRASS | 0 | 30 | 65 | 80 | 95 |
| FALL PANICUM | 0 | 20 | 40 | 50 | 75 |
| CRABGRASS | 0 | 0 | 0 | 20 | 30 |
| SIGNALGRASS | 0 | 20 | 30 | 50 | 65 |
| NUTSEDGE | 65 | 80 | 90 | 100 | 100 |

Table D (cont.)

Compound 9

| RATE RATE GM/H | 0031 | 0062 | 0125 | 0250 |
|---|---|---|---|---|
| PREEMERGENCE | | | | |
| SOYBEAN | 0 | 0 | 25 | 60 |
| CORN | 0 | 20 | 65 | 90 |
| VELVETLEAF | 0 | 20 | 80 | 95 |
| NIGHTSHADE | 50 | 70 | 85 | 95 |
| JIMSONWEED | 0 | 30 | 70 | 95 |
| SICKLEPOD | 0 | 0 | 30 | 60 |
| SESBANIA | 0 | 0 | 40 | 50 |
| COCKLEBUR | 80 | 75 | 80 | 95 |
| IVYLEAF M/G | 0 | 0 | 40 | 50 |
| PIGWEED | 70 | 85 | 100 | 100 |
| LAMBSQUARTER | 20 | 75 | 95 | 100 |
| PRICKLY SIDA | 40 | 70 | 80 | 90 |
| SMARTWEED | 70 | 90 | 90 | 100 |
| BARNYARDGRASS | 0 | 0 | 40 | 85 |
| GIANT FOXTAIL | 0 | 0 | 30 | 70 |
| GREEN FOXTAIL | 0 | 20 | 40 | 70 |
| JOHNSONGRASS | 85 | 90 | 95 | 95 |
| FALL PANICUM | 90 | 100 | 100 | 95 |
| CRABGRASS | 0 | 0 | 20 | 40 |
| SIGNALGRASS | 0 | 30 | 70 | 85 |
| NUTSEDGE | 80 | 90 | 100 | 100 |

## Claims

1. Compounds of Formula I

$$JSO_2NH\overset{W}{\overset{\|}{C}}NR_1A$$

I

wherein

J is 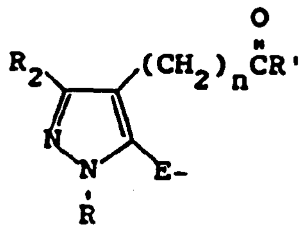 , 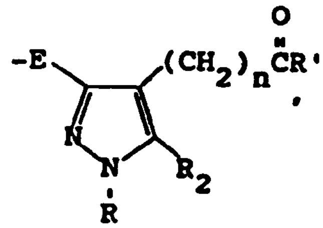 ,

J-1 J-2

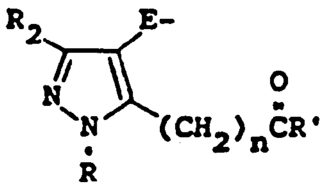 , 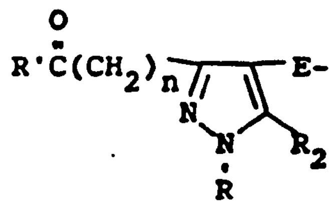 ,

J-3 J-4

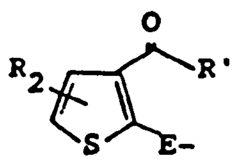 , 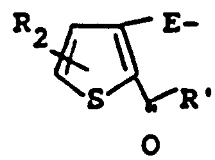 ,

J-5 J-6

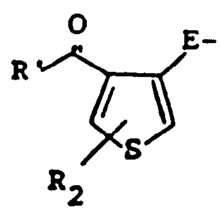 , 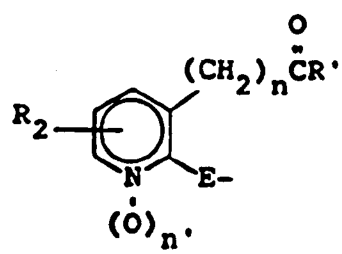 ,

J-7 J-8

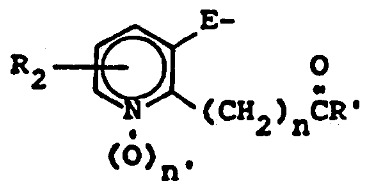 ,

J-9

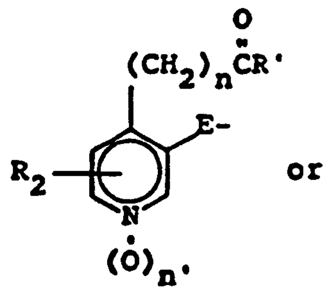 or

J-10

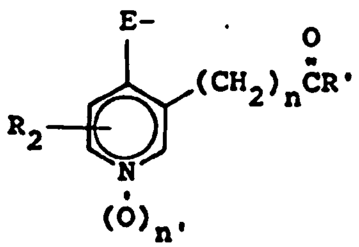 ;

J-11

R is H, $C_1$-$C_3$ alkyl, phenyl, $SO_2NR_aR_b$, $C_1$-$C_2$ haloalkyl, $C_2$-$C_4$ alkoxyalkyl, $C_2$-$C_3$ cyanoalkyl, $C_2$-$C_4$ alkylthioalkyl, $C_2$-$C_4$ alkylsulfinylalkyl, $C_2$-$C_4$ alkylsulfonylalkyl, $CO_2C_1$-$C_2$ alkyl, $C_1$-$C_4$ alkylcarbonyl, $C_1$-$C_2$ alkylsulfonyl, $C_3$-$C_4$ alkenyl, $C_3$-$C_4$ alkynyl or $C_1$-$C_2$ alkyl substituted with $CO_2C_1$-$C_2$ alkyl;

$R_1$ is H or $CH_3$;

$R_2$ is H, $C_1$-$C_3$ alkyl. $C_1$-$C_3$ haloalkyl, halogen, nitro. $C_1$-$C_3$ alkoxy, $SO_2NR_cR_d$, $C_1$-$C_3$ alkylthio, $C_1$-$C_3$ alkylsulfinyl, $C_1$-$C_3$ alkylsulfonyl, CN, $CO_2R_e$, $C_1$-$C_3$ haloalkoxy, $C_1$-$C_3$ haloalkylthio, amino, $C_1$-$C_2$ alkylamino, di($C_1$-$C_3$ alkyl)amino or $C_1$-$C_2$ alkyl substituted with $C_1$-$C_2$ alkoxy, $C_1$-$C_2$ haloalkoxy, $C_1$-$C_2$ alkylthio, $C_1$-$C_2$ haloalkylthio, CN, OH or SH;

$R_a$ and $R_b$ are independently $C_1$-$C_2$ alkyl;

$R_c$ is H, $C_1$-$C_4$ alkyl, $C_2$-$C_3$ cyanoalkyl, methoxy or ethoxy;

$R_c$ is H, $C_1$-$C_4$ alkyl or $C_3$-$C_4$ alkenyl; or

$R_c$ and $R_d$ may be taken together as $-(CH_2)_3-$, $-(CH_2)_4-$, $-(CH_2)_5-$ or $-CH_2CH_2OCH_2CH_2-$;

$R_e$ is $C_1$-$C_4$ alkyl, $C_3$-$C_4$ alkenyl, $C_3$-$C_4$ alkynyl, $C_2$-$C_4$ haloalkyl, $C_1$-$C_2$ cyanoalkyl, $C_5$-$C_6$ cycloalkyl, $C_4$-$C_7$ cycloalkylalkyl or $C_2$-$C_4$ alkoxyalkyl:

R′ is $C_1$-$C_5$ alkyl, $C_1$-$C_5$ haloalkyl, $C_1$-$C_5$ alkyl substituted with one or two $R_3$ groups, $C_2$-$C_5$ alkenyl, $C_2$-$C_5$ haloalkenyl, $C_3$-$C_5$ alkenyl substituted with one or two $R_3$ groups, $C_3$-$C_5$ alkynyl, $C_3$-$C_5$ haloalkynyl, $C_3$-$C_5$ alkynyl substituted with one or two $R_3$ groups, $C_3$-$C_5$ cycloalkyl, $C_3$-$C_5$ halocycloalkyl, $C_3$-$C_5$ cycloalkyl substituted with one or two $R_4$ groups, $C_4$-$C_7$ cycloalkylalkyl, $C_4$-$C_7$ halocycloalkylalkyl, $C_4$-$C_7$ cycloalkylalkyl substituted with one or two $R_4$ groups, phenyl or benzyl;

$R_3$ is $C_1$-$C_3$ alkoxy, $C_1$ -$C_3$ alkylthio, $C_1$-$C_3$ haloalkoxy, $C_1$-$C_3$ alkylsulfinyl, $C_1$-$C_3$ alkylsulfonyl, CN, $NO_2$, OH, $OR_5$ or di-($C_1$ -$C_3$ alkyl)amino;

$R_4$ is $C_1$-$C_3$ alkyl, $C_1$-$C_3$ alkoxy, $C_1$-$C_3$ haloalkoxy, CN, $NO_2$, OH, $OR_5$ or di-($C_1$-$C_3$ alkyl)amino;

$R_5$ is $SO_2CH_3$, $Si(CH_3)_3$, $C_2$-$C_3$ alkylcarbonyl or $CO_2C_1$-$C_2$ alkyl;

E is a single bond or $CH_2$;

W is O or S:

n is O or 1:

n′ is 0 or 1;

A is 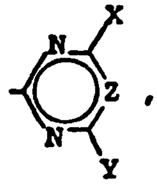, 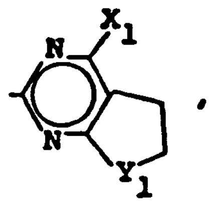, 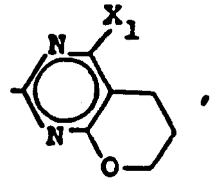,

A-1 A-2 A-3

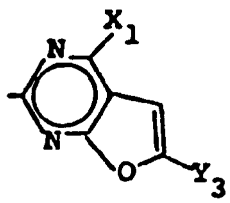, 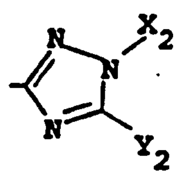, 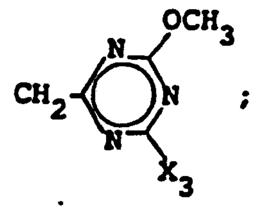;

A-4 A-5 A-6

or 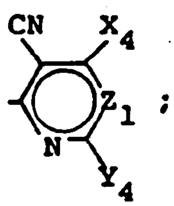;

A-7

X is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino, di($C_1$-$C_3$ alkyl)amino or $C_3$-$C_5$ cycloalkyl;

Y is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, $C_2$-$C_5$ alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkylthioalkyl, $C_1$-$C_4$ haloalkyl, $C_2$-$C_4$ alkynyl, azido, cyano, $C_2$-$C_5$ alkylsulfinylalkyl, $C_2$-$C_5$ alkylsulfonylalkyl,

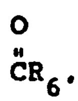, 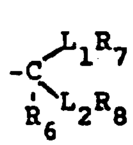, 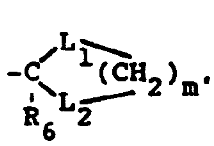, 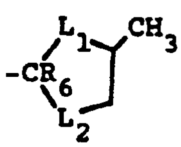

or $N(OCH_3)CH_3$;

m is 2 or 3;

$L_1$ and $L_2$ are independently O or S;

$R_6$ is H or $C_1$-$C_3$ alkyl;

$R_7$ and $R_6$ are independently $C_1$-$C_3$ alkyl;

Z is CH or N;

$Z_1$ is CH or N;

$Y_1$ is O or $CH_2$;

$X_1$ is $CH_3$, $OCH_3$, $OC_2H_5$ or $OCF_2H$;

$X_2$ is $CH_3$, $C_2H_5$ or $CH_2CF_3$;
$Y_2$ is $OCH_3$, $OC_2H_5$, $SCH_3$, $SC_2H_5$, $CH_3$ or $CH_2CH_3$;
$X_3$ is $CH_3$ or $OCH_3$;
$Y_3$ is H or $CH_3$;
$X_4$ is $CH_3$, $OCH_3$, $OC_2H_5$, $CH_2OCH_3$ or Cl; and
$Y_4$ is $CH_3$, $OCH_3$, $OC_2H_5$ or Cl;

and their agriculturally suitable salts; provided that

a) when X is Cl, F, Br or I, then Z is CH and Y is $OCH_3$, $OC_2H_5$, $N(OCH_3)CH_3$, $NHCH_3$, $N(CH_3)_2$ or $OCF_2H$;
b) when X or Y is $C_1$ haloalkoxy, then Z is CH;
c) $X_4$ and $Y_4$ are not simultaneously Cl;
d) when W is S, then $R_1$ is H, A is A-1 and Y is $CH_3$, $OCH_3$, $OC_2H_5$, $CH_2OCH_3$, $C_2H_5$, $CF_3$, $SCH_3$, $OCH_2CH{=}CH_2$, $OCH_2C{\equiv}CH$, $OCH_2CH_2OCH_3$, $CH(OCH_3)_2$ or 1,3-dioxolan-2-yl;
e) when the total number of carbons of X and Y is greater than four, then the number of carbons of R must be less than or equal to two;
f) when J is J-1, J-2, J-3 or J-4 then R′ is other than phenyl;
g) when J is J-5, J-6 or J-7 wherein E is a single bond, then R′ is other than $C_1$-$C_5$ alkyl, $C_3$-$C_5$ alkenyl, phenyl, benzyl, cyclopentyl or $C_4$-$C_7$ cycloalkylalkyl;
h) when either or both of X and Y are $OCF_2H$ then J is J-1, J-2, J-3, J-4, J-8, J-9, J-10 or J-11; and
i) when A is A-7 and $Z_1$ is N, then J is J-1, J-2, J-3 or J-4 and R′ is $C_3$-$C_5$ cycloalkyl;
j) when the total number of carbon atoms of X and Y is greater than four, then the total number of carbon atoms of $R_2$ and R′ must be less than or equal to 7.

2. Compounds of Claim 1 where E is a single bond; and
W is O.

3. Compounds of Claim 1 where E is $CH_2$; and
W is O.

4. Compounds of Claim 2 where
$R_2$ is H, $C_1$-$C_3$ alkyl, halogen, $C_1$-$C_3$ alkyl substituted with 1 to 3 halogen atoms selected from 1 to 3 Cl, 1 to 3 F or 1 Br, $OCH_3$, $SO_2NHCH_3$, $SO_2N(CH_3)_2$, $S(O)_nCH_3$. $CO_2CH_3$, $CO_2CH_2CH_3$, $OCF_2H$, $CH_2OCH_3$ or $CH_2CN$;
R is H, $C_1$-$C_3$ alkyl, phenyl, $CH_2CF_3$ or $CH_2CH{=}CH_2$;
X is $C_1$-$C_2$ alkyl, $C_1$-$C_2$ alkoxy, Cl, F, Br, I, $OCF_2H$, $CH_2F$, $CF_3$, $OCH_2CH_2F$, $OCH_2CHF_2$, $OCH_2CF_3$, $CH_2Cl$ or $CH_2Br$; and
Y is H, $C_1$-$C_2$ alkyl, $C_1$-$C_2$ alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH{=}CH_2$, $OCH_2C{\equiv}CH$, $OCH_2CH_2OCH_3$, $CH_2SCH_3$,

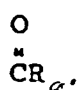, 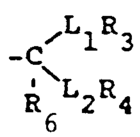, 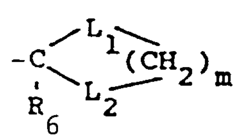, 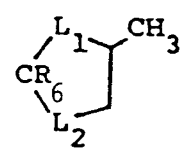.

$OCF_2H$, $OCF_2Br$, $SCF_2H$, cyclopropyl, $C{\equiv}CH$ or $C{\equiv}CCH_3$.

5. Compounds of Claim 4 where
R′ is $C_1$-$C_4$ alkyl, $C_1$-$C_3$ alkyl substituted with 1 to 3 halogen atoms selected from 1 to 3 Cl, 1 to 3 F or 1 Br, $C_2$-$C_4$ alkoxyalkyl, $C_2$-$C_4$ alkylthioalkyl, $C_2$-$C_4$ cyanoalkyl, $C_2$-$C_4$ alkenyl, $C_2$-$C_3$ alkenyl substituted with 1 to 3 halogen atoms selected from 1 to 3 Cl, 1 to 3 F or 1 Br, $C_3$-$C_4$ alkynyl, $C_3$-$C_5$ cycloalkyl, $C_3$-$C_5$ cycloalkyl substituted with 1 to 3 halogen atoms selected from 1 to 3 Cl, 1 to 3 F or 1 Br or cyclopropylmethyl.

6. Compounds of Claim 5 where
A is A-1;
n is O;
X is $CH_3$, $OCH_3$, $OCH_2CH_3$, Cl or $OCF_2H$; and
Y is $CH_3$, $OCH_3$, $C_2H_5$, $CH_2OCH_3$, $NHCH_3$, $CH(OCH_3)_2$ or cyclopropyl.

7. Compounds of Claim 6 where
$R_1$ is H;
$R_2$ is H, Cl, Br, $OCH_3$ or $CH_3$; and
R′ is $C_1$-$C_3$ alkyl, $C_1$-$C_3$ alkyl substituted with 1 to 3 F, $C_2$-$C_3$ alkoxyalkyl, $C_2$-$C_3$ alkylthioalkyl, $C_2$-$C_3$ cyanoalkyl, $C_2$-$C_3$ alkenyl, propargyl, $C_3$-$C_5$ cycloalkyl or cyclopropylmethyl.

8. Compounds of Claim 7 where J is J-1, J-2, J-3 or J-4.

9. Compounds of Claim 8 where

R′ is $C_1$-$C_3$ alkyl substituted with 1 to 3 F, $C_2$-$C_3$ alkoxyalkyl, $C_2$-$C_3$ alkylthiolalkyl, $C_2$-$C_3$ cyanoalkyl, $C_2$-$C_3$ alkenyl, propargyl, $C_3$-$C_5$ cycloalkyl or cyclopropylmethyl.

10. A compounds of Claim 1 which is 4-(cyclopropylcarbonyl)-N-[(4,6-dimethoxypyrimidin-2-yl)aminocarbinyl]-1-methyl-1H-pyrazole-5-sulfonamide.

11. A compound of Claim 1 which is 4-(1-oxopropyl)-N-[(4,6-dimethoxypyrimidin-2-yl)aminocarbonyl]-1-methyl-1H-pyrazole-5-sulfonamide.

12. A compound of Claim 1 which is 2-(cyclopropylcarbonyl)-N-[(4,6-dimethoxypyrimidin-2-yl)aminocarbonyl]-3-thiophenesulfonamide.

13. An agriculturally suitable composition for controlling the growth of undesired vegetation or for use as a plant growth regulant comprising an effective amount of a compound of any of Claims 1 to 12 and at least one of the following: surfactant, solid, or liquid diluent.

14. A composition of Claim 13 comprising a compound of Claim 10, 11, or 12 or an agriculturally suitable salt thereof.

15. A method for controlling the growth of undesired vegetation which comprises applying to the locus to be protected an effective amount of a compound of any of claims 1 to 12.

16. A method of Claim 15 wherein the compound of Claim 10, 11, or 12 is applied or an agriculturally suitable salt thereof.

17. A method for regulating the growth of plants which comprises applying to the locus of such plants an effective but substantially non-phytotoxic amount of a plant growth regulant selected from coupounds of any of Claims 1 to 12.

18. A process for the preparation of a compound of claim 1 which comprises:

(a) reacting a sulfonyl isocyanate or isothiocyanate of formula

$$JSO_2N = C = W \quad (II)$$

or a phenyl carbamate or thiocarbamate of formula

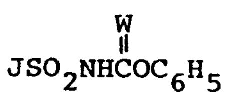

(VI)

with an aminoheterocycle of formula

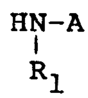

(III); or

(b) reacting a sulfonamide of formula

$$JSO_2NH_2 \quad (IV)$$

with a heterocyclic phenyl carbamate or thiocarbamate of formula

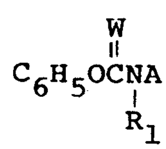

(VII);

wherein J, A, $R_1$ and W are as defined in claim 1.

19. Compounds of formulae

$$JSO_2N = C = W \quad (II)$$

$$JSO_2NH_2 \quad (IV)$$

and

$$JSO_2NH\overset{W}{\overset{\|}{C}}OC_6H_5 \qquad (VI)$$

wherein J and W are as defined in claim 1.

## Patentansprüche

1. Verbindungen der Formel I:

$$JSO_2NH\overset{W}{\overset{\|}{C}}NR_1A \qquad \underline{I}$$

worin J

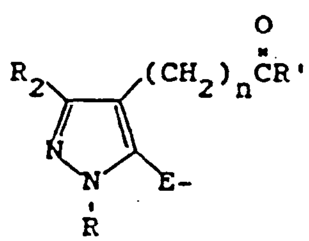

,

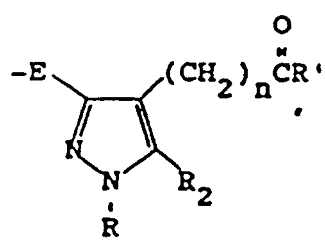

,

J-1

J-2

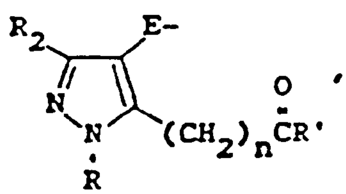

,

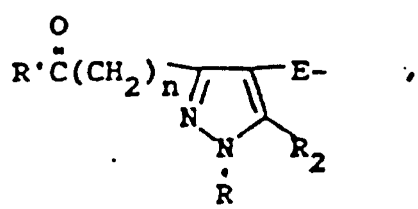

,

J-3

J-4

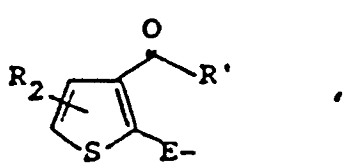

,

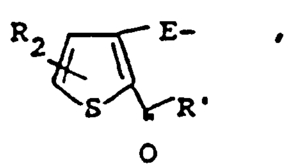

,

J-5

J-6

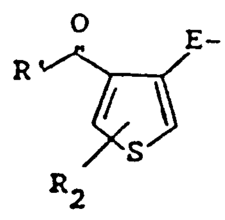

J-7

,

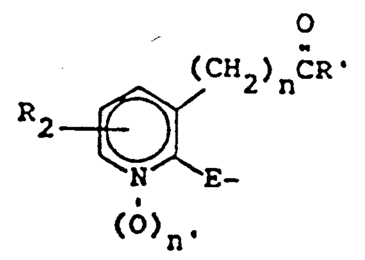

J-8

,

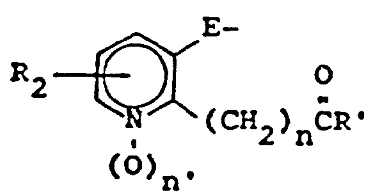

J-9

,

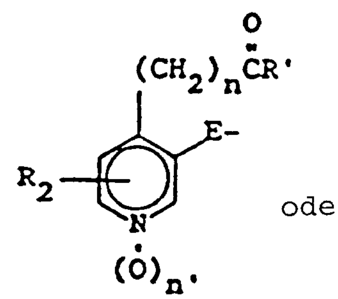

J-10

oder

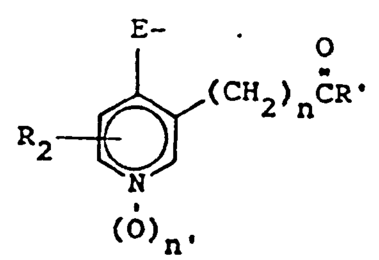

J-11

ist;

R H, $C_1$-$C_3$-Alkyl, Phenyl, $SO_2NR_aR_b$, $C_1$-$C_2$-Halogenalkyl, $C_2$-$C_4$-Alkoxyalkyl, $C_2$-$C_3$-Cyanalkyl, $C_2$-$C_4$-Alkylthioalkyl, $C_2$-$C_4$-Alkylsulfinylalkyl, $C_2$-$C_4$-Alkylsulfonylalkyl, $CO_2C_1$-$C_2$-Alkyl, $C_1$-$C_4$-Alkylcarbonyl, $C_1$-$C_2$-Alkylsulfonyl, $C_3$-$C_4$-Alkenyl, $C_3$-$C_4$-Alkinyl oder mit $CO_2C_1$-$C_2$-Alkyl substituiertes $C_1$-$C_2$-Alkyl ist;

$R_1$ H oder $CH_3$ ist;

$R_2$ H, $C_1$-$C_2$-Alkyl, $C_1$-$C_3$-Halogenalkyl, Halogen, Nitro, $C_1$-$C_3$-Alkoxy, $SO_2NR_cR_d$, $C_1$-$C_3$-Alkylthio, $C_1$-$C_3$-Alkylsulfinyl, $C_1$-$C_3$-Alkylsulfonyl, CN, $CO_2R_e$, $C_1$-$C_3$-Halogenalkoxy, $C_1$-$C_3$-Halogenalkylthio, Amino, $C_1$-$C_2$-Alkylamino, Di($C_1$-$C_3$-alkyl)amino oder mit $C_1$-$C_2$-Alkoxy, $C_1$-$C_2$-Halogenalkoxy, $C_1$-$C_2$-Alkylthio, $C_1$-$C_2$-Halogenalkylthio, CN, OH oder SH substituiertes $C_1$-$C_2$-Alkyl ist;

$R_a$ und $R_b$ unabhängig $C_1$-$C_2$-Alkyl sind;

$R_d$ H, $C_1$-$C_4$-Alkyl, $C_2$-$C_3$-Cyanalkyl, Methoxy oder Ethoxy ist;

$R_d$ H, $C_1$-$C_4$-Alkyl oder $C_3$-$C_4$-Alkenyl ist; oder

$R_d$ und $R_d$ als $-(CH_2)_3-$, $-(CH_2)_4-$, $-(CH_2)_5-$ oder $-CH_2CH_2OCH_2CH_2-$ zusammengenommen werden können;

$R_e$ $C_1$-$C_4$-Alkyl, $C_3$-$C_4$-Alkenyl, $C_3$-$C_4$-Alkinyl, $C_2$-$C_4$-Halogenalkyl, $C_1$-$C_2$-Cyanalkyl, $C_5$-$C_6$-Cycloalkyl, $C_4$-$C_7$-Cycloalkylalkyl oder $C_2$-$C_4$-Alkoxyalkyl ist;

R′ $C_1$-$C_5$-Alkyl, $C_1$-$C_5$-Halogenalkyl, mit einer oder zwei Gruppen $R_3$ substituiertes $C_1$-$C_5$-Alkyl, $C_2$-$C_5$-Alkenyl, $C_2$-$C_5$-Halogenalkenyl, mit einer oder zwei Gruppen $R_3$ substituiertes $C_3$-$C_5$-Alkenyl, $C_3$-$C_5$-Alkinyl, $C_3$-$C_5$-Halogenalkinyl, mit einer oder zwei Gruppen $R_3$ substituiertes $C_3$-$C_5$-Alkinyl, $C_3$-$C_5$-Cycloalkyl, $C_3$-$C_5$-Halogencycloalkyl, mit einer oder zwei Guppen $R_4$ substituiertes $C_3$-$C_5$-Cycloalkyl, $C_4$-$C_7$-Cycloalkylal-

kyl, $C_4$-$C_7$-Halogencycloalkylalkyl, mit einer oder zwei Gruppen $R_4$ substituiertes $C_4$-$C_7$-Cycloalkylalkyl, Phenyl oder Benzyl ist;

$R_3$ $C_1$-$C_3$-Alkoxy, $C_1$-$C_3$-Alkylthio, $C_1$-$C_3$-Halogenalkoxy, $C_1$-$C_3$-Alkylsulfinyl, $C_1$-$C_3$-Alkylsulfonyl, CN, $NO_2$, OH, $OR_5$ oder Di($C_1$-$C_3$-alkyl)amino ist;

$R_4$ $C_1$-$C_3$-Alkyl, $C_1$-$C_3$-Alkoxy, $C_1$-$C_3$-Halogenalkoxy, CN, $NO_2$, OH, $OR_5$ oder Di($C_1$-$C_3$-alkyl)amino ist;

$R_5$ $SO_2CH_3$, $Si(CH_3)_3$, $C_2$-$C_3$-Alkylcarbonyl oder $CO_2C_1$-$C_2$-Alkyl ist;

E eine Einfachbindung oder $CH_2$ ist;

W O oder S ist;

n 0 oder 1 ist;

n' 0 oder 1 ist;

A 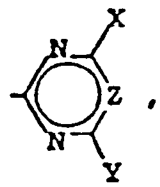 , 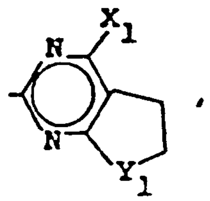 , 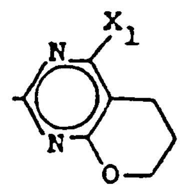 ,

A-1 A-2 A-3

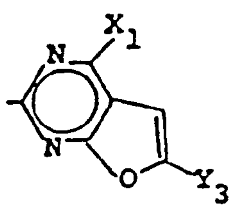 , 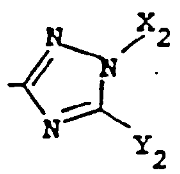 , 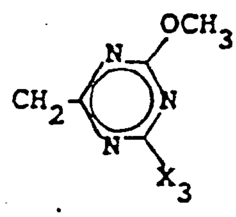 ;

A-4 A-5 A-6

oder 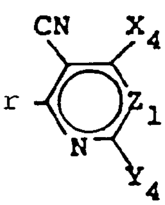 ist;

A-7

X H, $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)amino oder $C_3$-$C_5$-Cycloalkyl ist;

Y H, $C_1$-$C_4$-Alkyl, $C_1$-$C_4$-Alkoxy, $C_1$-$C_4$-Halogenalkoxy, $C_1$-$C_4$-Halogenalkylthio, $C_2$-$C_5$-Alkylthio, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)amino, $C_3$-$C_4$-Alkenyloxy, $C_3$-$C_4$-Alkinyloxy, $C_2$-$C_5$-Alkylthioalkyl, $C_1$-$C_4$-Halogenalkyl, $C_2$-$C_4$-Alkinyl, Azido, Cyano, $C_2$-$C_5$-Alkylsulfinylalkyl, $C_2$-$C_5$-Alkylsulfonylalkyl,

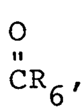 , 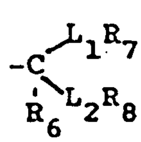 , 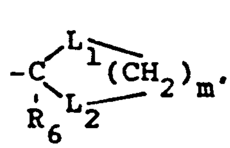 , 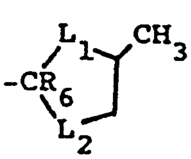 oder

$N(OCH_3)CH_3$ ist;

m 2 oder 3 ist;

$L_1$ und $L_2$ unabhängig O oder S sind;

$R_6$ H oder $C_1$-$C_3$-Alkyl ist;

$R_7$ und $R_6$ unabhängig $C_1$-$C_3$-Alkyl sind;

Z CH oder N ist;

$Z_1$ CH oder N ist;

$X_1$ O oder $CH_2$ ist;

$X_1$ $CH_3$, $OCH_3$, $OC_2H_5$ oder $OCF_2H$ ist;

$X_2$ $CH_3$, $C_2H_5$ oder $CH_2CF_3$ ist;

$Y_2$ $OCH_3$, $OC_2H_5$ , $SCH_3$, $SC_2H_5$, $CH_3$ oder $CH_2CH_3$ ist;

$X_3$ $CH_3$ oder $OCH_3$ ist;

$Y_3$ H oder $CH_3$ ist;

$X_4$ $CH_3$, $OCH_3$, $OC_2H_5$, $CH_2OCH_3$ oder Cl ist; und

$Y_4$ $CH_3$, $OCH_3$, $OC_2H_5$ oder Cl ist;

und ihre landwirtschaftlich geeigneten Salze, mit der Maßgabe, daß

a) wenn X Cl, F, Br oder I ist, dann Z CH ist und Y $OCH_3$, $OC_2H_5$, $N(OCH_3)CH_3$, $NHCH_3$, $N(CH_3)_2$ oder $OCF_2H$ ist;

b) wenn X oder Y $C_1$-Halogenalkoxy ist, dann Z CH ist;

c) $X_4$ und $Y_4$ nicht gleichzeitig Cl sein können;

d) wenn W S ist, dann $R_1$ H ist, A A-1 ist und Y $CH_3$, $OCH_3$, $OC_2H_5$, $CH_2OCH_3$, $C_2H_5$, $CF_3$, $SCH_3$, $OCH_2CH{=}CH_2$, $OCH_2C{\equiv}CH$, $OCH_2CH_2OCH_3$, $CH(OCH_3)_2$ oder 1,3-Dioxolan-2-yl ist;

e) wenn die Gesamtzahl der Kohlenstoffe von X und Y größer als vier ist, dann die Zahl der Kohlenstoffe von R kleiner oder gleich zwei sein muß;

f) wenn J J-1, J-2, J-3 oder J-4 ist, dann R′ von Phenyl verschieden ist;

g) wenn J J-5, J-6 oder J-7 ist, worin E eine Einfachbindung ist, dann R′ von $C_1$-$C_5$-Alkyl, $C_3$-$C_5$-Alkenyl, Phenyl, Benzyl, Cyclopentyl oder $C_4$-$C_7$-Cycloalkylalkyl verschieden ist;

h) wenn eines oder beide von X und Y $OCF_2H$ ist, dann J J-1, J-2, J-3, J-4, J-8, J-9, J-10 oder J-11 ist; und

i) wenn A A-7 ist und $Z_1$ N ist, dann J J-1, J-2, J-3 oder J-4 ist und R′ $C_3$-$C_5$-Cycloalkyl ist;

j) wenn die Gesamtzahl der Kohlenstoffatome von X und Y größer als vier ist, dann die Gesamtzahl der Kohlenstoffatome von $R_2$ und R′ kleiner oder gleich 7 sein muß.

2. Verbindungen nach Anspruch 1, worin E eine Einfachbindung ist und W O ist.

3. Verbindungen nach Anspruch 1, worin E $CH_2$ ist und W O ist.

4. Verbindungen nach Anspruch 2, worin

$R_2$ H, $C_1$-$C_3$-Alkyl, Halogen, mit 1 bis 3 aus 1 bis 3 Cl, 1 bis 3 F oder 1 Br ausgewählten Halogenatomen substituiertes $C_1$-$C_3$-Alkyl, $OCH_3$, $SO_2NHCH_3$, $SO_2N(CH_3)_2$, $S(O)_nCH_3$, $CO_2CH_3$, $CO_2CH_2CH_3$, $OCF_2H$, $CH_2OCH_3$ oder $CH_2CN$ ist;

R H, $C_1$-$C_3$-Alkyl, Phenyl, $CH_2CF_3$ oder $CH_2CH{=}CH_2$ ist;

X $C_1$-$C_2$-Alkyl, $C_1$-$C_2$-Alkoxy, Cl, F, Br, I, $OCF_2H$, $CH_2F$, $CF_3$, $OCH_2CH_2F$, $OCH_2CHF_2$, $OCH_2CF_3$, $CH_2Cl$ oder $CH_2Br$ ist; und

Y H, $C_1$-$C_2$-Alkyl, $C_1$-$C_2$-Alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH{=}CH_2$, $OCH_2C{\equiv}CH$, $OCH_2CH_2OCH_3$, $CH_2SCH_3$,

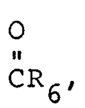 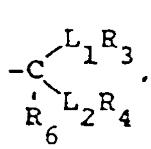, 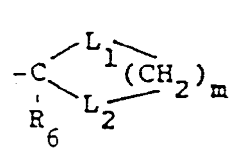, 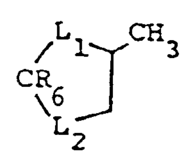

$OCF_2H$, $OCF_2Br$, $SCF_2H$, Cyclopropyl, $C{\equiv}CH$ oder $C{\equiv}CCH_3$ ist.

5. Verbindungen nach Anspruch 4, worin

R′ $C_1$-$C_4$-Alkyl, mit 1 bis 3 aus 1 bis 3 Cl, 1 bis 3 F oder 1 Br ausgewählten Halogenatomen substituiertes $C_1$-$C_3$-Alkyl, $C_2$-$C_4$-Alkoxyalkyl, $C_2$-$C_4$-Alkylthioalkyl, $C_2$-$C_4$-Cyanalkyl, $C_2$-$C_4$-Alkenyl, mit 1 bis 3 aus 1 bis 3 Cl, 1 bis 3 F oder 1 Br ausgewählten Halogenatomen substituiertes $C_2$-$C_3$-Alkenyl, $C_3$-$C_4$-Alkinyl, $C_3$-$C_5$-Cycloalkyl, mit 1 bis 3 aus 1 bis 3 Cl, 1 bis 3 F oder 1 Br ausgewählten Halogenatomen substituiertes $C_3$-$C_5$-Cycloalkyl

oder Cyclopropylmethyl ist.

6. Verbindungen nach Anspruch 5, worin
A A-1 ist;
n 0 ist;
X $CH_3$, $OCH_3$, $OCH_2CH_3$, Cl oder $OCF_2H$ ist; und
Y $CH_3$, $OCH_3$, $C_2H_5$, $CH_2OCH_3$, $NHCH_3$, $CH(OCH_3)_2$ oder Cycloprogyl ist.

7. Verbindungen nach Anspruch 6, worin
$R_1$ H ist;
$R_2$ H, Cl, Br, $OCH_3$ oder $CH_3$ ist; und
R′ $C_1$-$C_3$-Alkyl, mit 1 bis 3 F substituiertes $C_1$-$C_3$-Alkyl, $C_2$-$C_3$-Alkoxyalkyl, $C_2$-$C_3$-Alkylthioalkyl, $C_2$-$C_3$-Cyanalkyl, $C_2$-$C_3$-Alkenyl, Propargyl, $C_3$-$C_5$-Cycloalkyl oder Cyclopropylmethyl ist.

8. Verbindungen nach Anspruch 7, worin J J-1, J-2, J-3 oder J-4 ist.

9. Verbindungen nach Anspuch 8, worin
R′ mit 1 bis 3 F substituiertes $C_1$-$C_3$-Alkyl, $C_2$-$C_3$-Alkoxyalkyl, $C_2$-$C_3$-Alkylthioalkyl, $C_2$-$C_3$-Cyanalkyl, $C_2$-$C_3$-Alkenyl, Propargyl, $C_2$-$C_5$-Cycloalkyl oder Cyclopropylmethyl ist.

10. Verbindung nach Anspruch 1, welche 4-(Cyclopropylcarbonyl)-N-[(4,6-dimethoxypyrimidin-2-yl)aminocarbonyl]-1-methyl-1H-pyrazol-5-sulfonamid ist.

11. Verbindung nach Anspruch 1, welche 4-(1-Oxopropyl)-N-[(4,6-dimethoxygyrimidin-2-yl)aminocarbonyl]-1-methyl-1H-pyrazol-5-sulfonamid ist.

12. Verbindung nach Anpruch 1, welche 2-(Cyclopropylcarbonyl)-N-[(4,6-dimethoxypyrimidin-2-yl)aminocarbonyl]-3-thiophensulfonamid ist.

13. Landwirtschaftlich geeignete Zusammensetzung zur Bekämpfung des Wachstums unerwünschter Vegetation oder zur Verwendung als Pflanzenwachstumsregulans, welche eine wirksame Menge einer Verbindung nach einem der Ansprüche 1 bis 12 und wenigstens eines der folgenden umfaßt: oberflächenaktives Mittel, festes oder flüssiges Verdünnungsmittel.

14. Zusammensetzung nach Anspruch 13, welche eine Verbindung nach Anspruch 10, 11 oder 12 oder ein landwirtschaftlich geeignetes Salz davon enthält.

15. Verfahren zur Bekämpfung des Wachstums unerwünschter Vegetation durch Anwenden einer wirksamen Menge einer Verbindung nach einem der Ansprüche 1 bis 12 auf den zu schützenden Ort.

16. Verfahren nach Anspruch 15, worin die Verbindung nach Anspruch 10, 11 oder 12 oder ein landwirtschaftlich geeignetes Salz davon angewandt wird.

17. Verfahren zur Steuerung des Wachstums von Pflanzen durch Anwenden einer wirksamen, jedoch im wesentlichen nicht phytotoxischen Menge eines Pflanzenwachstumsregulans, ausgewählt aus Verbindungen nach einem der Ansprüche 1 bis 12, auf den Ort solcher Pflanzen.

18. Verfahren zur Herstellung einer Verbindung nach Anspruch 1 durch
(a) Umsetzen eines Sulfonylisocyanats oder -isothiocyanats der Formel

$$JSO_2N = C = W \quad (II)$$

oder eines Phenylcarbamats oder -thiocarbamats der Formel

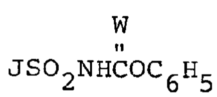

(VI)

mit einem Aminoheterocyclus der Formel

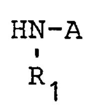

(III) oder

(b) Umsetzen eines Sulfonamids der Formel

$$JSO_2NH_2 \quad (IV)$$

mit einem heterocyclischen Phenylcarbamat oder -thiocarbamat der Formel

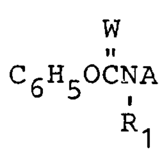

(VII)

worin J, à, $R_1$ und W wie in Anspruch 1 definiert sind.

19. Verbindung der Formeln

$$JSO_2N = C = W \quad (II)$$

$$JSO_2NH_2 \quad (IV)$$

und

$$JSO_2NH\overset{W}{\overset{\|}{C}}OC_6H_5 \qquad (VI)$$

worin J und W wie in Anspruch 1 definiert sind.

## Revendications

1. Composés de Formule I

$$JSO_2NH\overset{W}{\overset{\|}{C}}NR_1A$$

<u>I</u>

où

J est

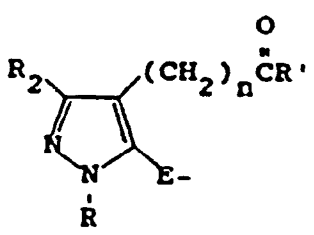

<u>J-1</u>

,

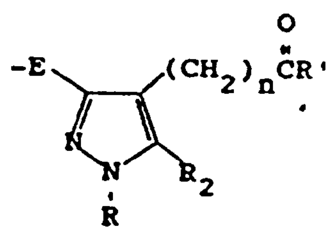

<u>J-2</u>

,

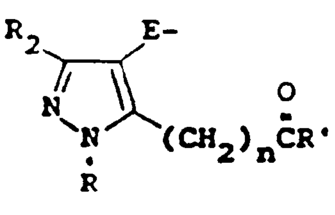

<u>J-3</u>

,

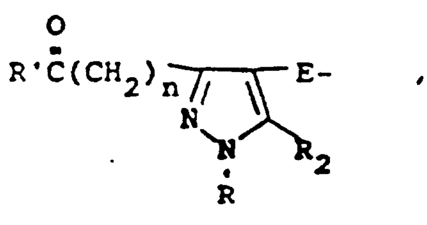

<u>J-4</u>

,

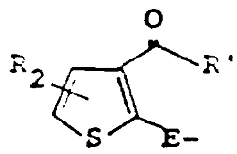 ,

J-5

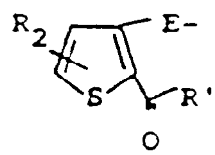 ,

J-6

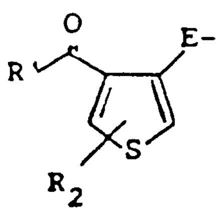 ,

J-7

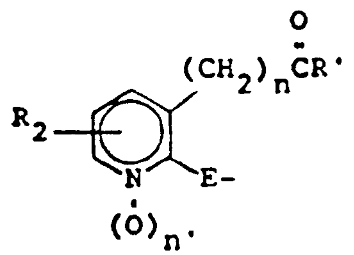 ,

J-8

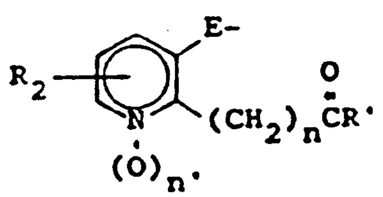 ,

J-9

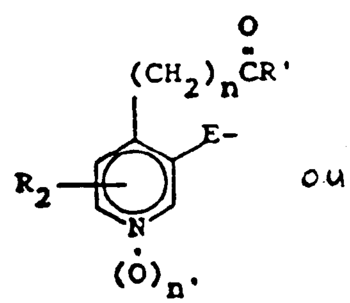 ou

J-10

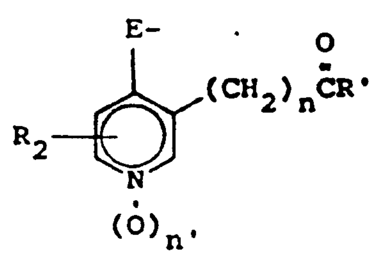 ;

J-11

R est H, un groupe alkyle en $C_1$-$C_3$, phényle, $SO_2NR_aR_b$, halogénalkyle en $C_1$-$C_2$, alcoxyalkyle en $C_2$-$C_4$, cyanalkyle en $C_2$-$C_3$, alkylthioalkyle en $C_2$-$C_4$, alkylsulfinylalkyle en $C_2$-$C_4$, alkylsulfonylalkyle en $C_2$-$C_4$, $Co_2$(alkyle en $C_1$-$C_2$), (alkyle en $C_1$-$C_4$)carbonyle, alkylsulfonyle en $C_1$-$C_2$, alcényle en $C_3$-$C_4$, alcynyle en $C_3$-$C_4$ ou alkyle en $C_1$-$C_2$substitué par $CO_2$(alkyle en $C_1$-$C_2$) ;

$R_1$ est H ou $CH_3$ ;

$R_2$ est H, un groupe alkyle en $C_1$-$C_3$, halogénalkyle en $C_1$-$C_3$, halogéno, nitro, alcoxy en $C_1$-$C_3$, $SO_2NR_cR_d$, alkylthio en $C_1$-$C_3$, alkylsulfinyle en $C_1$-$C_3$, alkylsulfonyle en $C_1$-$C_3$, CN, $CO_2R_e$, halogénalcoxy en

$C_1$-$C_3$, halogénalkylthio en $C_1$-$C_3$, amino, alkylamino en $C_1$-$C_2$, di(alkyle en $C_1$-$C_3$)amino ou alkyle en $C_1$-$C_2$ substitué par alcoxy en $C_1$-$C_2$, halogénalcoxy en $C_1$-$C_2$, alkylthio en $C_1$-$C_2$, halogénalkylthio en $C_1$-$C_2$, CN, OH ou SH ;

$R_a$ et $R_b$ sont indépendamment un groupe alkyle en $C_1$-$C_2$ ;

$R_c$ est H, un groupe alkyle en $C_1$-$C_4$, cyanalkyle en $C_2$-$C_3$, méthoxy ou éthoxy ;

$R_c$ est H, un groupe alkyle en $C_1$-$C_4$ ou alcényle en $C_3$-$C_4$ ; ou bien

$R_c$ et $R_d$ peuvent être pris ensemble sous la forme $-(CH_2)_3-$, $-(CH_2)_4-$, $-(CH_2)_5-$ ou $-CH_2CH_2OCH_2CH_2-$ ;

$R_e$ est un groupe alkyle en $C_1$-$C_4$, alcényle en $C_3$-$C_4$, alcynyle en $C_3$-$C_4$, halogénalkyle en $C_2$-$C_4$, cyanalkyle en $C_1$-$C_2$, cycloalkyle en $C_5$-$C_6$, cycloalkylalkyle en $C_4$-$C_7$ ou alcoxyalkyle en $C_2$-$C_4$ ;

R' est un groupe alkyle en $C_1$-$C_5$, halogénalkyle en $C_1$-$C_5$, alkyle en $C_1$-$C_5$ substitué par un ou deux groupes $R_3$, alcényle en $C_2$-$C_5$, halogénalcényle en $C_2$-$C_5$, alcényle en $C_3$-$C_5$ substitué par un ou deux groupes $R_3$, alcynyle en $C_3$-$C_5$, halogénalcynyle en $C_3$-$C_5$, alcynyle en $C_3$-$C_5$ substitué par un ou deux groupes $R_3$, cycloalkyle en $C_3$-$C_5$, halogénocycloalkyle en $C_3$-$C_5$, cycloalkyle en $C_3$-$C_5$ substitué par un ou deux groupes $R_4$, cycloalkylalkyle en $C_4$-$C_7$, halogénocycloalkylalkyle en $C_4$-$C_7$, cycloalkylalkyle en $C_4$-$C_7$ substitué par un ou deux groupes $R_4$, phényle ou benzyle ;

$R_3$ est un groupe alcoxy en $C_1$-$C_3$, alkylthio en $C_1$-$C_3$, halogénalcoxy en $C_1$-$C_3$, alkylsulfinyle en $C_1$-$C_3$, alkylsulfonyle en $C_1$-$C_3$, CN, $NO_2$, OH, $OR_5$ ou di(alkyle en $C_1$-$C_3$)amino ;

$R_4$ est un groupe alkyle en $C_1$-$C_3$, alcoxy en $C_1$-$C_3$, halogénalcoxy en $C_1$-$C_3$, CN, $NO_2$, OH, $OR_5$ ou di-(alkyle en $C_1$-$C_3$)amino ;

$R_5$ est $SO_2CH_3$, $Si(CH_3)_3$, un groupe alkylcarbonyle en $C_2$-$C_3$ ou $CO_2$(alkyle en $C_1$-$C_2$) ;

E est une liaison simple ou $CH_2$ ;

W est O ou S ;

n est 0 ou 1 ;

n' est 0 ou 1 ;;

A est

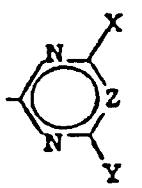
,
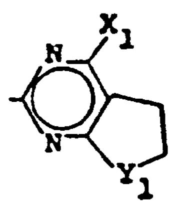
,
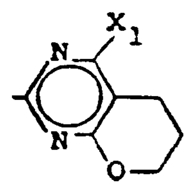
,

A-1 A-2 A-3

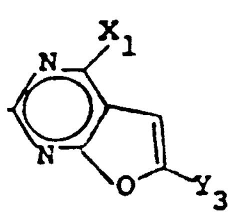
,
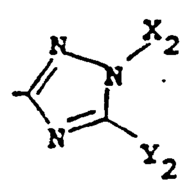
,
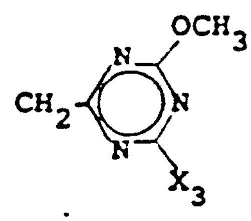
;

A-4 A-5 A-6

ou 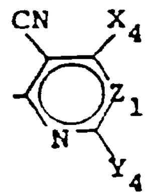 ;

A-7

X est H, un groupe alkyle en $C_1$-$C_4$, alkoxy en $C_1$-$C_4$, halogénalcoxy en $C_1$-$C_4$, halogénalkyle en $C_1$-$C_4$, halogénalkylthio en $C_1$-$C_4$, alkylthio en $C_1$-$C_4$, halogéno, alcoxyalkyle en $C_2$-$C_5$, alcoxyalcoxy en $C_2$-$C_5$, amino, alkylamino en $C_1$-$C_3$, di(alkyle en $C_1$-$C_3$)amino ou cycloalkyle en $C_3$-$C_5$ ;

Y est H, un groupe alkyle en $C_1$-$C_4$, alcoxy en $C_1$-$C_4$, halogénalcoxy en $C_1$-$C_4$, halogénalkylthio en $C_1$-$C_4$, alkylthio en $C_1$-$C_4$, alcoxyalkyle en $C_2$-$C_5$, alcoxyalcoxy en $C_2$-$C_5$, amino, alkylamino en $C_1$-$C_3$, di(alkyle en $C_1$-$C_3$)amino, alcényloxy en $C_3$-$C_4$, alcynyloxy en $C_3$-$C_4$, alkylthioalkyle en $C_2$-$C_5$, halogénalkyle en $C_1$-$C_4$, alcynyle en $C_2$-$C_4$, azido, cyano, alkylsulfinylalkyle en $C_2$-$C_5$, alkylsulfonylalkyle en $C_2$-$C_5$,

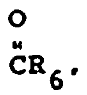, 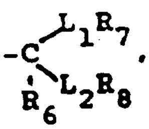, 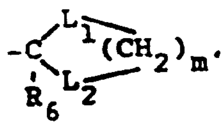, 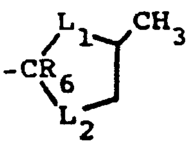

ou $N(OCH_3)CH_3$ ;

m est 2 ou 3 ;

$L_1$ et $L_2$ sont indépendemment O ou S ;

$R_6$ est H ou un groupe alkyle en $C_1$-$C_3$ ;

$R_7$ et $R_6$ sont indépendamment un groupe alkyle en $C_1$-$C_3$ ;

Z est CH ou N ;

$Z_1$ est CH ou N ;

$Y_1$ est O ou $CH_2$ ;

$X_1$ est $CH_3$, $OCH_3$, $OC_2H_5$ ou $OCF_2H$ ;

$X_2$ est $CH_3$, $C_2H_5$ ou $CH_2CF_3$ ;

$Y_2$ est $OCH_3$, $OC_2H_5$, $SCH_3$, $SC_2H_5$, $CH_3$ ou $CH_2CH_3$ ;

$Y_3$ est $CH_3$ ou $OCH_3$ ;

$Y_3$ est H ou $CH_3$ ;

$X_4$ est $CH_3$, $OCH_3$, $OC_2H_5$, $CH_2OCH_3$ ou Cl ; et

$Y_4$ est $CH_3$, $OCH_3$, $OC_2H_5$ ou Cl ;

et leurs sels utilisables en agriculture ; avec les conditions suivantes

a) si X est Cl, F, Br ou I, alors Z est CH et Y est $OCH_3$, $OC_2H_5$, $N(OCH_3)CH_3$, $NHCH_3$, $N(CH_3)_2$ ou $OCF_2H$ ;

b) si X ou Y est un groupe halogénalcoxy en $C_1$, alors Z est CH ;

c) $X_4$ et $Y_4$ ne sont pas simultanément Cl ;

d) si W est S, alors $R_1$ est H, A est A-1 et Y est $CH_3$ , $OCH_3$ , $OC_2H_5$ , $CH_2OCH_3$ , $C_2H_5$ , $CF_3$ , $SCH_3$, $OCH_2CH{=}CH_2$, $OCH_2C{\equiv}CH$, $OCH_2CH_2OCH_3$, $CH(OCH_3)_2$ ou 1,3-dioxolanne-2-yle ;

e) si le nombre total d'atomes de carbone de X et Y est supérieur à quatre, alors le nombre d'atomes de carbone de R doit être inférieur ou égal à deux ;

f) si J est J-1, J-2, J-3 ou J-4, alors R′ est autre chose qu'un groupe phényle ;

g) si J est J-5, J-6 ou J-7, où E est une liaison simple, alors R′ est autre chose qu'un groupe alkyle en $C_1$-$C_5$, alcényle en $C_3$-$C_5$, phényle, benzyle, cyclopentyle ou cycloalkylalkyle en $C_4$-$C_7$ ;

h) si l'un quelconque ou chacun de X et Y est $OCF_2H$, alors J est J-1, J-2, J-3, J-4, J-8, J-9, J-10 ou J-11 ; et

i) si A est A-7 et $Z_1$ est N, alors J est J-1, J-2, J-3 ou J-4 et R′ est un groupe cycloalkyle en $C_3$-$C_5$ ;

j) si le nombre total d'atomes de cabone de X et Y est supérieur à quatre, alors le nombre total d'atomes de carbone de $R_2$ et R′ doit être inférieur ou égal à 7.

2. Composés de la revendication 1, où E est une liaison simple ; et W est O.

3. Composés de la revendication 1, où E est $CH_2$ ;
et W est O.

4. Composés de la revendication 2, où

$R_2$ est H, un groupe alkyle en $C_1$-$C_3$, halogéno, alkyle en $C_1$-$C_3$ substitué par 1 à 3 atomes d'halogènes choisis entre 1 à 3 Cl, 1 à 3 F et 1 Br, $OCH_3$ , $SO_2NHCH_3$ , $SO_2N(CH_3)_2$, $S(O)_nCH_3$, $CO_2CH_3$, $CO_2CH_2CH_3$, $OCF_2H$, $CH_2OCH_3$, ou $CH_2CN$ ;

R est H, un groupe alkyle en $C_1$-$C_3$, phényle, $CH_2CF_3$ ou $CH_2CH{=}CH_2$ ;

X est un groupe alkyle en $C_1$-$C_2$, alcoxy en $C_1$-$C_2$, Cl, F, Br, I, $OCF_2H$, $CH_2F$, $CF_3$, $OCH_2CH_2F$, $OCH_2CHF_2$, $OCH_2CF_3$, $CH_2Cl$ ou $CH_2Br$ ; et

Y est H, un groupe alkyle en $C_1$-$C_2$, alcoxy en $C_1$-$C_2$, $CH_2OCH_3$, $CH_2OCH_2CH_3$, $NHCH_3$, $N(OCH_3)CH_3$, $N(CH_3)_2$, $CF_3$, $SCH_3$, $OCH_2CH{=}CH_2$, $OCH_2C{\equiv}CH$, $OCH_2CH_2OCH_3$, $CH_2SCH_3$,

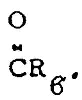, 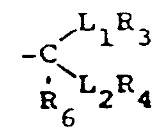, 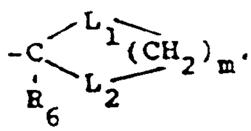, 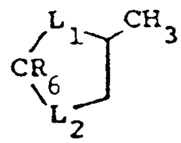.

$OCF_2H$, $OCF_2Br$, $SCF_2H$, cyclopropyle, $C{\equiv}CH$ ou $C{\equiv}CCH_3$.

5. Composés de la revendication 4, où

R′ est un groupe alkyle en $C_1$-$C_4$, alkyle en $C_1$-$C_3$ substitué par 1 à 3 atomes d'halogènes choisis parmi 1 à 3 Cl, 1 à 3 F et 1 Br, alcoxyalkyle en $C_2$-$C_4$, alkylthioalkyle en $C_2$-$C_4$, cyanalkyle en $C_2$-$C_4$, alcényle en $C_2$-$C_4$, alcényle en $C_2$-$C_3$ substitué par 1 à 3 atomes d'halogènes choisis parmi 1 à 3 Cl, 1 à 3 F et 1 Br, alcynyle en $C_3$-$C_4$, cycloalkyle en $C_3$-$C_5$, cycloalkyle en $C_3$-$C_5$ substitué par 1 à 3 atomes d'halogènes choisis parmi 1 à 3 Cl, 1 à 3 F et 1 Br, ou cyclopropylméthyle.

6. Composés de la revendication 5, où

A est A-1 ;

n est O ;

X est $CH_3$, $OCH_3$, $OCH_2CH_3$, Cl ou $OCF_2H$ ; et

Y est $CH_3$, $OCH_3$, $C_2H_5$, $CH_2OCH_3$, $NHCH_3$, $CH(OCH_3)_2$ ou un groupe cyclopropyle.

7. Composés de la revendication 6, où $R_1$ est H ;

$R_2$ est H, Cl, Br, $OCH_3$ ou $CH_3$ ; et

R′ est un groupe alkyle en $C_1$-$C_3$, alkyle en $C_1$-$C_3$ substitué par 1 à 3 F, alcoxyalkyle en $C_1$-$C_3$, alkylthioalkyle en $C_2$-$C_3$, cyanalkyle en $C_2$-$C_3$, alcényle en $C_2$-$C_3$, propargyle, cycloalkyle en $C_3$-$C_5$ ou cyclopropylméthyle.

8. Composés de la revendication 7, où J est J-1, J-2, J-3 ou J-4.

9. Composés de la revendication 8, où

R′ est un groupe alkyle en $C_1$-$C_3$ substitué par 1 à 3 F, alcoxyalkyle en $C_1$-$C_3$, alkylthioalkyle en $C_2$-$C_3$, cyanalkyle en $C_2$-$C_3$, alcényle en $C_2$-$C_3$, propargyle, cycloalkyle en $C_3$-$C_5$ ou cyclopropylméthyle.

10. Un composé de la revendication 1, qui est le 4-cyclopropylcarbonyl)-N-[(4,6-diméthoxypyrimidine-2-yl)aminocarbonyl]-1-méthyl-1H-pyrazole-5-sulfonamide.

11. Un composé de la revendication 1, qui est le 4-(1-oxopropyl)-N-[(4,6-diméthoxypyrimidine-2-yl)aminocarbonyl]-1-méthyl-1H-pyrazole-5-sulfonamide.

12. Un composé de la revendication 1, qui est le 2-(cyclopropylcarbonyl)-N-[(4,6-diméthoxypyrimidine-2-yl)aminocarbonyl]-3-thiophène-sulfonamide.

13. Une composition utilisable en agriculture pour combattre la croissance de végétation indésirable ou à utiliser comme régulateur de la croissance de plantes, comprenant une quantité efficace d'un composé de l'une quelconque des revendications 1 à 12 et au moins l'un des ingrésients suivants : agent tensio-actif, diluant solide ou liquide.

14. Une composition de la revendication 13, comprenant un composé de la revendication 10, 11 ou 12 ou un sel utilisable en agriculture de celui-ci.

15. Un procédé pour combattre la croissance de végétation indésirable, qui consiste à appliquer au lieu à protéger une quantité efficace d'un composé de l'une quelconque des revendications 1 à 12.

16. Un procédé de la revendication 15, dans lequel on applique le composé de la revendication 10, 11 ou 12 ou un sel utilisable en agriculture de celui-ci.

17. Un procédé pour réguler la croissance de plantes, qui consiste à appliquer au lieu où se trouvent ces plantes une quantité efficace, mais sensiblement non phytotoxique, d'un régulateur de croissance de plantes choisi parmi les composés de l'une quelconque des revendications 1 à 12.

18. Un procédé pour la préparation d'un composé de la revendication 1, qui consiste à :
(a) faire réagir un isocyanate ou isothiocyanate de sulfonyle de formule

$$JSO_2N = C = W \quad (II)$$

ou un carbamate ou thiocarbamate de phényle de formule

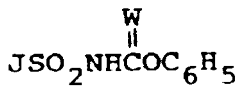

(VI)

avec un aminohétérocycle de formule

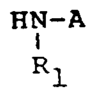

(III) ; ou

(b) faire réagir un sulfonamide de formule

$$JSO_2NH_2 \quad (IV)$$

avec un phényl carbamate ou thiocarbamate hétérocyclique de formule

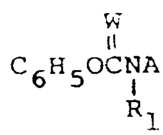

(VII) ;

où J, A, $R_1$ et W sont tels que dédinis dans la revendication 1.

19. Composés de formules

$$JSO_2N = C = W \quad (II)$$

$$JSO_2NH_2 \quad (IV)$$

et

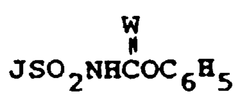

(VI)

où J et W sont tels que définis dans la revendication 1.